(19) Europäisches Patentamt
European Patent Office
Office européen des brevets

(11) Publication number: 0 352 000
A2

(12) **EUROPEAN PATENT APPLICATION**

(21) Application number: 89306995.5

(22) Date of filing: 10.07.89

(51) Int. Cl.4: C07K 7/06 , C07K 5/02 , A61K 37/64 , C07D 213/40 , C07D 207/10 , C07C 271/18 , C07C 271/20 , C07F 9/40 , //G01N33/68,C12Q1/37, C12Q1/70

(30) Priority: 08.07.88 US 216178
10.03.89 US 321937

(43) Date of publication of application:
24.01.90 Bulletin 90/04

(84) Designated Contracting States:
AT BE CH DE ES FR GB GR IT LI LU NL SE

(71) Applicant: SMITHKLINE BECKMAN CORPORATION
One Franklin Plaza P O Box 7929
Philadelphia Pennsylvania 19103(US)

(72) Inventor: Dreyer, Geoffrey Bainbridge
2 Marlin Drive
Malvern Pennsylvania 19355(US)
Inventor: Huffman, William Francis
40 Crest Avenue
Malvern Pennsylvania 19355(US)
Inventor: Meek, Thomas Dowing
500 East Lancaster Avenue
Saint Davids Pennsylvania 19087(US)
Inventor: Metcalf, Brian Walter
520 Woodland Drive
Radnor Pennsylvania 19087(US)
Inventor: Moore, Michael Lee
417 South Jackson Street
Media Pennsylvania 19063(US)

(74) Representative: Waters, David Martin, Dr. et al
Smith Kline & French Laboratories Ltd.
Patent Department Mundells
Welwyn Garden City Hertfordshire AL7 1EY(GB)

(54) **Retroviral protease binding peptides.**

(57) Peptide mimics of the retrovirus protease polyprotein substrate bind to viral proteases and are useful in assaying for protease activity or inhibiting protease activity and in treating viral disease.

EP 0 352 000 A2

# RETROVIRAL PROTEASE BINDING PEPTIDES

## BACKGROUND

Retroviruses, that is, viruses within the family of Retroviridae, are a class of viruses which transport their genetic material as ribonucleic acid rather than deoxyribonucleic acid. Also known as RNA-tumor viruses, their presence has been associated with a wide range of diseases in humans and animals. They are believed to be the causative agents in pathological states associated with infection by Rous sarcoma virus (RSV), murine leukemia virus (MLV), mouse mammary tumor virus (MMTV), feline leukemia virus (FeLV), bovine leukemia virus (BLV), Mason-Pfizer monkey virus (MPMV), simian sarcoma virus (SSV), simian acquired immunodeficiency syndrome (SAIDS), human T-lymphotropic virus (HTLV-I, -II) and human immunodeficiency virus (HIV-1, HIV-2), which is the etiologic agent of AIDS (acquired immunodeficiency syndrome) and AIDS related complexes, and many others. Although the pathogens have, in many of these cases, been isolated, no effective method for treating this type of infection has been developed. Among these viruses, the HTLV and HIV have been especially well characterized.

Although diverse in detail, all retroviruses are rather similar in overall structure. The extracellular virus particle is composed of an outer membrane studded with viral glycoproteins, a core of structural proteins, and a genome of single stranded ribonucleic acid. The retroviral genome has a distinctive regional organization, referred to as the 5′-gag-pol-env-3′ structure, wherein the gag region encodes the core structural proteins, the pol region encodes certain critical viral enzymes such as reverse transcriptase, integrase and protease, and the env region encodes the envelope glycoproteins. Viral replication occurs only within host cells and is dependent upon host cellular functions. Critical to this replication is the production of functional viral proteins. Protein synthesis is accomplished by translation of the open reading frames into polyprotein constructs, corresponding to the gag, pol and env reading frames, which are processed, at least in part, by a viral protease into the functional proteins. The proteolytic activity provided by the viral protease in processing the polyproteins cannot be provided by the host and is essential to the life cycle of the retrovirus. In fact, it has been demonstrated that retroviruses which lack the protease or contain a mutated form of it, lack infectivity. See Katoh et al., Virology, 145, 280-92(1985), Crawford, et al., J. Virol., 53, 899-907(1985) and Debouk, et al., Proc. Natl. Acad. Sci. USA, 84, 8903-6(1987). Inhibiton of retroviral protease, therefore, presents a method of therapy for retroviral disease.

Retroviral proteases have not been well characterized. Both the proteases and their polyprotein substrates are recovered from virion particles in very low yield. To assess their activity, it is necessary to provide a substrate to express the proteolytic activity. Use of the natural substrate to assay proteolytic activity presents experimental difficulty in production, purification and quantitation of the substrate and its hydrolysis products. Many of these disadvantages can be overcome by the use of a small peptide which can be produced synthetically, and in pure form, to assay proteolytic activity. It is therefore highly desirable and advantageous to produce small peptides which may be used as substrates for these enzymes.

Proteases are enzymes which cleave peptide bonds in proteins and polypeptides. They are present in most biological systems, where they serve both metabolic and regulatory functions. Their role in metabolism is to hydrolyze polypeptides to smaller peptides and ultimately to their constituent amino acids. Their role in biological regulation is pervasive and is the subject of several reviews. See as an example Biological Functions of Proteinases, edited by H. Holzer and H. Tschesche, Springer-Verlag, Berlin-Heidelberg-New York (1979).

One of their regulatory roles is that of post-translational processing of polypeptides to form functional proteins, enzymes and mediators. Essential to their regulatory function is their ability to act selectively upon polypeptides. This selectivity is comprised of two factors, 1.) their ability to act upon specific substrates and 2.) their ability to hydrolyze only specific peptide bonds. On a structural level this substrate specificity is the result of the primary amino acid sequence, the resultant local conformation of the substrate polypeptide and the amino acids which form the peptide bond at the cleavage site.

Sequence information regarding the putative cleavage site in polyprotein natural substrates is available for several retroviral proteases. However, peptide substrates for retroviral proteases are heretofore unknown. Further, since the binding interactions of the natural substrate are unknown, it is often difficult to predict what length peptide will be acceptable. In a similar manner, since the conformation of a large polypeptide may be significantly different than that of a small peptide, it is difficult to predict which amino acid sequence will bind optimally in a small peptide.

Examination of viral proteins by amino acid sequencing has suggested certain common amino acid

residues within the cleavage region for retroviral proteases. For instance, Casey, et al., J. Virol., 55, 417-23-(1985) disclose the amino terminal sequence of the mature p24 gag protein from HIV-1 as Pro-Ile-Val-Gln-Asn, indicating that a proline residue is involved in the cleavage site. diMarzo Veronese, et al., Science, 231, 1289-90(1986) have proposed a cleavage site (indicated by *) for the reverse transcriptase (RT) of Thr-Leu-Asn-Phe*-Pro which gives RT a proline amino terminus. Debouck, et al., Proc. Natl. Acad.Sci. USA, 84, 8903-06(1987), have suggested that the protease of HIV-1 is autocatalytic and disclose that the amino terminus of the protease contains the sequence Pro-Gln-Ile-Thr-Leu. Pearl, et al., Nature, 328, 482(1987) have compared certain core precursor polypeptide substrates for a large group of retroviruses and suggest a sequence preference for cleavage at X-Pro for the retroviral proteases, wherein X is usually either an aromatic (Phe, Tyr) or a large and hydrophobic (Met, Leu) amino acid. It is further suggested that this sequence is usually flanked on either side by a small and hydrophobic amino acid.

The structure of many retroviral proteases has been examined, based primarily upon their predicted amino acid sequence deduced from their nucleotide structure. They are generally encoded at the 5′ end of the pol region, the 3′ end of gag region or between (but out of frame with) the two regions. Yasunaga et al., FEBS Lett., 199, 2, 145-49(1986) disclose good sequence homology between families of retrovirus and demonstrate several highly conserved sequences among members of the lentivirus family and the HTLV/BLV subfamily of retroviruses.

Methods to express retroviral proteases in E. coli have been disclosed by Debouck, et al., Proc. Natl. Acad. Sci. USA, 8903-06(1987) and Graves, et al., Proc. Natl. Acad. Sci. USA, 85, 2449-53(1988) for the HIV-1 virus.

Little biochemical characterization of retroviral proteases has been undertaken to date. However, it has been suggested by Katoh, et al., Nature, 329, 654-6(1987), that certain retroviral proteases may be acid proteases. This is based upon the observation that pepstatin, a known acid protease inhibitor, has been shown to be a weak inhibitor of the retroviral proteases associated with bovine leukemia virus, Moloney murine leukemia virus and human T-cell leukemia virus.

Methods for producing protease inhibitors of retroviral proteases have been disclosed by Kettner, et al., U.S. Patent No. 4,636,492 and Kettner, et al., U.S. Patent No. 4,644,055. Accordingly, tri- and tetra-peptides which substantially correspond to the amino acid sequence of the cleavage site of the substrate of the protease are modified to possess a C-terminal halomethyl ketone moiety. The halomethyl ketone, being a reactive moiety, is presumed to react with the protease once binding of the peptide has been effected.

Although unrelated to retroviral proteases, Umezawa et al. have isolated inhibitors of a diverse group of proteases by screening microbial culture filtrates. See Proteinase Inhibitors: Medical and Biological Aspects, eds. Katunuma et al., pp. 3-15(1983), Japan Sci. Soc. Press, Tokyo/Springer-Verlag, Berlin. Certain of these peptide inhibitors, such as bestatin, amastatin and pepstatin, contain unusual amino acids which are not α-amino acids. These inhibitors are believed to function by presenting a non-hydrolyzable carbon-carbon bond to the proteolytic enzyme at the scissile site.

Pepstatin, a pentapeptide inhibitor isolated from a Streptomyces culture, has been shown to inhibit a wide range of acid proteases, and has attracted attention due to its ability to inhibit renin and pepsin. (See Umezawa, H. et al., J. Antibiot., Tokyo, 23, 259-263(1970).) Pepstatin contains the unusual amino acid, (3S,4S)-4-amino-3-hydroxy-6-methylheptanoic acid (AHMHA), called statine, in two positions of the pentapeptide. This unusual amino acid is not an α-amino acid, but is believed to function as a dipeptide mimic and present a carbon-carbon bond to the enzyme instead of a scissile peptide bond. Peptide derivatives containing statine, have been used to inhibit the human proteases renin and pepsin. (See Cazaubon et al., U.S. Patent No. 4,481,192.) Modifications of pepstatin to optimize its activity toward the inhibition of renin, have been made by several groups. Rich et al., J. Med. Chem., 23, 27-33(1980), have shown that the chirality of the statine incorporated into pepstatin is important for binding and have made modifications of statine to probe the relationship between structure and inhibitory activity. Thus, (3S,4S)-4-amino-3-hydroxy-5-phenylpentanoic acid (AHPPA) showed activity similar to statine when incorporated into peptides in place of statine.

Other "dipeptide analogues" which contain a carbon-carbon bond in place of a peptide bond have been synthesized and studied by several groups. Many of these attempts have involved structures closely related to statine by modification of the carbon bearing the hydroxyl group and the adjacent carbon. Thus, oxo derivatives, diamino derivatives, dihydroxy derivatives (Thaisrivongs et al., J. Med. Chem., 30, 976-82-(1987)), deshydroxy derivatives (Rich et al., J. Med. Chem., 23, 27-33(1980)), phosphorous-containing derivatives (Grannousis, et al,, J. Med. Chem., 1603-09(1987)) and fluoro derivatives (Fearon et al., J. Med. Chem., 30, 1617-22(1987) and Thaisivongs et al., J. Med. Chem., 20, 2080-87(1986)) of statine have all been synthesized and reported for their ability to inhibit renin or pepsin. Variations, in which a substituted methylene group is inserted α to the carboxyl group of statine have also been reported for inhibition of renin

(Luly et al., European Patent Application EP 229667, Fung et al., PCT Patent Application PCT/WO 88/05050; Evans, U.S. Patent 4,665,055; Hester et al., European Patent Application EP 173 481). These "dipeptide analogues" have shown variable abilities to inhibit renin and pepsin, their specific acitvity being also dependent upon adjacent moieties which mediate binding to these enzymes. The synthetic methods employed within these disclosures are incorporated herein by reference.

There exists a need for peptides which bind retroviral proteases in order to assay protease activity and to provide inhibitors of retroviral protease activity for pharmaceutical use. Such pharmaceutical use provides therapy for diseases which have been heretofore untreatable.

## SUMMARY OF THE INVENTION

This invention comprises peptides, hereinafter represented as formula (I), which bind to retroviral proteases. In one respect, these peptides are useful as substrates for assaying protease activity. In another respect, these peptides are inhibitors of viral protease and are useful for treating disease related to infection by these agents.

This invention is also a pharmaceutical composition, which comprises a compound of formula (I) and a pharmaceutically acceptable carrier.

This invention further constitutes a method for treating viral disease, which comprises administering to a mammal in need thereof an effective amount of a compound of formula (I).

This invention also provides a method for assaying viral protease activity.

In yet another aspect, this invention is a compound, as shown hereinafter as formula (IIb), which can be used as an intermediate in the preparation of the peptides of formula (I), which renders the peptide resistant to degradation by a viral protease.

## DETAILED DESCRIPTION OF THE INVENTION

The peptides of this invention are illustrated by formula (I):

$A\text{-}B\text{-}(Q)_a\text{-}(C)_b\text{-}(D)_c\text{-}M\text{-}(W)_d\text{-}(X)_e\text{-}Y\text{-}Z$ (I)

wherein:

A is BocNH, CbzNH, H, $R'R''N$, $R''CONR'$ or DnsNH, or if a,b and c are 0 and Y is a covalent bond, then A is H, Boc, Cbz, $R''$ or $R''CO$;

B is one or more D or L amino acids, β-Ala or is a covalent bond;

C and D are the same or different and are Glx, Asx, Ala, β-Ala, Arg, Gly, Ile, Leu, Lys, Ser, Thr, Val, Met or His;

Q is a D or L amino acid and is Ser, Thr, Asp, His, Cys, Arg or Ala;

W is Pro or Δ3-dehydro-Pro ;

X is Ala, Gly, Ile, Leu, Val, Met, Lys, Glx or Asx;

Y is one or more D or L amino acids, or is a covalent bond;

Z is $CO_2R''''$, $CONR'R''''$, $COR'$, $CH_2OH$, $CH_2NR'R''''$ or H, or if d and e are 0 and Y is a covalent bond, Z is $OR''''$ or $NR'R''''$;

a, b, c, d and e are each independently 0 or 1, provided that c and e are not simultaneouly 0;

M is Cha, Phe($4'R_a$) or $-NHCHR_1R_2-$;

wherein:

$R_1$ is independently $C_{1-5}Alk$, $(CH_2)_nSC_{1-5}Alk$, $(CH_2)_nOC_{1-5}Alk$, $(CH_2)_nC_{3-7}$cycloalkyl or $(CH_2)_mC_6H_4\text{-}R_a$;

$R_a$ is halogen, $OR'$, $NO_2$, $NH_2$ or H;

$R_2$ is $(CH_2)_n$-, $CHR_3(CH_2)_mCO$-, $CO(CH_2)_mCO$-, $CHR_3CHR_3'(CH_2)_mCO$-, $CHR_3CHR_3'CHR_1(CH_2)_mCO$-, $CHR_3CHR_1CHR'(CH_2)_mCO$-, $CHR_3CHR'CHR_1(CH_2)_mCO$-, $COCHR_1CHR'(CH2)_mCO$-, $COCF_2CR'R''(CH_2)_mCO$-, $COCF_2(CH_2)_mCO$-, $CH=CR'CHR''(CH_2)_mCO$-, $COCH=CR'(CH_2)_mCO$-, $CH_2S(O)_pCHR'(CH_2)_mCO$-, $CH_2NR'CHR''(CH_2)_mCO$-, $P=O(OR')(CH_2)_mCO$-,

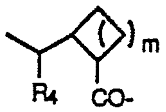 , 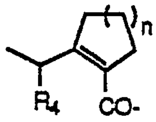 , 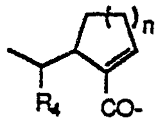 , 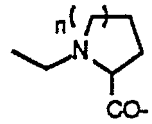 , 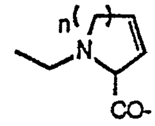 ,

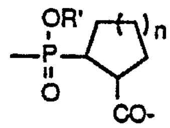 or 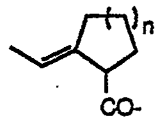 ;

$R_3$ and $R_3'$ are each independently OH, H or $NH_2$;
$R_4$ is OH, H, $NH_2$ or =0;
m is independently 0, 1, 2 or 3;
n is independently 1 or 2;
p is 0, 1 or 2; and
$R'$ is H or $C_{1-5}$Alk;
$R''$ is H or $C_{1-18}$Alk;
$R''''$ is H, $C_{1-5}$Alk, $C_{3-6}$cycloalkyl, $(CH_2)_nC_6H_5$, $(CH_2)_nC_5H_4N$, $(CH_2)_nOH$, $(CH_2)_nNH_2$, or $(CH_2)_nNHC(NH)$-$NH_2$;
and pharmaceutically acceptable salts thereof.

Also included in this invention are pharmaceutically acceptable addition salts, complexes or prodrugs of the compounds of this invention. Prodrugs are considered to be any covalently bonded carriers which release the active parent drug according to formula (I) in vivo. Inasmuch as it may differ from conventional notation, it should be noted in formula (I) that A comprises the terminal amino group of the peptide and Z comprises the terminal carboxyl group of the peptide. Thus, when A and Z are H, the terminal residues of the peptide are "des-amino" and "descarboxy" amino acids respectively.

Employing this representation, A comprises the terminal amino group of the residue corresponding to B; or, when B is a covalent bond, to Q; or, when a is 0 and B is a covalent bond, to C; or when B is a covalent bond and a and b are 0, to D. When B is a covalent bond and a, b, and c are 0, the amino group of M is substituted by an acyl or alkyl group, as specially provided by A in formula (I). In similar fashion, Z comprises the terminal carboxyl group of the amino acid residue corresponding to Y; or when Y is a covalent bond, to X; or When Y is a covalent bond and d and e are 0, the terminal carboxyl group of M is substituted by Z as specially provided in formula (I).

The operation of the subscripts a-c in formula (I) is to denote that residues may be removed from the amino terminus of the peptide with retention of utility in the compounds.. Such operation is intended to proceed in a sequential fashion; such that a is not 0 unless, Y is a covalent bond, b is not 0 unless a is 0, and c is not 0 where b is 0. At the carboxy terminus, e is not 0 unless Y is a covalent bond. As more fully described hereinafter, d operates independently and is 1 when M is Cha or Phe($R_a$), and either 1 or 0, preferably 0, when M is -$NHCHR_1R_2$.

Other abbreviations and symbols commonly used in the art are used herein to describe the peptides.

| Amino Acid | 3 letter code | 1 letter code | Amino Acid | 3 letter code | 1 letter code |
|---|---|---|---|---|---|
| Alanine | Ala | A | Leucine | Leu | L |
| Arginine | Arg | R | Lysine | Lys | K |
| Asparagine | Asn | N | Methionine | Met | M |
| Aspartic Acid | Asp | D | Phenylalanine | Phe | F |
| Cysteine | Cys | C | Proline | Pro | P |

| Amino Acid | 3 letter code | 1 letter code | Amino Acid | 3 letter code | 1 letter code |
|---|---|---|---|---|---|
| Glutamine | Gln | Q | Serine | Ser | S |
| Glutamic Acid | Glu | E | Threonine | Thr | T |
| Glycine | Gly | G | Tryptophan | Trp | W |
| Histidine | His | H | Tyrosine | Tyr | Y |
| Isoleucine | Ile | I | Valine | Val | V |
| Asparagine or Aspartic Acid | | | | Asx | B |
| Glutamine or Glutamic Acid | | | | Glx | Z |

In accordance with conventional representation, the amino terminus is on the left and the carboxy terminus is on the right. Unless specified otherwise, all chiral amino acids (AA) are assumed to be of the L absolute configuration. (4′$R_a$)Phe refers to phenylalanine substituted in the 4 position of the phenyl ring by $R_a$. It should be appreciated that when $R_a$ is hydrogen, the residue constitutes phenylalanine and when $R_a$ is OH, the residue constitutes tyrosine. β-Ala refers to 3-amino propanoic acid. Cha refers to cyclohexylalanine. Boc refers to the t-butyloxycarbonyl radical, Dns refers to the dansyl radical, which is 1-dimethylamino napthylene-5-sulfonyl, Cbz refers to the carbobenzyloxy radical, BrZ refers to the o-bromobenzyloxycarbonyl radical, Clz is the p-chlorocarbobenzyloxy radical, $Cl_2Z$ refers to the 2,4-dichlorocarbobenzyloxy radical, Bzl refers to the benyzl radical, MeBzl refers to the 4-methyl benzyl radical, Ac refers to acetyl, Alk or $C_{1-5}$Alk refers to $C_{1-5}$ alkyl, Ph refers to phenyl, DCC refers to dicyclohexylcarbodiimide, DMAP refers to dimethylaminopyridine, HOBT refers to 1-hydroxybenzotriazole, NMM is N-methylmorpholine, DTT is dithiothreitol, EDTA is ethylenediamine tetraacetic acid, DIEA is diisopropyl ethylamine, DBU is 1,8 diazobicyclo[5.4.0]undec-7-ene, DMSO is dimethylsulfoxide, DMF is dimethyl formamide and THF is tetrahydrofuran. HF refers to hydrofluoric acid and TFA refers to trifluoroacetic acid. $C_{1-5}$alkyl as applied herein is meant to include methyl, ethyl, propyl, isopropyl, butyl, isobutyl, sec-butyl, tertiary butyl, pentyl and isopentyl. As used herein in the compounds of this invention, Asp and Glu, which have carboxylic acid side chains, encompass free carboxylic acids, $C_{1-5}$alkyl and benzyl ester side chains. $C_{1-18}$Alk is intended to include any straight or branched chain alkyl group of 1 to 18 carbons.

The peptides of this invention bind to viral proteases in a manner which mimics the binding of the natural substrate. The peptides are generally dodecapeptides or smaller. However, longer peptides which encompass the residues defined herein as -Q-C-D-M-W-X-, as given in formula (I), are also believed to be active and are considered within the scope of this invention. The residues closest to the putative cleavage site of the peptide, designated as M-W, are most important for binding. X is preferably a neutral or acidic amino acid. Suitably, X is Ala, Gly, Ile, Leu, Val, Met, Lys, Glx or Asx. Valine is especially suitable. D is preferably a neutral and hydrophobic amino acid, although certain hydrophilic residues such as Asp, Glu and Ser are acceptable. Glx, Asx, Ala, β-Ala, Ile, Leu, Val and Met are preferred. Gln, Asn and Ala are especially preferred. The residue corresponding to C may be a neutral, acidic or basic amino acid. Glu, Gln, Arg, Lys, Ser, Ala, β-Ala, Asn and Gly are suitable. Gln, Asn and Ala are especially preferred for C. The residue Q is preferably a hydrophilic residue, such as D- or L-, Ser, Thr, Asp or His. Especially preferred are Thr and Ser.

B corresponds to one or more amino acids, which may be hydrophilic or hydrophobic, or it may be a covalent bond in the shorter peptides. The identity of B is not critical and a residue may be chosen for the favorable physico-chemical and biochemical properties it confers on the overall peptide, such as water solubility and resistance to exopeptidases. The choice of a D-amino acid often confers resistance to exopeptidases when the D-amino acid is at the terminus of the peptide. When B is a covalent bond, it may be advantageous for Q to be a D-amino acid. When B is not a covalent bond, B is preferably one or two D- or L-amino acids chosen from Ala, β-Ala, Gly, Ile, Val, Leu, Met, His, Lys, Arg, Glx, Asx, Cys, Ser or Thr. For producing the smallest peptides of this invention which bind to retroviral protease, B is preferably a covalent bond.

Y may correspond to one or more amino acids, or a covalent bond. If it is one or more amino acids, they may be hydrophilic or hydrophobic. One to three residues are preferred, but a longer chain is acceptable. In much the same manner as B, the residues of Y may be used to confer favorable biochemical

or physio-chemical properties to the peptide. Thereby, the use of hydrophilic residues may be used to confer desirable solubility properties or D-amino acids at the carboxy terminus may be used to confer resistance to exopeptidases. Preferably Y is one to three amino acids chosen from Ala, Gly, Ile, Leu, Met, Val, Arg, Lys, Thr, Ser, Cys, Glx or Asx. For the shorter peptides of this invention, Y may be a covalent bond but a single amino acid is especially suitable. Ala, Gly, Ile, Met, Arg, Asx and Val are preferred. Valine is especially preferred.

Peptides wherein M is Cha or Phe(4′Ra) and d is 1 may act as substrates, and are hydrolyzed to smaller peptides. Although the substrates may generally be of any length, 6-9 residues is preferred. These are conveniently used to assay for protease activity. In addition, they may compete with the natural viral substrates and thereby serve to inhibit viral replication and, hence, disease progression in vivo; although, due to their metabolic instability, their duration of action may be short. Typically, these peptides may be used in an assay for protease activity by subjecting the peptide to the protease in a suitably buffered medium. Analysis of the activity is carried out in such a manner as to detect the hydrolytic cleavage of the peptide. One such method constitutes the separation of at least one of the product peptides, or a derivative thereof, and its quantitation.

In such assay, chromatographic means can be used to effect the separation of the peptide products using conventional solid supports such as silica gel, octadecyl silane, Sephadex®, ion exchange resins or adsorption resins. Detection/quantitation of the products is effected by ultraviolet absorption or other spectroscopic analysis. Alternatively, incorporation or substitution of a radioactive isotope in the peptide, such as tritium or $^{13}C$ label in one or more of the amino acids, provides for detection/quantitation based upon radioactivity. In another alternative, incorporation of a fluorescent moiety, such as a dansyl group on the amino terminus of the peptide, provides for fluorescent detection/quantitation. Fluorogenic methods, which would incorporate a quenching agent into the peptide, such as a 3-(4-N-methyl-pyridyl)-propyl oxy ester of the carboxy terminus, in addition to the fluorescent marker, would also be useful to assay protease activity. Such methods are disclosed, for example, by Dunn, et al., Anal. Biochem., 129, 502(1983), and provide for continuous monitoring of the activity. These fluorogenic methods have advantages in that the hydrolysis is observed directly without a need for separation of the hydrolysis products.

The protease binding activity of the peptides of this invention, wherein M is Cha or (4′Ra)Phe, is demonstrated by their ability to act as substrates. The following table illustrates the substrate kinetics of these peptides.

Table I
Substrate Kinetics of
Peptides Binding to rHIV Protease

| Compound of Example No. | Rel. $V_o$ | $K_M$ (mM) | $K_{cat}$ ($min^{-1}$) |
|---|---|---|---|
| 1 | 1.0 | 6.7 | 42 |
| 39 | 1.0 | 6.9 | 41 |
| 40 | .35 | 8.6 | 16 |
| 41 | neg.* | - | - |
| 42 | 1.34 | 1.9 | 21 |
| 43 | .48 | 20 | 30 |
| 44 | .48 | 2.2 | 5 |

Table I (Continued)
Substrate Kinetics of
Peptides Binding to rHIV Protease

| Compound of Example No. | Rel. $V_o$ | $K_M$ (mM) | $K_{cat}$ ($min^{-1}$) |
|---|---|---|---|
| 45 | .46 | 11 | 21 |
| 46 | 1.23 | 2.3 | 12 |
| 47 | .36 | 8 | 15 |
| 48 | 1.0 | 6.8 | 35 |
| 49 | .65 | 6.0 | 18.4 |
| 50 | .32 | 5.0 | 1.8 |
| 51 | neg.* | - | - |
| 52 | neg.* | - | - |
| 53 | .71 | 1.6 | 12 |
| 54 | .39 | .71 | 2.03 |
| 55 | .86 | 6.5 | 23.0 |
| 56 | 1.45 | 1.2 | 31 |
| 57 | neg.* | - | - |
| 59 | .56 | 3.3 | 8.0 |
| 60 | .54 | 8.0 | 18 |
| 61 | .51 | 2.3 | 6.4 |
| 62 | .05 | - | - |
| 63 | .045 | 4.9 | 1.8 |
| 64 | 1.24 | - | - |
| 65 | .11 | 19 | 14 |
| 66 | 1.25 | 11.7 | 183 |
| 67 | .07 | - | - |
| 68 | .98 | - | - |
| 69 | .70 | - | - |
| 70 | .56 | - | - |
| 95 | neg.* | 12.8 | 5.3 |
| 96 | .34 | 17.7 | 90.4 |
| 98 | .16 | 25 | 65 |
| 99 | .09 | 6.2 | 1.4 |
| 100 | .045 | 1.8 | .38 |
| 101 | .06 | 6.2 | 7.8 |

Table I (Continued)
Substrate Kinetics of
Peptides Binding to rHIV Protease

| Compound of Example No. | Rel. $V_o$ | $K_M$ (mM) | $K_{cat}$ ($min^{-1}$) |
|---|---|---|---|
| 102 | 1.27 | 7.7 | 96 |
| 105 | 1.09 | - | - |
| 107 | .24 | - | - |
| 108 | neg.* | - | - |
| 109 | 1.24 | - | - |
| 110 | 1.29 | 9.8 | 103 |
| 111 | 1.33 | 34 | 374 |
| 112 | neg.* | - | - |
| 113 | neg.* | - | - |
| 115 | .48 | 7.1 | 16 |
| 118 | neg.* | - | - |
| 119 | neg.* | 30 | - |
| 121 | .80 | .96 | 16.6 |

*negligible
-not done

In another respect, these substrate peptides in which M is Cha or Phe(4′Ra) are also useful indicators for discovering amino acid sequences which bind to viral proteases and thereby inhibit the proteolytic activity. Modifications of such peptides which bind to viral proteases so as to retard the hydrolysis of the peptide provide a means for effecting longer lived inhibition of protease activity.

Accordingly, another subgeneric group of compounds of this invention comprises the peptides wherein the residue M is $-NHCHR_1R_2-$. These peptides contain an unnatural amino acid, which is not an α-amino acid. These peptides do not possess a peptide bond in the same manner as a substrate and therefore, although they bind to the protease in a manner which mimics the binding of the natural substrate, are resistant to hydrolysis. Peptides of 2-9 residues are suitable. Peptides of 3-6 residues are preferred. Preferably when M is $-NHCHR_1R_2-$, d = 0 and W is absent, since the unnatural amino acid may act as a dipeptide mimic. In one preferred embodiment $R_1$ is $CH_2C_6H_4-R_a$, $CH_2C_6H_{11}$ or $C_{1-5}Alk$. $R_2$ is preferably $CHR_3CHR_3'(CH_2)_mCO-$, $CHR_3CHR_3'CHR_1CO-$, $CHR_3CHR_1CHR'CO-$, $CHR_3(CH_2)_mCO-$, $CO(CH_2)_mCO-$, $COCHR_1CHR'(CH_2)_mCO-$, $CHR_3CF_2(CH_2)_mCO-$,$-COCF_2(CH_2)_mCO-$, $-P=O(OR')(CH_2)_mCO-$,

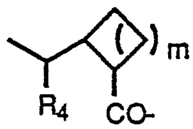 , 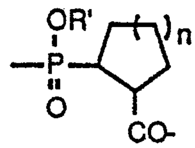 or 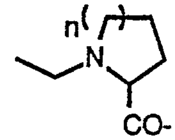 .,

wherein, R′, $R_3$, $R_3$′, m and n have the meanings defined in formula (I). In preferred embodiments, $R_1$ is $CH_2C_6H_4$-$R_a$ and $R_2$ is $CH(OH)CH_2CH_2CO$-. Suitably the peptides of formula (I) have the partial structure : -Ala-NHCH($CH_2$Ph)CH(OH)$CH_2CH_2$CO-Val-Val-.

When administered to an animal infected or potentially infected with a virus, which is dependent upon a virally encoded protease for processing of viral polyproteins, viral replication is inhibited, hence, disease progression is retarded. Inasmuch as there appear to exist consensus sequences for the polyprotein substrates of various retroviruses, an inhibitor or substrate is likely to be broadly active over the range of retroviruses. DNA viruses, which are dependant upon virally encoded proteases, such as the hepatitis virus, may also be susceptible to such treatment.

The following compounds are included in, but in no way limit, the scope of this invention:
2-(acetyl-serylglutaminylasparaginyl)amino-3-phenylpropyl-prolylvalyl valinamide;
2-(serylglutaminylasparaginyl)amino-3-phenylpropylprolylvalyl valinamide;
2-(acetyl-serylglutaminylasparaginyl)amino-3-hydroxy-1-oxo-5-phenylpentylprolylvalyl valinamide;
2-(acetyl-serylglutaminylasparaginyl)amino-3-hydroxy-1-oxo-5-phenylpentylvalyl valinamide;
2-(seryl glutaminylasparaginyl)amino-3(S)-hydroxy-1-oxo-5-phenyl pentylvalyl valinamide;
2-((2-(tert-butoxycarbonyl-serylalanylalanyl)amino-1-oxo-3-phenylpropyl)cyclopentylcarbonylvalyl valine methyl ester;
2-(2-(serylalanylalanyl)amino-1-oxo-3-phenylpropyl)cyclopentyl-carbonylvalylvaline methyl ester, trifluoracetic acetic acid salt;
2-(2-(tert-butoxycarbonyl-serylalanylalanyl)amino-1-hydroxy-3-phenylpropyl)cyclopentylcarbonylvalyl valine methyl ester;
2-(-1-hydroxy-3-phenyl-2-(serylalanylalanyl)aminopropyl)cyclopentylcarbonylvalylvaline methyl ester, trifluoracetic acid salt;
2-((1-methoxy-1-(2-phenyl-1-(tert-butyloxycarbonyl-serylalanylalanyl)amino)ethyl)phosphinyl)-cyclopentylcarbonylvalylvaline methyl ester;
2-((1-hydroxy-1-(2-phenyl-1-(serylalanylalanyl)amino) ethyl)phosphinyl)cyclopentylcarbonyl-valyl valine methyl ester;
5-(tert-butoxycarbonyl-serylalanylalanyl)amino-4-hydroxy-1-oxo-6-phenylhexyl-valyl valine methyl ester;
5-(tert-butoxycarbonyl-serylalanylalanyl)amino-4-hydroxy-1-oxo-6-(4-hydroxy-phenyl)hexyl-valyl valine methyl ester;
4-(tert-butoxycarbonyl-serylalanylalanyl)amino-2,2-difluoro-3-hydroxy-1-oxo-5-phenylpentyl-valyl valine methyl ester, hydrochloride;
4-(serylalanylalanyl)amino-2,2-difluoro-3-hydroxy-1-oxo-5-phenylpentyl-valyl valine methyl ester, hydrochloride;
4-(serylalanylalanyl)amino-2,2-difluoro-1,3-dioxo-5-phenylpentyl-valyl valine methyl ester, hydrochloride;
5-(t-butyloxycarbonylserylalanylalanyl)amino-4-hydroxy-6-(4-hydroxy)phenyl-1-oxo-hexyl-valyl amide
5-((carbobenzyloxy-D-seryl)alanylalanyl)amino-4-hydroxy-1-oxo-6-phenylhexyl-valyl valine methyl ester;
5-((D-seryl)alanylalanyl)amino-4-hydroxy-1-oxo-6-phenylhexyl-valyl valine methyl ester;
5-(t-butyloxycarbonylserylalanylalanyl)amino-6-(4-hydroxy)phenyl-4-hydroxy-1-oxo-hexyl-valyl valine;
5-(serylalanylalanyl)amino-6-(4-hydroxy)phenyl-4-hydroxy-1-oxo-hexyl-valyl valine, hydrochloride;
2-((1-hydroxy-2-(serylalanylalanyl)amino-3-cyclohexyl)propyl)cyclopentanecarbonyl-valyl valine methyl ester, hydrochloride;
2-((2-(tert-butoxycarbonyl-serylalanylalanyl)amino-1-oxo-3-phenylpropyl)cyclopentylcarbonylvalyl valinamide;
2-(2-(serylalanylalanyl)amino-1-oxo-3-phenylpropyl)cyclopentylcarbonylvalylvalinamide, trifluoracetic acetic acid salt;
2-(2-(tert-butoxycarbonyl-serylalanylalanyl)amino-1-hydroxy-3-phenylpropyl)cyclopentylcarbonylvalyl valinamide;
2-(-1-hydroxy-3-phenyl-2-(serylalanylalanyl)aminopropyl)-cyclopentylcarbonylvalylvalinamide, trifluoracetic acetic acid salt;
2-((1-methoxy-1-(2-phenyl-1-(tert-butyloxycarbonyl-serylalanylalanyl)amino)ethyl)phosphinyl)-cyclopentylcarbonylvalylvalinamide;

2-((1-hydroxy-1-(2-phenyl-1-(serylalanylalanyl)amino)ethyl)phosphinyl)cyclopentylcarbonyl-valyl valinamide;
5-(tert-butoxycarbonyl-serylalanylalanyl)amino-4-hydroxy-1-oxo-6-phenylhexyl-valyl valinamide;
5-(tert-butoxycarbonyl-serylalanylalanyl)amino-4-hydroxy-1-oxo-6-(4-hydroxy-phenyl)hexyl-valyl valinamide;
4-(tert-butoxycarbonyl-serylalanylalanyl)amino-2,2-difluoro-3-hydroxy-1-oxo-5-phenylpentyl-valyl valinamide, hydrochloride;
4-(serylalanylalanyl)amino-2,2-difluoro-3-hydroxy-1-oxo-5-phenylpentyl-valyl valinamide, hydrochloride;
4-(serylalanylalanyl)amino-2,2-difluoro-1,3-dioxo-5-phenylpentyl-valyl valinamide, hydrochloride;
5-((carbobenzyloxy-D-seryl)alanylalanyl)amino-4-hydroxy-1-oxo-6-phenylhexyl-valyl valinamide;
5-((D-seryl)alanylalanyl)amino-4-hydroxy-1-oxo-6-phenylhexyl-valyl valinamide;
5-(t-butyloxycarbonylserylalanylalanyl)amino-6-(4-hydroxy)phenyl-4-hydroxy-1-oxo-hexyl-valyl valinamide;
5-(serylalanylalanyl)amino-6-(4-hydroxy)phenyl-4-hydroxy-1-oxo-hexyl-valyl valinamide, hydrochloride;
2-((1-hydroxy-2-(serylalanylalanyl)amino-3-cyclohexyl)propyl)cyclopentanecarbonyl-valyl valinamide, hydrochloride;
(4S,5S)-5-(alanylalanyl)amino-4-hydroxy-1-oxo-6-(4-hydroxyphenyl)hexyl-valyl valine methyl ester, acetic acid salt;
(4S,5S)-5-((carbobenzyloxy-β-alanyl)alanyl)amino-4-hydroxy-1-oxo-6-(4-hydroxyphenyl)hexyl-valyl valine methyl ester;
(4S,5S)-5-((β-alanyl)alanyl)amino-4-hydroxy-1-oxo-6-(4-hydroxyphenyl)hexyl-valyl valine methyl ester, hydrochloride;
(4S, 5S)-5-(alanylalanyl)amino-4-hydroxy-1-oxo-6-cyclohexyl-hexyl-valyl valine methyl ester;
(4S,5S)-5-(carbobenzyloxyalanylalanyl)amino-4-hydroxy-1-oxo-6-phenylhexyl-valyl valine methyl ester;
(4S,5S)-5-(alanylalanyl)amino-4-hydroxy-1-oxo-6-phenylhexyl-valyl valine methyl ester;
(4S,5S)-5-(alanylalanyl)amino-4-hydroxy-1-oxo-6-(4-hydroxyphenyl)hexyl-valyl valinamide, acetic acid salt;
(4S,5S)-5-((carbobenzyloxy-β-alanyl)alanyl)amino-4-hydroxy-1-oxo-6-(4-hydroxyphenyl)hexyl-valyl valinamide;
(4S,5S)-5-((β-alanyl)alanyl)amino-4-hydroxy-1-oxo-6-(4-hydroxyphenyl)hexyl-valyl valinamide, hydrochloride;
(4S, 5S)-5-(alanylalanyl)amino-4-hydroxy-1-oxo-6-cyclohexyl-hexyl-valyl valinamide;
(4S,5S)-5-(carbobenzyloxyalanylalanyl)amino-4-hydroxy-1-oxo-6-phenylhexyl-valyl valinamide;
(4S,5S)-5-(alanylalanyl)amino-4-hydroxy-1-oxo-6-phenylhexyl-valyl valinamide;
(4S,5S)-5-(carbobenzyloxyalanyl)amino-4-hydroxy-1-oxo-6-phenylhexyl-valyl valine methyl ester;
(4S,5S)-5-(alanyl)amino-4-hydroxy-1-oxo-6-phenylhexylvalyl valine, acetic acid salt;
(4S,5S)-5-(tert-butyloxycarbonylalanyl)amino-4-hydroxy-1-oxo-6-(4-hydroxyphenyl)hexyl-valyl valine methyl ester;
(4S,5S)-5-alanylamino-4-hydroxy-1-oxo-6-(4-hydroxyphenyl)-hexyl-valyl valine methyl ester, acetic acid salt;
(4S,5S)-5-(carbobenzyloxyalanyl)amino-4-hydroxy-1-oxo-6-phenylhexyl-valyl valinamide;
(4S,5S)-5-(alanyl)amino-4-hydroxy-1-oxo-6-phenylhexyl-valyl valinamide;
(4S,5S)-5-(tert-butyloxycarbonylalanyl)amino-4-hydroxy-1-oxo-6-(4-hydroxyphenyl)hexyl-valyl valinamide;
(4S,5S)-5-(alanyl)amino-4-hydroxy-1-oxo-6-(4-hydroxyphenyl)-hexyl-valyl valinamide, acetic acid salt;
(4RS,5S)-5-amino-6-(4-hydroxy)phenyl-4-hydroxy-1-oxo-hexyl-valyl valine benzyl ester,hydrochloride;
(1R,2R)-2-(((1S,2S)-1-hydroxy-2-amino-3-cyclohexyl)propyl)-cyclopentanecarbonyl-valyl valine methyl ester, hydrochloride;
(4S,5S)-5-amino-4-hydroxy-1-oxo-6-phenylhexyl-valyl valine methyl ester, hydrochloride;
(4S,5S)-5-(tert-butyloxycarbonyl)amino-4-hydroxy-1-oxo-6-phenylhexyl-valyl valine methyl ester;
(4RS,5S)-5-amino-6-(4-hydroxy)phenyl-4-hydroxy-1-oxo-hexyl-valyl valinamide, hydrochloride;
(1R,2R)-2-(((1S,2S)-1-hydroxy-2-amino-3-cyclohexyl)propyl)-cyclopentanecarbonyl-valyl valinamide, hydrochloride;
(4S,5S)-5-amino-4-hydroxy-1-oxo-6-phenylhexyl-valyl valinamide, hydrochloride;
(4S,5S)-5-(tert-butyloxycarbonyl)amino-4-hydroxy-1-oxo-6-phenylhexyl-valyl valinamide;
(5S)-5(carbobenzyloxyalanyl)amino-4-hydroxy-1-oxo-6-phenyl-hexyl-valine, isobutyl amide;
(5S)-5-(myristyl)amino-4-hydroxy-1-oxo-6-phenyl-hexyl valine, isobutyl amide;
(5S)-5-(carbobenzyloxyalanylalanyl)amino-4-hydroxy-1-oxo-6-phenyl-hexyl valine, isobutylamide;
(5S)-5-(stearyl)amino-4-hydroxy-1-oxo-6-phenyl-hexyl valine, isobutyl amide;
5-(carbobenzyloxyalanyl)amino-4-hydroxy-2-methyl-1-oxo-6-phenyl-hexylvalyl valine, methyl ester;
7-methyl-5-(carbobenzyloxyalanyl)amino-3,4-dihydroxy-2-phenyl-1-oxo-octyl valine, isobutylamide;
5-(carbobenzyloxyalanylalanyl)amino-4-hydroxy-1-oxo-6-phenylhexyl valine, isobutyl amide;
5-(carbobenzyloxy-alanylalanyl)amino-6-phenyl-4-hydroxy(1-oxo)hexyl-valyl valinol;
carbobenzyloxy-alanyl-(3-hydroxy-5-amino-6-phenyl)hexanoyl-valyl-(4-aminomethyl)pyridine;
5-(carbobenzyloxyalanylalanyl)amino-4-hydroxy-6-phenyl(1-oxo)hexyl-valine isobutylamide;

(4S,5S)-5-(methoxycarbonyl-alanylalanyl)amino-6-phenyl-4-hydroxy-1-oxo-hexyl-valyl valine;
(4S,5S)-5-(carbobenzyloxy-alanylalanyl)amino-6-phenyl-4-hydroxy-1-oxo-hexyl-valyl valine;
(4S,5S)-5-(alanylalanyl)amino-6-phenyl-4-hydroxy-1-oxo-hexyl-valyl valine;
(4RS,5S)-5-((tert-butyloxycarbonyl)isoleucyl)amino-7-methyl-4-hydroxy-1-oxo-octyl-leucyl-(O-benzyl)aspartic acid methyl ester; and
(4RS,5S)-5-((tert-butyloxycarbonyl)isoleucyl)amino-7-methyl-4-hydroxy-1-oxo-octyl-leucyl-aspartic acid methyl ester.

Certain individual preferred compounds are:
(4S,5S)-5-(carbobenzyloxy-alanylalanyl)amino-6-phenyl-4-hydroxy-1-oxo-hexyl-valyl valine methyl ester;
(4S,5S)-5-(alanylalanyl)amino-6-phenyl-4-hydroxy-1-oxo-hexyl-valyl valine methyl ester;
(4S,5S)-5-(carbobenzyloxy-alanyl)amino-6-phenyl-4-hydroxy-1-oxo-hexyl-valyl valine methyl ester;
(4S,5S)-5-(alanyl)amino-6-phenyl-4-hydroxy-1-oxo-hexyl-valyl valine methyl ester; and
(4RS,5S)-5-((tert-butyloxycarbonyl)isoleucyl)amino-7-methyl-4-hydroxy-1-oxo-octyl-leucyl-(O-benzyl)aspartic acid methyl ester; and the carboxylic acids and carboxamides thereof.

The peptides of the invention are prepared by coupling the appropriate amino acid residues, optionally removing any protective groups and optionally modifying the amino or carboxy terminus of the peptide. They are prepared preferably by the solid phase technique of Merrifield (J. Am. Chem. Soc., 85, 2149 (1964)), although solution methods known to the art may be successfully employed. Solution methods or a combination of solid phase and solution methods may be used in a convergent synthesis in which di-, tri-, tetra-, or penta-peptide fragments are prepared by solid phase or solution synthesis and either coupled to other di-, tri- or tetra-peptides, or further modified by solution synthesis. The methods of peptide synthesis generally set forth in J. M. Stewart and J. D. Young, "Solid Phase Peptide Synthesis", Pierce Chemical Company, Rockford, Il (1984) or M. Bodonsky, Y. A. Klauser and M. A. Ondetti, "Peptide Synthesis", John Wiley & Sons, Inc., New York, N,Y. (1976) may be used to produce the peptides of this invention and are incorporated herein by reference.

Each amino acid or peptide is suitably protected as known in the peptide art. For example, the Boc- or carbobenzyloxy-group is preferred for protection of the amino group, especially at the α position. A benzyl group or suitably substituted benzyl group is used to protect the mercapto group of cysteine, or other thiol containing amino acids; or the hydroxyl of serine or threonine. The tosyl or nitro group may be used for protection of the guanidine of Arg or the imidazole of His, and a suitably substituted carbobenzyloxy group or benzyl group may be used for the hydroxyl group of Tyr, Ser or Thr, or the ε-amino group of lysine. Suitable substitution of the carbobenzyloxy or benzyl protecting groups is ortho and/or para substitution with chloro, bromo, nitro or methyl, and is used to modify the reactivity of the protective group. Cysteine and other sulfur-containing amino acids may also be protected by formation of a disulfide with a thioalkyl or thioaryl group. Except for the Boc group, the protective groups are, most conveniently, those which are not removed by mild acid treatment. These protective groups are removed by such methods as catalytic hydrogenation, sodium in liquid ammonia or HF treatment as known in the art.

If solid phase methods are used, the peptide is built up sequentially starting from the carboxy terminus and working toward the amino terminus of the peptide. Solid phase synthesis is begun by covalently attaching the C terminus of a protected amino acid to a suitable resin, such as a benzhydrylamine resin (BHA), methylbenzhydrylamine resin (MBHA) or chloromethyl resin (CMR), as is generally set forth in U.S. Patent No. 4,244,946. A BHA or MBHA support resin is used if the carboxy terminus of the product peptide is to be a carboxamide. A CMR support is generally used if the carboxy terminus of the product peptide is to be a carboxyl group, although this may also be used to produce a carboxamide or ester.

Once the first protected amino acid (AA) has been coupled to the desired resin, the amino group is hydrolyzed by mild acid treatment, and the free carboxyl of the second protected AA is coupled to this amino group. This process is carried out sequentially, without isolation of the intermediate, until the desired peptide has been formed. The completed peptide may then be deblocked and/or split from the carrying resin in any order.

Treatment of a CMR supported peptide with HF or HBr/acetic acid splits the peptide from the resin and produces the carboxy terminal amino acid as a carboxylic acid. Treatment of a CMR supported peptide with ammonia or alkyl amines in an alcoholic solvent provides a carboxamide or alkyl carboxamide at the carboxy terminus.

If an ester is desired, the CMR resin may be treated with an appropriate alcohol, such as methyl, ethyl, propyl, butyl or benzyl alcohol, in the presence of triethylamine to cleave the peptide from the resin and produce the ester directly.

Esters of the peptides of this invention may also be prepared by conventional methods from the carboxylic acid precursor. Typically, the carboxylic acid is treated with an alcohol in the presence of an acid

catalyst. Alternatively, the carboxylic acid may be converted to an activated acyl intermediate, such as an acid halide or activated anhydride or ester, and treated with an alcohol, preferably in the presence of a base.

The preferred method for cleaving a peptide from the support resin is to treat the resin supported peptide with anhydrous HF in the presence of a suitable cation scavenger, such as anisole or dimethoxy benzene. This method simultaneously removes all protecting groups, except a thioalkyl group protecting sulfur, and splits the peptide from the resin. Peptides hydrolyzed in this way from the CMR are carboxylic acids, those split from the BHA resin are obtained as carboxamides.

Modification of the terminal amino group of the peptide is accomplished by alkylation or acetylation as is generally known in the art. These modifications may be carried out upon the amino acid prior to incorporation into the peptide, or upon the peptide after it has been synthesized and the terminal amino group liberated, but before the protecting groups have been removed.

Typically, acetylation is carried out upon the free amino group using the acyl halide, anhydride or activated ester, of the corresponding alkyl acid, in the presence of a tertiary amine. Mono-alkylation is carried out most conviently by reductive alkylation of the amino group with an appropriate aliphatic aldehyde or ketone in the presence of a mild reducing agent, such as lithium or sodium cyanoborohydride. Dialkylation may be carried by treating the amino group with an excess of an alkyl halide in the presence of a base. The longer chain alkyl and acyl groups of this invention are believed to have advantageous properties due to their affinity for lipid membranes. Myristylation or stearylation, for example, is useful.

Solution synthesis of peptides is accomplished using conventional methods used to form amide bonds. Typically, a protected Boc-amino acid which has a free carboxyl group is coupled to a protected amino acid which has a free amino group using a suitable carbodiimide coupling agent, such as N, N′ dicyclohexyl carbodiimide (DCC), optionally in the presence of catalysts such as 1-hydroxybenzotriazole (HOBT) and dimethylamino pyridine (DMAP). Other methods, such as the formation of activated esters, anhydrides or acid halides, of the free carboxyl of a protected Boc-amino acid, and subsequent reaction with the free amine of a protected amino acid, optionally in the presence of a base, are also suitable. For example, a protected Boc-amino acid or peptide is treated in an anhydrous solvent, such as methylene chloride or tetrahydrofuran (THF), in the presence of a base, such as N-methyl morpholine, DMAP or a trialkyl amine, with isobutyl chloroformate to form the "activated anhydride", which is subsequently reacted with the free amine of a second protected amino acid or peptide. The peptide formed by these methods may be deprotected selectively, using conventional techniques, at the amino or carboxy terminus and coupled to other peptides or amino acids using similar techniques. After the peptide has been completed, the protecting groups may be removed as hereinbefore described, such as by hydrogenation in the presence of a palladium or platinum catalyst, treatment with sodium in liquid ammonia, hydrofluoric acid or alkali.

Esters are often used to protect the terminal carboxyl group of peptides in solution synthesis. They may be converted to carboxylic acids by treatment with an alkali metal hydroxide or carbonate, such as potassium hydroxide or sodium carbonate, in an aqueous alcoholic solution. The acids may be converted to other esters via an activated acyl intermediate as previously described.

The amides and substituted amides of this invention are prepared from carboxylic acids of the peptides in much the same manner. Thus, ammonia or a substituted amine may be reacted with an activated acyl intermediate to produce the amide. Use of coupling reagents, such as DCC, is convenient for forming substituted amides from the carboxylic acid itself and a suitable amine.

In addition, the methyl esters of this invention may be converted to the amides, or substituted-amides, directly by treatment with ammonia, or a substituted amine, in methanol solution. A methanol solution of the methyl ester of the peptide is saturated with ammonia and stirred in a pressurized reactor to yield the simple carboxamide of the peptides. Carboxamides are preferred embodiments of this invention due their enhanced stability relative to esters.

The amino acid which constitutes the M residue of the peptide comprises either phenylalanine, a derivative thereof, or an unnatural amino acid denoted as $-NHCHR_1R_2-$. The derivatives of phenylalanine include tyrosine; (O-$C_{1-5}$alkyl)tyrosine, 4′-halophenylalanine, 4′-nitrophenylalanine and 4′-aminophenylalanine as are generally well known in the peptide art.

An intermediate useful in the synthesis of the peptides of the invention wherein M is $-NHCHR_1R_2$ is represented by the formula (IIa):

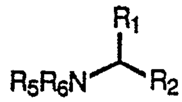

(IIa)

wherein:

$R_1$ is independently $C_{1-5}Alk$, $(CH_2)_nSC_{1-5}Alk$, $(CH_2)_nOC_{1-5}Alk$, $(CH_2)_nC_{3-7}$cycloalkyl or $(CH_2)_mC_6H_4\text{-}R_a$;

$R_a$ is halogen, $OR'$, $NO_2$, $NH_2$ or H;

$R_2$ is $(CH_2)_n$-, $CHR_3(CH_2)_mCO$-, $CO(CH_2)_mCO$-, $CHR_3CHR_3'(CH_2)_mCO$-, $CHR_3CHR_3'CHR_1(CH_2)_mCO$-, $CHR_3CHR_1CHR'(CH_2)_mC0$-, $CHR_3CHR'CHR_1(CH_2)_mCO$-, $COCHR_1CHR'(CH_2)_mCO$- $COCF_2CR'R''(CH_2)_mCO$-, $COCF_2(CH_2)_mCO$-, $CH=CR'CHR''(CH_2)_mCO$-, $COCH=CR'(CH_2)_mCO$-, $CH_2S(O)_pCHR'(CH_2)_mCO$-, $CH_2NR'CHR''(CH_2)_mCO$-, $P=O(OR')(CH_2)_mCO$-,

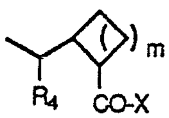 , 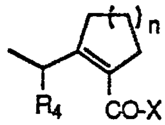 , 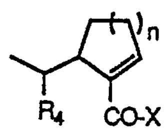 , 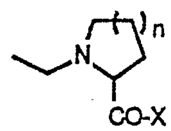 , 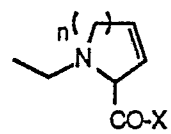 ,

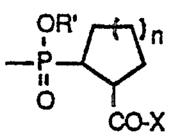 or 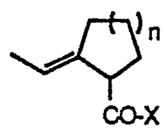 ;

$R_3$ and $R3'$ are each independently OH, H or $NH_2$; $R_4$ is OH, H, $NH_2$ or =0;

m is independently 0, 1, 2 or 3;

n is independently 1 or 2;

p is 0, 1 or 2;

$R_5$ is H;

$R_6$ is $CH_3CO$, $C_{1-5}Alk$, Dns, Cbz or Boc, or taken together $R_5$ and $R_6$ are phthalimido; X is H, halogen, $OR''$, $OC_{1-5}Alk$, $NHR'R''$ or $OCOC_{1-5}Alk$;

$R'$ H or $C_{1-5}Alk$; and

$R''$ is H or $C_{1-18}Alk$.

These unnatural amino acids have utility in themselves as intermediates which may be used to prepare protease inhibitors. Accordingly, a peptide substrate which binds to a protease may be converted to an inhibitor by replacement of one or both of the amino acids at the scissile site by a suitable compound of formula (IIa).

The unnatural amino acids are generally not α-amino acids, but are derived from α-amino acids. Thus, the identity of $R_1$ is established by the choice of an amino acid of formula (III), wherein $R_1$ is chosen as previously set forth.

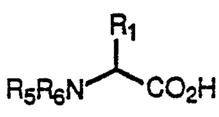

(III)

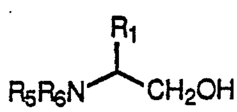

(IV)

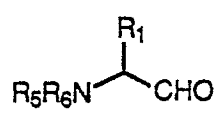

(V)

Protection of the amino group of the α-amino acid, such as by the Boc, acetyl or phthaloyl group, and conversion of the acid or ester to an alcohol of formula (IV) or an aldehyde of formula (V) thereby provides a versatile intermediate for the introduction of the $R_2$ substituent, and the preparation compounds of this invention which are designated by formula (IIa). The N-methoxy-methyl amides of these α-amino acids are also useful intermediates for this use. The α-amino acids may be obtained from natural sources, commercial sources or may be synthesized by a number of methods well known in the peptide art.

Alcohols of formula (IV) may be obtained from the diborane reduction of the corresponding protected

amino acid. Aldehydes of formula (V) may be obtained from the reduction of the methyl ester of the corresponding amino acid, for example with diisobutyl aluminum hydride (DIBAL). Alternatively the alcohol of formula (IV) may be oxidized to the aldehyde using a mild oxidizing agent, such as the Dess Martin periodinane or using the Swern method (DMSO, oxalyl chloride, triethylamine).

For instances in which $R_1$ is Alk, the amino acids alanine, valine, leucine and isoleucine are especially useful for producing chiral aldehydes. α-Amino butyric acid, α-amino isobutyric acid and α-amino pentanoic acid are commercially available as racemates. In general, any alkyl-α-amino acid can be synthesized by alkylation of a nitroacetic acid ester with an appropriate alkyl halide in the presence of a base, such as triethylamine, DBU or other tertiary amine. Alkoxides, such as sodium methoxide or ethoxide are also suitable bases. Subsequent reduction of the α-nitro alkyl acid or ester with hydrogen and a palladium or platinum catalyst yields the amino acid. Alternately, it is common to produce α-amino acids by alkylation of an N-benzylidine glycine ester, such as a methyl or ethyl ester. Thus treatment of the benzylidine with a strong base, such as sodium or potassium hydride or lithium diisopropylamide, followed by alkylation with an appropriate alkyl chloride and subsequent acid hydrolysis of the benzylidene group yields the desired amino acid. These methods are also adaptable to the formation of cycloalkyl amino acids, for instance, by use of the cycloalkyl methyl bromide or the 2-cycloalkyl ethyl bromide. Other methods for synthesis of chiral amino acids have been disclosed by Evans, et al., Tet. Let., 1123(1987).

Most amino acids in which $R_1$ is aryl or aralkyl are available commercially, or are well known in the art such as phenylalanine, 4′-halo and 4′-nitrophenylalanine, tyrosine, (O-alkyl)tyrosine, phenylglycine and (4′-hydroxy)phenylglycine. Homologs of these compounds may be prepared by conventional techniques of amino acid synthesis. Thus, alkylation of a nitro acetic acid ester or a N-benzylidene glycine with 1-phenyl-2-bromoethane or 1-phenyl-3-bromopropane, as described above, produces the homologous amino acids.

The α-amino acids in which $R_1$ contains an oxygen or sulfur are obtained from serine, homo-serine, threonine, cysteine or methionine or alkylation or these amino acids, excepting methionine. Alkylation is carried out by treating the corresponding hydroxyl or thiol compound with a base, such as triethylamine, DBU or sodium hydride, and an appropriate alkyl halide, such as methyl, ethyl, isopropyl or isobutylbromide. The di-homoanalogues are prepared by conventional techniques for producing amino acids as described above. Accordingly, an ester of nitro acetic acid is treated with a base, as previously described and acrolein in a Michael reaction. The aldehyde produced is subsequently reduced by a mild reducing agent, such as sodium borohydride, to yield the desired hydroxy group. Subsequent catalytic reduction of the nitro group yields the α-amino 5-hydroxy pentanoic acid. The corresponding mercapto analogue may be synthesized from the hydroxy intermediate. Thus, after protection of the amino and carboxyl groups as known in the art, the hydroxyl group is converted to a halide, such as by treatment with phosphorous tribromide or oxalyl bromide or chloride in the presence of triethylamine or another tertiary amine, or by treatment with triphenyl phosphine and carbon tetrabromide. Treatment of the halide with sodium sulfide or potassium thioacetate followed by saponification with sodium hydroxide, yields the 5-mercapto-2-amino pentanoic acid. This intermediate may be further alkylated in the same manner as cysteine or serine described herein above.

As previously indicated the α-amino acids hereinbefore described may be converted to aldehydes and alcohols of formula (IV) and (V). Subsequent elaboration of these intermediates to introduce the substituent $R_2$, and thereby form the compounds of this invention designated in formula (IIa), is dependent upon the nature of $R_2$.

Introduction of a hydrocarbon substituent at $R_2$ is suitably accomplished by reaction of the aldehyde of formula (V) with an organometallic hydrocarbon, such as a Grignard reagent or organo-lithium species. The incipient carboxylic acid functionality of $R_2$ is in protected form as in an ortho ester or disguised, as in an oxidizable olefin. In one embodiment, the organometallic species is a magnesium or lithium alkylide generated from lithium or magnesium and an alkylene halide, such as allyl bromide, 1-bromo-3-butene, 1-bromo-4-pentene, or 1-bromo-5-hexene, or an alkyl substituted derivative thereof.

Addition of the organometallic reagent to an aldehyde of formula (IV) yields a hydroxy-alkene of formula (VIa) or (VIb), wherein $R_0$ is OH, $R_1'$ is H, $C_{1-5}$Alk, $(CH_2)_nOC_{1-5}$Alk, $(CH_2)_nC_{3-7}$cycloalkyl or $(CH_2)_mC_6H_4$-$R_a$ and $R_1$, $R_5$, $R_6$, $R'$, $R''$, m and n are as defined for formula (IIa).

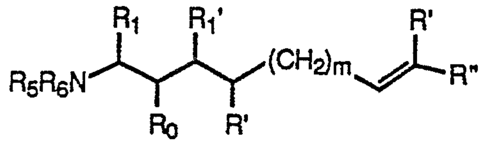

(VIa)

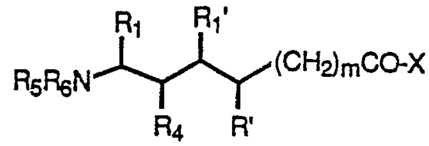

(VIIa)

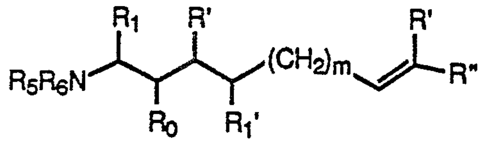

(VIb)

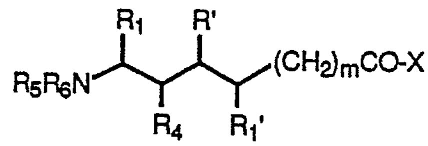

(VIIb)

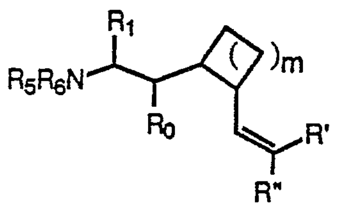

(VIc)

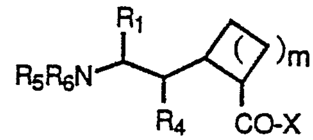

(VIIc)

Alternatively, addition of an organometallic alkylidene, such as a Grignard reagent, to the N-methoxy methyl amide of an amino acid of formula (III) yields the keto-alkenes of formula (VIa) and (VIb), wherein $R_0$ is =O. Using the same procedures, except substituting a 1-metallo-2-propenyl cyclopentane or 1-metallo-2-propenyl cyclohexane, the corresponding cycloalkyl alkenes of formula (VIc), wherein $R_1$, $R_5$, $R_6$, $R'$, $R''$ m and n are as defined in formula (IIa), are prepared. Accordingly, a process for preparing a compound of formula (VIIa), (VIIb) or (VIIc), wherein $R_1'$ is H, $C_{1-5}Alk$, $(CH_2)_nOC_{1-5}Alk$, $(CH_2)_nC_{3-7}$cycloalkyl or $(CH_2)_mC_6H_4$-$R_a$ and $R_1$, $R_4$, $R_5$, $R_6$, $R'$, $R''$, X, m and n are as defined in formula (IIa), comprises oxidizing a compound of formula (VIa), (VIb) or (VIc) to a keto-carboxylic acid (VIIa), (VIIb) or (VIIc), wherein $R_4$ is =O and X is OH, and thereafter in any order optionally reducing or reductively aminating the keto group, and optionally converting the carboxylic acid to an ester, amide, aldehyde, anhydride or acyl halide. Suitably $R'$ is H and m is 0, and in a preferred embodiment of compound (VIIa), $R_1'$ is H.

Oxidation of the hydroxy- or keto-alkane is accomplished by conventional methods, such as with potassium permanganate or by ozonolysis followed by standard dimethyl sulfide workup and subsequent oxidation with potassium permanganate or Jones reagent. As would be obvious to one skilled in the art, this method is inapplicable when $R_1$ contains a sulfur substituent. Other methods adaptable to prepare certain compounds of this type are disclosed by Holladay et al.,J. Med. Chem., 30, 374-83(1987) and Kempf, D., J. Org. Chem , 51, 21, 3921-26(1986).

Reduction of the keto-compounds of formula (VIIa), (VIIb) and (VIIc) ($R_0$ is =O) using a conventional reducing agent, such as sodium borohydride, yields the corresponding alcohol. If the alcohol is desired, it is often useful to protect it, such as by formation of a benzyl or silyl ether, or an acetate to prevent unwanted side reactions, such as lactonization, and to improve the efficiency of later incorporation into the peptides of this invention. Such protection of the alcohol is conveniently carried out prior or subsequent to the oxidation steps leading to the carboxylic acid. The hydroxyl may be further converted to chloride or bromide using a halogenating agent, such as phosphorous trichloride or phosphorous tribromide, and subsequently reduced to a methylene group with hydrogen and Raney® nickel. If an amino group is desired, the keto group may be reductively aminated with sodium cyanoborohydride and an ammonium halide, such as ammonium chloride or bromide. The carboxylic acid may be converted to an ester, amide, aldehyde, anhydride or acyl halide by common methods well known in the chemical art.

The introduction of unsaturation at $R_2$ may be derived from the amino acid products corresponding to formulas (VII). Reduction of the carbonyl group to a hydroxyl group, as previously indicated, and subsequent elimination of the hydroxyl group, provides the unsaturated compounds of this invention. The hydroxyl group may be eliminated by conversion to a mesylate or halide, as previously provided, and treatment with a base such as DBU or lithium diisopropylamide. Other methods of elimination of the hydroxyl group, such as treatment with warm mineral acid (HCl, $H_2SO_4$) may also be suitable. Another procedure for introduction of unsaturation at $R_2$ involves Wittig olefination of an aldehyde of formula (V). Reduction of these unsaturated compounds with hydrogen and a palladium catalyst provides an alternative

overall method for effecting removal of the ketone group in the compounds of formulas (VII) ($R_0$ is =O).

Introduction of a nitrogen substituent at $R_2$ is accomplished by reaction of an amino aldehyde of formula (V), under reducing conditions with an ester of a $C_{1-4}$amino alkanoic acid, or alkyl amino $C_{1-4}$alkanoic acid or a structure where the amino moeity is encompassed in a saturated or unsaturated 5 or 6 membered ring. Examples of such amines are the benzyl or methyl esters of glycine, alanine, 3-amino propionic acid, 4-amino-pentanoic acid butanoic acid, proline, Δ3-dehydro proline, and 2-carboxy piperidine. Suitable reducing conditions would include the use of hydrogen and a palladium or platinum catalyst, or sodium cyanoborohydride. Accordingly, compounds of formula (VIII):

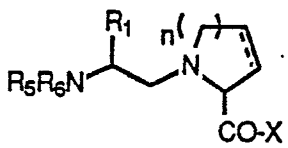

(VIII)

wherein $R_1$, $R_5$, $R_6$, X and n are as definded in formula (IIa), are prepared by a process which comprises reacting a compound of formula (V), under reducing conditions, with an ester or acid of proline, Δ3-dehydro-proline or 2-carboxypiperidine, and thereafter, in any order, converting the ester to a carboxylic acid, ester, aldehyde or amide; or converting the acid to an ester, amide, anhydride or acyl halide.

Introduction of a sulfur substituent at $R_2$ is accomplished by reaction of a thioalkanoic acid or ester with an α-amino acid derivative. Accordingly, a hydroxy-amino acid of formula (IV) is converted to the corresponding halide using a conventional reagent, such as phosphorous tribromide, phosphorous oxychloride or thionyl chloride in the presence of a base, such as triethylamine or pyridine, and treated with the thioalkanoic acid or ester again in the presence of a base. The sulfide produced in this way may be further oxidized to a sulfoxide by treatment with a mild oxidizing agent, such as sodium metaperiodate, or a sulfone, by treatment with potassium permanganate.

Introduction of a fluorocarbon substituent at $R_2$, wherein the fluorine is γ to the amino group, is accomplished by treatment of an aldehyde of formula (V) with ethyl difluorobromoacetate and activated zinc dust. Thaisrivongs, et al., J. Med. Chem., 29, 2080-87 (1986) disclose such chemistry for difluorostatine-type compounds. Homologues of these compounds may be prepared from α-keto diesters, such as dimethyl oxaloacetate.

Illustrative of the method, dimethyl oxaloacetate is treated with diethylamino sulfur trifluoride (DAST) to produce α-difluoro dimethyl succinate. Preferential reduction of the α-difluoro ester functionality with sodium borohydride yields the difluoro alcohol, which may be oxidized to the corresponding aldehyde using a mild oxidizing method such as Collins reagent, the Dess-Martin periodinane or the Swern method (DMSO, oxalyl chloride, triethylamine). Condensation of the α-difluoro aldehyde with a nitro compound of the formula $R_1CH_2NO_2$, such as 2-nitrophenyl ethane or a 1-nitro $C_{1-5}$alkane, in the presence of a base, such as DBU or $K_2CO_3$, and subsequent reduction of the nitro group with hydrogen and either a 5% Pd/C or platinum black catalyst yields the desired difluoro homologues. Alkylated derivatives of these homologues may be prepared by alkylation of the initial α-keto diester with a base and alkyl halide prior to conducting the sequence. For example, alkylation of dimethyl oxalacetate with 2 equivalents of methyl iodide in the presence of DBU yields 2-keto-3,3-dimethyl succinnate. Further conversion by the above sequence to 2,2 dimethyl-3,3-difluoro-4-oxo-methyl butanoate, condensation with 2-phenyl nitroethane and reduction with hydrogen and 5% Pd/C yields 2,2 dimethyl-3,3-difluoro-4-hydroxy-5-amino-6-phenyl methyl hexanoate. Further oxidation of these compounds to provide the fluoroketone may be carried out at this stage, but it is often more suitably carried out after the residue has been incorporated into the peptide. Moffatt oxidation (DMSO, acetic anhydride), Dess-Martin periodinane or pyridine/chromium trioxide are suitable oxidants.

Introduction of a phosphinyl substituent at $R_2$ is carried out by a condensation of benzyl carbamate with triphenyl phosphite and an aldehyde of the general formula $R_1CHO$, such as phenylacetaldehyde, isobutyraldehyde or 2-methyl butyraldehyde, in acetic acid to yield a phosphonate of formula (IX).

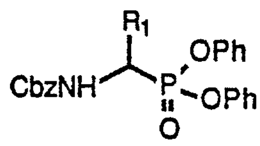

(IX)

Further treatment of the phosphonate with an alkoxide provides the corresponding phosphonic acid monoalkyl ester. Thus, treatment with sodium methoxide yields the phosphonic acid monomethyl ester. Conversion of this phosphonic acid to a phosphonyl halide with a suitable halogenating reagent, such as thionyl chloride or bromide, provides a suitable reactive intermediate for further elaboration of the side chain. Treatment with an organo metallic reagent, such as the lithium or Grignard reagent derived from alkyl bromide, bromo-3-butene, 2-(2-methylpropenyl)chlorocyclopentane or 2-(2-methyl propenyl)-cyclohexane, yields the alkenyl methyl phosphinates of this invention as given by formula (Xa) and (Xb):

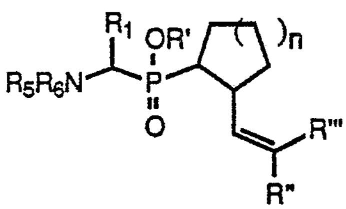

(Xa)

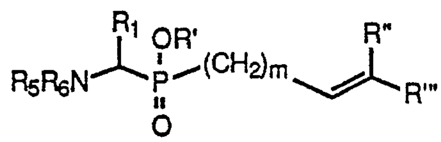

(Xb)

wherein $R_1$, $R_5$, $R_6$, $R'$, $R''$, m and n are as defined for formula (IIa) and $R'''$ is $C_{1-5}$ alkyl. Subsequent oxidation, such as with potassium permanganate or by ozonolysis as herein described affords the corresponding alkyl phosphinate carboxylic acids of this invention. Accordingly, the compounds of formulas (XI):

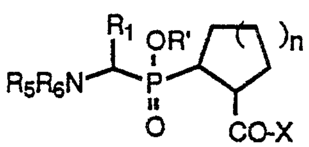

(XIa)

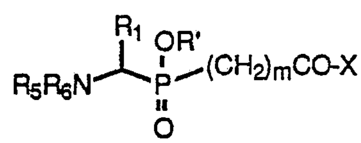

(XIb)

wherein $R_1$, $R_5$, $R_6$, $R'$, X and n are as defined as in formula (IIa), may be prepared by oxidizing a compound of formula (Xa) or (Xb) to a carboxylic acid, optionally hydrolyzing the alkyl phosphinate to the phosphinic acid, and optionally converting the carboxylic acid to an ester, amide, aldehyde, anhydride or acyl halide. Hydrolysis of the alkyl phosphinate to the phosphinic acid may be accomplished by treatment with a strong acid, such as hydrogen bromide. As would be obvious to one skilled in the art, this method is inapplicable when $R_1$ contains a sulfur substituent. Methods adaptable for synthesis of certain of these compounds are reported for instance by Giannousis et al., J. Med. Chem., 30, 1603-09(1987).

A further aspect of this invention relates to the preparation of completely novel amino acids which, when properly incorporated into peptides, provide a non-hydrolyzable imitation of a peptide bond. These compounds are prepared as hereinbefore described and are further illustrated in the Examples which follow. They are incorporated into the peptides of this invention by methods known to the art and are given by formula (IIb) as:

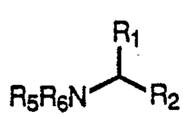

(IIb)

wherein:

$R_1$ is independently $C_{1-5}Alk$, $-(CH_2)_nSC_{1-5}Alk$, $-(CH_2)_nOC_{1-5}Alk$, $-(CH_2)_nC_{3-7}cycloalkyl$ or $-(CH_2)_mC_6H_4-R_a$;

$R_a$ is halogen, $NO_2$, OH, $OC_{1-5}Alk$, $NH_2$, or H;

$R_2$ is $(CH_2)_n$-X, $(CH_2)_nCO$-X, $CHR_3CHR_1CHR'(CH_2)_mCO$-, $COCR_1CHR'(CH_2)_mCO$-X, $CHR_3CF_2(CH_2)_mCO$-X, $COCF_2CR'R''(CH_2)_mCO$-X, $CHR_3(CH_2)_mCO$-X, $CO(CH_2)_mCO$-X,

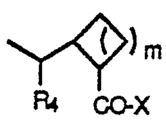 , 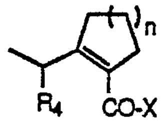 , 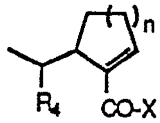 , 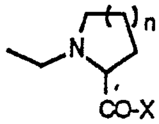 , 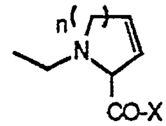 ,

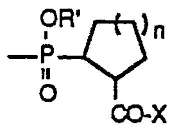 or 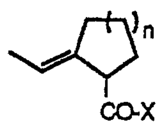 ;

$R_3$ is H, $NH_2$ or OH;

$R_4$ is H, $NH_2$, OH, or =O;

$R_5$ is H;

$R_6$ is $CH_3CO$, $C_{1-5}Alk$, Dns, Cbz or Boc, or taken together $R_5$ and $R_6$ are phthalimido;

X is H, halogen, OH, $OC_{1-5}Alk$, $NHR'R''$ or $OCOC_{1-5}Alk$;

$R'$ and $R''$ are H or $C_{1-5}Alk$;

m is independently 0, 1, 2 or 3; and

n is independently 1 or 2;

provided that $R_2$ is not $CHR_3CH_2CO$-X, $COCH_2CO$-X, $COCF_2CO$-X or $CHR_3CF_2CO$-X when $R_1$ is phenylmethylene, isobutyl or cyclohexylmethylene, and $R_2$ is not $CH(OH)CH_2CH_2CO$-X or $COCH_2CH_2CO$-X, when R1 is isobutyl.

Incorporating a compound of formula (IIb) into a peptide in the place of one or two amino acid residues which undergo cleavage by a protease, especially a retroviral protease, provides a method for producing protease inhibiting activity. It will be appreciated that the above synthetic methods introduce chiral centers in a non-selective, or racemic, manner. As is often the case in biological systems, a single configuration of chiral centers is usually optimal. In many of the compounds, there are more than one chiral center, which renders compounds with varying chiral centers diastereomeric. These diasteromers are usually separable by chromatography over silica gel or an octadecyl silane chromatographic support with a suitable mobile phase. If silica gel is used, a mixture of ethyl acetate and hexane or methanol and chloroform or methylene chloride are used to elute the diasteromers. If an octadecyl silane support is used, a mixture of water and methanol or acetonitrile may be used to purify the diastereomers. In the case of acidic or basic compounds, the mobile phase may be buffered by the addition of .02 to .5 % of acetic acid, trifluoracetic acid or phosphate buffer. If the residue, designated as M, possesses only a single chiral center, or the diasteromers are difficult to separate, it may be preferable to incorporate the residue into the peptide, wherein the chiral centers of the other constituent amino acids confer further asymetry, and render the resulting diasteromers separable at a later stage. However, while it may sometimes be preferable to separate diasteromers, it is by no means necessary to separate the chiral centers to practice this invention.

The unnatural amino acids of the M residue are incorporated into the peptides of this invention as hereinbefore described by methods common in the peptide art.

If the final peptide, after it has been deprotected, contains a basic group, an acid addition salt may be prepared. Acid addition salts of the peptides are prepared in a standard manner in a suitable solvent from the parent compound and an excess of an acid, such as hydrochloric, hydrobromic, sulfuric, phosphoric, acetic, maleic, succinic or methanesulfonic. The acetate salt form is especially useful. If the final peptide contains an acidic group, cationic salts may be prepared. Typically the parent compound is treated with an excess of an alkaline reagent, such as a hydroxide, carbonate or alkoxide, containing the appropriate cation. Cations such as $Na^+$, $K^+$, $Ca^{++}$ and $NH_4^+$ are examples of cations present in pharmaceutically acceptable salts. Certain of the compounds form inner salts or zwitterions which may also be acceptable.

The compounds of formula (I), wherein M is $-HNCHR_1R_2-$, are used to induce anti-viral activity in patients which are infected with susceptible viruses and require such treatment. The method of treatment comprises the administration orally, parenterally, buccally, trans-dermally, rectally or by insufflation, of an

effective quantity of the chosen compound, preferably dispersed in a pharmaceutical carrier. Dosage units of the active ingredient are selected from the range of .05 to 15 mg/kg. These dosage units may be administered one to ten times daily for acute or chronic infection.

The protease inhibiting properties of the peptides of this invention, wherein M is $-HNCHR_1R_2-$, are demonstrated by their ability to inhibit the hydrolysis of a peptide substrate by rHIV protease in the range of about 10 nM to about 250 µM. The following table is representative of the inhibition constants of these peptides.

Table II

Inhibition of rHIV Protease

| Compound of Example No. | $K_i$ (µM) |
|---|---|
| 15 | 14 |
| 16 | 1 |
| 17 | 68 |
| 18 | 6.7 |
| 19 | 7 |
| 20 | 32 |
| 21 | 160 |
| 22 | 47 |
| 23 | 27 |
| 24 | 6 |
| 25 | 40 |
| 26 | 60 |

Table II (Continued)
Inhibition of rHIV Protease

| Compound of Example No. | $K_i$ (μM) |
|---|---|
| 27 | .26 |
| 28 | .026 |
| 29 | ≥28 |
| 30 | 14 |
| 31 | 1.2 |
| 32 | 220 |
| 33 | 3.6 |
| 34 | .140 |
| 35 | .110 |
| 36 | 10 |
| 37 | 3.5 |
| 38 | .15 |

Pharmaceutical compositions of the peptides of this invention, or derivatives thereof, may be formulated as solutions or lyophilized powders for parenteral administration. Powders may be reconstituted by addition of a suitable diluent or other pharmaceutically acceptable carrier prior to use. The liquid formulation is generally a buffered, isotonic, aqueous solution. Examples of suitable diluents are normal isotonic saline solution, standard 5% dextrose in water or buffered sodium or ammonium acetate solution. Such formulation is especially suitable for parenteral administration, but may also be used for oral administration or contained in a metered dose inhaler or nebulizer for insufflation. It may be desirable to add excipients such as polyvinylpyrrolidone, gelatin, hydroxy cellulose, acacia, polyethylene glycol, mannitol, sodium chloride or sodium citrate.

A preferred composition for parenteral administration may additionally be comprised of a quantity of the compound encapsulated in a liposomal carrier. The liposome may be formed by dispersion of the peptides in an aqueous phase with phospholipids, with or without cholesterol, using a variety of techniques, including conventional handshaking, high pressure extrusion, reverse phase evaporation and microfluidization. A suitable method of making such compositions is more fully disclosed in copending Application Serial No. 06/763,484 and is incorporated herein by reference. Such a carrier may be optionally directed toward its site of action by an immunoglobulin or protein reactive with the viral particle or infected cells. The choice of such proteins would of course be dependent upon the antigenic determinants of the infecting virus. An example of such a protein is the CD-4 T-cell glycoprotein, or a derivative thereof, such as sCD-4 (soluble CD-4), which is reactive with the glycoprotein coat of the human immunodeficiency virus (HIV). Such proteins are disclosed in copending Application Serial No. 07/160,463, which is incorporated herein by reference. Similar targeting proteins could be devised, by methods known to the art, for other viruses and are considered within the scope of this invention.

Alternately, these peptides may be encapsulated, tableted or prepared in a emulsion or syrup for oral administration. Pharmaceutically acceptable solid or liquid carriers may be added to enhance or stabilize the composition, or to facilitate preparation of the composition. Liquid carriers include syrup, peanut oil, olive oil, glycerin, saline and water. Solid carriers include starch, lactose, calcium sulfate dihydrate, terra alba, magnesium stearate or stearic acid, talc, pectin, acacia, agar or gelatin. The carrier may also include a sustained release material such as glyceryl monostearate or glyceryl distearate, alone or with a wax. The amount of solid carrier varies but, preferably, will be between about 20 mg to about 1 g per dosage unit. The pharmaceutical preparations are made following the conventional techniques of pharmacy involving milling, mixing, granulating, and compressing, when necessary, for tablet forms; or milling, mixing and filling

for hard gelatin capsule forms. When a liquid carrier is used, the preparation will be in the form of a syrup, elixir, emulsion or an aqueous or non-aqueous suspension. Such a liquid formulation may be administered directly p.o. or filled into a soft gelatin capsule.

For rectal administration, a pulverized powder of the peptides of this invention may be combined with excipients such as cocoa butter, glycerin, gelatin or polyethylene glycols and molded into a suppository. The pulverized powders may also be compounded with an oily preparation, gel, cream or emulsion, buffered or unbuffered, and administered through a transdermal patch.

Suitably, a pharmaceutical composition comprises a peptide of this invention, azidothymidine and a pharmaceutically acceptable carrier.

The Examples which follow serve to illustrate this invention. The Examples are intended to in no way limit the scope of this invention, but are provided to show how to make and use the compounds of this invention.

In the Examples, all temperatures are in degrees Centigrade. Amino acid analyses were performed upon a Dionex Autoion 100. Analysis for peptide content is based upon Amino Acid Analysis. FAB mass spectra were performed upon a VG Zab mass spectrometer using fast atom bombardment. The abbreviations used to represent the eluent composition for thin layer chromatography and counter current distribution are B: n-butanol, A: acetic acid, W: water, E: ethyl acetate and IP: isopropanol. NMR were recorded at 250 MHz using a Bruker AM 250 spectrometer. Multiplicities indicated are: s = singlet, d = doublet, t = triplet, q = quartet, m = multiplet and br indicates a broad signal. 4(S)-Amino-3(R)-hydroxy-5-phenyl hexanoic acid [AHPPA] and its 3(R) epimer [3-R-AHPPA] were prepared by the method of Rich, et al., J. Med. Chem., 23, 27-33 (1980). The racemate [3-R,S-AHPPA] was obtained by omitting the final chromatography in this method.

## Purification of Recombinant HIV Protease

Methods for expressing recombinant HIV protease in E. coli have been described by Debouck, et al., Proc. Natl. Acad. Sci. USA, 84, 8903-6(1987). The enzyme used to assay the peptide of this invention was produced in this manner and purified from the cell pellet as follows. The E. coli cell pellet was resuspended in a buffer consisting of 50 mM Tris-HCl, pH 7.5; 1.0 mM each DTT, EDTA and PMSF (phenylmethylsulfonyl fluoride). The cells were lysed by sonication and insoluble material was removed by centrifugation at 15,000 x g av, for 15 min. The clarified supernatant was then brought to 40% of saturation with ammonium sulfate This suspension was stirred at room temperature for 30 min. and then centrifuged as above. The resulting precipitate was redissolved/resuspended in a minimal volume of 20 mM Tris-HCl, pH 7.5; 200 mM NaCl; 0.1 mM each DTT and EDTA. The sample was centrifuged again before application (in 5 ml aliquots) to a Beckman TSK G2000SW preparative HPLC gel filtration column (2.1 cm x 60 cm.). The column was equilibrated in the same buffer at a flow rate of 4 ml/min. The effluent of the column was monitored at 280 nm and 1 min. fractions collected. Typically, the rHIVPRT (recombinant HIV protease) eluted 45-46 min. into the run. At this stage, the protease was N85-95% pure. By immunoblot analysis >90% of the immunoreactive material was precipitated at the ammonium sulfate step. By activity assay, the highest peak of activity was found in the fractions collected at 45 and 46 minutes. Analysis of the TSK column fractions by RP-HPLC and SDS-PAGE indicated that the majority of the 11,000 Mr protein is also found in fractions 45 and 46. The activity itself cannot be used to obtain reliable recovery data as it is influenced by high salt, i.e., with increasing salt, increasing levels of activity were obtained. Thus, with each step in the purification, more total activity was recovered than was started with. The overall yield of rHIVPRT was N1 mg from a 50 gm E. coli cell pellet.

## Substrate Kinetics for HIV Protease Activity

Initial rate data for oligopeptides were determined at pH 6.0, 37° C. Reaction mixtures (0.01-0.1 mL) contained 50 mM Mes (2-N-morpholino)ethanesulfonic acid), 1 mM EDTA, 1 mM DTT, 0.2 M NaCl, 0.1% (v/v) Triton X-100 (pH 6.0), 10% DMSO, and variable concentrations of the peptides. After incubation at 37° C for several minutes, reaction was initiated by the addition of purified HIV protease (0.01-2 mg), and reactions were quenched after 10-30 min. with an equal volume of either ice-cold 0.6 N tricholoroacetic acid or 0.2% trifluoroacetic acid. Samples were centrifuged at 17,000 rpm for 5 min., and the peptide substrates

and products were separated and quantified (by peak integration) by reverse phase HPLC. Initial rates were determined as (mM peptide product formed)/min based on the percentage of conversion of substrate to product for a given concentration of substrate. Typically, reaction rates were linear over the time course of the reaction, and less than 15-20% of the substrate had been converted to product at reaction endpoint. Kinetic constants (Michaelis constants, maximal velocities) were determined by fitting initial rate data to the Michaelis-Menten equation ($v = V_{max}(S)/(K_m + (S))$) using the Fortran program of Cleland (W. W. Cleland, Adv. Enzymol. 29, 1, (1967)). The turnover number ($k_{cat}$) was obtained as $k_{cat} = V_{max}/E_t$, where it is assumed that one active site is present per 22-kD dimer of HIV protease.

An estimate of substrate viability was obtained by determination of a relative initial velocity (rel $V_o$). This assay was run in the same manner, except the percent hydrolysis of substrate after 10-20 min. was compared to the percent hydrolysis of Ac-Arg-Ala-Ser-Gln-Asn-Tyr-Pro-Val-Val-$NH_2$ (control) after a similar incubation. This established a comparison of initial reaction velocity at one time point relative to the control peptide.

## Inhibition of HIV protease activity

A typical assay contained 10 mL MENDT buffer (50 mM Mes (pH 6.0; 2-(N-morpholino)ethanesulfonic acid), 1 mM EDTA, 1 mM dithiothreitol, 200 mM NaCl, 0.1% Triton X-100); 2, 3, or 6 mM N-acetyl-L-arginyl-L-alanyl-L-seryl-L-glutaminyl-L-asparaginyl-L-tyrosyl-L-prolyl-L-valyl-L-valinamide (Ac-Arg-Ala-Ser-Gln-Asn-Tyr-Pro-Val-Val-$NH_2$; $K_m = 7$ mM); and micromolar and sub-micromolar concentrations of synthetic compounds. Following incubation at 37° C for several minutes, the reaction was initiated with purified 0.01-1 mg HIV protease. Reaction mixtures (37° C) were quenched after 10-20 minutes with an equal volume of cold 0.6 N trichloroacetic acid, and, following centrifugation to remove precipitated material, peptidolysis products were analyzed by reverse phase HPLC (Beckman Ultrasphere ODS, 4.5 mm x 25 mm; mobile phase: 5-20% acetonitrile/$H_2O$ - .1% TFA (15 min), 20% acetonitrile/$H_2O$ -.1% TFA (5 min) at 1.5 mL/min, detection at 220 nm. The elution positions of Ac-Arg-Ala-Ser-Gln-Asn-Tyr-Pro-Val-Val-$NH_2$ (17-18 min) and Ac-Arg-Ala-Ser-Gln-Asn-Tyr (10-11 min) were confirmed with authentic material. Initial rates of Ac-Arg-Ala-Ser-Gln-Asn-Tyr formation were determined from integration of these peaks, and typically, the inhibitory properties of the synthetic compounds were determined from slope/intercept analysis of a plot of 1/v vs. [inhibitor] (Dixon analysis). $K_i$ values resulting from this type of primary analysis are accurate for competitive inhibitors only, and under conditions in which the Michaelis constant of the substrate used is well-determined.

## Example 1

### General procedure for solid phase peptide synthesis

Peptide amides are synthesized by solid phase peptide synthesis using benzhydrylamine resin as the support. Protected amino acids are added sequentially starting from the carboxyl terminus until the desired sequence has been obtained. The t-butyloxycarbonyl (Boc) group is used for protection of the alpha-amino group. Side chain functional groups are protected as follows: arginine and histidine, tosyl (Tos); cysteine, p-methylbenzyl (MeBzl); serine and threonine, benzyl ether (Bzl); lysine, p-chlorocarbobenzoxy (Clz); glutamic acid and aspartic acid, benzyl ester (OBzl); tyrosine, p-bromocarbobenzoxy (BrZ). Removal of the Boc group is accomplished by treatment with 50% trifluoroacetic acid (TFA) in methylene chloride. Neutralization of the amine-TFA salt is accomplished by treatment with 7% diisopropylethylamine (DIEA) in methylene chloride. Amino acids are coupled to the growing peptide using 3 equiv. Boc-amino acid and 3 equiv. 1-hydroxybenzotriazole (HOBt) in DMF and 3 equiv. of dicyclohexylcarbodiimide (DCC) in methylene chloride. Completeness of coupling is checked by ninhydrin test and couplings repeated as necessary. The general protocol is given below.

| | | |
|---|---|---|
| 1. | Wash with $CH_2Cl_2$ | 1 x 1 min. |
| 2. | Wash with 50% TFA | 1 x 1 min. |
| 3. | Deblock with 50% TFA | 1 x 20 min. |
| 4. | Wash with $CH_2Cl_2$ | 6 x 1 min. |
| 5. | Neutralize with 7% DIEA | 3 x 2 min. |
| 6. | Wash with $CH_2Cl_2$ | 4 x 1 min. |
| 7. | Wash with DMF | 2 x 1 min. |
| 8. | Boc-AA + HOBt in DMF | do not drain |
| 9. | DCC in $CH_2Cl_2$ | 2 hr. |
| 10. | Wash with DMF | 2 x 1 min. |
| 11. | Wash with $CH_2Cl_2$ | 3 x 1 min. |

For attachment of the first (C-terminal) residue to the BHA resin, the synthesis is begun at step 5. For all subsequent amino acids, the synthesis is begun at step 1.

Preparation of Ac-Aro-Ala-Ser-Gln-Asn-Tyr-Pro-Val-Val-$NH_2$

The protected nonapeptide resin Boc-Arg(Tos)-Ala-Ser(Bzl)-Gln-Asn-Tyr(BrZ)-Pro-Val-Val-BHA was prepared in the usual way. After removal of the N-terminal Boc group with 50% TFA in $CH_2Cl_2$ and neutralizing the resulting TFA salt with 7% DIEA in $CH_2Cl_2$, the resin-bound peptide was acetylated with acetic anhydride (2 ml) in $CH_2Cl_2$ (30 ml) for 30 minutes.

The peptide was cleaved from the resin with removal of the side chain protecting groups by treatment with anhydrous liquid HF (10 ml) in the presence of anisole (1 ml) at 0° C for 50 minutes. After removal of the HF under vacuum, the resin was washed with ethyl ether and air-dried. The resin was then extracted with 2 x 30 ml 1% HOAc/$H_2O$ followed by 2 x 30 ml 10% HOAc/$H_2O$. The combined extracts were lyophilized to yield 476 mg crude peptide.

The crude peptide was purified by countercurrent distribution using the system n-BuOH/HOAc/$H_2O$ 4:1:5. The appropriate fractions were pooled, evaporated to dryness and the residue lyophilized from 1% HOAc/$H_2O$ to yield 294 mg partially purified peptide. An aliquot of 100 mg partially purified peptide was further purified by gel filtration on a 2.6 x 70 cm G-15 Sephadex® column using 1% HOAc as eluant. The appropriate fractions were pooled and lyophilized to yield 89 mg of the purified title compound.

TLC (n-BuOH/HOAc/$H_2O$ 4:1:1) $R_f = 0.49$.

HPLC: (Hamilton PRP-1, $CH_3CN/H_2O$/0.1% TFA, 5% to 40% $CH_3CN$, 15 minutes, linear gradient, 1.5 ml/min.) $k' = 2.6$; FAB-MS: m/z 1074 ($M + H^+$).

## Example 2

Preparation of (2S)-N-(2-N-tert-butyloxycarbonyl)amino-3-phenyl)propyl-proline

N-tert-butyloxycarbonyl-L-phenylalanine methyl ester

a) L-phenylalanine methyl ester hydrochloride (10.0 g, 46.4 mmol) was dissolved in a mixture of triethylamine (12.9 mL, 92.8 mmol) and DMF (60 mL). N-t-butyl dicarbonate ( 16.0 mL, 69.6 mmol, 50% excess) was added over 5 min. at room temperature. After stirring for 16 h, the mixture was filtered and concentrated under reduced pressure. Flash chromatography over silica gel using 20% EtOAc in hexane gave 13.0 g of the titled compound.

TLC: (20% EtOAc in hexane) $R_f = 0.31$.

NMR ($CDCl_3$): $\delta$ 7.23 (m,5H), 5.16 (d,1H,J = 8 Hz), 4.57 (q,1H,J = 7 Hz), 3.66 (s,3H), 3.07 (d,2H,J = 6 Hz), 1.40 (s,9H).

a) Neat ethyl diazoacetate (53 ml, 0.50 mol) was added via a syringe drive over a 4 hour period to a stirred mixture of rhodium acetate dimer (1.0 g, 0.0023 mol) and cyclopentene (250 ml, 2.5 mol) immersed in a 20-25° C water bath. After an additional 30 minutes, excess cyclopentene was removed by rotary evaporation. The residue was diluted with $CH_2Cl_2$, filtered through Celite® and concentrated by rotary evaporation. The residue was distilled at 44-46° C/0.5 mmHg to provide the titled compound (46.5 g, 0.302 mol) as a mixture of carboethoxy epimers in 60% yield.
TLC: (80:20 Hexanes: ethyl acetate) $R_f = 0.67$.
NMR ($CDCl_3$): δ 4.1 (2H, overlapping quartets), 2.0-1.5 (7H, m), 1.45-1.1 (5H, m).

6-(1-hydroxy-1-methylethyl)bicyclo[3.1.0]hexane

b) A 1.4 M solution of methyl lithium in ether (350 ml, 0.50 mol) was stirred rapidly under Ar at -78° C while the product of Example 3(a) (27.67 g, 0.180 mol) was added neat via syringe over a 15 minute period. The mixture was stirred 1 hour at -78°, then was allowed to warm to room temperature over a 2 hour period. Water (400 ml) was added cautiously, followed by 3N HCl (ca. 200 ml) until the solution was neutral. The ether layer was separated and the aqueous layer was extracted twice with ether. The combined ether extracts were dried over $MgSO_4$ and concentrated by rotary evaporation. The residue was distilled (80°-90°/15 mmHg) to provide the titled product (19.79 g, 0.141 mol) as a mixture of epimers in 79% yield.
TLC: (80:20 Hexanes: ethyl acetate) $R_f = 0.36$, 0.17.
NMR ($CDCl_3$): δ 2.0 - 1.6 (5H, m), 1.5 - 1.0 (9H, m), 0.55 (1H, m).

(±)-trans-2-(2-methylpropenyl)chlorocyclopentane

c) A solution of the product of example 3(b), above, (19.6 g, 0.140 mol) in THF (125 ml) was added at once to a rapidly stirring mixture of 37% aqueous HCl (250 ml) and THF (125 ml) chilled in an ice bath. After 3.5 - 4.0 minutes the mixture was poured into ice water (1 l.) and was quickly extracted with pentane (3 x 250 ml). The pentane extracts were washed with 5% $NaHCO_3$ (200 ml), dried over $MgSO_4$ and concentrated by rotary evaporation (25° C/15mm). The residue was distilled at 78°-80° C/15 mmHg through an 8 inch Vigreux column to provide the titled compound (18.65 g, 0.118 mol) in 84% yield.
TLC: (hexanes) $R_f = 0.71$
NMR ($CDCl_3$): δ 4.95 (1H, dm), 3.84 (1H, apparent quartet), 2.87 (1H, apparent quintet), 2.2 (1H, m), 2.07 - 1.75 (4H, m), 1.72 (3H, d; J = 1.2 Hz), 1.68 (3H, d; J = 1.2 Hz), 1.33 (1H, m).
GC-MS (isobutane): m/z 158 $(M)^+$, 123 $(M+H-HCl)^+$.
IR (film): 2980, 1455, 1382, 838 $cm^{-1}$

2-(2-methylpropenyl)lithiocyclopentane

d) A dry 50 ml flask fitted with a reflux condenser was purged with Ar. Powdered lithium (0.34 g, 49 mmol; 1% Na content; 25 wt % dispersion in mineral oil) was introduced and was rinsed with 2 x 10 ml dry pentane to remove mineral oil. A solution of the product of Example 3(c) (1.58 g, 10.0 mmol) in dry freeze-thaw degassed pentane (20 ml) was added. The mixture was stirred under reflux in a 55° C oil bath for 19 hr., then was allowed to settle. The supernatant was withdrawn via syringe and filtered under Ar through a dry fritted glass filter. The resulting clear yellow solution (17.6 ml) was 0.45 M in the titled compound by titration against menthol with 1,10-phenanthroline as indicator. The titled product was produced in 79% yield.

(1R,2R)-2-((2S)-2-tert-butyloxycarbonylamino-3-phenyl-1-oxo)propyl-2-methylpropenylcyclopentane

e) To a stirred solution of Boc-L-phenylalanine (1.59 g, 6.00 mmol) in ether (40 ml) at -78° under Ar was added n-butyllithium (4.00 ml, 1.50 M in hexanes; 6.00 mmol). After 10 minutes, the product of Example 3(d) (37 ml, 0.32 M in pentane; 11.8 mmol) was added dropwise over a 10 minute period. After 10 minutes, the solution was warmed to 0° and stirred 1 hour 45 minutes, then water (200 ml) was added with vigorous stirring. The mixture was extracted with ethyl acetate and the crude product was purified three times by flash chromatography (95:5 hexanes:ethyl acetate) to provide isomer B (0.356 g, 9.60 mmol; 16%

N-tert-butyloxycarbonyl-L-phenylalaninal

b) Boc-Phe-OMe (12.9 g, 46.4 mmol), prepared as above, was dissolved in dry toluene (50 mL) and cooled to -78 °C. Diisobutylaluminum hydride (25% in toluene, 77.3 mL, 116 mmol, 150% excess) was added over 2 min. After stirring for 15 min. at -78 °C, methanol (15 mL) was added slowly to control effervescence. The mixture was poured into Rochelle salt (94 g potassium sodium tartrate in 1 L water, 150 mL) and shaken with ether (100 mL) until extractable. The aqueous phase was washed with ether (3 X 100 mL). The organic phases were combined, dried ($MgSO_4$), and evaporated. The resulting product was used directly in the next step.
TLC: (20% EtOAc in hexane) $R_f = 0.21$.
NMR ($CDCl_3$): δ 9.69 (s,1H), 7.28 (m,5H), 5.05 (s,1H), 4.43 (q,1H,J=7 Hz), 3.10 (d,2H,J=7 Hz), 1.43 (s,9H).

(2S)-N-(2-(N-tert-butyloxycarbonyl)amino-3-phenyl)propyl-L-proline benzyl ester

c) The crude aldehyde, prepared as above, 2(b), was dissolved in 1% acetic acid in methanol (250 mL) with L-proline benzyl ester hydrochloride ( 18.1 g, 75 mmol). Sodium cyanoborohydride (3.06 g, 48.7 mmol, 5% excess) was added, and the mixture was allowed to stir at room temperature for 16 h. The solution was then evaporated, and the residue was partitioned between EtOAc and 1N HCl. The organic layer was washed with 1N HCl (2X), 1N $NaHCO_3$ (3X), and water (1X). Evaporation of the organic phase gave 13.5 g of the titled benzyl ester. Flash chromatography of a portion of the crude material (silica gel, 20% EtOAc in hexane) gave pure product which was crystallized from $CHCl_3$ and hexane. These crystals gave a satisfactory x-ray crystal structure (S,S).
mp: 79-79.5 °C.
TLC: (20% EtOAc in hexane); $R_f = 0.31$.
NMR ($CDCl_3$): δ 7.33 (s,5H), 7.20 (s,5H), 5.14 (s,2H), 4.95 (d,1H,J=9 Hz), 4.05 (d,1H,J=7 Hz), 3.17 (m,2H), 2.63 (m,5H), 1.87 (m,4H), 1.35 (s,9H).
MS: m/z 439 $(M+H)^+$, 383, 365, 347, 339, 303, 231, 218, 91, 79. Anal. Calc'd for $C_{26}H_{34}N_2O_4$: C, 71.21; H, 7.81; N, 6.39;
Found C, 71.00; H, 7.83; N, 6.25.

(2S)-N-(2-(N-tert-butyloxycarbonyl)amino-3-phenyl)propyl-L-proline

d) The protected reduced dipeptide (0.44 g, 1.0 mmol), prepared as above, 2(c), was dissolved in methanol (100 mL) with 10% Pd/C (0.50 g) and hydrogenated under 45 psi $H_2$ for 1 h. The mixture was filtered and concentrated to give 0.35 g (100%) of the titled compound.
TLC: (B:A:W 4:1:1); $R_f = 0.66$.
NMR (DMSO-$d_6$): δ 7.26 (s,5H), 6.80 (d,1H,J=9 Hz), 6.00 (s,2H), 3.78 (s,1H), 2.9 (m,6H), 1.85 (m,4H), 1.30 (s,9H).

Performing the same sequence, except substituting benzyl piperidine-2-carboxylate for proline benzyl ester, yields (2S)-N-(2-(N-t-butyloxycarbonyl)amino-3-phenyl)propylpiperdine-2-carboxylic acid.

Performing the sequence 2(a)-2(c), except substituting Δ3-dehydro-proline methyl ester for proline benzyl ester, yields methyl (2S)-N-(2-(N-t-butyloxycarbonyl)amino-3-phenyl)propyl-L-proline. Saponification of the methyl ester with 5 equivalents of $K_2CO_3$ in 4:1 methanol:water yields 2(S)-N-(2-(N-t-butyloxycarbonyl)amino-3-phenyl)propyl-L-proline.

## Example 3

Preparation of (1R,2R)-2-(((2S)-2-tert-butyloxycarbonylamino-3-phenyl-1-oxo)propyl)cyclopentanecarboxylic acid

6-carbomethoxybicyclo[3.1.0]hexane

yield based on Boc-L-Phe), which elutes second, and isomer A (0.314 g, 8.46 mmol; 14% yield) which elutes first.

(isomer B)

(1R,2R)-2-((2S)-2-tert-butyloxycarbonylamino-3-phenyl-1-oxo)propyl-2-methylpropenylcyclopentane
TLC: (95:5 hexanes: ethyl acetate) $R_f = 0.19$.
NMR ($CDCl_3$): δ 7.28-7.08 (5H), 5.06 (1H, d; J = 7.8Hz; NH), 4.95(1H, d; J = 9.6Hz; vinyl), 4.61 (1H, apparent quartet), 3.11 (1H, m), 3.10 (1H, dd; benzylic), 2.83 (1H, dd; benzylic), 2.77 (1H, m), 2.05 - 1.25 (6H), 1.68 (3H; methyl), 1.62 (3H; methyl), 1.39 (9H, s; Boc).
MS ($DCI/NH_3$): m/z 372 $(M+H)^+$, 316, 272.

(isomer A)

(1S,2S)-2-((2S)-2-tert-butyloxycarbonylamino-3-phenyl-1-oxo)propyl-2-methylpropenylcyclopentane
mp. 42-44° C.
TLC: (95:5 hexanes: ethyl acetate) $R_f = 0.26$.
NMR ($CDCl_3$): δ 7.27-7.08 (5H), 5.18 (1H, d; J = 7.7Hz; NH), 5.00 (1H, d; J = 9.3 Hz; vinyl), 4.57 (1H, apparent quartet), 3.05 (1H, dd; J = 13.9, 6.7 Hz; benzylic), 2.95 (1H, dd; J = 13.9, 5.8 Hz; benzylic), 2.79 - 2.58 (2H, m), 1.95-1.20 (6H, m), 1.70 (3H; methyl), 1.52 (3H; methyl), 1.41 (9H; Boc).
MS ($DCI/NH_3$): m/z $(M+H)^+$, 372, 316, 272.

(1R,2R)-2-(((2S)-2-tert-butyloxycarbonylamino-3-phenyl-1-oxo)propyl)cyclopentanecarboxylic acid

f) A solution of the product of Example 3(e) isomer B (186 mg, 0.50 mmol) in 4:1 $CH_2Cl_2/CH_3OH$ (5 ml) was stirred at -78°, and ozone was introduced in a stream until a blue color persisted (ca. 2 min.). The solution was purged with argon, $(CH_3)_2S$ (0.5 ml) was added and the solution was allowed to warm to 20° C. After 2 hr. the volatile components were removed under vacuum. The resulting crude aldehyde was dissolved in 5 ml acetone and treated with Jones Reagent (0.25 ml, 2.0M) at 0° with stirring for 3 min. Isopropanol (0.2 ml) was added. The mixture was diluted with water and extracted with $CH_2Cl_2$, and the extracts were concentrated. Flash chromatography (80:20 hexanes:ethyl acetate followed by 80:20:4 hexanes:ethyl acetate:acetic acid) provided the titled compound (148 mg, 0.410 mmol) as a glass in 82% yield.
m.p. 94.5-96° C
TLC: (80:20:4 hexanes:ethyl acetate:acetic acid) $R_f = 0.27$.
NMR ($CDCl_3$): δ 7.26-7.13(5H, m), 5.12(1H, d; J = 8.0 Hz), 4.70(1H, apparent quartet), 3.46-3.27(2H, m), 3.16(1H, dd; J = 14 Hz, 6.2 Hz), 2.93(1H, dd; J = 14.0 Hz, 6.5 Hz), 2.14-1.67(6H, m), 1.39(12H, s).
MS ($DCI/NH_3$): m/z 362 $(M+H)^+$.

Performing the same sequence, except substituting cyclohexene for cyclopentene in 3(a), yields 2-((-(2S)-2-tert-butyloxycarbonylamino-3-phenyl-1-oxo)propyl)cyclohexanecarboxylic acid

## Example 4

### Preparation of (1S,2S)-2-(((2S)-2-tert-butyloxycarbonylamino-1-oxo-3-phenyl)propyl)cyclopentanecarboxylic acid

By the same procedure used to prepare the compound of Example 3(f), except substituting isomer A (186 mg, 0.50 mmol) from Example 3(e), the titled compound was obtained (135 mg, 0.37 mmol) as a white solid in 75% yield.
TLC: (80:20:5 hexanes:ethyl acetate:acetic acid) $R_f = 0.41$.
NMR (CDCL3): δ 7.32-7.12(5H, m), 5.13(1H,d; J = 8.1 Hz), 4.71(1H, apparent quartet), 3.42(1H, apparent quartet), 3.22-3.08(2H, m), 2.91(1H, dd; J = 13.8, 6.9 Hz; benzylic), 2.20-1.62(6H, m), 1.38(12H, s).
MS ($DCI/NH_3$): m/z 362$(M+H)^+$, 323 $(M+NH_3+H\text{-}tBu)^+$, 262, 120.

Example 5

Preparation of (1R,2R)-2-(((1RS,2S)-1-hydroxy-2-tertbutyloxycarbonylamino-3-phenyl)propyl)-cyclopentanecarboxylic acid

To a solution of the compound of Example 7(f) (216 mg, 0.598 mmol) in methanol (5 ml) at 0° was added $NaBH_4$ (25 mg, 0.66 mmol) with stirring. After 20 min. dilute HCl was added and the mixture was extracted with $CH_2Cl_2$ to provide the titled compound as a mixture of epimers (216 mg, 0.595 mmol) in 100% yield and approximately 2:1 ratio.

NMR ($CDCl_3$): δ 7.35-7.15 (5H), 5.05 (1H; NH), 3.87-3.56 (2H), 2.95-2.60 (3H), 2.38 (1H), 2.19-1.55 (6H), 1.38 (9H).

MS ($DCI/NH_3$): m/z 364$(M+H)^+$, 325, 308$(M-C(CH_3)_3+H)^+$, 264 $(M-OCOC(CH_3)_3+H)^+$, 183, 160, 120

Performing the same reduction upon 2-(((2S)-2-tert-butyloxycarbonylamino-3-phenyl-1-oxo)propyl)-cyclohexanecarboxylic acid yields the corresponding 2-(((2S)-2-tert-butyloxycarbonylamino-3-phenyl-1-hydroxoy)propyl)cyclohexanecarboxylic acid

Example 6

Preparation of (1R,2R)-2-(((1S,2S)-1-hydroxy-2-tert-butyloxycarbonylamino-3-phenyl)propyl)-cyclopentanecarboxolic acid

(1R,2R)-methyl-2-(((1S,2S)-2-tert-butyloxycarbonylamino-1-hydroxy-3-phenyl)propyl)-cyclopentanecarboxylate

a) A solution of the epimers of Example 5 (140 mg, 0.386 mmol) in ether (5 ml) at 0° was treated with excess ethereal diazomethane for about 5 min. (until a yellow color persisted). Acetic acid (0.1 ml) was added and the solution was concentrated by rotary evaporation. Separation by HPLC (80:20 ethyl acetate: hexanes) on silica provided isomer A (47.0 mg, 0.125 mmol), which eluted first, followed by isomer B (50.6 mg, 0.134 mmol).

isomer A

(1R,2R)-methyl-2-(((1S,2S)-2-*tert*-butyloxycarbonylamino-1-hydroxy-3-phenyl)propyl)-cyclopentanecarboxylate

TLC: (2:1 hexanes:ethyl acetate) $R_f = 0.54$.

NMR ($CDCl_3$): δ 7.32-7.18 (5H, m), 4.85 (1H, d; J = 8.7 Hz; NH), 3.72 (1H, m), 3.66 (3H, s), 3.52 (1H, dd), 2.90 (2H, apparent d; benzylic), 2.58 (1H, apparent quartet), 2.40 (1H, m), 1.92-1.50 (6H, m), 1.39 (9H, s).

MS ($DCI/NH_3$): m/z 378$(M+H)^+$, 339, 322$(M-C(CH_3)_3+H)^+$, 321, 304$(M-OC(CH_3)_3+H)^+$, 278 $(M-OCOC(CH_3)_3+H)^+$, 120.

The 2-hydroxy configuration was assigned as follows. Isomer A was converted to its oxazolidinone derivative by treatment with TFA to remove the BOC protecting group, followed by treatment with phosgene and triethylamine. The oxazolidinone ring hydrogens had an NMR coupling constant consistent with a trans relationship (J = 5.3 Hz), which was confirmed by a lack of observed NOE, thus establishing the 2-hydroxy configuration to be (S).

isomer B

(1R,2R)-methyl-2-(((1R,2S)-2-*tert*-butyloxycarbonylamino-1-hydroxy-3-phenyl)propyl)-cyclopentanecarboxylate

TLC: (2:1 hexanes: ethyl acetate) $R_f = 0.42$.

NMR ($CDCl_3$): δ 7.31-7.16 (5H, m), 4.92 (1H, d; J = 8.7 Hz), 3.89 (1H, m), 3.72 (3H, s), 3.64 (1H, apparent d), 3.41 (1H, dd; J = 14.2, 4.2 Hz; benzylic), 2.72 (2H, m), 2.36 (1H, apparent quintet), 2.06-1.40 (6H, m), 1.32 (9H, s).
MS ($DCI/NH_3$): m/z 378$(M+H)^+$, 322, 278, 120.

The 2-hydroxy configuration of isomer B was determined in the same manner as isomer A.

In an alternate procedure, the two epimers were cleanly separated by preparative HPLC (Rainin Dynamax® 1 in. x 25 cm silica column, 20 ml/min, 75:25:1 hexanes:ethyl acetate:formic acid) to provide Isomer A (49% yield) and Isomer B (35% yield).

(1R,2R)-2-(((1S,2S)-1-hydroxy-2-tert-butyloxycarbonylamino-3-phenyl)propyl)cyclopentanecarboxolic acid

b) To a solution of isomer A of Example 6(a) (23.7 mg, 63.2 m mol) in 2:1:1 THF:water:methanol (2 ml) was added 10% NaOH (ca. 0.2 mmol). After 2.5 hr. the mixture was diluted with ethyl acetate and washed with 5% HCl. Drying over $MgSO_4$ and evaporation provided the titled compound (21.4 mg, 59.0 m mol) in 94% yield.
m.p.: 120-122 °C
TLC: (80:20:4 hexanes:ethyl acetate:acetic acid) $R_f$ = 0.34.
NMR ($CD_3OD$): δ 7.23 (5H), 3.73 (1H, m), 3.53 (1H, m), 2.85 (1H, dd; benzylic), 2.73 (1H, dd; benzylic), 2.55 (1H, m), 2.37 (1H, m), 1.90-1.57 (6H), 1.35 (9H, s).

## Example 7

Preparation of (1R,2R)-2-(((1R,2S)-1-hydroxy-2-tert-butyloxoycarbonylamino-3-phenyl)propyl)-cyclopentanecarboxylic acid

By the procedure of Example 6(b), isomer B of Example 6(a) (20.1 mg, 53.6 m mol) provided the titled compound (19.3 mg, 53.2 mmol; 99% yield).
m.p.: 133-135 °C
TLC: (80:20:4 hexanes:ethyl acetate:acetic acid) $R_f$ = 0.27.
NMR ($CD_3OD$): δ 7.21 (5H), 3.75 (1H, m), 3.49 (1H, dd), 2.96 (1H, dd; benzylic), 2.77-2.42 (3H), 1.95-1.83 (3H), 1.78-1.60 (2H), 1.50 (1H, m), 1.30 (9H, s).

## Example 8

Preparation of (1S,2S)-2-(((1RS,2S)-2-tert-butyloxycarbonylamino-1-hydroxy-3-phenyl)propyl)-cyclohexanecarboxylic acid

Using the procedure of Example 5 except substituting the Compound of Example 4 (38 mg, 0.105 mmol), the titled compound was obtained (35.5 mg, 0.0978 mmol) in a 1:1 ratio and 93% yield as a white solid.
TLC: (80:20:5 hexanes:ethyl acetate:acetic acid) $R_f$ = 0.26.
NMR ($CDCl_3$): δ 7.32-7.14 (5H, m), 3.93-3.60 (2H, m), 3.38-3.21(1H, two multiplets), 3.05-2.70(2H, m), 2.55-2.38(1H, m), 2.35-1.54(7H, m), 1.31-1.14(9H, two singlets).

## Example 9

Preparation of valyl valine methyl ester hydrochloride phenylmethoxycarbonyl-valyl valine methyl ester

a) Isobutyl chloroformate (1.30 ml, 10.0 mmol) was added dropwise over 10 min. to a stirring solution of N-Cbz-L-valine (2.51g, 10.0 mmol) and N-methylmorpholine (1.21 ml, 11.0 mmol) in THF (30 ml) at -40 °C under argon. After 1 hr., solid L-valine methyl ester hydrochloride (1.68 g, 10.0 mmol) was added, followed by more N-methylmorpholine (1.21 ml, 11.0 mmol). The mixture was stirred with warming to 20 °C over 24 hr., then was filtered and concentrated to a solid by rotary evaporation. Flash chromatography (gradient, 0 to 5% $CH_3OH$ in $CHCl_3$) provided the titled compound (3.44 g, 9.50 mmol) as a white solid in 95% yield.
NMR ($CDCl_3$): δ 7.34(5H, m), 6.80(1H,d), 5.62(1H, d), 5.13 (2H, s), 4.56 (1H, dd), 4.17(1H, dd), 3.74(3H, s), 2.15(2H, m), 1.00-0.88(12H, overlapping doublets).

valylvaline methyl ester hydrochloride

b) A solution of the compound of Example 9(a) (535 mg, 1.47 mmol) in acetic acid (4 ml) was stirred with 10% Pd on carbon (50 mg) under an $H_2$ atmosphere for 5 hr. The mixture was filtered, diluted with $CH_3OH$, acidified with 37% HCl (0.2 ml), and concentrated under vacuum to a gum. The residue was triturated with ether and dried under vacuum to yield the titled compound (395 mg, 1.47 mmol) as a white powder.
NMR ($CD_3OD$): δ 4.36(1H, d; J=5.8 Hz), 3.81(1H, d; J = 5.7 Hz), 3.73(3H, s), 2.21 (2H, septet), 1.09(3H, d; J = 7.0 Hz), 1.06 (3H,d; J=6.9 Hz), 0.998 (6H, overlapping doublets).
MS (FAB + VE): m/z 231 $(M + H)^+$, 138, 171, 132, 72, 55.

Example 10

Preparation of tert-butyloxycarbonyl-(O-phenylmethyl)serylalanyl alanine

tert-butyloxycarbonyl-(O-phenylmethyl)seryl alanine methyl ester

a) Isobutyl chloroformate (2.59 ml, 20.0 mmol) was added dropwise over 15 min. to a stirred solution of N-Boc-L-serine-O-benzyl ester (5.90 g, 20.0 mmol) and N-methylmorpholine (2.74 ml, 25.0 mmol) in THF (100 ml) at -40 °C under argon. After 45 min. N-methylmorpholine (2.75 ml, 25.0 mmol) and solid L-alanine methyl ester hydrochloride (3.22 g, 23.0 mmol) were added. The mixture was allowed to stir with gradual warming to 20 °C. After 18 hr. the mixture was diluted with ethyl acetate and washed successively with 5% HCl, 5% $NaHCO_3$ and brine. Filtration and removal of solvent under vacuum provided the titled compound (7.54 g, 19.8 mmol) as an oil in 99% yield.
TLC: (1:1 ethyl acetate:hexanes) $R_f$=0.5.
NMR ($CDCl_3$): δ 7.32(5H, m), 7.11(1H, br m), 5.41(1H, br m), 4.65-4.53(3H,m), 4.30(1H, br m), 3.92(1H, dd; J=9.2 Hz, 3.9 Hz), 3.73(3H, s), 3.58(1H,dd; J=9.2 Hz, 616 Hz), 1.45(9H, s), 1.39(3H, d; J=7.1 Hz).

tert-butyloxycarbonyl-(O-phenylmethyl)seryl alanine

b) Sodium hydroxide (0.8 g, 20 mmol) in water (50 ml, was added in portions over 2 hr. to a solution of the compound of Example 10(a) (7.5 g, 19.7 mmol) in 2:1 methanol:water (225 ml). Stirring was continued 1 hr. longer until the solution was approximately neutral and TLC indicated complete reaction. The solution was concentrated by rotary evaporation, diluted with water, and extracted with ethyl acetate. The aqueous layer was acidified with 10% HCl and extracted twice with ethyl acetate. Drying over $MgSO_4$ and concentration under vacuum provided the titled compound (6.89 g, 18.8 mmol) as a glass in 96% yield.
NMR ($CDCl_3$): δ 7.32(5H, m), 5.62 (1H, broad), 4.60 (1H, m), 4.55 (2H, s), 4.36 (1H, broad), 3.87 (1H, dd), 3.61 (1H, dd), 1.46-1.40 (12H, m).

tert-butyloxycarbonyl-(O-phenylmethyl)serylalanyl alanine methyl ester

c) Isobutyl chloroformate (2.42 ml, 18.8 mmol) was added dropwise over 18 min. to a stirring solution of

the compound of Example 10(b) (6.89 g, 18.8 mmol) and N-methyl morpholine (2.74 ml, 25.0 mmol) in THF (100 ml) at -40° C under argon. After 20 min. N-methyl morpholine (2.74 ml, 25.0 mmol) and solid L-alanine methyl ester hydrochloride (3.07 g, 22.0 mmol) were added. The mixture was allowed to stir with gradual warming to 20° C for 20 hr., then was diluted with ethyl acetate and washed successively with 5% HCl, 5% $NaHCO_3$ and brine. Filtration and removal of solvent under vacuum yielded the crude product (7169 g) as a solid. Recrystallization (2:1 hexanes:ethyl acetate) provided the titled compound (7.55 g, 16.7 mmol) in 89% yield as white crystals.

TLC: (1:1 ethyl acetate:hexanes) $R_f = 0.15$.

NMR($CDCl_3$): δ 7.32(5H, m), 7.00 (1H,d), 6.90(1H,d), 5.44(1H, d), 4.58(2H, s), 4.54(2H, m), 4.30(1H, broad), 3.90(1H, dd), 3.74(3H, s), 3.64(1H, dd), 1.44(9H, s), 1.38(6H, overlapping doublets).

tert-butyloxycarbonyl-(O-phenylmethyl)serylalanyl alanine

d) Sodium hydroxide (0.25 g, 6.2 mmol) was added in portions over 4 hr. to a solution of the compound of Example 10(c) (2.79 g, 6.19 mmol) in 2:1 methanol:water (75 ml), at such a rate that the pH remained between 7 and 9. Stirring was continued for an additional 10 hr., then the solution was concentrated by rotary evaporation. Water was added and the solution was extracted twice with ethyl acetate. The aqueous layer was acidified with 10% HCl and extracted three times with ethyl acetate. Drying ($MgSO_4$) of the organic extract and removal of solvent umder vacuum provided the titled compound (2.58 g, 5.90 mmol) in 95% yield as a white powder.

NMR ($CDCl_3$): δ 7.30 (7H, m; aromatic + NH), 5.57 (1H, broad), 4.64-4.46(4H, m), 4.38(1H, broad), 3.86 (1H,dd), 3.65(1H, dd), 1.44(9H,s), 1.39(6H, overlapping doublets).

MS (FAB + VE): m/z 438$(M+H)^+$, 382, 338, 293, 249, 221, 203.

MS (FAB-VE): m/z 436$(M-H)^-$, 336, 254.

## Example 11

### Preparation of trans-2-((1-methoxy-1-(2-phenyl-1-phenylmethoxycarbonylamino)ethyl)phosphinyl)-cyclopentanecarboxylic acid

(±)-2-phenyl-1-phenylmethoxycarbonylaminoethyl phosphonic acid, monomethyl ester

(a) (±)-diphenyl-(2-phenyl-1-phenylmethoxycarbonylamino)ethyl phosphonate was prepared as described in Synthesis, 985(1979). Briefly, a mixture of triphenyl phosphite(0.1 mol), phenylacetaldehyde(.15 mol, freshly distilled), benzyl carbamate(.1 mol), and glacial acetic acid(15 ml) was stirred for 1 hr. until the exothermic reaction subsites. The mixture was then heated at 80-85° C for 1 hr. and volatile products were removed on a rotary exaporator under reduced pressure with heating on a boiling water bath. The oily residue was dissolved in methanol(180 ml) and left for crystallization at -10° C. After 1-3 hr. the crystalline ester was collected by filtration and recrystallized by dissolving it in a minimum amount of hot chloroform-(30-40 ml) and adding a 4-fold volume of methanol.

A portion of the resulting diphenyl phosphonate(2.44 g, 5.00 mmol) was stirred for 2 days in methanol (75 ml) with sodium methoxide (1.6 g, 30 mmol). The solution was diluted with 5% HCl (250 ml) and extracted with ether. The ether layer was extracted with 5% $Na_2CO_3$ and brine, then was concentrated. The crude dimethyl phosphonate was stirred for 1 day as a solution in 50 ml methanol and 50 ml 10% NaOH. The solution was acidified with 10% HCl (300 ml) and extracted with $CH_2Cl_2$. Removal of solvent provided a solid which was dissolved in hot $CHCl_3$ (100 ml). Hexane (50 ml) was added and the solution was stored overnight at -20° C. The resulting crystals were collected by filtration to provide the title compound (1.53 g, 4.38 mmol) in 88% yield.

2-(2-methylpropenyl)cyclopentylmagnesium bromide

b) To a stirring suspension of magnesium turnings (432 mg, 18 mmol) in dry ether (15 ml) and dry

benzene (5 ml) under Ar in a flask equipped with a reflux condenser was added neat 1,2-dibromoethane (1.55 ml, 18 mmol) cautiously in dropwise fashion over 30 min. A 10.0 ml aliquot (9.0 mmol) of the resulting clear $MgBr_2$ solution was added to a -78° solution of 2-(2-methyl propenyl)lithiocyclopentane (the compound of Example 3(d)) (8.6 mmol) in pentane (20 ml) and THF (20 ml). The resulting heterogenous mixture was warmed to 0° to give a clear orange solution of the corresponding Grignard reagent (8.6 mmol) which was recooled to -78° for use in step (b).

trans-2-(1-methoxy-1-(2-phenyl-1-phenylmethoxycarbonylamino)ethyl)-phosphinyl-2-methylpropenylcyclopentane

c) To a solution of the compound of Example 11(a), (1.92 g, 5.50 mmol) in dry $CH_2Cl_2$ (15 ml) under Ar was added $SOCl_2$ (0.40 ml, 5.5 mmol). After 2.5 hr the solvent was removed under vacuum. Residual HCl was removed by addition and re-evaporation of dry $CH_2Cl_2$ (10 ml) followed by dry THF (10 ml). The remaining gummy phosphonyl chloride was diluted with dry THF to a volume of 11.0 ml; an aliquot of the solution (8.6 ml; 4.3 mmol) was added with stirring to a -78° solution of Grignard reagent (8.6 mmol) as prepared in step (a) above. The homogenous mixture was allowed to stir 1 hr. at -78° C, then 1 hr. at -20° C. Aqueous 10% HCl (50 ml) was added, followed by water (200 ml). The mixture was extracted three times with ether. The combined ether extracts were rinsed with 5% $NaHCO_3$, dried over $MgSO_4$ and concentrated to an oil (2.1 g). Flask chromatography ($CHCl_3$) provided the titled compound as a gum (864 mg, 1.90 mmol; 44% yield) and as a mixture of four diastereoisomers.
TLC: (25:1 $CHCl_3$:$CH_3OH$) $R_f$ = 0.4.
GC (Column: CHROMPACK 51 mm x 0.22 mm i.d.; liquid phase CP sil 5 CB. Oven: 240° C): RT (rel. area) 4.04 (1.0), 4.17 (1.3), 4.42 (1.2), 4.58 (1.9).
NMR ($CDCl_3$): δ 7.35-7.03 (10H, m), 5.56-5.10 (1H, m; NH), 5.05-4.87 (3H, m), 4.33 (1H, m), 3.76-3.60 (3H, m), 3.29-2.69 (3H, m), 2.08-1.56 (12H, m), 1.29 (1H, m).
MS ($DCI/NH_3$): m/z 456 $(M + H)^+$, 378, 365, 348, 322, 213, 203, 120, 108, 91.

trans-2-((1-methoxy-1-(2-phenyl-1-phenylmethoxycarbonylamino)ethyl) phosphinyl)cyclopentanecarboxolic acid

d) Using the ozonolysis procedure of Example 3(f), the above compound of Example 11(c) (680 mg, 1.49 mmol) provided the titled compound as a crude gum. Flash chromatography (25:1 $CHCl_3$:$CH_3OH$ followed by 25:1:1 $CHCl_3$:$CH_3OH$: acetic acid) provided the pure product (365 mg, 0.820 mmol) in 55% yield as a crispy foam.
NMR ($CDCl_3$): δ 7.32-7.12 (10H, m), 5.00-4.91 (2H, m), 4.45 (1H, m), 3.78-3.64 (3H, m), 3.35-2.68 (4H, m), 2.08-1.60 (7H, m).

Performing the same sequence except substituting 4-bromo-1-butene for 2-(2-methyl propenyl)-lithiocyclopentane, yields the corresponding 2-((1-methoxy-1-(2-phenyl-1-phenylmethoxycarboxylamino)-ethylphosphinyl)propanoic acid.

## Example 12

### Preparation of (5S)-4-oxo-5-(t-butoxycarbonylamino)-6-phenylhexanoic acid

(6S)-7-phenyl-6-(t-butoxycarbonylamino)hept-1-en-5-one

a) To a stirring suspension of magnesium powder (1.82 g, 75 mmol) in dry diethyl ether (10 ml) under argon were added several drops of 4-bromo-1-butene. Once spontaneous reflux had begun, 4-bromo-1-butene (6.75 g, 50 mmol) was added dropwise at a rate that maintained gentle reflux. The mixture was stirred for an additional 30 minutes with heating to reflux. After cooling, the solution of Grignard reagent was added to a stirring solution of (Boc)-L-phenylalanine-N- methoxy-N-methylamide (1.0 g, 3.2 mmol) in dry diethyl ether (10 ml) at 0°. After 1.5 hours the reaction mixture was diluted with 10% HCl and extracted 3x

with diethyl ether. The organic layer was washed successively with 1M HCl, saturated aqueous sodium bicarbonate, saturated aqueous sodium chloride, dried ($MgSO_4$) and evaporated under reduced pressure to yield the titled compound (780 mg, 81.8% yield) as white crystals.

NMR ($CDCl_3$): δ 7.20(5H,m); 5.73(1H,m); 5.1(1H,d); 5.05(2H,m); 4.55(1H,quartet); 3.05 (2H,m); 2.48-(2H,quartet); 2.30(2H,m); 1.40(9H,s).

MS (DP/$NH_3$): m/z $(M+H)^+$ 304; 248, 204, 120.

(5-S)-4-oxo-5-(t-butoxycarbonylamino)-6-phenyl-hexanoic acid

b) Ozone was introduced into a solution of the compound of Example 12(a) (400 mg, 1.32 mmol) in 4:1 $CH_2Cl_2$:MeOH (5 ml) at -78° C until a blue color persisted. The solution was purged with argon for several minutes, then dimethyl sulfide (2 ml) was added. The mixture was warmed to room temperature for 2 hrs., then concentrated under reduced pressure to an oil. The oily residue was dissolved in acetone and cooled with stirring to 0° C. Jones reagent (1.32 mmol, 2M soln.) was added dropwise. After 5 minutes isopropanol was added. The mixture was diluted with $H_20$ and extracted 3x with dichloromethane. The organic layer was dried ($MgSO_4$), filtered and concentrated to an oil. Flash chromatography over silica gel(60:40 hexane:ethyl acetate, followed by 65:35:5 hexane:ethyl acetate:acetic acid) provided the titled compound as an oil (175 mg, 41% yield

NMR ($CDCl_3$): δ 7.24(5H,m); 5.1(1H,d); 4.55,(1H,quartet); 3.05(2H,m); 2.68(4H,m); 1.38(9H,s).

MS ($FAB^+$): m/z 322; ($FAB^-$): m/z 320.

Using the same sequence, except substituting Boc-phenylglycine for Boc-(O-benzyl)tyrosine yields 4-oxo-5-(tert-butyloxycarbonyl-amino)-5-phenylpentanoic acid.

## Example 13

Preparation of (5S)-4-oxo-5-(tert-butoxycarbonylamino)-6-(4-benzyloxophenyl)-hexanoic acid

Boc-(O-benzyl)tyrosine-N-methoxy-N-methyl amide

a) To a solution of Boc-(O-benzyl)tyrosine (7.42 g; 30.0 mmol) in dry $CH_2Cl_2$ (20 ml) were added triethylamine (3.0 ml; 21.5 mmol) and benzotriazol-1-yloxytris(dimethylamino) phosphonium hexafluorophosphate (6.96 g; 20.0 mmol). After 10 minutes, triethylamine (3.0 ml; 21.5 mmol) and O,N dimethylhydroxylamine (2.15 g; 22.0 mmol). After 16 hr., the solution was diluted with ethyl acetate and washed successively with 5% HCl, saturated aqueous sodium bicarbonate, and saturated aqueous sodium chloride. The organic solution was dried ($MgSO_4$), filtered, and concentrated to a foam. Flash chromatography over silica gel(50:50 hexane/ethyl acetate) provided the titled compound(5.87 g, 71% yield) as a white powder.

NMR ($CDCl_3$): δ 7.40 (5H, m); 7.02, (4H), dd; 5.12 (1H, d); 5.05 (2H, s); 4.90 (1H, broad); 3.62 (3H, s); 3.15 (3H, s); 2.92 (2H, m); 1.38 (9H, s).

MS: m/z $(M+H)^+$ 415; 359, 341, 315

(6S)-7-(4-benzyloxyphenyl)-6-(tert-butoxycarbonylamino)hept-1-ene-5-one

b) To a suspension of magnesium powder (6.68 g, 0.275 mol) in anhydrous diethyl ether (100 ml), under an argon atmosphere was added several drops of 4-bromo-1-butene. Once spontaneous reflux was initiated, 4-bromo-1-butene (24.72 g, 0.183 mol) was added at a rate as to maintain a gentle reflux. Upon completion of the addition, the mixture was refluxed an additional 30 min. A solution of t-butyloxycarbonyltyrosine-(O-benzyl)-methoxymethyl amide (18.95 g, 0.046 mol) in anhydrous THF was prepared and slowly added to the previously prepared Grignard reagent at 0°C. After 90 min, the reaction was complete as indicated by TLC and was quenched with 10% aqueous HCl. The reaction mixture was extracted with ether three times, and the combined organic extracts were washed successively with 1 M HCl, saturated aqueous $NaHCO_3$, and saturated aqueous NaCl. The solution was dried ($MgSO_4$), filtered,

and evaporated under reduced pressure to give a white solid. Purification by column chromatography on silica gel eluting with 17% ethyl acetate/ hexanes provided the titled compound as a white solid (18.1 g, 96%).

NMR ($CDCl_3$): δ 7.42 (5H, m); 6.96 (4H, dd); 5.72 (1H, m); 5.10 (1H, br d)); 5.06 (2H, s);4.95 (2H, m); 4.48 (1H, g); 2.95 (2H, m);2.5-2.0 (4H, m); 1.38 (9H, s).

MS: m/z $(M+H)^+$ 410, 371, 354, 310

(5S)-4-oxo-5-(tert-butoxycarbonylamino)-6-(4-benzyloxophenyl)-hexanoic acid

c) To a solution of the compound of Example 13(b) (8.0 g, 19.6 mmol) in 4:1 $CHCl_3$: MeOH at -78°C was introduced ozone until a blue color persisted. The solution was then purged with argon for several min, followed by the addition of dimethyl sulfide (6 ml). The resulting mixture was stirred for 2 h at room temperature and then evaporated. The oily residue was dissolved in acetone (175 ml) and cooled to 0°C. Jones reagent (12 ml of a 2 M solution) was added dropwise, and the mixture was stirred for 10 min before quenching with isopropanol. The solution was poured into $H_2O$ and extracted with $CH_2Cl_2$ three times. The combined organic extracts were dried ($MgSO_4$), filtered and evaporated under reduced pressure. The oily residue was purified by column chromatography using silica gel, eluting first with 2:3 ethyl acetate: hexanes, followed by 5: 35: 60 acetic acid: ethyl acetate: hexanes to give the title compound as a tan solid (3.39 g, 40% yield).

NMR ($CDCl_3$): δ 7.40 (5H, m); 7.0 (4H, dd);

NMR ($CD_3OD$): δ 5.05 (1H, obsc.); 5.03 (2H, s); 4.52 (1H, quartet); 3.2- 2.5 (7H, m); 1.40 (9H, s).

## Example 14

### Preparation of (3R,4S)-4-(N-benzyloxycarbonyl)amino-2,2-difluoro-3-hydroxy-5-phenylpentanoic acid

Ethyl(3R,4S)-4-(N-benzyloxycarbonyl)amino-2,2-difluoro-3-hydroxy-5-phenylpentanoate

a) A solution of N-Cbz-L-phenylalanol (0.77g, 2.75 mmol) in 10 ml of dry $CH_2Cl_2$ was added to a solution of the Dess-Martin periodinane (1.28g, 3.02 mmol) in 15 ml of dry $CH_2Cl_2$ in a slow steady stream. After stirring for 30 min., 100 ml of a solution of 1 part 10% aqueous sodium thiosulfate and 1 part saturated aqueous $NaHCO_3$ was introduced and the mixture was stirred vigorously for 30 min. The mixture was extracted three times with $CH_2Cl_2$ and the combined organic extracts were dried over $MgSO_4$, filtered, and concentrated in vacuo to give N-Cbz-L-phenylalanal as a yellow solid. A suspension of zinc (0.54g, 8.24 mmol) and CuBr (60 mg, 0.22 mmol) in 15 ml of dry THF was treated with diethylaluminum chloride (6.04 ml, 6.04 mmol, 1M in hexanes) and cooled to -20°C. To this suspension was added a solution of the crude N-Cbz-L-phenylalanal and ethyl bromodifluoroacetate in 25 ml of dry THF in a dropwise manner over 20 min. The reaction mixture was stirred for 45 min. then was quenched by addition of 5% aqueous HCl and allowed to warm to room temperature. The mixture was extracted three times with $Et_2O$ and the combined organic extracts were dried over $MgSO_4$, filtered, and concentrated. Flash chromatography over silica gel-(1.5:1 $Et_2O$:hexanes) yielded 0.78g (70%) of a 3:2 mixture of diastereomers. The diastereomers were separated by repeated flash chromatography (1:1 hexanes:$Et_2O$) to provide 468 mg (42%) of the titled product as a white solid.

NMR ($CDCl_3$): δ 7.27 (10H, m); 5.02 (3H, m); 4.31 (2H, q; J=7.5 Hz); 4.23 (1H, m); 4.09 (1H, m); 3.86 (1H, br d; J=6.2 Hz); 3.01 (2H, m); 1.31 (1H, t, J=7.5 Hz)

(3R,4S)-4-(N-benzyloxycarbonyl)amino-2,2-difluoro-3-hydroxy-5-phenylpentanoic acid

b) The ester of Example 14(a) (1.0g, 2.45 mmol) was dissolved in 40 ml of 3:2 MeOH:THF. This solution was added to $K_2CO_3$ (2.7 g, 19.6 mmol) in water (25 ml) and the mixture was stirred vigorously for 3 hr. The aqueous solution was washed with $Et_2O$ (discarded), acidified with HCl and extracted once with $Et_2O$ and twice with $CH_2Cl_2$. The combined organic extracts were dried over $MgSO_4$ and filtered. Evaporation of

the solvent in vacuo furnished the titled acid (0.93g, 90%) as a white solid.
NMR ($CDCl_3$): δ 7.21 (10H, m); 4.91 (3H, m); 4.22 (3H, m); 2.97 (2H, m)
MS: m/z 380

## Example 15

### Preparation of 2-(acetyl seryl glutaminyl asparaginyl)amino-3-phenyl-propyl-prolylvalylvalinamide

The compound of Example 2(d) (0.35 g,1.0 mmol) was coupled to Val-Val-BHA (1.0 mmol) using DCC (0.21 g, 1.0 mmol) and HOBT (0.15 g, 1 mmol) in 50% $CH_2Cl_2$/DMF overnight. Quantitative ninhydrin test indicated 83% coupling. The peptide resin was acetylated with $Ac_2O$ (1 mL in 30 mL $CH_2Cl_2$) until the ninhydrin test was negative. The peptide was then completed and acetylated, according to the procedure of Example 1, using half of the resin.The peptide-resin intermediate was cleaved with 10 mL anhydrous HF with 1 mL anisole for 1 h at 0° C. The peptide was extracted from the resin with glacial HOAc. Lyophilization afforded 150 mg crude peptide (37%). The peptide was purified by counter current distribution (n-butanol: acetic acid: water; 4:1:5) yielding 60 mg. The peptide was further purified by preparative HPLC, k′ 4.00 (Hamilton PRP-1 305 X 7 mm semi-preparative HPLC column, water-acetonitrile- 0.1% TFA, 95:5 to 78:22 in 13 min, 78:22 to 60:40 in 6.5 min) giving 17.9 mg..
TLC: $R_f$ 0.34 (B:A:W 4:1:1), 0.68 (B:E:A:W 1:1:1:1).
HPLC: (Hamilton PRP-1 250 X 4.1 mm analytical HPLC column, water-acetonitrile- 0.1% TFA, 95:5 to 60:40 over 15 min.) k′ 2.73.
FAB-MS: m/z 817 $(M+H)^+$.
Amino acid analysis: Ser 0.57, Gln 1.00, Asn 1.00, Val 2.00.

Performing the same sequence, except substituting 1.1 molar equivalents of butyryl chloride for acetic anhydride, yields 2-(butyryl-serylglutaminylasparaginyl)amino-3-phenyl-propyl-prolylvalyl valinamide.

## Example 16

### Preparation of 2-(seryl glutaminylasparaginyl)amino-3-phenylpropyl-prolylvalyl valinamide

The compound of Example 2(d) (0.87 g, 2.5 mmol) was coupled to Val-Val-BHA (1.2 mmol) with DCC (0.52 g, 2.5 mmol) and HOBt (0.38 g, 2.5 mmol) in 30 mL of 50% $CH_2Cl_2$/DMF overnight. Ninhydrin test showed complete coupling. The peptide was completed using 0.6 g (0.4 mmol) of the resin. The peptide was cleaved from the resin using 15 mL HF with 1.5 mL anisole at 0° C for 1 h. The peptide resin mixture was washed with ether (3X) followed by HOAc (4X). Lyophilization of the HOAc gave 192.5 mg crude peptide (62%). A 100 mg portion of the crude peptide was purified by gel filtration through Sephadex® G-15 using 1% HOAc. This afforded 33.3 mg of peptide, which was further purified by preparative HPLC (Hamilton PRP-1 305 X 7 mm semi-preparative HPLC column, water-acetonitrile- .1% TFA, 85:15 to 60:40 in 15 min) to give 8.10 mg.
$R_f$ 0.93 (B:A:W 4:1:1), 0.97 (B:E:A:W 1:1:1:1).
HPLC: (Hamilton PRP-1 250 X 4.1 mm analytical HPLC column, water-acetonitrile- .1% TFA, 95:5 to 60:40 over 15 min.) k′ 2.87
FAB-MS m/z 775 $(M+H)^+$

## Example 17

### Preparation of 4-(acetyl-serylglutaminylasparaginyl)amino-3(RS)-hydroxy-1-oxo-5-phenylpentyl-prolylvalyl valinamide

The resin supported intermediate Ac-Ser(Bzl)-Gln-Asn-[3(RS)-AHPPA]-Pro-Val-Val-BHA was prepared on a 1 mm scale in the usual manner according to Example 1. Boc-AHPPA used was racemic at the 3 position. The peptide was cleaved from the resin and the benzyl group was removed from the serine hydroxyl by treatment at 0° with 10 ml of anhydrous HF and 1 ml of anisole for 60 min. The HF was removed in vacuo at 0°. The residue was triturated with diethyl ether, the peptide was extracted with acetic acid (3 x 20 ml) and lyophilized to yield 302 mg. Purification was accomplished by counter current distribution (n-Butanol:Acetic Acid:water, 4:1:5; 200 transfers). The major fractions were pooled, concentrated, diluted with acetic acid and lyophilized to yield 103 mg of the titled compound.
TLC: (B:E:A:W, 1:1:1:1) $R_f$ = .51;
FAB-MS: m/z 875 $(M+H)^+$;
Amino Acid Analysis: Asp = 1.00, Ser = .14, Glu = .94, Pro = 1.09, Val = 2.24; Peptide Content (based on Asp)-= 80.8%.

## Example 18

Preparation of 4-(acetyl-serylglutaminylasparaginyl)amino-3(RS)-hydroxy-1-oxo-5-phenylpentyl-valyl valinamide

The resin supported intermediate Ac-Ser(Bz)-Gln-Asn-[3(RS)-AHPPA]-Val-Val-$NH_2$ was prepared in the usual manner on a .5 mm scale. The Boc-AHPPA used was racemic in the 3 position. The peptide was cleaved from the resin with removal of the benzyl protecting group by treatment with anhydrous HF (10 ml) in the presence of anisole (1 ml) at 0° for 60 min. After removal of the HF at 0° under vacuum, the resin was triturated with diethyl ether and air-dried. The peptide was extracted from the resin with acetic acid (4 x 20 ml) and lyophilized to yield 110 mg. The peptide was purified using counter current distribution (B:A:W, 4:1:5; 200 transfers). The major fractions were pooled, concentrated, diluted with acetic acid and lyophilized to yield 21 mg of the titled compound.
TLC: (B:E:A:W, 1:1:1:1) $R_f$ = .53
HPLC: (4.5 mm X 25 cm Altex Ultrasphere 5m ODS, acetonitrile-water-.1% TFA, gradient of 10-50% acetonitrile over 20 min.), $k'$ = 4.47, 4.37
FAB-MS: m/z 778 $(M+H)^+$;
Amino Acid Analysis: Asp = 1.00, Ser = .71, Glu = 1.00, Val = 2.09; Peptide Content (based on Asp) = 72.75%.

## Example 19

Preparation of 4-(serylglutaminylasparaginyl)amino-3(R)-hydroxy-1-oxo-5-phenylpentyl-valyl valinamide

The resin supported intermediate Boc-Ser(Bzl)-Gln-Asn-[3(R)-AHPPA]-Val-Val-BHA was prepared in the usual manner on a 1 mm scale. Omitting the acetylation step, the peptide was cleaved from the resin with removal of the benzyl protecting group by treatment with HF (10 ml) and anisole (1 ml) at 0° for 60 min. The HF was removed under vacuum at 0°. The residue was triturated with diethyl ether and the peptide was extracted from the residue with acetic acid (4 x 10 ml). The acetic acid extract was lyophilized to yield 85 mg. 40 mg of the crude peptide was purified by preparative HPLC (Ultrasphere ODS, 15% acetonitrile/$H_2O$-.1% TFA). The fractions containing the desired product were combined, concentrated in vacuo, diluted with acetic acid and lyophilized to yield 20 mg of the titled peptide.
HPLC: (4.6 mm X 25 cm Altex Ultrasphere ODS, 15% acetonitrile-water-.1% TFA) $k'$ = 6.33.
FAB MS: m/z 736 $(M+H)^+$
Amino Acid Analysis: Asp 1.00, Ser .71, Glu .99, Val 1.96

## Example 20

Preparation of 4-(serylglutaminylasparaginyl)amino-3(S)-hydroxy-1-oxo-5-phenylpentyl-valyl valinamide

The resin supported peptide intermediate Boc-Ser(Bz)-Gln-Asn-[3(S)-AHPPA]-Val-Val-$NH_2$ was prepared in the usual manner on a 1 mm scale. The peptide was cleaved from the resin, deblocked and purified in the same manner as in Example 19 to yield 12 mg of the titled compound.
HPLC (4.6 mm X 25 cm Altex Ultrasphere ODS, 15% acetonitrile-water-.1% TFA): $k' = 6.33$
FAB MS: m/z 736 $(M+H)^+$
Amino Acid Analysis: Asp 1.00, Ser .71, Glu .99, Val 1.96

## Example 21

Preparation of (1R,2R)-2-(((2S)-2-(tert-butyloxycarbonylserylalanylalanyl)amino-1-oxo-3-phenyl)propyl)-cyclopentylcarbonyl-valyl valine methyl ester

(1R,2R)-2-(((2S)-2-tert-butyloxycarbonylamino-1-oxo-3-phenyl)propyl)cyclopentylcarbonyl-valyl valine methyl ester

(a) To a solution of the compound of Example 3(f) (10.5 mg, 29.0 m mol) in THF (0.4 ml) under argon at -40°C were added N-methylmorpholine (6 ml, 55 mmol) and isobutyryl chloroformate (3.8 ml, 29.1 mmol). After 20 min. more N-methylmorpholine (6 ml) was added, followed by a solution of valyl valine methyl ester hydrochloride (16 mg, 60 mmol) in THF (0.5 ml). The mixture was stirred at -40°C for 30 min., then at 20°C for 17 hr. The mixture was diluted with ethyl acetate, washed with 5% HCl, dried over $MgSO_4$ and concentrated. Flash chromatography (50:1 $CHCl_3$:$CH_3OH$) provided the titled compound as a white solid (13.4 mg, 23 mmol; 81% yield).
TLC: (50:1 $CHCl_3$:$CH_3OH$) $R_f = 0.28$.
NMR ($CDCl_3$): δ 7.26 (3H, m; aromatic), 7.10(2H, m, aromatic), 6.50(1H, d; J=8.7 Hz; NH), 6.35(1H, d; J=8.6 Hz; NH), 5.11 (1H, d; J=7.9 Hz; BocNH),4.70(1H, apparent quartet), 4.52 (1H dd; J=8.7, 4.9 Hz), 4.26 (1H, dd; J=8.6, 7.1 Hz), 3.73 (3H, s), 3.51 (1H, apparent quartet), 3.16 (1H, dd; J=14.1, 5.5 Hz; benzylic), 3.07 (1H, apparent quartet), 2.86 (1H, dd; J=14.1 6.7 Hz; benzylic), 2.25-1.60 (8H, m), 1.38 (9H, s), 0.941-0.874 (12H, overlapping doublets).
MS (FAB + VE): m/z 574 $(M + H)^+$, 518, 474, 443, 387, 343, 244, 229.

(1R,2R)-2-(((2S)-2-amino-1-oxo-3-phenyl)propyl)cyclopentylcarbonyl-valyl valine methyl ester, trifluoroacetic acid salt

(b) The compound of Example 21(a) (13 mg, 23 mmol, was dissolved in trifluoroacetic acid (0.5 ml). After 2 hr. the solution was concentrated under vacuum, and the residue was combined with methylene chloride and reconcentrated to provide the titled compound (13 mg) as a white solid.

(1R,2R)-2-(((2S)-2-(tert-butyloxycarbonyl-(O-phenylmethylene)serylalanyl-alanyl)amino-1-oxo-3-phenyl)-propyl)cyclopentylcarbonyl-valyl valine methyl ester

(c) To a solution of Boc-Ser(Bzl)-Ala-Ala (15.1 mg, 34.5 mmol) in THF at -40°C under argon, N-methyl morpholine (7.7 ml) and isobutyl chloroformate (4.5 ml, 35 mmol) were added. After stirring 20 min., additional N-methylmorphonine(7.7 ml) was added, followed by a solution of the tripeptide of Example 21(b) (13 mg) in THF. The mixture was warmed to 20°C and stirred overnight. The reaction mixture was diluted with $CH_2Cl_2$ and washed with 5% HCl, 5% $NaHCO_3$ and water. Evaporation of the solvent afforded a crude product which was purified by flash chromatography (gradient elution, 50:1 to 25:1 $CHCl_3$:$CH_3OH$) to give the titled product (17.9 mg, 20 mmol) as a white solid.
TLC: (95:5 $CHCl_3$:$CH_3OH$) $R_f = 0.35$.
NMR (1:1 $CD_3OD$:$CDCl_3$): δ 7.35 - 7.20 (5H, m), 4.80 (1H, dd; J=8.7, 4.8 Hz), 4.54 (2H, s; Ph$CH_2$O), 4.42-4.16 (5H, m), 3.73 (3H, s), 3.78-3.62 (2H, m; BnO$CH_2$), 3.40 (1H, m), 3.20 (1H, dd; J=14.4, 4.7 Hz;

benzylic), 3.09 (1H, m), 2.92 (1H, dd; J = 14.4, 8.7 Hz; benzylic), 2.25-1.95 (4H, m), 1.85-1.65 (4H, m), 1.47 (9H, s), 1.35 (3H, d; J=7.2 Hz), 1.23 (3H, d; J=7.2Hz), 0.956-0.922 (12H, overlapping doublets).
MS (FAB + VE): m/z 893 $(M + H)^+$, 793, 762, 706, 662, 645, 634, 514, 474, 420, 364.

(1R,2R)-2-(((2S)-2-(tert-butyloxycarbonylserylalanylalanyl)amino-1-oxo-3-phenyl)propyl)cyclopentylcarbonyl-valyl valine methyl ester

(d) A solution of the hexapeptide of Example 21(c) (8.1 mg, 9.1 mmol) in methanol (4 ml) was stirred with 10% Pd/C (7 mg) under an $H_2$ atmosphere for 15 hr. Filtration and evaporation provided the titled compound (7.2 mg, 9 mmol) as a white solid.
TLC (95:5 $CHCl_3$:$CH_3OH$): $R_f$=0.15.
NMR (1:1 $CD_3OD$:$CDCl_3$): δ 7.21(5H, m), 4.80 (1H, m), 4.36-4.12 (5H, m), 3.81 (1H, dd; J=10.9, 5.3 Hz; $CH_2OH$), 3.72 (3H, s), 3.67(1H, dd; J=10.9, 5.8 Hz; $CH_2OH$), 3.41(1H, m), 3.20 (1H, dd; J=14.3, 4.6 Hz; benzylic), 3.07 (1H, m), 2.88 (1H, dd; J=14.3, 9.0 Hz; benzylic), 2.22-1.96 (4H, m), 1.81-1.67 (4H, m), 1.47 (9H, s), 1.38 (3H, d; J=7.3 Hz), 1.27 (3H, d; J=7.3 Hz), 0.960-0.932 (12 H, overlapping doublets).
MS (FAB + VE): m/z 803$(M + H)^+$, 787, 703, 672, 616, 572, 544, 473, 330, 315, 298, 274, 244, 203.
MS (FAB - VE): m/z 802 698, 307, 273, 241, 201, 185, 153.

## Example 22

Preparation of (1R,2R)-2-(((2S)-2-(serylalanylalanyl)amino-1-oxo-3-phenyl)propyl)cyclopentylcarbonyl-valyl valine methyl ester, hydrochloride

The compound of Example 21(d) (5 mg, 6.2 mmol) was dissolved in trifluoroacetic acid (.25 ml). After 90 min., the solution was concentrated under vacuum, the residue was dissolved in methanol (.5 ml) and concentrated HCl (about .025 ml) was added. The solution was concentrated, the resulting gum was triturated with ether and dried under vacuum to afford the titled compound (4.5 mg.) as a white solid.
NMR ($CD_3OD$): δ 7.20 (5H, m), 4.72 (1H, dd; J=8.5, 5.0 Hz), 4.38 (1H, m), 4.31 (2H, m), 4.19 (1H, d), 3.92 (3H, m), 3.70 (3H, s), 3.48 (1H, m), 3.19 (1H, dd; J=14.3, 5.0 Hz; benzylic), 3.08 (1H, m), 2.83 (1H, dd; J=14.3, 8.5 Hz; benzylic), 2.21-1.98 (4H, m), 1.84-1.65 (4H, m), 1.35 (3H, d; J=7.2 Hz), 1.29 (3H, d; J=7.1 Hz), 0.958-0.921 (12H, overlapping doublets).
MS (FAB + VE): m/z 703 $(M + H)^+$.

Preparation of (1S,2S)-2-(((1S,2S)-2-(tert-butyloxycarbonylserylalanylalanyl)amino-1-hydroxy-3-phenyl)-propyl)cyclopentylcarbonyl-valyl valine methyl ester

(1S,2S)-2-(((1RS,2S)-2-tert-butyloxycarbonylamino-1-hydroxy-3-phenyl)propyl)cyclopentanecarbonyl-L-valyl-L-valine methyl ester

a) The product of Example 8 (35.5 mg, 97.8 mmol) and HOBT (26 mg, 196 mmol) were dissolved in dioxane(0.5 ml) and DMF(0.1 ml), and DCC (20.2 mg, 98.1 mmol) was added. After 20 min. stirring, N-methyl morpholine (32 ml, 290 mmol) and valyl valine methyl ester hydrochloride(52 mg, 195 mmol) were added. After 3 days, the mixture was diluted with ethyl acetate, filtered and extracted with dilute HCl. The organic layer was concentrated. Flash chromatography (50:1 $CHCl_3$:$CH_3OH$) of the crude product provided isomer A (26 mg, 45.9 mmol; 47% yield) which eluted first, and isomer B (25 mg, 43.1 mmol; 44% yield).

isomer A:

(1S,2S)-2-(((1R,2S)-2-(tert-butyloxycarbonyl)amino-1-hydroxy-3-phenyl)propyl)cyclopentanecarbonyl-valyl valine methyl ester
TLC: (25:1 $CHCl_3$:$CH_3OH$) $R_f$=0.55.

NMR ($CDCl_3$): δ 7.28 (5H, m), 6.73(1H, d; J=8.7 Hz; NH), 6.42(1H, d; J=8.6 Hz; NH), 5.16(1H, d; J=9.5 Hz; BocNH), 4.58(1H, dd; J=8.8, 5.2 Hz), 4.32 (1H, dd; J=8.6, 6.0 Hz), 3.91 (1H, apparent quartet), 3.76(3H, s), 3.25 (1H, apparent doublet; J=10.2 Hz), 2.95-2.83 (2H; benzylic), 2.52 (1H, apparent quartet), 2.30-2.10(3H, m), 2.00-1.55(6H, m), 1.39(9H, s), 0.990-0.907 (12H, apparent quartet).
MS(FAB + VE): m/z 576$(M + H)^+$, 476, 389, 345, 317, 246, 225.

isomer B:

(1S,2S)-2-(((1S,2S)-2-(tert-butyloxycarbonyl)amino-1-hydroxy-3-phenyl)propyl)cyclopentanecarbonyl-valyl valine methyl ester
TLC: (25:1 $CHCl_3$:$CH_3OH$) $R_f$=0.45.
NMR ($CDCl_3$): δ 7.24-7.15(5H, m), 6.77(1H, d; J=8.3 Hz), 6.56 (1H, d; J=8.2 Hz), 4.80(1H, d; J=8.4 Hz), 4.47 (1H, dd; J=8.4, 4.9 Hz), 4.36(1H, apparent triplet), 3.80-3.60(5H, m), 3.08 (1H, dd), 2.69(2H, m; benzylic), 2.53(1H, m), 2.25-2.05(2H, m), 2.00-1.60 (6H, m), 1.32(9H, s), 0.979-0.855 (12H, overlapping doublets).
MS(FAB + VE): m/z 576$(M + H)^+$, 476, 389, 246, 231, 152, 132.

(1S,2S)-2-(((1S,2S)-2-amino-1-hydroxy-3-phenyl)propyl)cyclopentanecarbonyl-valyl valine methyl ester, hydrochloride

b) Isomer B of Example 23(a) (25 mg, 43 mmol) was dissolved in trifluoracetic acid (0.25 ml). After 1 hr., the solution was concentrated by rotary evaporation. The residue was dissolved in $CH_3OH$, treated with conc. HCl (.05 ml) and re-concentrated. Trituration with $Et_2O$ and drying under vacuum provided the titled compound as a white powder (22 mg, 43 mmol).

(1S,2S)-2-(((1R,2S)-2-amino-1-hydroxy-3-phenyl)propyl)cyclopentanecarbonyl-valyl valine methyl ester hydrochloride

c) Isomer A of Example 23(a) (26 mg, 45 mmol) was treated with trifluoroacetic acid and HCl according to the procedure of Example 23(b) to provide the titled compound (19.9 mg, 39 mmol).

(1S,2S)-2-(((1S,2S)-2-(tert-butyloxycarbonyl-L-(O-phenylmethylene)serylalanylalanyl)amino-1-hydroxy-3-phenyl)propyl)cyclopentylcarbonyl-valyl valine methyl ester

d) To a solution of Boc-Ser(Bzl)-Ala-Ala (19.4 mg, 44.4 mmol) in THF (0.5 ml) under Argon at -40°C were added N-methyl morpholine (8.8 ml) and isobutyl chloroformate (5.8 ml, 44.4 mmol). After stirring 20 min., additional N-methyl morpholine (8.8 ml) was added followed by a solution of the compound of Example 23(b) (22 mg, 43 mmol) in THF (1.0 ml). The mixture was warmed to 20°C and stirred overnight. The reaction mixture was diluted with $CH_2Cl_2$ and washed with 5% HCl, dried($MgSO_4$) and concentrated. Flash chromatography of the residue provided the titled compound as a white solid (25 mg, 28 mmol; 65% yield).
NMR ($CDCl_3$): δ 7.40-7.05 (11H, m), 7.00-6.80 (4H, m; NH), 5.53 (1H, d; BocNH), 4.52 (2H, s), 4.47-4.30 (4H, m), 4.19-4.06 (2H, m), 3.97 (1H, broad), 3.80-3.62 (2H, m), 3.69 (3H, s), 3.07 (1H, dd), 2.83 (1H, dd), 2.66 (1H, m), 2.52 (1H, m), 2.34 (1H, broad), 2.23-2.03 (2H, m), 2.00-1.84 (2H, m), 1.80-1.60 (4H, m), 1.46 (9H, s), 1.32 (3H, d; J=7.0 Hz), 1.15 (3H, d; J=7.1 Hz), 0.939 (6H; two overlapping doublets), 0.871 (6H, two overlapping doublets).
MS (FAB + VE): m/z 895 $(M + H)^+$, 795 $(M\text{-}Boc + H)^+$, 764, 708, 664, 618, 565, 547, 476, 416, 388.

(1S,2S)-2-(((1R,2S)-2-(tert-butyloxycarbonyl-L-(O-phenylmethyl)serylalanylalanyl)amino-1-hydroxy-3-phenyl)-propyl)cyclopentylcarbonyl-valyl valine methyl ester

e) By a procedure substantially similar to Example 23(d), using the tripeptide prepared in Example 25(c) (19.9 mg, 39 mmol), Boc-Ser(Bzl)-Ala-Ala (17.5 mg, 40 mmol), isobutyryl chloroformate (5.2 ml, 40 mmol) and N-methylmorpholine (2x8.8 ml), the titled compound was obtained as a white solid (23.2 mg, 26 mmol; 67% yield).

NMR ($CDCl_3$): δ 7.38-7.17 (10H, m), 7.10 (2H, m; NH), 6.96 (1H, d; J=8.3 Hz; NH), 6.87 (1H, d; J=9.1 Hz; NH), 6.63 (1H, d; J=8.4 Hz; NH), 5.50 (1H, d; J=6.4 Hz; BocNH), 4.56 (2H, s; benzylic), 4.53-4.36 (4H, m), 4.39 (2H, m), 4.15 (1H, apparent quartet), 3.84 (1H, dd; J=9.4, 4.6 Hz), 3.74 (3H, s), 3.66 (1H, dd; J=9.4, 5.8 Hz), 3.33 (1H, apparent triplet), 2.94-2.91 (2H, m; benzylic), 2.57 (1H, apparent quartet), 2.26-2.08 (4H, m), 1.95-1.75 (3H, m), 1.67-1.50 (2H, m), 1.45 (9H, s), 1.37 (3H, d; J=7.1 Hz), 1.30 (3H, d; J=7.1 Hz), 0.950-0.892 (12H, overlapping doublets).

(1S,2S)-2-(((1S,2S)-2-(tert-butyloxycarbonyl-L-serylalanylalanyl)-amino-1-hydroxy-3-phenyl)propyl)-cyclopentanecarbonyl-valyl valine methyl ester

f) A solution of the compound of Example 23(d) (25 mg, 28 mmol) in methanol (1 ml) was stirred with 10% Pd/C (12 mg) under an $H_2$ atmosphere for 15 hrs. Filtration and evaporation of the solvent provided the titled compound (20.7 mg, 25.7 mmol; 92% yield) as a white solid.
TLC: (95:5 $CHCl_3$:$CH_3OH$) $R_f$=0.39.
NMR ($CDCl_3$): δ 7.71 (1H, broad; NH), 7.58 (1H, broad d; NH), 7.16 (5H, m), 6.97 (2H, m; NH), 6.12 (1H, broad; NH), 4.75 (1H, broad; BocNH), 4.42 (1H, dd), 4.40-4.04 (5H, m), 3.89 (1H, broad), 3.70 (3H, s), 3.64 (2H, m), 3.03 (1H, dd), 2.82-2.44 (4H, m), 2.23-1.87 (4H, m), 1.80-1.60 (4H, m), 1.45 (9H, s), 1.35 (3H, d; J=6.8 Hz), 1.09 (3H, d; J=6.9 Hz), 0.963 (6H; two doublets), 0.853 (6H, two doublets).
MS (FAB + VE): m/z 805 $(M + H)^+$, 789, 705, 674, 618, 574, 476, 416, 388, 300.
MS (FAB-VE): m/z 803 $(M-H)^-$.

## Example 24

Preparation of (1S,2S)-2-(((1S,2S)-2-(L-seryl-L-alanyl-L-alanyl)-amino-1-hydroxy-3-phenyl)propyl)-cyclopentylcarbonyl-valyl valine methyl ester, trifluoroacetic acid salt

The product of Example 23(f) (1.3 mg, 1.6 mmol), was dissolved in trifluoroacetic acid. After 90 min. the solution was concentrated to dryness under vacuum. The residue was triturated with ether and the ether was removed under vacuum to yield the titled hexapeptide as a white solid.

## Example 25

Preparation of (1S,2S)-2-(((1R,2S)-2-(tert-butyloxycarbonylserylalanylalanyl)amino-1-hydroxy-3-phenyl)-propyl)cyclopentylcarbonyl-L-valyl-L-valine methyl ester

Using the procedure of Example 23(f), the hexapeptide of Example 23(e) (23 mg, 26 mmol) was debenzylated to provide the titled compound as a white solid (18.9 mg, 23.5 mmol; 90% yield).
TLC: (95:5 $CHCl_3$:$CH_3OH$) $R_f$=0.45.
NMR ($CDCl_3$): δ 7.47 (1H, d; J=7.3 Hz; NH), 7.34 (1H, d; J=6.8 Hz; NH), 7.29-7.16 (5H, m), 7.09 (1H, d; J=8.9 Hz, NH), 6.79 (1H, d; J=8.5 Hz; NH), 5.79 (1H, d; J=6.5 Hz; NH), 4.76 (1H, broad), 4.45-4.20 (5H, m), 4.11 (1H, apparent quartet), 3.94 (1H, dd; J=10.9, 4.3 Hz), 3.74 (3H, s), 3.66 (1H, dd), 3.31 (1H, broad doublet), 2.91 (2H, apparent doublet), 2.58 (1H, apparent quartet), 2.44 (2H, broad), 2.28-2.08 (3H, m), 1.95-1.71 (3H, m), 1.59 (2H, m), 1.45 (9H, s), 1.41 (3H, d; J=7.1 Hz), 1.30 (3H, d; J=7.1 Hz), 0.951-0.906 (12H, m).
MS (FAB + VE): m/z 805 $(M + H)^+$, 787, 731, 705, 674, 618, 547, 519, 476.
MS (FAB-VE): m/z 803, 711, 699.

## Example 26

Preparation of (1S,2S)-2-(((1R,2S)-2-(serylalanylalanyl)amino-1-hydroxy-3-phenyl)propyl)-cyclopentylcarbonyl-valyl valine methyl ester, trifluoroacetic acid salt

The compound of Example 25 (1.2 mg, 1.5 mmol) was dissolved in trifluoroacetic acid. After 90 min. the solution was concentrated to dryness under vacuum. The residue was triturated with ether to yield a white solid.

## Example 27

Preparation of (1R,2R)-2-(((1S,2S)-2-(tert-butyloxy-carbonylserylalanylalanyl)amino-1-hydroxy-3-phenyl)-propyl)cyclopentylcarbonyl-valyl valine methyl ester

(1R,2R)-2-(((1RS,2S)-1-hydroxy-2-tert-butyloxycarbonylamino-3-phenyl)propyl)cyclopentylcarbonyl-valyl valine

a) To a solution of the epimers of Example 5 (36 mg, 0.10 mmol) in dioxane (0.4 ml) were added N-hydroxy benzotriazole (27 mg, 0.20 mmol) and DCC (22 mg, 0.11 mmol). After 20 min. stirring, N-methyl morpholine (27 ml, 0.25 mmol) was added, followed by valyl valine methyl ester hydrochloride (53 mg, 0.20 mmol). The mixture was stirred 5 days, then was filtered and concentrated. Flash chromatography (25:1 $CHCl_3/CH_3OH$) provided the titled epimeric compounds in 89% yield as a white foam (51 mg, 0.089 mmol).
TLC (20:1 $CHCl_3:CH_3OH$): $R_f = 0.37$.
NMR ($CDCl_3$): δ 7.35-7.15 (5H, m), 7.05-6.75 (2H, m; NH), 5.13 (1H, m; BocNH), 4.50 (1H, m), 4.30 (1H, m), 3.85-3.60(5H, m), 2.95-2.58(3H, m), 2.43-1.55(8H, m), 1.45-1.25(9H, two singlets; Boc), 1.05-0.90(12H, m).
MS($FAB^+$): m/z 576 $(M+H)^+$, 476$(M-OCOC(CH_3)_3)^+$.

(1R,2R)-2-(((1RS,2S)-1-hydroxy-2-amino-3-phenyl)propyl)cyclopentylcarbonyl-valyl valine methyl ester, hydrochloride

b) The peptides of Example 27(a) (48mg, 83 mmol) were dissolved in trifluoroacetic acid (0.5 ml). After 1 hr. the solution was concentrated by rotary evaporation, and the residue was dissolved in $CH_3OH$, treated with concentrated HCl (0.1 ml) and reconcentrated. Trituration with ether and drying under vacuum provided the epimeric titled compounds as a white powder (42 mg, 82 mmol).

(1R,2R)-2-(((1RS,2S)-2-(tert-butyloxycarbonyl-(O-phenylmethyl)-serylalanylalanyl)amino-1-hydroxy-3-phenyl)propyl)cyclopentyl-carbonyl-valyl valine methyl ester

c) To a solution of Boc-Ser(Bzl)-Ala-Ala (35 mg; 0.080 mmol) in THF (0.4 ml) under argon at -40°C were added N-methyl morpholine (11 ml, 0.10 mmol) and isobutyryl chloroformate (10.4 ml, 0.080 mmol). After 20 minutes stirring, N-methylmorpholine (11 ml) was added, followed by a solution of the compounds of Example 27(b) (42 mg, 0.082 mmol) in THF (1 ml). The mixture was stirred with warming to 20°C overnight, then was dissolved in $CH_2Cl_2$ and washed with 5% HCl, 5% $NaHCO_3$ and water. Evaporation of solvent provided 61 mg of a crude product. A 50 mg portion of the crude product was purified by flash chromatography (gradient elution, 50:1 to 25:1 $CHCl_3:CH_3OH$) to elute pure isomer A (16 mg), followed by a mixed fraction (11 mg), and finally pure isomer B (13 mg).

isomer B:

(1R,2R)-2-(((1S,2S)-2-(tert-butyloxycarbonyl-L-(O-phenylmethyl)serylalanylalanyl)amino-1-hydroxy-3-phenyl)-propyl)cyclopentylcarbonyl-valyl valine methyl ester
TLC: (25:1 $CHCl_3:CH_3OH$) $R_f = 0.40$.
NMR ($CDCl_3$): δ 7.40-6.95 (15H, m; aryl + NH), 5.58 (1H,d; BocNH), 4.53 (2H,s; benzylic), 4.51(1H,dd),

4.37(2H,m), 4.26-4.20 (3H, m), 3.75 (2H, m), 3.71 (3H, s), 3.54(1H, broad), 2.96-2.90 (2H; benzylic), 2.69(1H, m), 2.38 (1H, m), 2.15 (2H, m; $CH(CH_3)_2$), 1.90-1.60 (6H, m), 1.46(9H, s; Boc), 1.37(3H,d; J=7.2 Hz; $CH_3$), 1.20 (3H, d; J=7.2 Hz; $CH_3$), 0.937 - 0.980 (12H, overlapping doublets; $CH_3$).

isomer A

(1R,2R)-2-(((1R,2S)-2-(tert-butyloxycarbonyl-L-(O-phenylmethyl)serylalanylalanyl)amino-1-hydroxy-3-phenyl)-propyl)cyclopentylcarbonyl-valyl valine methyl ester
TLC: (25:1 $CHCl_3$:$CH_3OH$) $R_f$=0.45
NMR ($CDCl_3$): δ 7.79 (14H, d; NH), 7.40-7.10 (11H, m; aryl + NH), 6.70 (2H; NH), 5.59 (1H,d; J=3.3 Hz;NH), 4.93 (1H, d; J=3.2 Hz), 4.54 (2H, s; benzylic), 4.47 (1H, dd; J = 8.5, 5.0 Hz), 4.30-4.20 (4H, m), 4.14 (1H, m), 3.78 (1H, m), 3.71 (3H, s), 3.67 (1H, m), 2.90 (1H, dd; benzylic), 2.77 (2H, m), 2.35-2.10 (4H, m), 1.95-1.55 (6H, m), 1.49 (9H, s; Boc), 1.40 (3H, d; J=7.3 Hz; $CH_3$), 1.12 (3H, d; J=7.3 Hz; $CH_3$), 0.99 (6H, two doublets; $CH_3$), 0.91 (6H, two doublets; $CH_3$).

(1R,2R)-2-(((1S,2S)-2-(tert-butyloxycarbonylserylalanylalanyl)amino-1-hydroxy-3-phenyl)propyl)-cyclopentylcarbonylvalyl valine methyl ester

d) A solution of isomer B of Example 27(c) (13 mg, 15 mmol) in methanol (5 ml) was stirred with 10% Pd/C (10 mg) under an $H_2$ atmosphere for 15 hr. Filtration and evaporation provided the titled peptide (12 mg, 15 mmol) as a white solid.
TLC: (95:5 $CHCl_3$:$CH_3OH$) $R_f$=0.21.
NMR ($CD_3OD$): δ 7.21 (5H, m), 4.35 (1H, m), 4.30-4.13 (4H, m), 4.08 (1H, broad), 3.85 (1H, dd; J=10.8, 5.4 Hz) $CH_2OH$), 3.73 (1H, m), 3.72 (3H, s), 3.58 (1H, apparent t; J=4.3 Hz), 2.92 (1H, dd; J=13.7, 6.2 Hz; benzylic), 2.75 (1H, dd; benzylic), 2.71 (1H, m), 2.37 (1H, broad m), 2.19-2.02 (2H, m; $CH(CH_3)_2$), 1.85-1.60 (6H, m), 1.48 (9H, s; Boc), 1.41 (3H, d; J=7.3 Hz; $CH_3$), 1.21 (3H, d; J=7.3; $CH_3$), 0.934-0.928 (12H; overlapping doublets; $CH_3$).
MS (FAB + VE): m/z 805$(M + H)^+$, 674, 618, 574, 476, 309, 274, 246, 228, 203.

## Example 28

Preparation of (1R,2R)-2-(((1S,2S)-2-(serylalanylalanyl)amino-1-hydroxy-3-phenyl)propyl)-cyclopentylcarbonyl-valyl valine methyl ester, hydrochloride

The product of Example 27(d) (12 mg, 15 mmol) was dissolved in trifluoroacetic acid (0.5 ml). After 90 min the solution was concentrated under vacuum; the residue was dissolved in methanol (1 ml) and conc. HCl (ca .05 ml) was added. The solution was concentrated, the resulting gum was triturated with ether and dried under vacuum to afford the titled compound (11 mg) as a white powder.
NMR ($CD_3OD$): δ 7.23 (5H, m), 4.50-4.25 (3H, m), 4.16 (1H, m), 4.05-3.90 (3H, m), 3.70 (3H, s), 3.51 (1H, dd), 3.00-2.70 (2H, m), 2.56 (1H, m), 2.32 (1H, m), 2.20-1.95 (2H, m), 1.80-1.58 (6H, m), 1.40 (3H, d; J=7.1 Hz), 1.27 (3H, d; J=7.2 Hz), 0.959-0.931 (12H, overlapping doublets).
MS (FAB + VE): m/z 705$(M+H)^+$, 574, 476.

## Example 29

Preparation of (1R,2R)-2-(((1R,2S)-2-(tert-butyloxycarbonylserylalanylalanyl)amino-1-hydroxy-3-phenyl)-propyl)cyclopentylcarbonyl-valyl valine methyl ester

Isomer A of Example 27(c) (15.5 mg, 17.3 mmol) was stirred with 10% Pd/C (10 mg) in methanol (5 ml) under an $H_2$ atmosphere for 15 hr. Filtration and evaporation provided the titled peptide (13.8 mg, 17.2 mmol).
TLC: (95:5 $CHCl_3$:$CH_3OH$) $R_f$=0.27.

NMR ($CD_3OD$):δ 7.22 (5H, m), 4.40 - 4.10 (6H, m), 3.86 (1H, dd; J=11.0, 5.5 Hz; $CH_2OH$), 3.78 (1H, dd; J=11.0, 6.0; $CH_2OH$), 3.72 (3H, s), 3.58(1H, dd; J=10.0, 1.8), 2.98(1H, dd; J=14.1, 3.4 Hz; benzylic), 2.81-2.71 (2H, m), 2.34 (1H, m),2.24-1.60 (8H, m), 1.50 (9H, s), 1.40 (3H, d; J=7.3 Hz; $CH_3$), 1.11 (3H, d; J=7.4 Hz; $CH_3$), 1.02-0.940 (12H, four doublets; $CH(CH_3)_2$).
MS (FAB + VE): m/z 805 $(M + H)^+$, 674, 618, 574, 519, 475, 308, 264.

## Example 30

Preparation of (1R,2R)-2-(((1R,2S)-2-(serylalanyl-L-alanyl)amino-hydroxy-3-phenyl)propyl)cyclopentylcarbonyl-valyl alanine methyl ester, hydrochloride

Using the procedure of Example 28, the hexapeptide of Example 29 (13.8 mg, 17.2 mmol) was treated with trifluoroacetic acid and HCl to provide the titled compound 13 mg).
NMR ($CD_3OD$): δ 7.20 (5H, m), 4.39-4.17 (4H, m), 4.07-3.92 (4H, m), 3.70 (3H, s), 3.52 (1H, dd; J=9.5, 2.9 Hz), 2.98 (1H, dd; J=14.2, 3.4 Hz; benzylic), 2.82-2.68 (2H, m), 2.38 (1H, m), 2.21-1.55 (8H, m), 1.35 (3H, d; J=7.2 Hz), 1.20 (3H, d; J = 7.1 Hz), 1.01-0.889 (12H, four doublets).
MS (FAB + VE): m/z 705 $(M + H)^+$, 575, 476.

## Example 31

Preparation of (1R,2R)-2-(((1RS,2S)-2-(acetyl-serylalanylalanyl)amino-1-hydroxy-3-phenyl)propyl)-cyclopentylcarbonyl-valyl valine methyl ester

(1R,2R)-2-(((1RS,2S)-2-(acetyl-(O-phenylmethyl)serylalanyl alanyl)amino-1-hydroxy-3-phenyl)propyl)-cyclopentylcarbonyl-valyl valine methyl ester

a) A 1:1 mixture of the epimers of the compound in Example 27(c) (10.8 mg, 12.2 mmol) was dissolved in trifluoroacetic acid (0.5 ml). After 75 min. the solution was concentrated under vacuum. The residue was dissolved in $CH_2Cl_2$ and extracted with 5% $NaHCO_3$. The $CH_2Cl_2$ layer was dried over $MgSO_4$ and concentrated to a white solid (8.8 mg, TLC: (25:1 $CHCl_3$:$CH_3OH$) $R_f$=0.13. The solid was stirred as a suspension in 1:1 $CH_2Cl_2$: dioxane (2 ml) and acetic anhydride (1.1 ml, 12 mmol). After 30 min. the solvent was removed by rotary evaporation. The residue was purified by flash chromatography (9:1 $CHCl_3$:$CH_3OH$) to provide the titled epimeric mixture as a white solid (9.2 mg, 11.0 mmol).
TLC: (25:1 $CHCl_3$: $CH_3OH$) $R_f$=0.25, 0.18.
NMR ($CD_3OD$): δ 7.38-7.11 (10H, m), 4.57 (2H, s), 4.53-3.97 (6H, m), 3.78 (2H, apparent triplet), 3.70 (3H, s), 3.68 (1H, m), 3.53 (1H, m), 3.00-2.59 (2H, m), 2.35 (1H, m), 2.18-1.98 (5H, m), 1.95-1.55 (6H, m), 1.37 (3H; overlapping doublets), 1.17-1.07 (3H, two doublets), 0.94 (12H, overlapping doublets).

(1R,2R)-2-(((1RS,2S)-2-(acetyl-serylalanylalanyl)amino-1-hydroxy-3-phenyl)propyl)cyclopentylcarbonyl-valyl valine methyl ester

b) A solution of the epimers of Example 31(a) (9.2 mg, 11.0 mmol) in acetic acid (1 ml), was stirred, with 10% Pd on carbon (6.0 mg) under hydrogen (1 atm.) for 18 hr. The solution was filtered and concentrated under vacuum to provide the titled epimeric mixture as a white solid (8.2 mg, 11.0 mmol).
TLC: (9:1 $CHCl_3$: $CH_3OH$) $R_f$=0.35, 0.27.
NMR ($CD_3OD$): δ 7.21 (5H, m), 4.42-4.09 (6H, m), 3.89-3.60 (2H, m), 3.70 (3H, s), 3.04 (1H, m), 2.99-2.59 (2H, m), 2.34 (1H, m), 2.18-1.99 (5H, m), 1.92-1.55 (6H, m), 1.38 (3H, overlapping doublets), 1.20-1.08 (3H, two doublets), 0.93 (12H, overlapping doublets).
MS (FAB + VE): m/z 747 $(M + H)^+$, 616, 517, 476, 317, 272, 246, 228, 201, 183.

## Example 32

Preparation of trans-2-((1-methoxy-1-(2-phenyl-1-(tertbutyloxycarbonyl-serylalanylalanyl)amino)ethyl)-phosphinyl)cyclopentylcarbonyl-valyl valine methyl ester

trans-2-((1-methoxy-1-(2-phenyl-1-phenylmethoxycarbonylamino)ethyl)phosphinyl)cyclopentylcarbonyl-valyl valine methyl ester

a) Isobutyl chloroformate (13 ml, 0.10 mmol) was added to a stirred solution of the compound of 11(d) (45 mg, 0.10 mmol) and N-methyl morpholine (17 ml, 0.15 mmol) in THF at -40° C. After 30 min. N-methyl morpholine (17 ml, 0.15 mmol) was added, followed by solid valyl valine methyl ester hydrochloride (40 mg. 0.15 mmol). The mixture was allowed to warm to 20° C with stirring. After 14 hr. the mixture was diluted with 5% HCl and extracted with $CH_2Cl_2$. The $CH_2Cl_2$ extract was concentrated and purified by flash chromatography (25:1 $CHCl_3$:$CH_3OH$) to provide the titled compound as a white solid (65 mg, 98.9 mmol) in 99% yield.
TLC: (9:1 $CHCl_3$: $CH_3OH$) $R_f = 0.6$.
NMR ($CDCl_3$): δ 7.33-7.10 (10H, m), 5.08-4.77 (2H, m), 4.70-4.24 (3H, m), 3.82-3.66 (6H, m), 3.41-2.63 (3H, m), 2.24-1.51 (8H, m), 1.03-0.89 (13H, m).

trans-2-((1-methoxy-1-(1-amino-2-phenyl)ethyl)phosphinyl)cyclopentylcarbonyl-valyl valine methyl ester

b) A solution of the compound of Example 32(a) (30 mg, 46 mmol) in methanol (1.5 ml) was stirred with 10% Pd/C (30 mg) under hydrogen (1 atm) for 9 hr. The mixture was filtered and concentrated under vacuum to provide the titled compound as a glass (20 mg, 38 mmol; 84% yield).
NMR ($CDCl_3$): δ 7.38-7.18 (5H, m), 4.51 (1H, m), 4.30 (1H, m), 3.90-3.68 (6H, m), 3.44-3.03 (2H, m), 2.93-2.48 (1H, m), 2.25-1.67 (8H, m), 1.27 (1H, m), 1.01-0.85 (13H, m).

trans-2-((1-methoxy-1-(2-phenyl-1-(tert-butyloxycarbonyl-(O-phenylmethyl)serylalanylalanyl)amino)ethyl)-phosphinyl)cyclope ntyl-carbonyl-valyl valine methyl ester

c) By a procedure substantially similar to Example 27(c), using the compound of Example 32(b) (20 mg, 38 mmol), Boc-Ser(Bzl)-Ala-Ala (18.4 mg, 42.0 mmol), N-methylmorpholine (7.0 ml, 63 mmol) and isobutyryl chloroformate (5.5 ml, 42 mmol), and subsequent flash chromatography (50:1 $CHCl_3$:$CH_3OH$), the titled compound was obtained as a glass (23.5 mg, 24.9 mmol; 65% yield).
TLC: (9:1 $CHCl_3$:$CH_3OH$) $R_f = 0.5$.
NMR ($CDCl_3$): δ 7.39-7.13 (10H, m), 4.80-4.20 (8H, m), 3.90-3.60 (8H, m), 3.45-2.70 (3H, m), 2.22-1.64 (8H, m), 1.50-1.25 (12H, m), 1.10 (3H, m), 1.04-0.85 (13H, m).

trans-2-((1-methoxy-1-(2-phenyl-1-(tert-butyloxycarbonylserylalanylalanyl)amino)ethyl)phosphinyl)-cyclopentylcarbonylvalyl valine methyl ester

d) Using the procedure of Example 27(d), the above compound of Example 32(c) (23 mg, 24 mmol) was hydrogenolyzed to provide the titled compound (19.1 mg, 22 mmol; 93% yield) as a white solid.
NMR ($CD_3OD$): δ 7.32-7.16 (5H), 4.75-4.10 (6H), 3.87-3.66 (8H), 3.32-2.72 (3H), 2.20-1.62 (8H), 1.50-1.28 (12H), 1.13 (3H), 1.05-0.85 (13H).
MS (FAB + VE): 853 $(M + H)^+$, 753.

## Example 33

Preparation of trans-2-((1-hydroxy-1-(2-phenyl-1-(serylalanylalanyl)amino)ethyl)phosphinyl)-cyclopentylcarbonylvalyl valine methyl ester hydrobromide

Trimethylsilyl bromide (4.0 ml, 30 mmol) was added to a solution of the compound of Example 32(d) (9.6 mg, 11.3 mmol) in dry $CH_2Cl_2$ (1 ml). After 4.5 hr 1:1 water:acetic acid (0.5 ml) was added. The mixture was concentrated and dried to a solid under vacuum. The residue was dissolved in methanol (1 ml) and treated with 30% HBr in acetic acid (50 ml), then reconcentrated and dried under vacuum to provide the titled compound (9 mg) as a light yellow powder.
NMR ($CD_3OD$): δ 7.30-7.15 (5H, m), 4.55-3.92 (5H, m), 3.83-3.66 (5H, m), 3.30-2.08 (4H, m), 2.20-1.64 (8H, m), 1.47-1.25 (4H, m), 1.12 (3H, m), 1.05-0.90 (12H, m).
MS (FAB + VE): m/z 739, $(M\text{-}HBr + H)^+$, 260, 232, 159.

## Example 34

Preparation of (5S,4RS)-5-(tert-butyloxycarbonylserylalanylalanyl)-amino-4-hydroxy-1-oxo-6-phenylhexyl-valyl valine methyl ester

(5S)-5-(t-butyloxycarbonylamino)-1,4-dioxo-6-phenylhexylvalyl valine methyl ester

a) To a solution of the compound of 12(b) (160 mg, .495 mmol) in THF (5 ml) at -40° C under argon was added N-methyl morpholine (66 mg, .66 mmol), followed by isobutyl chloroformate (68 mg, .495 mmol). The reaction mixture was stirred for 15 minutes, then N-methyl morpholine (66 mg, .66 mmol) was added, followed by a solution of valyl valine methyl ester hydrochloride (120 mg, .45 mmol) in THF (6 ml) in a dropwise manner. The mixture was stirred at -40° C for 30 min, then at 20° C for 17 hours. The reaction mixture was then diluted with 1:1 ethyl acetate:dichloromethane (100 ml) and washed successively with 5% HCl, 5% sodium bicarbonate, and saturated aqueous sodium chloride. The organic extracts were dried ($MgSO_4$), filtered, and evaporated under reduced pressure to an oil. Flash chromatography over silica gel-(25:1 chloroform:methanol) provided the titled compound 160 mg; 70% yield) as a white foam.
NMR ($CDCl_3$): δ 7.25(5H,m), 6.34(1H,d), 6.15(1H,d), 5.24(1H,d), 4.58(2H,m), 4.31(1H,dd), 3.78(3H,s), 3.19-(1H,m), 2.95(1H,m), 2.87(2H,m), 2.52(2H,t), 2.21(2H,pentet),1.43(9H,s), 0.98(12H,m).
MS ($FAB^+$): m/z 534;
MS ($FAB^-$): m/z 532

(5S,4RS)-5-(tert-butyloxycarbonyl)amino-4-hydroxy-1-oxo-6-phenylhexyl-valyl valine methyl ester)

b) To a solution of the compound of Example 34(a) (160 mg, 3.0 mmol) in methanol at 0° C was added sodium borohydride (5.7 mg, 1.5 x $10^{-4}$ mole) in portions. After 20 min. the mixture was diluted with 5% HCl and extracted (3x) with dichloromethane. The organic extracts were dried with $MgSO_4$, filtered, and evaporated to white flakes. Flash chromatography over silica gel(40:1 chloroform:methanol) yielded the titled compound as white flakes (112 mg,70%).
NMR ($CDCl_3$/MeOD): δ 7.15(5H,m); 4.38(1H,dd); 4.10(1H,m); 3.63(3H,s); 3.0 to 1.5(8H,m); 1.28(9H,s); 0.88-(12H,m).

(5S,4RS)-5-(tert-butyloxycarbonyl-(O-phenylmethyl)serylalanyl-alanyl)amino-4-hydroxy-1-oxo-6-phenylhexyl-valyl valine methyl ester

c) The peptide of Example 34(b) (98 mg, .183 mmol) was dissolved in neat trifluoroacetic acid (3 ml) and stirred at room temperature for two hours. The solution was then concentrated under reduced pressure, dissolved in methanol, treated with concentrated HCl (3 drops), and evaporated under reduced pressure to give 86 mg of the tripeptide amine hydrochloride as white flakes.

To a solution of Boc-Ser(OBz)-Ala-Ala (87.4 mg, .20 mmol) in THF (5 ml) at -40° C under argon, N-methyl morpholine (25 ml, 0.27 mmol) was added, followed by isobutyl chloroformate (27 mg, 0.20 mmol).

The mixture was stirred for 15 minutes, then N-methylmorpholine (25 ml, 0.27 mmol) was added, followed by the previously prepared 5-amino-4-hydroxy-1-oxo-6-phenylhexyl-valyl valine hydrochloride (86 mg, 0.18 mmol) in THF (1 ml) dropwise. The mixture was stirred at -40 °C for 30 minutes, then at 20 °C for 17 hours. The reaction mixture was diluted with 1:1 ethyl acetate:dichloromethane (100 ml) and washed successively with 5% HCl, 5% aqueous sodium bicarbonate, saturated aqueous sodium chloride, and evaporated to a white solid. The solid was purified by flash chromatography over silica gel (20:1 chloroform:methanol) to yield the titled hexapeptide as white crystals (98 mg; 63% yield).

NMR ($CDCl_3/CD_3OD$): δ 7.1 (5H, m), 6.95 (5H, m), 3.48 (3H, s), 1.29 (9H, s), 1.05 (6H, m), 4.2-1.5 (m), 0.70 (12H, m).

MS ($FAB^+$): m/z 855;

MS ($FAB^-$): m/z 853

(5S,4RS)-5-((tert-butoxycarbonyl-serylalanylalanyl)amino)-4-hydroxy-1-oxo-6-phenylhexyl-valyl valine methyl ester

d) To a solution of the compound of Example 34(c) (31 mg, 36.2 mmol) in 9:1 methanol:acetic acid, 10% Pd/C (35 mg) was added. The mixture was stirred under $H_2$ (1 atm) for 6 hours. The reaction mixture was filtered through a pad of Celite® and the Celite® was rinsed successively with acetic acid, methanol, and ethyl acetate. The combined solution was evaporated under reduced pressure to yield the titled compound as white crystals (21 mg; 75% yield).

NMR ($CDCl_3/CD_3OD$): δ 7.14 (5H, m), 4.33 (1H,m), 4.08 (4H, m), 3.60 (3H, s), 2.95 (1H, dd), 2.70 (1H, m), 2.35 (1H, m), 2.06 (2H, m), 1.72 (1H, m), 1.40 (9H, s), 1.22 (6H, m); 0.85 (12H, m).

MS ($FAB^+$): m/z 765;

MS ($FAB^-$): m/z 764.

## Example 35

### Preparation of (4RS,5S)-5-(tert-butyloxycarbonylserylalanylalanyl)amino-4-hydroxy-1-oxo-6-(4-hydroxyphenyl)hexyl-valyl valine methyl ester

(5S)-5-(tert-butyloxycarbonylamino)-1,4-dioxo-6-(4-benzyloxyphenyl)hexyl-valyl valine methyl ester

a) To a solution of the compound of Example 13(c) (810 mg, 1.9 mmol) in THF (20 ml) at -40°C under an argon atmosphere was added N-methyl morpholine (0.27 ml, 2.5 mmol), followed by isobutyl chloroformate (0.246 ml, 1.9 mmol). The reaction mixture was stirred for 15 min, and additional N-methyl morpholine (0.27 ml, 2.5 mmol) was added followed by valyl valine methyl ester hydrochloride salt (459 mg, 1.7 mmol). The resulting mixture was stirred at -40°C for 30 min, then allowed to warm to room temperature and stirred for 17 h. The reaction mixture was diluted with 1: 1 ethyl acetate: $CH_2Cl_2$ and washed successively with 5% HCl, 5% aqueous $NaHCO_3$, and saturated aqueous NaCl. The solution was dried ($MgSO_4$), filtered and evaporated under reduced pressure. The solid residue was purified by column chromatography using silica gel, eluting with 40:1 $CHCl_3$: MeOH to give the titled compound (838 mg, 76%).

NMR ($CDCl_3$): δ 7.4 (5H, m), 7.02 (4H, dd), 6.42 (1H, br d), 6.18 (1H, br d), 5.19 (1H, br d), 5.0 (2H, s), 4.51 (2H, m), 4.25 (1H, m), 3.70 (3H, s), 3.04 (1H, m), 2.82 (3H, m), 2.48 (2H, m), 2.15 (2H, m), 1.39 (9H, s), 0.91 (12H, m).

(4RS 5S)-5-(tert-butyloxycarbonyl)amino-4-hydroxy-1-oxo-6-(4-benzyloxyphenyl)hexyl-valyl valine methyl ester

b) To a solution of the compound of Example 35(a) (144 mg, .225 mmole) in methanol at 0 °C, sodium borohydride (4.28 mg, .113 mmole) was added in portions. After 20 min. the mixture was diluted with 5% HCl and extracted (3x) with dichloromethane. The organic extracts were dried ($MgSO_4$), filtered, and evaporated to a white solid. The crude product was purified by flash chromatography over silica gel(20:1

chloroform:methanol)to yield the tripeptide as white crystals (118 mg; 82% yield).
NMR ($CDCl_3$): δ 7.4 (5H, m), 7.02 (4H, dd), 6.40 (2H, m), 5.03 (2H, s), 4.54 (2H, m), 4.30 (1H, m), 3.70 (3H, s), 3.61 (2H, m), 3.0 to 1.6 (11H, m), 1.38 (9H, s), 0.91 (12H, m).

(4RS,5S)-5-(tert-butyloxycarbonyl-(O-phenylmethyl)serylalanylalanyl)amino-4-hydroxy-1-oxo-6-(4-benzyloxyphenyl)hexyl-valyl valine methyl ester

c) The tripeptide of Example 35(b) (115 mg, .179 mmol) is treated with trifluoroacetic acid and HCl according to the procedure of Example 34(c). The resulting hydrochloride salt was coupled to Boc-Ser(Bzl)-Ala-Ala (83 mg, .19 mmol) using N-methyl morpholine (50 mg, .51 mmol), and isobutylchloroformate (26 mg, .19 mmol). The titled hexapeptide was obtained as white crystals (40 mg; 25% yield).
NMR ($CDCl_3/CD_3OD$): δ 7.35 (10H, m), 6.91 (4H, dd), 4.95 (2H, d), 4.48 (2H, d), 4.37 (1H, d), 4.07 (4H, m), 3.62 (4H, s), 3.60 (1H, obsc m), 2.5 to 1.5 (3H, m), 1.38 (9H, s), 1.2 (6H, m), 0.80 (12H, m).

(4RS,5S)-5-(tert-butoxycarbonyl-serylalanylalanyl)amino-4-hydroxy-1-oxo-6-(4-hydroxy-phenyl)hexyl-valyl valine methyl ester

d) The compound of Example 35(c) (39 mg, 40.6 mmol) was deprotected by the hydrogenolysis procedure of Example 34(d). The titled compound was obtained as white crystals (2.3 mg; 73% yield).
NMR ($CDCl_3/CD_3OD$): δ 6.79 (4H, m), 4.35 (1H, q), 4.2 to 3.8 (5H, m), 3.67 (3H, s), 3.0 to 2.5 (2H, m), 2.5 to 1.5 (4H, m), 1.41 (9H, s), 1.20 (6H, m), 0.89 (12H, m).

## Example 36

### Preparation of (3R,4S)-4-(tert-butyloxycarbonylserylalanylalanyl)-amino-2,2-difluoro-3-hydroxy-1-oxo-5-phenylpentyl-valyl valine methyl ester

(3R,4S)-4-(benzyloxycarbonyl)amino-2,2-difluoro-3-hydroxy-5-phenylpentanoyl-valyl valine methyl ester

a) Triethylamine(0.66 ml, 4.83 mmol) was added to a solution of the compound of Example 14(b) (0.82 g, 2.20 mmol), benzotriazol-1-yloxy tris(dimethylaminophosphonium hexafluorophosphate (1.07 g, 2.41 mmol) and valyl valine methyl ester hydrochloride (0.64g, 2.41 mmol) in 75 ml of dry acetonitrile. After 4 hr. of stirring, additional neat triethylamine (0.66 ml, 4.83 mmol) was introduced and the reaction mixture was stirred overnight. The solution was diluted with ethyl acetate and washed successively with 2N HCl, 5% $NaHCO_3$, and $H_2O$. After drying over $MgSO_4$ and filtering, the solution was concentrated *in vacuo*. Flash chromatography with 40:1 $CHCl_3$:methanol as the eluent afforded the titled tripeptide as a white flakey solid (466 mg; 36% yield).
NMR ($CDCl_3$):δ 7.34 (10H, m), 6.93 (1H, br, J=7.1 Hz), 6.75 (1H, br d, J=8.9 Hz), 5.03 (2H, s), 4.87 (1H, m), 4.60 (1H, m), 4.38 (3H, m), 3.63 (3H, s), 3.07 (2H, m), 2.32 (1H, m), 2.14 (1H, m), 1.93 (1H, m), 0.98 (12H, m)
MS: m/z 592

(3R,4S)-4-amino-2,2-difluoro-3-hydroxy-1-oxo-5-phenylpentylvalyl-valine methyl ester, hydrochloride

b) A solution of the tripeptide of Example 36(a) (466 mg, 0.79 mmol) in glacial acetic acid(40 ml) containing 10% Pd/C (450 mg) was saturated with $H_2$ gas and stirred overnight under an atmosphere of $H_2$. After filtering through a pad of Celite®, the solution was diluted with methanol and treated with concentrated HCl (100 ul, 1.0 mmol). Concentration of the solution *in vacuo* yielded the crude tripeptide hydrochloride as a fluffy white crystalline solid (390 mg, 100%).

(3R,4S)-4-(tert-butyloxycarbonyl-(O-benzyl)serylalanylalanyl)amino-2,2-difluoro-3-hydroxy-1-oxo-5-phenylpentylvalyl valine methyl ester

c) To a solution of Boc-Ser(Bzl)-Ala-Ala (380 mg, 0.87 mmol) and N-methyl morpholine (108 ul, 0.99 mmol) in dry THF (6 ml) at -40 °C was added isobutyl chloroformate (112 ml, 0.87 mmol) in a dropwise manner. After stirring for 30 min., N-methyl morpholine (112 ml, 0.87 mmol) was added followed by addition of the compound of Example 36(b) as a solid. The reaction mixture was stirred overnight with gradual warming to room temperature. The solution was diluted with $CH_2Cl_2$ and washed successively with 10% HCl, 5% $NaHCO_3$ and $H_2O$. The organic portion was dried over $MgSO_4$, filtered and concentrated in vacuo. Flash chromatography with 30:1 $CHCl_3$:methanol as the eluent provided the titled hexapeptide as a light yellow solid (636 mg, 83%).
NMR (DMSO-$d_6$): δ 8.31 (1H, brd; J=7.1 Hz), 8.12 (1H, brd; J=9.5 Hz), 7.98 (1H, brd; J=6.5 Hz), 7.78 (2H, m), 7.31 (5H, m), 7.18 (5H, m), 6.99 (1H, brd; J=7.1 Hz), 6.28 (1H, m), 4.48 (2H, s), 4.22 (4H, m), 3.62 (3H, s), 3.57 (1H, m), 2.98 (1H, m), 2.71 (1H, m), 2.17 (2H, m), 1.41 (9H, s), 1.15 (3H, d; J=6.5 Hz), 1.03 (3H, d; J=7.1 Hz), 0.92 (12H, m).
MS (FAB +): m/Z=877 ($M+H^+$)

(3R,4S)-4-(tert-butyloxycarbonyl-serylalanylalanyl)amino-2,2-difluoro-3-hydroxy-1-oxo-5-phenylpentyl-valyl-L-valine methyl ester

d) The protected hexapeptide of Example 36(c) (13 mg, 15 mmol) was dissolved in 5:1 acetic acid:methanol (6 ml). To this solution, 10% Pd/C (15 mg) was added and the mixture was saturated with $H_2$ gas. After stirring overnight under an atmosphere of $H_2$, the solution was filtered through a pad of Celite® and concentrated in vacuo to provide the titled product as a white crystalline solid (11.5 mg, 99%).
NMR ($CDCl_3$/$CD_3OD$): δ 7.24 (5H, m), 4.18 (6H, m), 3.77 (1H, m), 3.65 (3H, s), 3.52 (1H, m), 3.09 (1H, m), 2.87 (1H, m), 2.03 (3H, m), 1.39 (9H, s), 1.31 (3H, d, J=5.94 Hz), 1.06 (3H, d, J=6.6 Hz), 0.90 (12H, m)
MS: m/z 787

## Example 37

Preparation of (3R,4S)-4-(serylalanylalanyl)amino-2,2-difluoro-3-hydroxy-1-oxo-5-phenylpentyl-valyl valine methyl ester, trifluoroacetic acid salt

Two drops of TFA were added to the N-protected hexapeptide of Example 36(d) (1.1 mg, 1.4 mmol). After 2 hr., a slow stream of Argon was passed over the mixture to evaporate the excess TFA. Three drops of diethyl ether were added and evaporated in the same manner three times. The residue was dried in vacuo.

## Example 38

Preparation of 4-(tert-butyloxycarbonylserylalanylalanyl)amino-2,2-difluoro-1,3-dioxo-5-phenylpentyl-valyl valine methyl ester

4-(tert-butyloxycarbonyl-(O-benzyl)serylalanylalanyl)amino-2,2-difluoro-1,3-dioxo-5-phenylpentyl-valyl valine methyl ester

a) To a solution of α,α-difluoro alcohol of Example 37 (102 mg, 0.12 mmol) and Dess-Martin periodinane (247 mg, 0.58 mmol) in 2 ml of dry DMF was introduced neat TFA (45 ul, 0.58 mmol) in a dropwise manner. After stirring for 16 h., the solution was diluted with $Et_2O$ and poured over a mixture of saturated aqueous $NaHCl_3$ (20 ml) and 10% aqueous sodium thiosulfate (7 ml). This mixture was stirred vigorously for

1 hr and extracted with ethyl acetate. The combined extracts were dried over $MgSO_4$, filtered, and concentrated in vacuo. Flash chromotography using a gradient elution of 0-30% methanol in chloroform yielded the titled α,α-difluoro ketone as a white solid (70 mg, 69% yield).

NMR ($CDCl_3/CD_3OD$): δ 7.27 (10H, m), 4.50 (2H, s), 4.34 (6H, m), 3.71 (3H, s), 3.66 (2H, m), 3.25 (1H, m), 2.99 (1H, m), 2.24 (2H, m), 1.42 (9H, s), 1.34 (3H, m), 1.08 (3H, m), 0.92 (12H, m).

MS (FAB +): m/z 875 ($M+H^+$).

4-(tert-butyloxycarbonyl-serylalanylalanyl)amino-2,2-difluoro-1,3-dioxo-5-phenylpentyl-valyl valine methyl ester

b) A solution of the protected hexapeptide of Example 38(a) (15 mg, 17 µmol ) in glacial acetic acid (4 ml) containing 10% Pd/C (15 mg) was saturated with $H_2$ gas and stirred overnight under an atmosphere of $H_2$ gas. The mixture was filtered through a pad of Celite® and concentrated in vacuo. Flash chromatography using a gradient elution of 0-50% methanol in chloroform provided the titled debenzylated product as a white crystalline solid (9 mg, 67%).

NMR (DMSO-$d_6$): δ 9.01 (1H, m), 8.36 (2H, m), 7.95 (2H, m), 7.17 (5H, m), 6.68 (1H, m), 4.85 (1H, m), 4.18 (4H, m), 3.91 (1H, m), 3.58 (3H, s), 3.45 (2H, m), 3.08 (2H, m), 2.66 (1H, m), 2.00 (2H, m), 1.45 (9H, s), 1.11 (6H, m), 0.88 (12H, m).

MS (FAB +): m/z 785 ($M+H^+$).

## Example 39

### Preparation of Ac-Ser-Gln-Asn-Tyr-Pro-Val-Val-$NH_2$

The protected heptapeptide resin Boc-Ser(Bzl)-Gln-Asn-Tyr(BrZ)-Pro-Val-Val-BHA was prepared in the usual manner according to Example 1. After removal of the N-terminal Boc group with 50% TFA in $CH_2Cl_2$ and neutralizing the resulting TFA salt with 7% DIEA in $CH_2Cl_2$, the resin-bound peptide was acetylated with acetic anhydride (2 ml) in $CH_2Cl_2$ (30 ml) for 30 minutes.

The peptide was cleaved from the resin with removal of the side chain protecting groups by treatment with anhydrous liquid HF (10 ml) in the presence of anisole (1 ml) at 0°C for 50 minutes. After removal of the HF under vacuum, the resin was washed with ethyl ether and air-dried. The resin was then extracted with 2 x 30 ml 1% $HOAc/H_2O$ followed by 2 x 30 ml 10% $HOAc/H_2O$. The combined extracts were lyophilized to yield 318 mg crude peptide.

An aliquot of 130 mg crude peptide was purified by gel filtration on a 2.6 x 70 cm column of G-15 Sephadex® using 1% HOAc as eluant. The appropriate fractions were pooled and lyophilized to yield 120 mg of the titled compound.

TLC: (n-BuOH/HOAc/$H_2O$ 4:1:1) $R_f=0.34$; (n-BuOH/EtOAc/HOAc/$H_2O$ 1:1:1:1) $R_f=0.66$; HPLC $k'=3.0$ (Hamilton PRP-1, $CH_3CN/H_2O$/0.1% TFA, 10% to 40% $CH_3CN$, 15 minutes, linear gradient, 1.5 ml/min.); FAB-MS: m/z 847.6 ($M+H^+$).

Substituting Boc-Thr(Bzl) for Boc-Ser(Bzl) in the above method yields Ac-Thr-Gln-Asn-Tyr-Pro-Val-Val-$NH_2$.

## Example 40

### Preparation of Ac-Ser-Ala-Ala-Tyr-Pro-Val-Val-$NH_2$

The protected heptapeptide resin Boc-Ser(Bzl)-Ala-Ala-Tyr(BrZ)-Pro-Val-Val-BHA was prepared in the usual manner. After removal of the N-terminal Boc group with 50% TFA in $CH_2Cl_2$ and neutralizing the resulting TFA salt with 7% DIEA in $CH_2Cl_2$, the resin-bound peptide was acetylated with acetic anhydride (2 ml) in $CH_2Cl_2$ (30 ml) for 30 minutes.

The peptide was cleaved from the resin with removal of the side chain protecting groups by treatment

with anhydrous liquid HF (10 ml) in the presence of anisole (1 ml) at 0° C for 50 minutes. After removal of the HF under vacuum, the resin was washed with ethyl ether and air-dried. The resin was then extracted with 2 x 30 ml 1% HOAc/$H_2O$ followed by 2 x 30 ml 10% HOAc/$H_2O$. The combined extracts were lyophilized to yield 599 mg crude peptide.

The crude peptide was purified by countercurrent distribution using the system n-BuOH/HOAc/$H_2O$ 4:1:5. The appropriate fractions were pooled, evaporated to dryness and the residue lyophilized from 1% HOAc to yield 435 mg partially purified peptide. An aliquot of 100 mg partially purified peptide was further purified by gel filtration on a 2.6 x 70 cm G-15 Sephadex® column using 1% HOAc as eluant. The appropriate fractions were pooled and lyophilized to yield 95 mg of the purified title compound.

TLC: (n-BuOH/HOAc/$H_2O$ 4:1:1) $R_f$=0.72; HPLC k′=2.8 (Hamilton PRP-1, $CH_3CN/H_2O$/0.1% TFA, 5% to 40% $CH_3CN$, 15 minutes, linear gradient, 1.5 ml/min.);
FAB-MS: m/z 747 (M+H$^+$).

## Example 41

### Preparation of Ac-Ser-Gln-Asn-Tyr-D-Pro-Val-Val-$NH_2$

One mmole of the protected peptidyl resin Ac-Ser(Bzl)-Gln-Asn-Tyr(BrZ)-D-Pro-Val-Val-BHA was prepared as above. The peptide was cleaved from the resin by treatment with 10 ml HF / 1 ml anisole at 0° for 50 min. After removal of the HF under vacuum, the resin was washed with ether, air dried and extracted with 3 x 30 ml glacial HOAc. The HOAc extracts were combined and lyophilized to yield 406 mg crude peptide.

The crude peptide was purified by countercurrent distribution in nBuOH-HOAc-$H_2O$ (4:1:5). The appropriate fractions were located by tlc, pooled, evaporated to dryness and the residue lyophilized from 1% HOAc, yielding 260 mg purified peptide.

TLC: (n-BuOH-HOAc-$H_2O$ 4:1:1), $R_f$=0.15; (n-BuOH-HOAc-$H_2O$ 1:1:1), $R_f$=0.54; (n-BuOH-EtOAc-HOAc-$H_2O$ 1:1:1:1), $R_f$=0.72
FAB-MS: m/z 847 $(M+H)^+$
Amino Acid Analysis (hydrolysis in HCl/TFA 2:1 containing .005% w/v phenol at 160° for one hr): Asp, 1.00; Ser, 0.75; Glu, 0.99; Pro, 0.99; Val, 0.96; Tyr, 1.10

## Example 42

### Preparation of Dns-Ser-Gln-Asn-Tyr-Pro-Val-Val-$NH_2$

One-half mmole of the protected peptidyl resin Dns-Ser(Bzl)-Gln-Asn-Tyr(BrZ)-Pro-Val-Val-BHA was prepared as above. The peptide was cleaved from the resin by treatment with 10 ml HF / 1 ml anisole at 0° for 50 min. After removal of the HF under vacuum, the resin was washed with ether, air dried and extracted with 30 ml 10% HOAc followed by 2 x 30 ml 1% HOAc. The HOAc extracts were combined and lyophilized to yield 290 mg crude peptide.

The crude peptide (73 mg) was purified by gel filtration on Sephadex® G-15 using 10% HOAc as eluant. The appropriate fractions were lyophilized, yielding 56 mg purified peptide.

TLC: (n-BuOH-HOAc-$H_2O$ 4:1:1), $R_f$=0.15
HPLC (Hamilton PRP-1, $CH_3CN$ / $H_2O$ / 0.1% TFA, gradient of 5-40% CH3CN, 15 min), k′ 3.32.
FAB-MS: m/z 1038 $(M+H)^+$
Amino Acid Analysis (hydrolysis in 6N HCl at 110° for 24 hr): Asp, 1.00; Ser, 0.17; Glu, 1,00; Pro, 1.00; Val, 1.38; Tyr, 1.78

## Example 43

Preparation of Ac-Ser-Gln-Asn-Tyr-Pro-Val-Arg-$NH_2$

One mmole of the protected peptidyl resin Ac-Ser(Bzl)-Gln-Asn-Tyr(BrZ)-Pro-Val-Arg(Tos)-BHA was prepared as above. The peptide was cleaved from the resin by treatment with 10 ml HF / 1 ml anisole at 0° for 50 min. After removal of the HF under vacuum, the resin was washed with ether, air dried and extracted with 3 x 30 ml glacial HOAc. The HOAc extracts were combined and lyophilized to yield 639 mg crude peptide.

The crude peptide was purified by countercurrent distribution in nBuOH-HOAc-$H_2O$ (4:1:5). The appropriate fractions were located by tlc, pooled, evaporated to dryness and the residue lyophilized from 1% HOAc, yielding 518 mg partially purified peptide. A 100 mg aliquot of the partially purified peptide was further purified by gel filtration on G-15 with 1% HOAc as eluant. The appropriate fractions were pooled and lyophilized, yielding 61.5 mg purified peptide.

TLC: (n-BuOH-pyridine-HOAc-$H_2O$ 15:10:3:12), $R_f = 0.43$; (nBuOH-EtOAC-HOAc-$H_2O$ 1:1:1:1), $R_f = 0.34$;
HPLC (Hamilton PRP-1, $CH_3CN$ / $H_2O$ / 0.1% TFA, gradient of 5-40% CH3CN, 15 min), k′ 1.94.
FAB-MS: m/z 904 $(M+H)^+$
Amino Acid Analysis (hydrolysis in 6N HCl at 110° for 18 hr): Asp, 1.00; Ser, 0.98; Glu, 1.00; Pro, 0.88; Val, 0.56; Tyr, 0.86; Arg, 0.67

## Example 44

Preparation of Ac-Ser-Gln-Ala-Tyr-Pro-Val-Val-$NH_2$

One-half mole of the protected peptidyl resin Ac-Ser(Bzl)-Gln-Ala-Tyr(BrZ)-Pro-Val-Val-BHA was prepared as above. The peptide was cleaved from the resin by treatment with 10ml HF/1ml anisole 1 hr at 0° C. The peptidyl resin was washed with ether and the peptide was extracted with glacial acetic acid. The extract was lyophilized to afford 220 mg, 55% yield. 100mg of crude peptide was purified by gel filtration on Sephadex® G-15 using 1% HOAc as eluant, yielding 85 mg.
Gradient HPLC: 5% to 40%, 0.1% TFA($CH_3CN$), linear, 15min, k′ = 2.61
TLC: (B:A:W 1:1:1), $R_f = 0.67$.
FAB-MS: m/z 804 $(M+H)^+$
Amino Acid Analysis: (HCl:TFA 2:1, 0.005% w/v phenol, 160° C, 1 hr.), Ser 0.89, Glu 1.00, Pro 1.04, Ala 1.00, Val 1.75, Tyr 0.89. Peptide Content is 98.94%.

## Example 45

Preparation of Ac-Ser-Ser-Asn-Tyr-Pro-Val-Val-$NH_2$

One-half mole of the protected peptide resin Ac-Ser)(Bzl)-Ser(Bzl)-Asn-Tyr(BrZ)-Pro-Val-Val-BHA was prepared as above. The peptide was cleaved from the resin by treatment with 10ml HF/1ml anisole 1 hr at 0° C. The peptidyl resin was washed with ether and the peptide was extracted with glacial acetic acid. The extract was lyophilized to afford 283 mg, 70% yield.100mg of crude peptide was purified by gel filtration on Sephadex® G-15 using 1% HOAc as eluant, yielding 78 mg.
Gradient HPLC: 5% to 40%, 0.1% TFA($CH_3CN$), linear, 15min, k′ = 2.58
TLC: rf = 0.70, B:A:W 1:1:1
FAB-MS: m/z 806 $(M+H)^+$
Amino Acid Analysis: (HCl:TFA 2:1, 0.005% w/v phenol, 160° C, 1 hr.), Asp 1.00, Ser 1.59, Pro 0.90, Val 1.91, Tyr 0.91. Peptide Content was 80.6%.

## Example 46

Preparation of Ac-Ser-Arg-Asn-Tyr-Pro-Val-Val-$NH_2$

One-half mmole of the protected peptidyl resin Ac-Ser(Bzl)-Arg-Asn-Tyr(BrZ)-Pro-Val-Val-BHA was prepared as above. The peptide was cleaved from the resin by treatment with 10ml HF/1ml anisole 1 hr at 0° C. The peptidyl resin was washed with ether and the peptide was extracted with glacial acetic acid. The extract was lyophilized to afford 348.5mg, 80% yield. 100mg of crude peptide was purified by gel filtration on Sephadex® G-15 using 1% HOAc as eluant, yielding 87 mg.
Gradient HPLC: 5% to 40%, 0.1% TFA($CH_3CN$), linear, 15min, $k' = 2.64$
TLC: (B:A:W 1:1:1), $R_f = 0.60$
FAB-MS: m/z 875 $(M+H)^+$
Amino Acid Analysis: HCl/TFA/Phenol, Asp 1.00, Ser 0.80, Pro 0.83, Val 1.85, Tyr 0.94, Arg 0.97. Peptide Content was 101.3%.

## Example 47

Preparation of Ac-Ser-Lys-Asn-Tyr-Pro-Val-Val-$NH_2$

One-half mmole of the protected peptidyl resin Ac-Ser(Bzl)-Lys-Asn-Tyr(BrZ)-Pro-Val-Val-BHA was prepared as above. The peptide was cleaved from the resin by treatment with 10ml HF/1ml anisole 1 hr at 0° C. The peptidyl resin was washed with ether and the peptide was extracted with glacial acetic acid. The extract was lyophilized to afford 343.79mg, 81% yield.
100mg of crude peptide was purified by gel filtration on Sephadex® G-15 using 1% HOAc as eluant, yielding 87 mg.
Gradient HPLC: 5% to 40%, 0.1% TFA($CH_3CN$), linear, 15min, $k' = 2.47$
TLC: (B:A:W 1:1:1), $R_f = 0.60$
FAB-MS: m/z 847 $(M+H)^+$
Amino Acid Analysis: (HCl:TFA 2:1, 0.005% w/v phenol, 160° C, hr.), Asp 1.00, Ser 0.81, Pro 0.95, Val 1.87, Tyr 1.00, Lys 0.84. Peptide Content was 92.3%.

## Example 48

Preparation of Ac-Ser-Glu-Asn-Tyr-Pro-Val-Val-$NH_2$

One-half mmole of the protected peptidyl resin Ac-Ser(Bzl)-Glu(OBzl)-Asn-Tyr(BrZ)-Pro-Val-Val-BHA was prepared as above. The peptide was cleaved from 10ml HF/1ml anisole 1 hr at 0° C. The peptidyl resin was washed with ether and the peptide was extracted with glacial acetic acid. The extract was lyophilized to afford 286.22mg, 67% yield. 100mg of crude peptide was purified by gel filtration on Sephadex® G-15 using 1% HOAc as eluant, yielding 77 mg.
TLC: (B:A:W 1:1:1), $R_f = 0.38$
Gradient HPLC: 5% to 40%, 0.1% TFA($CH_3CN$), linear, 15 min, $k' = 2.72$
FAB-MS: m/z 848 $(M+H)^+$
Amino Acid Analysis: HCl/TFA/Phenol, Asp 1.06, Ser 0.84, Glu 1.00, Pro 0.88, Ala 1.00, Val 1.85, Tyr 1.00. Peptide Content was 94.3%.

## Example 49

Preparation of Ac-Ser-Ala-Asn-Tyr-Pro-Val-Val-$NH_2$

One-half mmole of the protected peptidyl resin Ac-Ser(Bzl)-Ala-Asn-Tyr(BrZ)-Pro-Val-Val-BHA was

prepared as above. The peptide was cleaved from 10ml HF/1ml anisole 1 hr at 0° C. The peptidyl resin was washed with ether and the peptide was extracted with glacial acetic acid. The extract was lyophilized to afford 330 mg, 84% yield. 100mg of crude peptide was purified by gel filtration on Sephadex® G-15 using 1% HOAc as eluant, yielding 86 mg.
TLC: (B:A:W 1:1:1), $R_f$ = 0.70
Gradient HPLC: 5% to 40%, 0.1% TFA($CH_3CN$), linear, 15min, k′ = 2.62
FAB-MS: m/z 790 $(M+H)^+$
Amino Acid Analysis: HCl/TFA/Phenol, Asp 1.00, Ser 0.81, Pro 0.94, Ala 1.00, Val 1.87, Tyr 0.76. Peptide Content was 72.77%.

## Example 50

### Preparation of Ac-Ser-Gln-Ser-Tyr-Pro-Val-Val-$NH_2$

One-half mmole of the protected peptidyl resin Ac-Ser(Bzl)-Gln-Ser(Bzl)-Tyr(BrZ)-Pro-Val-Val-BHA was prepared as above. The peptide was cleaved from 10ml HF/1ml anisole 1 hr at 0° C. The peptidyl resin was washed with ether and the peptide was extracted with glacial acetic acid. The extract was lyophilized to afford 285 mg, 70% yield. 100 mg of crude peptide was purified by gel filtration on Sephadex® G-15 using 1% HOAc as eluant, yielding 87 mg.
TLC: (B:A:W 1:1:1), $R_f$ = 0.68
Gradient HPLC: 5% to 40%, 0.1% TFA($CH_3CN$), linear, 15min, k′ = 2.51
FAB-MS: m/z 820 $(M+H)^+$
Amino Acid Analysis: HCl/TFA/Phenol, Ser 1.60, Glu 1.00, Pro 0.91, Val 1.83, Tyr 0.94. Peptide Content was 84%.

## Example 51

### Preparation of Ac-Ser-Gln-Arg-Tyr-Pro-Val-Val-$NH_2$

One-half mmole of the protected peptidyl resin Ac-Ser(Bzl)-Gln-Arg-Tyr(BrZ)-Pro-Val-Val-BHA was prepared as above. The peptide was cleaved from 10ml HF/1ml anisole 1 hr at 0° C. The peptidyl resin was washed with ether and the peptide was extracted with glacial acetic acid. The extract was lyophilized to afford 380.54 mg, 86% yield. 100 mg of crude peptide was purified by gel filtration on Sephadex® G-15 using 1% HOAc as eluant, yielding 89 mg.
TLC: (B:A:W 1:1:1), $R_f$ = 0.66
Gradient HPLC: 5% to 40%, 0.1% TFA($CH_3CN$), linear, 15min, k′ = 2.58
FAB-MS: m/z 889 $(M+H)^+$
Amino Acid Analysis: HCl/TFA/Phenol, Ser 0.80, Glu 1.00, Pro 1.00, Val 1.88, Arg 1.01, Tyr 0.81. Peptide Content was 85.33%.

## Example 52

### Preparation of Ac-Ser-Gln-Lys-Tyr-Pro-Val-Val-$NH_2$

One-half mmole of the protected peptidyl resin Ac-Ser(Bzl)-Gln-Lys(Clz)-Tyr(BrZ)-Pro-Val-Val-BHA was prepared as above. The peptide was cleaved from 10ml HF/1ml anisole 1 hr at 0° C. The peptidyl resin was washed with ether and the peptide was extracted with glacial acetic acid. The extract was lyophilized to afford 332 mg, 77.32% yield. 100mg of crude peptide was purified by gel filtration on Sephadex® G-15 using 1% HOAc as eluant, yielding 85 mg.

TLC: (B:A:W 1:1:1), $R_f = 0.69$
Gradient HPLC: 5% to 40%, 0.1% TFA($CH_3CN$), linear, 15min, $k' = 2.48$
FAB-MS: m/z 861 $(M+H)^+$
Amino Acid Analysis: HCl/TFA/Phenol, Ser 0.79, Glu 1.00, Pro 0.94, Val 1.85, Tyr 0.96, Lys 0.90. Peptide Content is 71.26%.

## Example 53

### Preparation of Ac-Ser-Gln-Asp-Tyr-Pro-Val-Val-$NH_2$

One-half mmole of the protected peptidyl resin Ac-Ser(Bzl)-Gln-Asp(OBzl)-Tyr(BrZ)-Pro-Val-Val-BHA was prepared as above. The peptide was cleaved from 10ml HF/1ml anisole 1 hr at 0° C. The peptidyl resin was washed with ether and the peptide was extracted with glacial acetic acid. The extract was lyophilized to afford 340 mg, 80% yield. 100mg of crude peptide was purified by gel filtration on Sephadex® G-15 using 1% HOAc as eluant, yielding 84 mg.
TLC: (B:A:W 1:1:1), $R_f = 0.73$
Gradient HPLC: 5% to 40%, 0.1% TFA($CH_3CN$), linear, 15min, $k' = 2.63$
FAB-MS: m/z 848 $(M+H)^+$
Amino Acid Analysis: HCl/TFA/Phenol, Asp 1.02, Ser 0.79, Glu 1.00, Pro 0.97, Val 1.86, Tyr 0.82. Peptide Content is 79.13%.

## Example 54

### Preparation of Ac-Asp-Gln-Asn-Tyr-Pro-Val-Val-$NH_2$

One-half mmole of the protected peptidyl resin Ac-Asp(OBzl)-Gln-Asn-Tyr(BrZ)-Pro-Val-Val-BHA was prepared as above. The peptide was cleaved and deprotected using 10 mL of anhydrous HF with 10% anisole at 0° C for 1 h. The HF was removed *in vacuo* at 0° C and the resin was washed with diethyl ether. The peptide was extracted with glacial acetic acid and lyophilized to yield 288 mg (65.9%). The peptide was purified by countercurrent distribution in 1-butanol-acetic acid-water (4:1:5 v/v/v). Tubes were checked by thin layer chromatography (TLC) and the appropriate fractions were pooled, evaporated and lyophilized from 0.2 N HOAC to yield 234 mg (81.2%).
TLC: (B:E:A:W 1:1:1:1) $R_f = 0.77$; (B:P:A:W 15:10:3:12) $R_f = 0.56$.
FAB MS: m/z 875 $(M+H)^+$
HPLC: 4.5 mm X 25 cm Altex Ultrasphere 5 m ODS, UV detection at 220 nm, water-acetonitrile- 0.1% TFA gradient of 5 to 40% acetonitrile over 15 min., $k' = 3.71$
Isocratic HPLC: 85:15 water-acetonitrile- 0.1% TFA, $k' = 4.77$
Amino Acid Analysis: (hydrolysis at 160° C for 1 h. in HCl/TFA 2:1 containing 0.005% w/v phenol) Asp 2.05, Glu 1.00, Pro 1.03, Val 1.94, Tyr 0.84

## Example 55

### Preparation of Ac-His-Gln-Asn-Tyr-Pro-Val-Val-$NH_2$

One-half mmole of the protected peptidyl resin Ac-His(Tos)-Gln-Asn-Tyr(BrZ)-Pro-Val-Val-BHA was prepared as above. The peptide was cleaved and deprotected using 10 mL of anhydrous HF with 10% anisole at 0° C for 1 h. The HF was removed *in vacuo* at 0° C and the resin was washed with diethyl ether. The peptide was extracted with glacial acetic acid and lyophilized to yield 311 mg (69.4%). The peptide was partially purified by countercurrent distribution in 1-butanol-acetic acid-water (4:1:5 v/v/v). Tubes were

checked by thin layer chromatography (TLC) and the appropriate fractions were pooled, evaporated and lyophilized from 0.2 N HOAC to yield 200 mg (65.3%). Final purification was obtained by subjecting 87 mg to gel filtration on Sephadex® G-15 using 0.2 N HOAC as the eluent to yield 48 mg (55.2%). TLC: (B:P:A:W 15:10:3:12) $R_f = 0.51$; (B:A:W 1:1:1) $R_f = 0.71$.
HPLC: 4.5 mm X 25 cm Altex Ultrasphere 5 m ODS, UV detection at 220 nm, water-acetonitrile- 0.1% TFA gradient of 5 to 40% acetonitrile over 15 min., $k' = 4.94$
Isocratic HPLC: 85:15 water-acetonitrile- 0.1% TFA, $k' = 3.76$
FAB MS: m/z 897 $(M+H)^+$
Amino Acid Analysis: (hydrolysis at 160° C for 1 h. in HCl/TFA 2:1 containing 0.005% w/v phenol) Asp 1.03, Glu 1.00, Pro 1.13, Val 2.00, Tyr 1.97, His 1.28.

## Example 56

### Preparation of Ser-Gln-Asn-Tyr-Pro-Val-Val-$NH_2$

One-half mmole of the protected peptidyl resin Boc-Ser(Bzl)-Gln-Asn-Tyr(BrZ)-Pro-Val-Val-BHA was prepared as above. The peptide was cleaved and deprotected using 10 mL of anhydrous HF with 10% anisole at 0° C for 1 h. The HF was removed in vacuo at 0° C and the resin was washed with diethyl ether. The peptide was extracted with glacial acetic acid and lyophilized to yield 225 mg (55.9%). The peptide was purified by countercurrent distribution in 1-butanol-acetic acid-water (4:1:5 v/v/v). Tubes were checked by thin layer chromatography (TLC) and the appropriate fractions were pooled, evaporated and lyophilized from 0.2 N HOAC to yield 188 mg (83.5%).
TLC: (B:E:A:W 1:1:1:1) $R_f = 0.68$; (B:P:A:W 15:10:3:12) $R_f = 0.54$.
HPLC: 4.5 mm X 25 cm Altex Ultrasphere 5 m ODS, UV detection at 220 nm, water-acetonitrile- 0.1% TFA gradient of 5 to 40% acetonitrile over 15 min., $k' = 4.89$
Isocratic HPLC: 85:15 water-acetonitrile- 0.1% TFA, $k' = 2.78$
FAB MS: m/z 805.6 $(M+H)^+$
Amino Acid Analysis: (hydrolysis at 160° C for 1 h. in HCl/TFA 2:1 containing 0.005% w/v phenol) Asp 1.03, Ser 0.78, Glu 1.00, Pro 0.88, Val 1.93, Tyr 0.66.

Substituting 3-benzyloxy-propanoic acid for Boc-Ser(Bzl) in the above sequence yields 3-hydroxypropionylglutaminylasparaginyltyrosylprolylvalyl valinamide, which is a des-amino peptide.

Substituting Boc-D-Ser(Bzl) for Boc-Ser(Bzl) in the above sequence of reactions yields D-Ser-Gln-Asn-Tyr-Pro-Val-Val-$NH_2$.

## Example 57

### Preparation of Ac-Ser-Gln-Gly-Tyr-Pro-Val-Val-$NH_2$

One-half mmole of the protected peptidyl resin Ac-Ser(Bzl)-Gln-Gly-Tyr(BrZ)-Pro-Val-Val-BHA was prepared as above. The peptide was cleaved and deprotected using 10 mL of anhydrous HF with 10% anisole at 0° C for 1 h. The HF was removed in vacuo at 0° C and the resin was washed with diethyl ether. The peptide was extracted with glacial acetic acid and lyophilized to yield 200 mg (50.6%). The peptide was purified by countercurrent distribution in 1-butanol-acetic acid-water (4:1:5 v/v/v). Tubes were checked by thin layer chromatography (TLC) and the appropriate fractions were pooled, evaporated and lyophilized from 0.2 N HOAC to yield 145 mg (72.5%).
TLC: (B:E:A:W 1:1:1:1) $R_f = 0.63$; (B:A:W 4:1:1) $R_f = 0.40$.
HPLC: 4.5 mm X 25 cm Altex Ultrasphere 5 m ODS, UV detection at 220 nm, water-acetonitrile- 0.1% TFA gradient of 5 to 40% acetonitrile over 15 min., $k' = 4.56$
Isocratic HPLC: 85:15 water-acetonitrile- 0.1% TFA, $k' = 3.46$
FAB MS: m/z 790 $(M+H)^+$
Amino Acid Analysis: (hydrolysis at 160° C for 1 h. in HCl/TFA 2:1 containing 0.005% w/v phenol) Ser 0.71, Glu 0.97, Gly 1.00, Pro 1.02, Val 1.70, Tyr 0.76.

Example 58

Preparation of Ala-Ser-Gln-Asn-Tyr-Pro-Val-Val-$NH_2$

One-half mmole of the protected peptidyl resin Boc-Ala-Ser(Bzl)-Gln-Asn-Tyr(BrZ)-Pro-Val-Val-BHA was prepared as above. The peptide was cleaved and deprotected using 10 mL of anhydrous HF with 10% anisole at 0 °C for 1 h. The HF was removed in vacuo at 0 °C and the resin was washed with diethyl ether. The peptide was extracted with glacial acetic acid and lyophilized to yield 245 mg (56.0%). The peptide was purified by countercurrent distribution in 1-butanol-acetic acid-water (4:1:5 v/v/v). Tubes were checked by thin layer chromatography (TLC) and the appropriate fractions were pooled, evaporated and lyophilized from 0.2 N HOAC to yield 185 mg (75.5%).
TLC: (B:E:A:W 1:1:1:1) $R_f = 0.64$; (B:P:A:W 15:10:3:12) $R_f = 0.60$.
HPLC: 4.5 mm X 25 cm Altex Ultrasphere 5 m ODS, UV detection at 220 nm, water-acetonitrile- 0.1% TFA gradient of 5 to 40% acetonitrile over 15 min., $k' = 3.86$
Isocratic HPLC: 85:15 water-acetonitrile- 0.1% TFA, $k' = 2.71$
FAB MS: m/z 876 $(M+H)^+$
Amino Acid Analysis: (hydrolysis at 160 °C for 1 h. in HCl/TFA 2:1 containing 0.005% w/v phenol) Asp 1.00, Ser 0.69, Glu 0.98, Pro 0.95, Ala 0.99, Val 1.91, Tyr 0.94.

Example 59

Preparation of Ac-Ser-Gln-Asn-(4'$NO_2$)Phe-Pro-Val-Val-$NH_2$

One-half mmole of the protected peptidyl resin Ac-Ser(Bzl)-Gln-Asu-(4'$NO_2$)Phe-Pro-Val-Val-BHA was prepared as above. The peptide was cleaved from 15ml HF/1ml anisole 1 hr at 0 °C. The peptidyl resin was washed with ether and the peptide was extracted with glacial acetic acid. The extract was lyophilized to afford 701.8mg, 80% yield.
100mg of crude peptide was purified by gel filtration on Sephadex® G-15 using 1% HOAc as eluant, yielding 68 mg. A 35mg aliquot was further purified by preparative HPLC (PRP-1 semi prep, 18% 0.1% TFA($CH_3CN$), 6ml/min) yielding 22 mg.
TLC: (B:A:W 1:1:1), $R_f = 0.54$
Gradient HPLC: 5% to 40%, 0.1% TFA($CH_3CN$), linear, 15min, $k' = 3.18$
FAB-MS: m/z 876 $(M+H)^+$
Amino Acid Analysis: (hydrolysis at 160 °C for 1 h. in HCl/TFA 2:1 containing 0.005% w/v phenol) Asp 1.01, Ser 0.81, Glu 1.00, Pro 0.90, Val 2.00, nitroPhe 0.91. Peptide Content is 103.67%.

Example 60

Preparation of Dns-Arg-Ala-Ser-Gln-Asn-Tyr-Pro-Val-Val-OH

One-half mmole of the protected peptidyl resin Boc-Arg(Tos)-Ala-Ser(Bzl)-Gln-Asn-Tyr(BrZ)-Pro-Val-Val-Merrifield resin was prepared as above. After removal of the Boc group with 50% TFA/$CH_2Cl_2$ and neutralizing with 7% DIEA/$CH_2Cl_2$, the peptide was dansylated by the addition of 3 equiv. dansyl chloride in DMF and reacting for one hour. The dansyl peptide was cleaved from the resin by treatment with 10 ml HF / 1 ml anisole at 0° for 50 min. After removal of the HF under vacuum, the resin was washed with ether, air dried and extracted with 3 x 30 ml glacial HOAc. The HOAc extracts were combined and lyophilized to yield 321 mg crude peptide.

An aliquot of 100 mg of the crude peptide was purified by gel filtration on a 2.6 x 70 cm column of Sephadex® G-15 using 1% HOAc as eluant. The appropriate fractions were pooled and lyophilized, yielding 74.6 mg of the titled peptide.

TLC: (B:A:W 1:1:1) $R_f = 0.84$
HPLC: (Hamilton PRP-1, $CH_3CN$ / $H_2O$ / 0.1% TFA, gradient of 5-40% $CH_3CN$, 15 min), $k' = 3.61$.
FAB-MS: m/z 1266 $(M+H)^+$
Amino Acid Analysis: (hydrolysis in HCl/TFA 2:1 containing .005% w/v phenol at 160° for one hr): Asp, 1.00; Ser, 0.31; Glu, 0.91; Pro, 0.99; Ala, 0.96; Val, 1.96; Tyr, 1.19; Arg, 0.85.

## Example 61

### Preparation of Ac-Ser-Gln-Asn-Tyr-Pro-Val-Val-Gln-Asn-$NH_2$

One mmole of the protected peptidyl resin Ac-Ser(Bzl)-Gln-Asn-Tyr(BrZ)-Pro-Val-Val-Gln-Asn-BHA was prepared as above. The peptide was cleaved from the resin by treatment with 10 ml HF / 1 ml anisole at 0° for 50 min. After removal of the HF under vacuum, the resin was washed with ether, air dried and extracted with 3 x 30 ml glacial HOAc. The HOAc extracts were combined and lyophilized to yield 862.3 mg crude peptide.

The crude peptide was purified by countercurrent distribution in nBuOH-HOAc-$H_2O$ (4:1:5). The appropriate fractions were located by tlc, pooled, evaporated to dryness and the residue lyophilized from 1% HOAc, yielding 712.9 mg of the titled peptide.
TLC: (B:A:W 1:1:1) $R_f = 0.61$
HPLC: (Hamilton PRP-1, $CH_3CN$ / $H_2O$ / 0.1% TFA, gradient of 5-40% $CH_3CN$, 15 min), $k' = 2.17$.
FAB-MS: m/z 1089 $(M+H)^+$
Amino Acid Analysis: (hydrolysis in HCl/TFA 2:1 containing .005% w/v phenol at 160° for one hr): Asp, 1.81; Ser, 0.97; Glu, 2.00; Pro, 0.88; Val, 1.23; Tyr, 1.81.

## Example 62

### Preparation of Ac-Ala-Thr-Leu-Asn-Phe-Pro-Ile-Ser-Pro-Ile-Glu-$NH_2$

One mmole of the protected peptidyl resin Ac-Ala-Thr(Bzl)-Leu-Asn-Phe-Pro-Ile-Ser(Bzl)-Pro-Ile-Glu-(OBzl)-BHA was prepared as above. The peptide was cleaved from the resin by treatment with 10 ml HF / 1 ml anisole at 0° for 50 min. After removal of the HF under vacuum, the resin was washed with ether, air dried and extracted with 3 x 30 ml 1% HOAc and 3 x 30 ml 50% HOAc. The HOAc extracts were combined, diluted with water and lyophilized to yield 862.3 mg crude peptide.

The crude peptide was purified by countercurrent distribution in nBuOH-HOAc-$H_2O$ (4:1:5). The appropriate fractions were located by tlc, pooled, evaporated to dryness and the residue lyophilized from 1% HOAc. The peptide was further purified by gel filtration on a 2.6 x 70 cm Sephadex® G-15 column using 1% HOAc as eluant. The appropriate fractions were collected and lyophilized. An aliquot of 100 mg of the peptide was further purified by preparative hplc on a Hamilton PRP-1 column using $CH_3CN$ / $H_2O$ / 0.1% TFA, gradient of 20-40% $CH_3CN$, 15 min. The appropriate fractions were pooled, evaporated to dryness and lyophilized from glacial HOAc, yielding 22.6 mg of the titled peptide.
HPLC: (Hamilton PRP-1, $CH_3CN$ / $H_2O$ / 0.1% TFA, gradient of 5-40% $CH_3CN$, 15 min), $k' = 2.94$.
FAB-MS: m/z 1241 $(M+H)^+$

## Example 63

### Preparation of Ac-Ser-Gln-Asn-Tyr-Pro-Val-$NH_2$

One mmole of the protected peptidyl resin Ac-Ser(Bzl)-Gln-Asn-Tyr(BrZ)-Pro-Val-BHA was prepared as above. The peptide was cleaved from the resin by treatment with 10 ml HF / 1 ml anisole at 0° for 50 min.

After removal of the HF under vacuum, the resin was washed with ether, air dried and extracted with 3 x 30 ml 1% HOAc and 3 x 30 ml 10% HOAc. The HOAc extracts were combined and lyophilized to yield 144.7 mg crude peptide.

The crude peptide was purified by countercurrent distribution in nBuOH-HOAc-$H_2O$ (4:1:5). The appropriate fractions were located by tlc, pooled, evaporated to dryness and the residue lyophilized from 1% HOAc, yielding 150 mg peptide. The peptide was further purified by gel filtration on a 2.6 x 70 cm Sephadex® G-15 column using 1% HOAc as eluant. The appropriate fractions were pooled and lyophilized to yield 101.3 mg of the titled peptide.

TLC: (B:A:W 4:1:1) $R_f = 0.65$

HPLC: (Hamilton PRP-1, $CH_3CN$ / $H_2O$ / 0.1% TFA, gradient of 5-40% $CH_3CN$, 15 min), $k' = 2.08$.

FAB-MS: m/z 748 $(M+H)^+$

Amino Acid Analysis: (hydrolysis in HCl/TFA 2:1 containing .005% w/v phenol at 160° for one hr): Asp, 1.00; Ser, 0.55; Glu, 0.98; Pro, 1.19; Val, 0.95; Tyr, 0.99.

## Example 64

### Preparation of Ac-Ser-Gln-Asn-Tyr-Pro-Val-Val-Arg-$NH_2$

One-half mmole of the protected peptidyl resin Ac-Ser(Bzl)-Gln-Asn-Tyr(BrZ)-Pro-Val-Val-Arg(Tos)-BHA was prepared as above. The peptide was cleaved from the resin by treatment with 10 ml HF / 1 ml anisole at 0° for 50 min. After removal of the HF under vacuum, the resin was washed with ether, air dried and extracted with 3 x 30 ml glacial HOAc. The HOAc extracts were combined and lyophilized to yield 329 mg crude peptide.

The crude peptide was purified by countercurrent distribution in nBuOH-HOAc-$H_2O$ (4:1:5). The appropriate fractions were located by tlc, pooled, evaporated to dryness and the residue lyophilized from 1% HOAc, yielding 79 mg of the titled peptide.

TLC: (B:E:A:W 1:1:1:1) $R_f = 0.46$

HPLC: (Altex Ultrasphere C18, $CH_3CN$ / $H_2O$ / 0.1% TFA, gradient of 5-40% $CH_3CN$, 15 min), $k' = 3.71$.

FAB-MS: m/z 1003 $(M+H)^+$

Amino Acid Analysis: (hydrolysis in HCl/TFA 2:1 containing .005% w/v phenol at 160° for one hr): Asp, 1.00; Ser, 0.73; Glu, 0.98; Pro, 0.98; Val, 1.84; Tyr, 1.08; Arg, 0.99.

## Example 65

### Preparation of Ac-Ala-Gln-Asn-Tyr-Pro-Val-Val-$NH_2$

One-half mmole of the protected peptidyl resin Ac-Ser(Bzl)-Gln-Asn-Tyr(BrZ)-Pro-Val-Val-Arg(Tos)-BHA was prepared as above. The peptide was cleaved from the resin by treatment with 10 ml HF / 1 ml anisole at 0° for 50 min. After removal of the HF under vacuum, the resin was washed with ether, air dried and extracted with 3 x 30 ml glacial HOAc. The HOAc extracts were combined and lyophilized to yield 275 mg crude peptide.

The crude peptide was purified by countercurrent distribution in nBuOH-HOAc-$H_2O$ (4:1:5). The appropriate fractions were located by tlc, pooled, evaporated to dryness and the residue lyophilized from 1% HOAc, yielding 235 mg of the titled peptide.

TLC: (B:E:A:W 1:1:1:1) $R_f = 0.69$

HPLC: (Altex Ultrasphere C18, $CH_3CN$ / $H_2O$ / 0.1% TFA, gradient of 5-40% $CH_3CN$, 15 min), $k' = 4.48$.

FAB-MS: m/z 831 $(M+H)^+$

Amino Acid Analysis: (hydrolysis in HCl/TFA 2:1 containing .005% w/v phenol at 160° for one hr): Asp, 1.00; Ala, 0.99; Glu, 0.98; Pro, 0.95; Val, 1.97; Tyr, 1.85.

## Example 66

Preparation of Ala-Ala-Gln-Asn-Tyr-Pro-Val-Val-$NH_2$

One-half mmole of the protected peptidyl resin Boc-Ala-Ala-Gln-Asn-Tyr(BrZ)-Pro-Val-Val-BHA was prepared as above. The peptide was cleaved from the resin by treatment with 10 ml HF / 1 ml anisole at 0° for 50 min. After removal of the HF under vacuum, the resin was washed with ether, air dried and extracted with 3 x 30 ml glacial HOAc. The HOAc extracts were combined and lyophilized to yield 265 mg crude peptide. An aliquot of 75 mg crude peptide was purified by gel filtration on a 2.6 x 70 cm Sephadex® G-15 column using 1% HOAc as eluant. The appropriate fractions were pooled and lyophilized, yielding 50 mg of the titled peptide.

TLC: (B:A:W 1:1:1) $R_f = 0.54$

HPLC: (Altex Ultrasphere C18, $CH_3CN$ / $H_2O$ / 0.1% TFA, gradient of 5-40% $CH_3CN$, 15 min), $k' = 4.67$.

FAB-MS: m/z 947 $(M+H)^+$

Amino Acid Analysis: (hydrolysis in HCl/TFA 2:1 containing .005% w/v phenol at 160° for one hr): Asp, 1.00; Ser, 0.61; Ala, 1.52; Glu, 0.99; Pro, 0.86; Val, 1.90; Tyr, 0.98.

## Example 67

Preferential of Ac-Cys-Gln-Asn-Tyr-Pro-Val-Val-$NH_2$

One-half mmole of the protected peptidyl resin Ac-Cys(Mbz)-Gln-Asn-Tyr(BrZ)-Pro-Val-Val-BHA was prepared as above. The peptide was cleaved from the resin by treatment with 10 ml HF / 1 ml anisole at 0° for 50 min. After removal of the HF under vacuum, the resin was washed with ether, air dried and extracted with 3 x 30 ml glacial HOAc. The HOAc extracts were combined and lyophilized to yield 292 mg crude peptide.

The crude peptide was purified by countercurrent distribution in nBuOH-HOAc-$H_2O$ (4:1:5). The appropriate fractions were located by tlc, pooled, evaporated to dryness and the residue lyophilized from 1% HOAc, yielding 188 mg of the titled peptide.

TLC: (B:E:A:W 1:1:1:1) $R_f = 0.67$

HPLC: (Altex Ultrasphere C18, $CH_3CN$ / $H_2O$ / 0.1% TFA, gradient of 5-40% $CH_3CN$, 15 min), $k' = 3.7$.

FAB-MS: m/z 863 $(M+H)^+$

Amino Acid Analysis: (oxidation with performic acid at 0° C for 4 hr followed by hydrolysis in HCl/TFA 2:1 containing .005% w/v phenol at 160° for one hr): Cys-$SO_3H$, 0.98; Asp, 1.03; Glu, 1.00; Pro,1.13; Val, 1.94; Tyr, 1.78.

## Example 68

Preparation of Ac-Ser-Gln-Asn-Tyr-Δ3-Pro-Val-Val-$NH_2$

One-half mmole of the protected peptidyl resin Ac-Ser(Bzl)-Gln-Asn-Tyr(BrZ)-Δ3-Pro-Val-Val-BHA was prepared as above. The peptide was cleaved from the resin by treatment with 10 ml HF / 1 ml anisole at 0° for 50 min. After removal of the HF under vacuum, the resin was washed with ether, air dried and extracted with 4 x 20 ml glacial HOAc. The HOAc extracts were combined and lyophilized to yield 250 mg crude peptide.

An aliquot of 100 mg crude peptide was purified gel filtration on a 2.6 x 70 cm Sephadex® G-10 column using 0.2 N HOAc as eluant. The appropriate fractions were pooled and lyophilized, yielding 41 mg of the titled peptide.

HPLC: (Altex Ultrasphere C18, $CH_3CN$ / $H_2O$ / 0.1% TFA, gradient of 10-40% $CH_3CN$, 30min), $k' = 3.00$.

FAB-MS: m/z 803 $(M+H)^+$

Amino Acid Analysis: (hydrolysis in HCl/TFA 2:1 containing .005% w/v phenol at 160° for one hr): Asp,

1.00; Ser, 0.66; Glu, 0.95; Val, 1.88; Tyr, 0.95.

Example 69

Preparation of Ac-Ser-Gly-Asn-Tyr-Pro-Val-Val-$NH_2$

The protected peptidyl resin Boc-Ser(Bzl)-Gly-Asn-Tyr(BrZ)-Pro-Val-Val-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lypohilized to yield 167 mg crude product. The crude peptide was dissolved in 1% HOAc and purified on a 2.6 x 70 cm Sephadex® G-15 column. The appropriate fractions were pooled and lyophilized to yield 138 mg purified product.
HPLC: $k'$ = 4.17 (Beckman Ultrasphere® ODS, 5% $CH_3CN$/ 0.1% TFA to 40% $CH_3CN$/ 0.1% TFA, 15 min.)
MS (FAB): m/z 776 $(M+H)^+$
Amino Acid Analysis: Asp 1.00,Gly 1.03, Pro 0.90, Val 1.88, Tyr 0.80, Ser 0.68.

Example 70

Preparation of 2-isopropylamino-3-phenyl-propyl-prolylvalyl valinamide

The protected peptidyl resin Boc-Asn-(2-amino-3-phenylpropyl-prolyl)-Val-Val-BHA was prepared in the usual manner on a 0.5 mmol scale using the compound of Example 2(d) and the procedure of Example 15. The peptide was deprotected and removed from the resin by treatment with anhydrous HF (10 ml) in the presence of anisole (1 ml) at 0 °C for one hour. After removal of the HF under vacuum, the resin was washed with ethyl ether, air-dried and then extracted with 2 x 30 ml glacial HOAc. The HOAc extracts were combined and lyophilized to give 139.6 mg crude peptide. The peptide was purified by gel filtration on G-15 Sephadex® using 1 °% HOAc as eluant. The appropriate fractions were combined to yield 81.1 mg purified peptide, single peak by hplc.
HPLC: (Hamilton PRP-1, 5-40% $CH_3CN$ / 0.1 % TFA, 15 min.) $k'=2.47$
FAB-MS: m/z 560.5 $(M+H)^+$.

Example 71

Preparation of (4S,5S) 5-amino-4-hydroxy-1-oxo-6-(4-hydroxyphenyl)hexyl-valyl valine methyl ester, hydrochloride

(4S,5S) 5-(tert-butyloxycarbonyl)amino-4-hydroxy-1-oxo-6-(4-benzyloxyphenylhexyl-valyl valine methyl ester

a) To a solution of the compound of Example 35(a) (1.08 g, 1.70 mmol) in MeOH at 0°C was added sodium borohydride (32 mg, 0.85 mmol) in portions. After 30 min, the reaction was complete as indicated by TLC, was diluted with 5% aqueous HCl and extracted with $CH_2Cl_2$ three times. The combined organic extracts were dried ($MgSO_4$), filtered and evaporated under reduced pressure to give a mixture of isomers as a white solid (1.05g, 97%). The isomers were eluted through a 1" x 25 cm HPLC silica column with 40:1 $CH_2Cl_2$: MeOH at a rate of 20 ml/ min. The desired (4S) isomer which eluted first at 14.5 min, was collected and evaporated to give the titled compound as a white solid (295 mg, 34%).
NMR ($CDCl_3$): δ 7.43 (5H, m), 7.02 (4H, dd), 6.40 (1H, d), 6.32 (1H, d), 5.04 (2H, s), 4.50 (2H, m), 4.26 (2H, m), 3.70 (3H, s), 2.71 (2H, m), 2.43 (2H, m), 2.11 (2H, m), 1.39 (9H, s), 0.91 (12H, m).

MS (FAB): m/z 642.4 $(M+H)^+$.

(4S,5S) 5-amino-4-hydroxy-1-oxo-6-(4-benzyloxyphenyl)hexyl-valyl valine methyl ester, hydrochloride

b) The compound of Example 71(a) (295 mg, 0.46 mmol) was dissolved in neat trifluoroacetic acid (1 ml) and stirred for 3 min. The solution was evaporated under reduced pressure, dissolved in MeOH, treated with conc. HCl (3 drops) and evaporated under reduced pressure to give the titled compound as a white solid.

## Example 72

Preparation of (4S,5S) 5-(alanylalanyl)amino-4-hydroxy-1-oxo-6-(4-hydroxyphenyl)hexyl-valyl valine methyl ester, acetic acid salt

(4S,5S)-5-(carbobenzyloxyalanylalanyl)amino-6-(4-benzyloxy)phenyl-4-hydroxy-(1-oxo)hexyl-valyl valine methyl ester

a) A solution of carbobenzyloxyalanyl alanine (56 mg, 0.19 mmol) in THF was cooled to -40°C under an argon atmosphere. To this solution was added N-methyl morpholine (28 ml, 0.251 mmol), followed by isobutyl chloroformate (25 ml, 0.19 mmol). The reaction mixture was stirred for 15 min, and additional N-methyl morpholine (28 ml, 0.251 mmol) was added, followed by the compound of Example 71(b) (100 mg, 0.173 mmol). The resulting mixture was stirred for 30 min at -40°C, warmed to room temperature and stirred for 16 h. The mixture was diluted with 1:1 ethyl acetate: $CH_2Cl_2$ and washed successively with 5% aqueous HCl, 5% aqueous $NaHCO_3$ and saturated NaCl. The solution was dried ($MgSO_4$), filtered and evaporated under reduced pressure. The solid residue was eluted through a 1" x 25 cm HPLC silica gel column with 95: 5: 0.5 $CHCl_3$: MeOH: $H_2O$ to give the titled compound as a white solid (96 mg, 77%).
NMR ($CD_3OD/CDCl_3$): $\delta$ 7.15 (10H, m), 6.80 (4H, dd), 4.90 (2H, s), 4.81 (2H, s), 3.90 (2H, g), 3.75 (1H, m), 3.51 (3H, s), 3.36 (1H, m), 2.59 (2H, m), 2.11 (2H, m), 1.88 (3H, m), 1.48 (2H, m), 1.10 (8H, m), 0.76 (12H, d).
MS (FAB): m/z 818.3 $(M+H)^+$.

(4S,5S) 5-(alanylalanyl)amino-4-hydroxy-1-oxo-6-(4-hydroxyphenyl)hexyl-valyl valine methyl ester, acetic acid salt

b) To a solution of the compound of Example 72(a) (90 mg, 0.11 mmol) in acetic acid was added 10% palladium on activated carbon (90 mg). Hydrogen gas was bubbled via balloon through the solution for 60 min, then the stirring mixture was maintained under a hydrogen atmosphere for 14 h. The suspension was filtered through a pad of Celite® and evaporated under reduced pressure to give the titled compound as a white solid (74 mg, 98%).
NMR ($CD_3OD/CDCl_3$): $\delta$ 7.06 (1H, d), 6.78 (4H, dd), 4.29 (1H, d), 4.16 (1H, m), 4.03 (1H, d), 3.60 (3H, s), 2.62 (2H, m), 1.85 (7H, br m), 1.57 (2H, m), 1.16 (6H, m), 0.80 (12H, d).
MS (FAB): m/z 594.4 $(M+H)^+$.

## Example 73

Preparation of (4S,5S)-5-alanylamino-4-hydroxy-1-oxo-6-(4-hydroxyphenyl)-hexyl-valyl valine methyl ester, acetic acid salt

(4S,5S)-5-(carbobenzyloxyalanyl)amino-4-hydroxy-1-oxo-6-(4-benzyloxyphenyl)hexyl-valyl valine methyl ester

a) Using the procedure of Example 72(b), substituting carbobenzyloxyalanine (34 mg, 0.15 mmol) for carbobenzyloxyalanyl alanine, and using N-methyl morpholine (44 ml, 0.40 mmol; added in two equal portions), isobutyl chloroformate (20 ml, 0.15 mmol), and the compound of Example 71(b) (79 mg, 0.137 mmol), the titled compound was prepared. The crude product was purified by column chromatography using silica gel, eluting with 5: 95 MeOH: $CHCl_3$ to give the titled compound as a white solid (60 mg, 82%).
NMR ($CD_3OD/CDCl_3$): δ 7.13 (10H, m), 6.81 (4H, dd), 4.92 (2H, s), 4.70 (2H, s), 4.21 (1H, m), 3.96 (2H, d), 3.76 (1H, m), 3.55 (3H, s), 3.34 (1H, m), 2.61 (2H, m), 2.12 (2H, m), 1.89 (3H, m), 1.48 (2H, m), 1.04 (4H, d), 0.71 (12H, d).
MS (FAB): m/z 747.3 $(M+H)^+$.

(4S,5S)-5-alanylamino-4-hydroxy-1-oxo-6-(4-hydroxyphenyl)hexyl-valyl valine methyl ester, acetic acid salt

b) The titled compound was made by a procedure identical to that of 72(b), substituting the compound of Example 73(a) (60 mg) for the compound of Example 72(a) to give the titled compound as a white solid (52 mg, 100%).
NMR ($CD_3OD/CDCl_3$): δ 8.19 (1H, d), 7.95 (1H, d), 7.70 (1H, d), 6.78 (4H, dd), 4.15 (2H, m), 3.95 (1H, m), 3.73 (1H, q), 3.58 (3H, s), 3.43 (1H, m), 2.66 (2H, m), 2.21 (2H, t), 1.90 (3H, m), 1.53 (2H, q), 1.36 (3H, d), 1.17 (2H, br), 0.93 (2H, m), 0.78 (12H, d).
MS (FAB): m/z 523.2 $(M+H)^+$.

## Example 74

Preparation of (4S,5S) 5-(tert-butyloxycarbonylalanyl)amino-4-hydroxy-1-oxo-6-(4-hydroxyphenyl)hexyl-valyl valine methyl ester

The titled compound was prepared by a prodedure identical to that of Example 72(a), substituting t-butyloxycarbonylalanine (52 mg, 0.275 mmol) for carbobenzyloxyalanyl alanine, and using the compound of Example 71(b) (144 mg, 0.25 mmol), N-methyl morpholine (79 ml, 0.724 mmol; in two portions) and isobutylchloroformate (36 ml, 0.275 mmol). The crude product was purified by eluting through a silica gel column with 1: 25 MeOH: $CH_2Cl_2$ to a white solid (55 mg, 55%). A portion of this product (12.5 mg, 0.0172 mmol) was stirred in acetic acid with 10% palladium on activated carbon (10 mg). Hydrogen gas was bubbled through the solution via balloon for 60 min, then the mixture was maintained under a positive hydrogen pressure for 14 h. The suspension was filtered through a pad of Celite® and evaporated under reduced pressure to give the titled compound as a white solid (7.5 mg, 70%).
NMR ($CD_3OD/CDCl_3$): δ 6.72 (4H, dd), 4.23 (1H, d), 3.65-3.92 (3H, m), 3.54 (3H, s), 3.38 (2H, m), 2.60 (2H, m), 1.62-2.20 (5H, m), 1.49 (3H, m), 1.26 (9H, s), 1.04 (5H, m), 0.77 (12H, d).
MS (FAB): m/z 623.3 $(M+H)^+$.

## Example 75

Preparation of (4S,5S) 5-((β-alanyl)alanyl)amino-4-hydroxy-1-oxo-6-(4-hydroxyphenyl)hexyl-valyl valine methyl ester, hydrochloride

(4S,5S) 5-((carbobenzyloxy-β-alanyl)alanyl)amino-4-hydroxy-1-oxo-6-(4-hydroxyphenyl)hexyl-valyl valine methyl ester

a) To a solution of carbobenzyloxy-β-alanine (23 mg, 0.105 mmol), and hydroxybenzotriazole (26 mg,

0.19 mmol) in DMF (0.8 ml) was added dicyclohexylcarbodiimide (22 mg, 0.105 mmol), and the mixture was stirred at room temperature for 20 min. To this solution was added N-methylmorpholine (32 ml, 0.29 mmol) and the compound of Example 73(b) (50 mg, 0.0956 mmol). The solution was allowed to stir at room temperature for 6 days and was then diluted with $CHCl_3$ and washed successively with 10% HCl, 5% aqueous $NaHCO_3$ and $H_2O$. The organic layer was dried ($MgSO_4$), filtered, and evaporated under reduced pressure to a white solid (44 mg). The product was eluted through a 1" x 25 cm HPLC silica column with 6: 96 MeOH: $CHCl_3$ to give the titled compound as a white solid (12 mg, 20%).
NMR ($CD_3OD/CDCl_3$): δ 7.51 (1H, d), 7.30 (1H, d), 7.18 (5H, br s), 6.94 (1H, d), 6.69 (4H, dd), 4.90 (2H, s), 4.19 (1H, m), 3.82 (1H, m), 3.56 (3H, s), 3.42 (1H, m), 3.18 (3H, m), 2.59 (2H, m), 2.15 (4H, m), 1.90 (2H, m), 1.49 (2H, m), 1.08 (3H, d), 0.80 (12H, d).
MS (FAB): m/z 728.3 $(M+H)^+$.

(4S,5S) 5-((β-alanyl)alanyl)amino-4-hydroxy-1-oxo-6-(4-hydroxyphenyl)hexyl-valyl valine methyl ester hydrochloride

b) To a solution of the compound of Example 75(a) (11 mg, 0.015 mmol) in MeOH was added 10% palladium on activated carbon (5 mg). Hydrogen gas was bubbled through the solution via balloon for 1 h, and then the stirring mixture was maintained under a hydrogen (1 atm) for 14 h. The suspension was filtered through a pad of Celite® and several drops of conc. HCl were added. The solution was evaporated under reduced pressure to give the titled compound as a white solid (10 mg, 95%).
NMR ($CD_3OD/CDCl_3$): δ 7.60 (1H, d), 6.59 (4H, dd), 4.06 (1H, t), 3.89 (2H, m), 3.66 (1H, m), 3.44 (3H, s), 3.27 (1H, m), 2.72 (2H, m), 2.41 (5H, m), 1.98 (2H, t), 1.79 (3H, m), 1.32 (2H, m), 1.07 (1H, m), 0.94 (3H, d), 0.67 (12H, d).

## Example 76

### Preparation of (4S,5S) 5-amino-4-hydroxy-1-oxo-6-phenylhexyl-valyl valine methyl ester, hydrochloride

(4S,5S)-5-(tert-butyloxycarbonyl)amino-4-hydroxy-1-oxo-6-phenylhexyl-valyl valine methyl ester

a) To a solution of the compound of Example 34(a) (4.59 g, 8.61 mmol) in MeOH (150mL) at 0°C was added sodium borohydride (0.33 g, 8.7 mmol). The resulting mixture was allowed to warm to room temperature and stirred for 30 min. The mixture was poured into 5% HCl (500 ml) and extracted with $CH_2Cl_2$ (3 x 100 ml). The combined organic extracts were dried ($MgSO_4$), filtered and evaporated under reduced pressure to afford a mixture of stereoisomers. The residue was dissolved in 2% $MeOH/CHCl_3$ (70 ml), filtered and purified in batches by HPLC using a 2" x 25 cm silica column and eluting with 2% $MeOH/CHCl_3$. The desired (4S) isomer eluted first, was collected and evaporated under reduced pressure to afford the titled compound (1.23 g, 27% yield).
NMR ($CDCl_3$): δ 7.27 (5H, m), 6.94 (1H, d), 6.76 (1H, d), 5.10 (1H, d), 4.51 (1H, dd), 4.36 (1H, br t), 3.74 (3H, s), 3.70-3.50 (3H, m), 2.89 (2H, d), 2.40 (2H, m), 2.24-2.00 (2H, m), 1.95-1.65 (2H, m), 1.38 (9H, s), 0.91 (12H, m).
MS (FAB): m/z 536.2 $(M+H)^+$.

(4S,5S) 5-amino-4-hydroxy-1-oxo-6-phenylhexyl-valyl valine methyl ester, hydrochloride

b) The peptide of Example 76(a) (1.0 g, 1.87 mmol) was treated with trifluoroacetic acid (5 ml), and the resulting mixture was stirred for 10 min. Methanol was added (25 ml) followed by conc. HCl (0.20 ml). The solvent was evaporated under reduced pressure; $Et_2O$ was added to the residue and re-evaporated, to produce the titled compound as a white powder which was used without further purification.
NMR ($CD_3OD$): δ 7.30 (5H, m), 4.26 (2H, m), 3.69 (3H, s), 3.60 (1H, m), 3.34 (1H, m), 3.07 (1H, dd), 2.88 (1H, dd), 2.40 (2H, m), 2.08 (2H, m), 1.80 (2H, m), 0.94 (12H, m).
MS (FAB): m/z 436.2 $(M+H)^+$.

Example 77

Preparation of (4S,5S) 5-(alanyl)amino-4-hydroxy-1-oxo-6-dicyclohexyl-valyl valine, methyl ester acetic acid salt

(4S,5S) 5-phenylpropylprolylvalyl)amino-4-hydroxy-1-oxo-6-phenylhexyl-valyl valine diethyl ester

a) To a solution of carbobenzyloxyalanine (413 mg, 1.85 mmol) in anhydrous THF (5 ml) at -40°C under an argon atmosphere was added N-methyl morpholine (0.24 ml, 2.2 mmol) followed by isobutyl chloroformate (0.240 ml, 1.85 mmol). After stirring 20 min, additional N-methyl morpholine (0.24 ml, 2.2 mmol) was added followed by a solution of the compound of Example 76(b) (0.83 g, 1.76 mmol) in THF (10 ml). The resulting mixture was allowed to warm to room temperature and stirred overnight. The reaction mixture was dissolved in EtOAC and washed successively with 10% HCl, 5% aqueous $NaHCO_3$ and $H_2O$, then dried ($MgSO_4$). Filtration and removal of the solvent in vacuo gave a white solid which was purified by flash chromatography eluting with a solvent gradient from 4% to 10% $MeOH/CHCl_3$ (0.975 g, 86% yield).
NMR ($CD_3OD$): δ 7.34 (5H, m), 7.23 (5H, m), 5.09 (2H, s), 4.32 (1H, d), 4.19 (1H, d), 4.09 (2H, m), 3.71 (3H, s), 3.60 (1H, m), 2.85 (2H, m), 2.30 (2H, m), 2.10 (2H, m), 1.70 (2H, m), 1.23 (3H, d), 0.94 (12H, m).
MS (FAB): m/z 641.3 $(M+H)^+$.

(4S,5S) 5-(alanyl)amino-4-hydroxy-1-oxo-6-phenylhexyl-valyl valine, methyl ester acetic acid salt

b) To a solution of the compound of Example 77(a) (969 mg, 1.51 mmol) in 1:1 MeOH: HOAc (15 ml) was added 10% palladium on activated carbon (100 mg). Hydrogen gas was introduced into the mixture, which was then maintained under a hydrogen atmosphere for 18 h. The reaction mixture was filtered through a pad of Celite® and evaporated under reduced pressure to afford the titled compound as a white solid (0.84 g, 98% yield).
NMR ($CD_3OD$): δ 7.25 (5H, m), 4.32 (1H, d), 4.21 (1H, d), 4.10 (1H, br t), 3.77 (1H, apparent q), 3.71 (3H, s), 3.60 (1H, br t), 2.96 (1H, dd), 2.83 (1H, dd), 2.35 (2H, t), 2.08 (2H, m), 1.96 (3H, s), 1.70 (2H, m), 1.44 (3H, d), 0.95 (12H, d).
MS (FAB): m/z 507.2 $(M+H)^+$.

Example 78

Preparation of (4S,5S) 5-(alanylalanyl)amino-4-hydroxy-1-oxo-6-phenylhexyl-valyl valine methyl ester

(4S,5S) 5-(carbobenzyloxyalanylalanyl)amino-4-hydroxy-1-oxo-6-phenylhexyl-valyl valine methyl ester

a) To a solution of carbobenzyloxyalanine (337 mg, 1.51 mmol) and N-methyl morpholine (0.198 ml, 1.80 mmol) in anhydrous THF (10 ml) at -40°C under an argon atmosphere was added isobutyl chloroformate (0.196 ml, 1.51 mmol) over a 10 min period. After stirring an additional 20 min, more N-methyl morpholine (0.198 ml, 1.80 mmol) was added followed by a solution of the compound of 77(b) (0.84 g, 1.48 mmol) in THF (20 ml). Dimethylformamide (10 ml) was added, and the cold temperature bath was removed briefly to facilitate stirring. The reaction mixture was recooled to -40°C, allowed to gradually warm to room temperature and stirred overnight. The reaction mixture was dissolved in 10% $MeOH/CHCl_3$ (500 ml) and washed successively with $H_2O$ (200 ml), 10% HCl (200 ml) and $H_2O$ (200 ml). The organic extract was evaporated under reduced pressure, and the residue was dissolved in 80:20:2 $CHCl_3$: MeOH: $H_2O$ (22 ml). The solution was purified in three portions by flash chromatography eluting with 90:10:1 $CHCl_3$: MeOH: $H_2O$ to provide the crude product as an off-white solid (950 mg). This material was dissolved in 5% $MeOH/CHCl_3$ (75 ml), filtered and further purified in portions of 8 ml each by HPLC using a 1" x 25 cm silica column eluting with 5% $MeOH/CHCl_3$ to afford the titled compound as a white solid (721 mg, 68%

yield).
NMR ($CD_3OD/CDCl_3$): δ 7.35 (5H, m), 7.22 (5H, m), 5.12 (2H, d), 4.36 (1H, d), 4.29 (1H, g), 4.16 (2H, m), 4.05 (1H, m), 3.73 (3H, s), 3.57 (1H, m), 2.92 (1H, dd), 2.81 (1H, dd), 2.32 (2H, m), 2.09 (2H, m), 1.72 (2H, m), 1.36 (3H, d), 1.27 (3H, d), 0.94 (12H, 2 overlapping d's).
MS (FAB): m/z 712.4 $(M+H)^+$.

(4S,5S) 5-(alanylalanyl)amino-4-hydroxy-1-oxo-6-phenylhexyl-valyl valine methyl ester

b) To a solution of the compound of Example 78(a) (715 mg, 1.01 mmol) in MeOH (45 ml) was added 10% palladium on activated carbon (100 mg). Hydrogen gas was introduced into the reaction mixture which was then maintained under a hydrogen atmosphere for 48 h. The mixture was filtered, and the solvent was removed in vacuo to afford the titled compound as a white solid (599 mg, 100% yield).
NMR ($CD_3OD$): δ 7.24 (5H, m), 4.32 (2H, m), 4.21 (1H, d), 4.03 (1H, br t), 3.94 (1H, g), 3.71 (3H, s), 3.59 (1H, br t), 2.86 (2H, 2 overlapping dd's), 2.31 (2H, t), 2.07 (2H, m), 1.68 (2H, m), 1.52 (3H, d), 1.32 (3H, d), 0.94 (12H, overlapping d's).
MS (FAB): m/z 578.3 $(M+H)^+$.

## Example 79

Preparation of (4S,5S) 5-(alanylalanyl)amino-4-hydroxy-1-oxo-6-cyclohexyl-hexyl-valyl valine methyl ester

To a solution of the compound of Example 78(b) (5.4 mg, 9.4 mmol) in MeOH (5 ml) was added $PtO_2$ - (10 mg), and the resulting mixture was shaken on a Parr hydrogenator at 60 psig $H_2$ for 2 d. The mixture was filtered, and the solvent was removed in vacuo to afford the titled compound as a white solid (3.4 mg, 62% yield).
NMR ($CD_3OD$): δ 4.42-4.15 (3H, m), 3.97 (2H, m), 3.71 (3H, s), 3.52 (1H, m), 2.35 (2H, t), 2.10 (2H, m), 1.85 (1H, br d), 1.80-1.15 (21H, m), 1.65 (12H, m).
MS (FAB): m/z 584.4 $(M+H)^+$.

## Example 80

Preparation of (4S,5S) 5-(serylalanylalanyl)amino-4-hydroxy-1-oxo-6-phenylhexyl-valyl valine methyl ester, acetic acid salt

(4S,5S) 5-(carbobenzyloxy-(O-phenylmethyl)serylalanylalanyl) amino-4-hydroxy-1-oxo-6-phenylhexyl-valyl valine methyl ester

a) To a solution of carbobenzyloxy-(O-benzyl)serine (311 mg, 0.945 mmol) and N-methyl morpholine (130 ml, 1.20 mmol) in THF (20 ml) at -40°C was added isobutyl chloroformate (123 ml, 0.945 mmol) over a 10 min period. After stirring an additional 30 min, the compound of Example 78(b) (520 mg, 0.900 mmol) was added in 20% DMF/THF (25 ml). The resulting mixture was allowed to slowly warm to room temperature and stirred for 20 h. The mixture was diluted with $CHCl_3$ (150 ml) and washed with $H_2O$ (2x). The organic extract was evaporated under reduced pressure. The solid residue (870 mg) was purified by flash chromatography eluting with 10% $MeOH/CHCl_3$ to afford material which was still slightly impure (640 mg). The product was dissolved in 4% $MeOH/CHCl_3$ (20 ml), filtered and further purified by HPLC in 6 portions using a 1" x 25 cm silica column eluting with 4% $MeOH/CH_2Cl_2$ to afford the titled compound as a white solid (414 mg, 52% yield).
NMR ($CD_3OD/CDCl_3$): δ 7.10 (10H, m), 7.01 (5H, m), 4.94 (2H, s), 4.35 (2H, s), 4.25-3.80 (6H, m), 3.55 (2H, m), 3.52 (3H, s), 3.38 (1H, m), 2.73 (1H, dd), 2.63 (1H, dd), 2.10 (2H, m), 1.90 (2H, m), 1.57 (2H, m), 1.17 (3H, d), 0.93 (3H, d), 0.73 2H, overlapping d's).

MS (FAB): m/z 889.4 $(M+H)^+$.

(4S,5S) 5-[(O-phenylmethyl)serylalanylalanyl]amino-1-oxo-6-phenylhexyl-valyl valine methyl ester, acetic acid salt

b) To a mixture of the compound of Example 80(a) (409 mg, 0.461 mmol) in 2:1 MeOH: HOAc (15 ml) was added 10% palladium on activated carbon (80 mg). Hydrogen gas was introduced into the reaction mixture which was then maintained under a hydrogen atmosphere for 16 h. The mixture was filtered through Celite® and evaporated under reduced pressure to afford the titled compound as a white solid (353 mg, 94% yield).
NMR ($CDCl_3$): δ 7.31 (5H, m), 7.24 (5H, m), 4.58 (2H, s), 4.42-4.18 (4H, m), 4.05 (1H, m), 3.94 (1H, m), 3.85-3.70 (2H, m), 3.70 (3H, s), 3.58 (1H, m), 2.91 (1H, dd), 2.81 (1H, dd), 2.30 (2H, m), 2.12 (2H, m), 1.98 (3H, s), 1.72 (2H, m), 1.37 (3H, d), 1.22 (3H, d), 0.94 (12H, m).
MS (FAB): m/z 755.3 $(M+H)^+$.

(4S,5S) 5-(serylalanylalanyl)amino-4-hydroxy-1-oxo-6-phenylhexyl-valyl valine methyl ester, acetic acid salt

c) To a solution of the compound of Example 80(c) (353 mg, 0.46 mmol) in MeOH (20 ml) was added 10% palladium on activated carbon (85 mg). Hydrogen gas was introduced into the mixture which was then maintained under a hydrogen atmosphere for 2 days. Acetic acid (5 ml) was added followed by additional 10% Pd/C (150 mg). After stirring an additional 4 days under a hydrogen atmosphere, the reaction mixture was filtered through Celite®, and the solvent was evaporated under reduced pressure to afford the titled compound (273 mg, 88% yield).
NMR ($CD_3OD$): δ 7.23 (5H, m), 4.43-4.18 (4H, m), 4.05 (1H, br t), 3.85 (2H, m), 3.70 (3H, s), 3.59 (2H, m), 2.92 (1H, dd), 2.80 (1H, dd), 2.30 (2H, m), 2.07 (2H, m), 1.96 (3H, s), 1.71 2H, m), 1.40 (3H, d), 1.25 (3H, d), 0.94 (12H, m).
MS (FAB): m/z 665.3 $(M+H)^+$.

## Example 81

Preparation of (4S,5S) 5-[(D-seryl)alanylalanyl]amino-4-hydroxy-1-oxo-6-phenylhexyl-valyl valine methyl ester

(4S,5S) 5-((carbobenzyloxy-D-seryl)alanylalanyl)amino-4-hydroxy-1-oxo-6-phenylhexyl-valyl valine methyl ester

a) To a solution of carbobenzyloxy-D-serine (8.4 mg, 35 mmol) and hydroxybenzotriazole (9.5 mg, 70 mmol) in DMF (8 drops) was added dicyclohexylcarbodiimide (7.2 mg, 35 mmol), and the resulting mixture was stirred for 20 min. The compound of Example 78(b) (20 mg, 35 mmol) was added followed by additional DMF (7 drops), and the mixture was stirred for 2 days. The mixture was diluted with 10% $MeOH/CHCl_3$ and washed successively with 10% HCl, 5% aqueous $NaHCO_3$ and $H_2O$. The solvent was removed in vacuo, and the residue was purified by flash chromatography eluting with 10% $MeOH/CHCl_3$ to afford the titled compound (4.2 mg, 14% yield).
NMR ($CDCl_3/CD_3OD$): δ 7.26 (5H, s), 7.17 (5H, m), 5.11 (1H, d), 4.96 (1H, d), 4.35-4.10 (5H, m), 3.96 (1H, m), 3.83 (1H, dd), 3.72 (1H, dd), 3.64 (3H, s), 3.47 (1H, m), 2.87 (1H, dd), 2.72 (1H, dd), 2.34-1.95 (4H, m), 1.70 (2H, m), 1.34 (3H, d), 1.24 (3H, d), 0.87 (12H, d).
MS (FAB): m/z 799.5 $(M+H)^+$.

(4S,5S) 5-((D-seryl)alanylalanyl)amino-4-hydroxy-1-oxo-6-phenylhexyl-valyl valine methyl ester

b) To a solution of the compound of Example 81(a) (3.9 mg, 4.9 mmol) in MeOH (2 ml) was added 10%

palladium on activated carbon (2 mg). Hydrogen gas was introduced into the reaction mixture which was then maintained under a hydrogen atmosphere for 2 d. The mixture was filtered through Celite® and evaporated under reduced pressure to afford the titled compound as a white solid (3.4 mg, 98% yield).
NMR ($CD_3OD$): δ 7.22 (5H, m), 4.25 (4H, m), 4.05 (1H, m), 3.82 (2H, m), 3.69 (3H, s), 3.55 (1H, m), 2.90 (1H, dd), 2.78 (1H, dd), 2.29 (2H, br t), 2.05 (2H, m), 1.69 (2H, m), 1.37 (3H, d), 1.25 (3H, d), 0.92 (12H, overlapping d's).
MS (FAB): m/z 665.4 $(M+H)^+$.

## Example 82

Preparation of (4RS,5S) 5-amino-6-(4-benzyloxy)phenyl-4-hydroxy-1-oxo-hexyl-valyl valine benzyl ester,hydrochloride

(5S) 6-(4-benzyloxy)phenyl-5-(t-butyloxycarbonyl)amino-(1,4-dioxo)hexyl-valyl valine benzyl ester

a) To a solution of the compound of Example 13(c) (43 mg, 0.10 mmol) in THF at -40°C under an argon atmosphere was added N-methyl morpholine (16 ml, 0.14 mmol) followed by isobutyl chloroformate (13 ml, 0.10 mmol). After stirring 15 min, additional N-methyl morpholine (16 ml, 0.14 mmol) was added followed by valyl valine benzyl ester hydrochloride (51 mg, 0.15 mmol). The resulting mixture was allowed to warm to room temperature and stirred overnight. The reaction mixture was diluted with 5% HCl and extracted with $CHCl_3$ (3x). The combined organic extracts were evaporated, and the residue was purified by flash chromatography eluting with 1: 25 MeOH: $CHCl_3$ to afford the titled compound as a white solid (62 mg, 100% yield).
NMR ($CDCl_3$): δ 7.34 (10H, m), 7.07 (2H, d), 6.89 (2H, d), 6.52 (1H, d), 6.31 (1H, d), 5.2 (1H, m), 5.16 (2H, dd), 5.02 (2H, s), 4.59 (1H, dd), 4.49 (1H, dd), 4.30 (1H, dd), 3.08 (1H, dd), 2.84 (3H, m), 2.46 (2H, m), 2.13 (2H, m), 1.86 (1H, br s), 1.39 (9H, s), 0.91 (12H, m).

(4RS,5S) 6-(4-benzyloxy)phenyl-5-(t-butyloxycarbonyl)amino-4-hydroxy-1-oxo-hexyl-valyl valine benzyl ester

b) To a solution of the compound of Example 82(a) (61 mg, 0.10 mmol) in 5:1 MeOH: $CH_2Cl_2$ (6 ml) was added sodium borohydride (5 mg, 0.13 mmol) and the resulting mixture was stirred for 20 min. The reaction mixture was diluted with 5% aqueous HCl and extracted several times with $CH_2Cl_2$. The combined organic extracts were dried ($MgSO_4$), filtered, and evaporated under reduced pressure to give the titled compound as a white solid (61 mg, 100% yield).
NMR ($CDCl_3/CD_3OD$): δ 7.35 (10H, m), 7.12 (2H, d), 6.90 (2H, d), 5.17 (2H, dd), 5.02 (2H, s), 4.47 (1H, m), 4.17 (1H, apparent t), 3.68 (1H, m), 3.53 (1H, m), 2.96-2.55 (2H, m), 2.36 (2H, m), 2.19 (1H, m), 2.10-1.81 (2H, m), 1.70 (1H, m), 1.35 (9H, 2 singlets), 0.91 (12H, m).
MS (FAB): m/z 718.5 $(M+H)^+$.

(4RS,5S) 5-amino-6-(4-benzyloxy)phenyl-4-hydroxy-1-oxo-hexyl-valyl valine benzyl ester,hydrochloride

c) To a mixture of the compound of Example 82(b) (60 mg, 97 mmol) in $CH_2Cl_2$ (2 ml) there was added trifluoroacetic acid (0.5 ml), and the resulting solution was stirred for 2 h. Methanol was added followed by conc. HCl (2 drops), and the solution was evaporated under reduced pressure. The residue was triturated with $Et_2O$, giving the titled compound as a white solid (56 mg, 100% yield).
NMR ($CD_3OD$): δ 7.36 (10H, m), 7.20 (2H, d), 6.97 (2H, d), 5.15 (2H, m), 5.07 (2H, s), 4.34 (1H, m), 4.21 (1H, m), 3.84-3.53 (1H, m), 3.44-3.20 (1H, m), 2.97 (1H, m), 2.80 (1H, m), 2.44 (2H, m), 2.15 (1H, m), 2.02 (1H, m), 1.94-1.62 (2H, m), 0.94 (12H, m).

(4RS,5S) 5-amino-6-(4-hydroxy)phenyl-4-hydroxy-1-oxo-hexyl-valyl valine benzyl ester,hydrochloride

c) The compound of Example 82(c) (50 mg) is dissolved in acetic acid and stirred with 10% palladium on activated carbon(50 mg). Hydrogen gas is bubbled through the solution for 2 h. and the reaction is stirred for 20 h. under a hydrogen atmosphere. The suspension is filtered through a pad of Celite® and evaporated under reduced pressure to yield the titled compound.

## Example 83

Preparation of (4RS,5S) 5-(serylalanylalanyl)amino-6-(4-hydroxy)phenyl-4-hydroxy-1-oxo-hexyl-valyl valine, hydrochloride

(4RS,5S) 5-[(t-butyloxycarbonyl-(O-phenylmethyl)serylalanylalanyl)-amino]-6-(4-benzyloxy)phenyl-4-hydroxy-1-oxohexyl-valyl valinol benzyl ester

a) To a solution of t-butyloxycarbonyl-(O-benzyl)serylalanyl alanine (46.8 mg, 107 mmol) in THF (1 ml) at -40°C under an argon atmosphere was added N-methyl morpholine (16.5 ml, 150 mmol) followed by isobutyl chloroformate (13.8 ml, 107 mmol). After stirring 15 min, additional N-methyl morpholine (16.5 ml, 150 mmol) was added followed by a solution of the compound of Example 82(c) (56 mg, 97 mmol) in THF (0.75 ml). The resulting mixture was allowed to warm to room temperature and stirred overnight. The reaction mixture was diluted with 5% HCl and extracted with $CH_2Cl_2$ several times. The combined organic extracts were evaporated under reduced pressure, and the solid residue was purified by flash chromatography eluting with 1: 9 MeOH: $CHCl_3$ to give the titled compound (79.5 mg, 87% yield).
NMR ($CDCl_3$/$CD_3OD$): δ 7.32 (15H, m), 7.14 (2H, m), 6.84 (2H, m), 5.16 (2H, dd), 5.00 (2H, d), 4.56-4.40 (2H, 2d), 4.21-3.53 (9H, m), 3.10-2.65 (2H, m), 2.48-2.28 (2H, m), 2.25-2.0 (2H, m), 1.96-1.68 (2H, m), 1.50 (9H), 1.39 (3H, overlapping d's), 1.17 (3H, 2d's), 0.96 (12H, m).
MS (FAB): m/z 1037.4 $(M+H)^+$.

(4RS,5S) 5-(t-butyloxycarbonylserylalanylalanyl)amino-6-(4-hydroxyl)phenyl-4-hydroxy-1-oxo-hexyl-valyl valine

b) To a solution of the compound of Example 83(a) (79 mg, 76 mmol) prepared above in 3:1 MeOH: HOAc (5 ml) was added 10% palladium on activated carbon (45 mg). Hydrogen was introduced into the mixture which was then maintained under 1 atm $H_2$ for 24 h. The reaction mixture was filtered through a pad of Celite®, and the solvent was removed under reduced pressure. The residue was purified by flash chromatography eluting with a gradient from 90: 10: 1: 1 to 80: 20: 2: 1 $CHCl_3$: MeOH: $H_2O$: $HCO_2H$ to afford the titled compound as a white solid (37.5 mg, 64% yield).
NMR ($CD_3OD$): δ 7.03 (2H, m), 6.66 (2H, m), 4.4-4.05 (5H, m), 4.03-3.50 (4H, m), 3.06-2.54 (2H, m), 2.38 (2H, m), 2.14 (2H, m), 1.96-1.62 (2H, m), 1.48 (9H), 1.38 (3H, m), 1.22 (3H, 2d's), 0.96 (12H, d).
MS (FAB): m/z 767.3 $(M+H)^+$.

(4RS,5S) 5-(serylalanylalanyl)amino-6-(4-hydroxy)phenyl-4-hydroxy-1-oxo-hexyl-valyl valine, hydrochloride

c) The compound of Example 83(b) (5.9 mg, 7.7 mmol) was treated with trifluoroacetic acid (0.25 ml), and the resulting solution was stirred for 90 min. Methanol was added followed by conc. HCl (1 drop), and the mixture was evaporated under reduced pressure. The residue was triturated with $Et_2O$ to afford the titled compound as a white powder.
NMR ($CD_3OD$): δ 7.03 (2H, m), 6.68 (2H, m), 4.42-4.17 (5H, m), 3.92 (4H, m), 3.04-2.54 (2H, m), 2.33 (2H, m), 2.22-1.97 (2H, m), 1.95-1.55 (2H, m), 1.35 (3H, overlapping d's), 1.26 (3H, overlapping d's), 0.98 (12H, m).
MS (FAB): m/z 667.4 $(M+H)^+$.

## Example 84

Purification of (1RS,5S) 5-(t-butyloxycarbonylserylalanylalanyl)amino-4-hydroxy-6-(4-hydroxy)phenyl-1-oxo-hexyl-valyl amide

(5S) 6-(4-benzyloxydiphenyl-5-(t-butyloxycarbonyl)amino-1,4-dioxo-hexyl-valyl amide

a) To a solution of the compound of Example 13(c) (25 mg, 58.5 µmol) in THF at -40°C under an argon atomosphere was added N-methyl morpholine (9.9 µl,90 µmol) followed by isobutyl chloroformate (7.6 ml, 58.5 mmol). After stirring for 15 min, valinamide (14 mg, 120 mmol) was added, and the resulting mixture was allowed to warm to room temperature and stirred overnight. The reaction mixture was diluted with 5% HCl and extracted with $CHCl_3$ three times. The solvent was removed in vacuo, and the residue was purified by flash chromatography eluting with 1: 9 MeOH: $CHCl_3$ to afford the titled compound as a yellow solid (23.5 mg, 75% yield).
NMR ($CD_3OD/CDCl_3$): δ 7.36 (5H, m), 7.12 (2H, d), 6.91 (2H, d), 5.05 (2H, s), 4.33 (1H, m), 4.22 (1H, m), 3.07 (1H, dd), 2.83 (3H, m), 2.52 (2H, t), 2.13 (1H, septet), 1.39 (9H, s), 0.97 (6H, overlapping d's).

(4RS,5S) 6-(4-benzyloxy)phenyl-5-(t-butyloxycarbonyl)amino-4-hydroxy-1-oxo-hexyl-valyl amide

b) To a solution of the compound of Example 84(a) (23 mg, 44 mmol) prepared above in 1: 4 $CH_2Cl_2$: MeOH (2.5 ml) was added $NaBH_4$ (3 mg, 80 mmol). After stirring 15 min, the mixture was diluted with 5% HCl and extracted with $CH_2Cl_2$ several times. The combined organic extracts were washed with $H_2O$ and dried ($MgSO_4$). Filtration and removal of the solvent in vacuo provided the titled compound as a white solid (23 mg, 100% yield).
NMR ($CD_3OD/CDCl_3$): δ 7.36 (5H, m), 7.13 (2H, d), 6.90 (2H, d), 5.04 (2H, s), 4.20 (1H, apparent t), 3.67 (1H, m), 3.53 (1H, m), 3.02-2.30 (4H, m), 2.10 (1H, m), 2.02-1.61 (2H, m), 1.35 (9H, 2 singlets), 0.97 (6H, overlapping d's).
MS (FAB): m/z 528.3 $(M+H)^+$.

(4RS,5S)-6-(4-benzyloxy)phenyl-5-amino-4-hydroxy-1-oxo-hexyl-valyl amide, hydrochloride

c) The compound of Example 84(b) (22 mg, 42 mmol) was dissolved in neat trifluoroacetic acid (0.1 ml). After 1 h, methanol was added followed by conc. HCl (2 drops). Removal of the solvent in vacuo gave the titled compound as a hygroscopic solid (22 mg).
NMR ($CD_3OD$): δ 7.35 (5H, m), 7.20 (2H, 2 doublets), 6.98 (2H, 2 doublets), 5.07 (2H, s), 4.21 (1H, m), 3.83-3.53 (1H, m), 3.47-3.21 (1H, m), 2.98 (1H, m), 2.81 (1H, m), 2.43 (2H, m), 2.09 (1H, m), 1.98-1.62 (2H, m), 0.98 (6H, m).

(4RS,5S) 5-(t-butyloxycarbonyl-(O-phenylmethyl)serylalanylalanyl)amino-6-(4-benzyloxy)phenyl-4-hydroxy-1-oxo-hexyl-valyl amide

d) To a solution of t-butyloxycarbonyl-(O-phenylmethyl)serylalanyl alanine (20 mg, 46 mmol) in THF (1 ml) at -40°C under an argon atmosphere was added N-methyl morpholine (8.2 ml, 75 mmol) followed by isobutyl chloroformate (6.0 ml, 46 mmol). The resulting mixture was stirred for 15 min, and additional N-methyl morpholine (8.2 ml, 75 mmol) was added followed by a solution of the compound of Example 84(c) (22 mg) in THF (0.8 ml). The resulting mixture was allowed to warm to room temperature and stirred overnight. The reaction mixture which solidified overnight was dissolved in 1:1 $CHCl_3$: MeOH and washed with 5% HCl. The organic extract was evaporated under reduced pressure. The residue (51 mg) was dissolved in 9:1:0.5 $CHCl_3$: MeOH: acetic acid (2 ml), applied to a 6" x 15 mm flash silica column and eluted with 9:1 $CHCl_3$: MeOH to afford a yellow solid (19.5 mg). Analytical HPLC (silica; 9:1 $CH_2Cl_2$: MeOH mobile phase; detector 275 nm) indicated a 2: 1 ratio of 4-hydroxy diastereomers. Further purification was effected by flash chromatography on silica using 80: 20: 2 $CHCl_3$: MeOH: $H_2O$, to yield the titled compound (13.8 mg, 39% yield) as a white solid.

NMR ($CD_3OD/CDCl_3/DMSO-d_6$): δ 7.32 (10H, m), 7.12 (2H, m), 6.82 (2H, m), 5.00 (2H, s), 4.19 (5H, m), 4.00 (1H, m), 3.68 (4H, m), 3.10-2.60 (2H, m), 2.42 (2H, m), 2.13 (1H, m), 1.99-1.63 (2H, m), 1.50 (9H, s), 1.37 (3H, overlapping d's), 1.12 (3H, d), 0.95 (6H, m).
MS (FAB): m/z 847.5 $(M+H)^+$.

(4RS,5S) 5-(t-butyloxycarbonylserylalanylalanyl)amino-4-hydroxy-6-(4-hydroxy)phenyl-1-oxo-hexyl-valyl amide

e) To a solution of the compound of Example 84(d) (13.8 mg, 16.3 mmol) prepared above in MeOH (5 ml) was added 10% palladium on activated carbon (7 mg). Hydrogen gas was bubbled into the resulting mixture which was then maintained at 1 atm $H_2$ for 24 h. Filtration and evaporation of the solvent under reduced pressure provided the titled compound as a white solid (10.7 mg, 99% yield).
NMR ($CD_3OD$): δ 7.05 (2H, 2 doublets), 6.64 (2H, 2 doublets), 4.18 (5H, m), 3.95 (1H, m), 3.88-3.67 (3H, m), 3.60 (1H, m), 3.09-2.53 (2H, m), 2.37 (2H, m), 2.11 (1H, m), 2.00-1.65 (2H, m), 1.47 (9H, 2s), 1.38 (3H, overlapping d's), 1.21 (3H, 2 doublets), 0.95 (6H, m).
MS (FAB): m/z 667.2 $(M+H)^+$.

## Example 85

Preparation of (1R,2R)-2-(((1S,2S)-1-hydroxy-2-amino-3-phenylhexyl) propyl)cyclopentanecarbonyl-valyl valine methyl ester, hydrochloride

(1R,2R) 2-(((1S,2S)-1-hydroxy-2-t-butyloxycarbonylamino-3-phenyl)propyl)-cyclopentanecarbonyl-valyl valine methyl ester

a) Using the procedure of Example 27(a), the compound of Example 6(b) (54 mg, 150 µmol) was reacted with N-hydroxy benzotriazole (40 mg, 300 µmol), DCC (33 mg, 170 µmol), N-methyl morpholine (40 µl, 380 µmol) and valyl valine methyl ester hydrochloride (80 mg, 300 µmol) to afford the titled compound.

(1R,2R) 2-(((1S,2S)-1-hydroxy-2-t-butyloxycarbonylamino-3-cyclohexyl)propyl)-cyclopentanecarbonyl-valyl valine methyl ester

b) A solution of the compound of Example 85(a) (10 mg, 17.4 mmol) in MeOH (3 ml) was shaken with $PtO_2$ (10 mg) on a Parr hydrogenator at 60 psig $H_2$. After 26 h, the mixture was filtered through a pad of Celite® and evaporated under reduced pressure. The residue was purified by flash chromatography eluting with 25:1 $CHCl_3$: MeOH to afford the titled compound (9 mg, 89% yield).
NMR ($CDCl_3$): δ 6.58 (1H, d), 6.44 (1H, d), 4.68 (1H, d), 4.51 (1H, dd), 4.23 (1H, br t), 3.74 (3H, s), 3.60 (1H, m), 3.47 (1H, m), 2.68 (1H, br t), 2.39-2.02 (4H, m), 2.0-1.5 (12H, m), 1.45 (9H, s), 1.40-1.08 (6H, m), 0.94 (12H, m).

(1R,2R)-2-(((1S,2S)-1-hydroxy-2-amino-3-cyclohexyl)propyl)cyclopentanecarbonyl-valyl valine methyl ester, hydrochloride

c) The compound of Example 85(b) (9 mg, 15.5 mmol) was dissolved in neat trifluoroacetic acid and stirred for several min. The solution was evaporated under reduced pressure, the residue dissolved in MeOH, and treated with conc. HCl. Removal of the solvent in vacuo gave the titled compound.

## Example 86

Preparation of (1R,2R) 2-(((1S,2S)-1-hydroxy-2-(serylalanylalanyl)amino-3-cyclohexyl)propyl) cyclopentanecarbonyl-valyl valine methyl ester, hydrochloride

(1R,2R) 2-(((1S,2S)-1-hydroxy-2-(t-butyloxycarbonyl-(O-phenylmethyl)serylalanylalanyl)amino-3-cyclohexyl)-propyl) cyclopentanecarbonyl-valyl valine methyl ester

a) To a solution of t-butyloxycarbonyl-(O-benzyl)serylalanyl alanine (6.6 mg, 15 mmol) in THF (0.5 ml) at -40°C under an argon atmosphere was added N-methyl morpholine (2.2 ml, 20 mmol) followed by isobutyl chloroformate (2.0 ml, 15 mmol). The resulting mixture was stirred for 15 min, and additional N-methyl morpholine (2.2 ml, 20 mmol) was added followed by a solution of the compound of Example 85(c) (15 mmol) in THF (0.5 ml). The resulting mixture was allowed to warm to room temperature and stirred overnight. The mixture was partitioned between EtOAC and dilute HCl. The organic extract was washed with aqueous $NaHCO_3$, saturated aqueous NaCl and dried ($MgSO_4$). Filtration and removal of the solvent in vacuo gave a white solid (12 mg). The product was purified by flash chromatography eluting with 25:1 $CHCl_3$: MeOH to afford the titled compound as a colorless glass (6.2 mg, 46% yield).
NMR ($CDCl_3$): δ 7.35 (5H, m), 7.0-6.6 (5H, m), 5.49 (1H, br d), 4.54 (2H, s), 4.52-4.08 (5H, m), 3.99 (1H, br), 3.78 (2H, m), 3.72 (3H, s), 3.42 (1H, br t), 2.71 (1H, m), 2.20 (4H, m), 1.95-1.55 (12H, m), 1.46 (9H, s), 1.40 (6H, d), 1.35-1.06 (6H, m), 0.94 (12H, m).

(1R,2R) 2-(((1S,2S)-1-hydroxy-2-(t-butyloxycarbonylserylalanyl-alanyl)amino-3-cyclohexyl)propyl)-cyclopentanecarbonyl-valyl valine methyl ester

b) To a solution of 86(a) (6.2 mg, 6.9 mmol) in MeOH (1 ml) was added 10% palladium on activated carbon (5mg). Hydrogen gas was introduced into the reaction mixture which was then maintained under 1 atm $H_2$ for 6 h. The mixture was filtered through a pad of Celite®, and the solvent was removed in vacuo to afford the titled compound as a colorless glass (6 mg).
NMR ($CDCl_3/CD_3OD$): δ 4.43 (1H, d), 4.38-4.05 (4H, m), 3.85 (2H, m), 3.73 (3H, s), 3.62 (1H, m), 3.45 (1H, m), 2.68 (1H, m), 2.30-2.05 (4H, m), 1.9-1.55 (12H, m), 1.46 (9H, s), 1.40 (6H, overlapping d's), 1.35-1.10 (6H, m), 0.94 (12H, m).
MS (FAB): m/z 811.5 $(M+H)^+$.

(1R,2R) 2-(((1S,2S)-1-hydroxyl-2-(serylalanylalanyl)amino-3-cyclohexyl)propyl)cyclopentanecarbonyl-valyl valine methyl ester, hydrochloride

c) The compound of Example 86(b) (6 mg, 7 mmol) was dissolved in a few drops of trifluoroacetic acid. After stirring at room temperature for 40 min, the solution was diluted with MeOH and one drop of conc. HCl/dioxane was added. The resulting mixture was evaporated under reduced pressure to afford the titled compound.
NMR ($CD_3OD$): δ 4.50-4.15 (4H, m), 3.93 (4H, m), 3.71 (3H, s), 3.45 (1H, t), 2.65 (1H, m), 2.29 (1H, m), 2.2-2.0 (3H, m), 1.93-1.53 (12H, m), 1.5-1.1 (6H, m), 1.41 (3H, d), 1.35 (3H, d), 0.96 (12H, m).
MS (FAB): m/z 711.5 $(M+H)^+$.

## Example 87

(4S,5S)-5-(methoxycarbonyl-alanylalanyl)amino-6-phenyl-4-hydroxy-(1-oxo)hexyl-valyl valine

(5S)-5-(tert-butyloxycarbonyl)amino-6-phenyl-(1,4-dioxo)hexyl-valyl valine benzyl ester

a) To a solution of (5S)-5-(tert-butyloxycarbonyl)amino-4-oxo-6 phenylhexanoic acid (160 mg, 0.500 mmol) and N-methyl morpholine (66 µl, 0.60 mmol) in THF (4 ml) at -40°C was added isobutyl chloroformate (65 µl, 0.50 mmol) over a 5 min period. After stirring an additional 15 min, more N-methylmorpholine (66 µl) was added, followed by valyl valine benzyl ester hydrochloride (205 mg, 0.60 mmol) in DMF (1.5 ml). The resulting mixture was allowed to slowly warm to room temperature and stirred for 15 h. The mixture was diluted with $CHCl_3$ and washed with 10% HCl then $H_2O$. The organic extract was evaporated under reduced pressure and the residue was purified by flash chromatography (25:1 $CHCl_3$:MeOH) to provide the titled compound as a white solid (253 mg, 85% yield).

(4S,5S)-5-(tert-butyloxycarbonyl)amino-4-hydroxy-6-phenyl-(1-oxo)hexyl-valyl valine benzyl ester

b) The product of Example 87(a) (253 mg, 0.415 mmol) was dissolved in 5 ml MeOH, and $NaBH_4$ (17 mg, 0.45 mmol) was added. After 20 min stirring, the mixture was diluted with 5% HCl and extracted with $CH_2Cl_2$. The solvent was removed and the residue was purified by HPLC on silica eluting with 2% MeOH in $CH_2Cl_2$ to provide the titled compound (75.6 mg), which eluted first, followed by the corresponding (4R) diastereomer (135 mg).

(4S,5S)-5-amino-4-hydroxy-6-phenyl-(1-oxo)hexyl-valyl valine benzyl ester hydrochloride

c) The product of Example 87(b) (75 mg) was dissolved in TFA (0.5 ml). After 5 min MeOH (2 ml) was added followed by conc. HCl (50 µl). The solution was concentrated to a gum, triturated with ether and dried to yield a white powder (70 mg).

(4S,5S)-5-(methoxycarbonyl-alanylalanyl)amino-4-hydroxy-6-phenyl-(1-oxo)hexyl-valyl valine benzyl ester

d) To a solution of methoxycarbonylalanylalanine (29.7 mg, 0.136 mmol) and N-methyl morpholine (17 µl, 0.15 mmol) in THF (2 ml) at -40°C was added isobutyl chloroformate (17.6 µl, 0.136 mmol) over a 5 min period. After stirring an additional 15 min, more N-methylmorpholine (17 µl) was added, followed by a solution of (4S,5S)-5-amino-4-hydroxy-6-phenyl-(1-oxo)hexyl-valyl valine benzyl ester hydrochloride (68 mg, 0.124 mmol) in 2:1 THF:DMF (1.5 ml). The resulting mixture was allowed to slowly warm to room temperature and stirred for 15 h. The mixture was diluted with 9:1 $CHCl_3$:MeOH and washed with 10% HCl then $H_2O$. The organic extract was evaporated under reduced pressure to provide the titled compound as a white solid (95 mg).
TLC (9:1 $CHCl_3$:MeOH): $R_f$ = 0.5.
NMR ($CD_3OD$/ $CDCl_3$): δ 7.4-7.25 (10H, m), 5.17 (2H, dd), 4.45 (1H, obsc.), 4.30 (1H, m), 4.18-4.00 (3H, m), 3.69 (3H, s), 3.56 (1H, m), 2.88 (2H, ddd), 2.32 (2H, m), 2.19 (1H, m), 2.03 (1H, m), 1.72 (2H, m), 1.34 (3H, d), 1.28 (3H, d), 0.91 (12H, overlapping d's).
MS (FAB): m/z 712.4 $(M+H)^+$.

(4S,5S)-5-(methoxycarbonyl-alanylalanyl)amino-6-phenyl-4-hydroxy-(1-oxo)hexyl-valyl valine

e) To a mixture of the product of Example 87(d) (85 mg, 0.12 mmol) in MeOH (5 ml) was added 10% palladium on activated carbon (13 mg). Hydrogen gas was introduced into the reaction mixture which was then maintained under a hydrogen atmosphere for 3.5 h. The mixture was filtered through Celite® and evaporated under reduced pressure to afford the titled compound as a white solid (70.5 mg, 95% yield).
NMR ($CD_3OD$): δ 7.23 (5H, m), 4.37-4.00 (5H, m), 3.66 (3H, s), 3.58 (1H, m), 2.88 (2H, ddd), 2.32 (2H, m), 2.18 (1H, m), 2.06 (1H, m), 1.73 (2H, m), 1.32 (3H, d), 1.26 (3H, d), 0.95 (12H, m).
MS (FAB): m/z 622.3 $(M+H)^+$.

## Example 88

Preparation of (4S,5S)-5-(carbobenzyloxy-alanylalanyl)amino-6-phenyl-4-hydroxy-(1-oxo)hexyl-valyl valinol

To a solution of (4S,5S)-5-(carbobenzyloxy-alanylalanyl)amino-6-phenyl-4-hydroxy-(1-oxo)hexyl-valyl

valine methyl ester (4.8 mg) in 9:1 $CHCl_3$:MeOH (1 ml) was added excess $LiBH_4$ (ca. 5 mg). After 10 min the solution was diluted with 5% HCl, and extracted with 9:1 $CHCl_3$:MeOH. The organic layer was washed with water and concentrated. The residue was purified by flash chromatography 9:1 $CHCl_3$:MeOH) to provide the titled compound (1.9 mg) as a white solid.
NMR: ($CD_3OD/CDCl_3$): δ 7.2-7.0 (10H, m), 4.93 (2H, d), 4.1 (1H, obsc.), 4.00-3.80 (3H, m), 3.50-3.35 (4H, m), 2.68 (2H, ddd), 2.13 (2H, m), 1.92 (1H, m), 1.69 (1H, m), 1.54 (2H, m), 1.16 (3H, d), 1.08 (3H, d), 0.75 (12H, m).
MS (FAB): m/z 684.3 $(M+H)^+$.

## Example 89

### Preparation of (4S,5S)-5-(4S,5S) 5-(carbobenzyloxyalanylalanyl)-amino-4-hydroxy-6-phenyl-(1-oxo)hexyl-valine isobutylamide

(5S)-5-(tert-butyloxycarbonyl)amino-4-oxo-6-phenylhexanoic acid benzyl ester

a) To a solution of (5S)-5-(tert-butyloxycarbonyl)amino-4-oxo-6-phenylhexanoic acid (1 mmol) in dry acetonitrile (10 ml) was added DBU (1 mmol) followed by benzyl bromide (1.5 mmol) with stirring. After 2 h the mixture was diluted with 5% HCl and extracted with $CH_2Cl_2$. The organic layer was concentrated, and the residue was purified by flash chromatography ($CHCl_3$) to provide the ester (370 mg).
NMR ($CDCl_3$): δ 7.4-7.0 (10H, m), 5.10 (2H, s), 5.03 (1H, d), 4.51 (1H, q), 3.18-2.83 (2H, m), 2.73 (2H, m), 2.60 (2H, m), 1.39 (9H, s).
MS (FAB): m/z 412 $(M+H)^+$.

(4S,5S)-5-(tert-butyloxycarbonyl)amino-4-(tert-butyldimethylsiloxy)-6-phenylhexanoic acid benzyl ester.

b) The product of Example 89(a) is reduced with $NaBH_4$ in MeOH by the procedure of Example 87(b) (96 - 98% yield). The resulting crude diastereomeric mixture of alcohols is dissolved in DMF at a concentration of ca. 2M, and imidazole (2.4 equiv.) and tert-butyldimethylsilyl chloride (1.2 equiv.) are added. The mixture is stirred for 2 days, then diluted with water and extracted with ethyl acetate. The organic layer is dried ($MgSO_4$) and concentrated. The residue is purified by flash chromatography (5:1 hexanes:EtOAc) followed by HPLC (15:1 hexanes:EtOAc) to provide the titled compound (25 % yield), which elutes first, followed by the corresponding (4R)-siloxy benzyl ester (70 % yield).
(4S)-isomer:
NMR ($CDCl_3$):δ 7.4-7.12 (10H, m), 5.08 (2H, s), 4.67 (1H, d), 3.85 (1H, q), 3.68 (1H, m), 2.72 (2H, m), 2.36 (2H, m), 1.78 (2H, m), 1.41 (9H, s), 0.88 (9H, s), 0.10 (6H, d).
MS (FAB): m/z 529 $(M+H)^+$.

(4S,5S)-5-(tert-butyloxycarbonyl)amino-4-(tert-butyldimethylsiloxy)-6-phenylhexanoic acid

c) Hydrogenolysis of the product of Example 89(c) by the procedure of Example 87(e) provided the titled compound (99 % yield) as a crispy foam.
NMR ($CDCl_3$): δ 7.35-7.10 (5H, m), 4.69 (2H, br d), 3.88 (1H, q), 3.70 (1H, m), 2.72 (2H, m), 2.31 (2H, m), 1.78 (2H, m), 1.30 (9H, s), 0.89 (9H, s), 0.10 (6H, d).
MS (FAB): m/z 438.2 $(M+H)^+$.

(4S,5S)-5-(carbobenzyloxyalanylalanyl)amino-4-(tert-butyldimethylsiloxy)-6-phenyl-(1-oxo)hexyl-valine isobutylamide

d) The product of Example 89(c) was coupled with valine isobutylamide hydrochloride by the procedure of Example 87(a) to provide crude (4S,5S)-5-(tert-butyloxycarbonyl)amino-4-(tert-butyldimethylsiloxy)-6-phenylhexanoyl-valine isobutylamide (80 % yield), which was converted directly to the corresponding hydrochloride by the procedure of Example 87(c). The crude hydrochloride was in turn coupled to carbobenzyloxyalanine by the procedure of 1-step-1 followed by flash chromatography (20:1 $CHCl_3$:MeOH) to yield the titled compound (60 % yield) as a white solid.
NMR ($CDCl_3$): δ 7.45-7.10 (10H, m), 6.38 (1H, m), 5.08 (2H, m), 4.21 (2H, m), 3.70 (1H, m), 2.73 (2H, m), 2.33 (1H, m), 2.12 (2H, m), 1.25(4H, m), 0.91 (22H, m), 0.10 (6H, d).
MS (FAB): m/z 697.3 $(M+H)^+$.

(4S,5S)-5-(carbobenzyloxyalanylalanyl)amino-4-hydroxy-6-phenyl-(1-oxo)hexyl-valine isobutylamide

e) To the product of Example 89(d) in THF was added tetrabutylammonium bromide (5 equiv.). After stirring for 14 h, the mixture was diluted with water and extracted with dichloromethane. Removal of solvent afforded the titled compound as a white solid.
NMR ($CDCl_3/CD_3OD$): δ 7.28-7.05 (10H, m), 4.96 (2H, s), 4.02-3.76 (4H, m), 3.47 (1H, m), 2.9 -2.60 (4H, m), 2.13 (2H, m), 1.82 (1H, q), 1.55(3H, m), 1.09 (4H, d), 0.70 (12H, d).
MS (FAB): m/z 583.3 $(M+H)^+$.

## Example 90

Preparation of (4RS, 5S)-5-((tert-butyloxycarbonyl)isoleucyl)amino-7-methyl-4-hydroxy-1-oxo-octyl-leucyl-aspartic acid methyl ester

(5S)-5-(tert-butyloxycarbonyl)amino-7-methyl-4-oxo-octanoic acid

a) The titled compound was prepared by the procedures of examples 12(a,b), except using Boc-leucine N-methoxy-N-methyl amide in place of Boc-phenylalanine N-methoxy-N-methyl amide .
NMR ($CDCl_3$): δ 5.0 (1H, d), 4.35 (1H, m), 3.5 (1H, s), 2.8 (2H, m), 2.6 (3H, t), 1.5 (9H, s), 0.90 (8H, d).

(5S)-5-(tert-butyloxycarbonyl)amino-7-methyl-(1,4)-dioxo-octyl-leucyl-(O-benzyl)aspartic acid methyl ester

b) The titled compound was prepared by the coupling procedure of Example 34(a), using the product of Example 90(a) and leucyl-(O-benzyl)aspartic acid methyl ester hydrochloride.

(4RS,5S)-5-(tert-butyloxycarbonyl)amino-7-methyl-4-hydroxy-1-oxo-octyl-leucyl-(O-benzyl)aspartic acid methyl ester

c) The product of Example 90(b) was reduced with excess $NaBH_3CN$ in acetic acid • (2 days) to provide the titled compound as a 1:1 mixture of 4R and 4S diastereomers after aqueous workup and flash chromatography.
NMR ($CDCl_3$): δ 7.35 (5H, s), 7.05 (1H, d), 6.15 (1H, m), 5.15 (2H, s), 4.85 (2H, m), 4.55 (1H, d), 4.4 (1H, m), 3.65 (3H, s), 2.9 (2H, m), 2.4 (2H, t), 1.5 (9H, s), 0.90 (12H, m).
MS (FAB): 622.3 $(M+H)^+$.

(4RS,5S)-5-((tert-butyloxycarbonyl)isoleucyl)amino-7-methyl-4-hydroxy-1-oxo-octyl-leucyl-(O-benzyl)aspartic acid methyl ester

d) The titled compound was prepared from the product of Example 90(c) by the procedures of examples 76(b) and 77(a), except substituting Boc-isoleucine in place of Cbz-alanine.
NMR ($CDCl_3$): δ 7.4 (5H, s), 7.0 (1H, d), 6.3 (0.5H, d), 6.2 (0.5H, d), 5.15 (2H, s), 5.05 (2H, m), 4.9 (1H, m), 4.5 (1H, m), 3.7 (3H, d), 3.0 (2H, m) 2.45 (3H, m), 1.5 (15H, s), 1.3 (15H, s), 0.90 (19H, m).

MS (FAB): 735.5 $(M+H)^+$.

(4RS,5S)-5-((tert-Butyloxycarbonyl)isoleucyl)amino-7-methyl-4-hydroxy-1-oxo-octyl-leucyl-aspartic acid methyl ester

e) The titled compound was prepared from the product of Example 90(d) by the procedure of example 77(b).
NMR: δ 4.55 (1H, t), 4.35(1H, m), 3.8 (2H, m), 3.65 (3H, s), 3.45 (1H, m), 2.7 (1H, m), 2.5 (1H, m), 2.3 (2H, m), 1.4 (9H, s), 1.2 (6H, s), 0.90 (15H, m).
MS (FAB): 667.4 $(M+Na)^+$; 643.5 $(M-H)^-$.

Example 91

Preparation of (4R,5S) and (4S,5R)-carbobenzyloxy-alanyl-(3-hydroxy-5-amino-6-phenyl)hexanoyl-valyl-(4-aminomethyl)pyridine

Boc-valyl-(4-aminomethyl)pyridine

a) Boc-L-valine (5.0 g, 23 mmol) was dissolved in 100 ml $CH_2Cl_2$ along with 4-amino-methylpyridine (2.5 g, 23 mmol). Dicyclohexylcarbodiimide (4.75 g, 23 mmol) was added and the reaction allowed to stir overnight. The reaction mixture was filtered, washed with 1 N $NaHCO_3$ (3 x 50 ml), water (1 x 50 ml) and dried over $Na_2SO_4$. The solvent was removed under reduced pressure and the residue was triturated with hexane, filtered and crystallized. The partially purified product was purified by passing it over a bed of silica gel in ethyl acetate. Evaporation of the solvent gave 3.64 g product as a white solid.
NMR ($CDCl_3$): δ 0.83 (d), 0.90 ( 6H, d), 1.30 (9H, s), 3.8-4.0 (1H, dd) 4.38 (2H, d), 5.2 (1H, d), 7.1 (d), 8.5 (4H, d).

(5S)-carbobenzyloxy-alanyl-(4-oxo-5-amino-6-phenyl)hexanoyl-valyl-(4-aminomethyl)pyridine

b) Boc-L-valyl-(4-aminomethyl)pyridine (2.0 g) was treated with 4 N HCl/dioxane at room temperature for 30 min, evaporated to dryness and the residue dissolved in water, filtered and lyophilized to yield L-valyl-(4- aminomethyl)pyridine dihydrochloride salt.

L-valyl-(4-aminomethyl)pyridine dihydrochloride salt (0.16 g, 0.5 mmol) and Boc-4-oxo-5(S)-amino-6-phenylhexanoic acid were dissolved in 10 ml DMF. To this was added benzotriazol-1-yloxytris-(dimethylamino)phosphonium hexafluorophosphate (0.332 g, 0.75 mmol), 1-hydroxybenzotriazole (.101 g, 0.75 mmol) and diisopropylethylamine (.097 g, 0.75 mmol) and the reaction mixture was stirred overnight at room temperature. Additional benzotriazol-1-yloxytris(dimethylamino)phosphonium hexafluorophosphate (0.332 g, 0.75 mmol), 1-hydroxybenzotriazole (.101 g, 0.75 mmol) and diisopropylethylamine (.097 g, 0.75 mmol) was added and the reaction allowed to proceed an additional 24 hr. at room temperature. After evaporation of solvent, the reaction mixture was dissolved in ethyl acetate, washed with 10% $K_2CO_3$, saturated $NaHSO_4$ and water, dried over $Na_2SO_4$ and concentrated to an oil. Purification on silica gel using 5% methanol/methylene chloride gave 99 mg (4S) Boc-(3-oxo-4-amino-5-phenyl)hexanoyl-valyl-(4-aminomethyl)pyridine.

The 5(S) Boc-(4-oxo-5-amino-6-phenyl)hexanoyl-valyl-(4-aminomethyl)pyridine from above was treated with 4 N HCl/dioxane at room temperature for 30 min, evaporated to dryness and the residue dissolved in water, filtered and lyophilized to yield 70 mg (5S) 3-oxo-5-amino-6-phenyl)hexanoyl-valyl-(4-aminomethyl)-pyridine dihydrochloride salt.

(5S) 3-oxo-5-amino-6-phenyl)hexanoyl-valyl-(4-aminomethyl)pyridine dihydrochloride salt (67 mg, 0.15 mmol) was dissolved in DMF along with carbobenzyloxy-L-alanine (51 mg, 0.23 mmol), benzotriazol-1-yloxytris(dimethylamino)phosphonium hexafluoro-phosphate (102 mg, 0.23 mmol), 1-hydroxybenzotriazole (31 mg, 0.23 mmol) and diisopropylethylamine (30 mg, 0.23 mmol) and the reaction was stirred at room temperature overnight. The reaction mixture was evaporated, dissolved in acetonitrile and purified by

preparative hplc on Beckman Ultrasphere® ODS using a gradient of 10% acetonitrile-water-0.1% TFA to 50% acetonitrile-water-0.1% TFA over 30 min. at 4 ml/min. The appropriate fractions were pooled, evaporated to dryness and lyophilized from 1% acetic acid to yield 29 mg of the desired product.
FAB-MS m/z 616.2 $(M+H)^+$, 614.2 $(M-H)^-$.

(3R, 4S) and (3S, 4R)-carbobenzyloxy-alanyl-(3-hydroxy-4-amino-5-phenyl)hexanoyl-valyl-(4-aminomethyl)-pyridine

c) (4S) carbobenzyloxy-alanyl-(3-oxo-4-amino-5-phenyl)hexanoyl-valyl-(4-aminomethyl)-pyridine (14) (20 mg, .033 mmol) was dissolved in 5 ml methanol and sodium borohydride (1.4 mg, .036 mmol) was added. After 30 min at room temperature, and additional 1 mg sodium borohydride was added and the reaction allowed to proceed an additional 30 min at room temperature. The solvent was removed by evaporation and the residue dissolved in acetic acid and lyophilized. The diastereomeric mixture of alcohols was separated by prep hplc on Beckman Ultrasphere® ODS using 28.5% acetonitrile-71.5% water-0.1% TFA. The appropriate fractions were pooled, evaporated to dryness and lyophilized from 1% acetic acid to yield 7 mg isomer A and 6 mg isomer B.

Isomer A:

FAB-MS m/z 618.5 $(M+H)^+$, 616.4 $(M-H)^-$.
HPLC: $k'$ = 9.6 (Beckman Ultrasphere® ODS using a gradient of 10% acetonitrile-water-0.1% TFA to 50% acetonitrile-water-0.1% TFA over 30 min.).

Isomer B:

FAB-MS m/z 618.5 $(M+H)^+$, 616.4 $(M-H)^-$.
HPLC: $k'$ = 9.9 (Beckman Ultrasphere® ODS using a gradient of 10% acetonitrile-water-0.1% TFA to 50% acetonitrile-water-0.1% TFA over 30 min.).

## Example 92

### Preparation of (3S)-Boc-valyl-4-methyl-3-aminopentan-2-one

Boc-L-valine N,O-dimethylamide

a) Dimethylhydroxylamine hydrochloride (3.91 g, 40 mmol) was dissolved in 25 ml $CH_2Cl_2$ and cooled to 0 °C. Triethylamine (4.14 g, 40 mmol) was added to give a thick suspension which was allowed to warm to room temperature.

Boc-L-valine (8.68 g, 40 mmol) was dissolved in 50 ml THF and 180 ml $CH_2Cl_2$ and cooled to -20 °C. N-Methylpiperidine (4.88 ml, 40 mmol) was added followed by ethyl chloroformate (4.34 g, 40 mmol). After 5 minutes, the dimethylhydroxylamine solution was added and the reaction was allowed to warm to room temperature. After 3 hours, the reaction mixture was washed with 1 N HCl (2 x 100 ml), 1 N NaOH (2 x 100 ml), dried over $Na_2SO_4$ and evaporated to a colorless oil which was used without further purification.

3(S)-4-methyl-3-t-butyloxycarbonylaminopentan-2-one

b) Boc-L-valine N,O-dimethylamide (2.6 g, 10 mmol) was dissolved in 30 ml diethyl ether and added dropwise to 17 ml of a 3 M solution of methylmagnesium bromide in diethyl ether (51 mmol). The reaction mixture was stirred for 3 hours at room temperature and then poured into ice-water, acidified to pH 2 with 1 N HCl and extracted with ethyl acetate (3 x 50 ml). The extracts were dried over $Na_2SO_4$ and concentrated to a colorless oil which was purified by silica gel chromatography using 2% methanol/$CH_2Cl_2$ to give 1.74 g of the desired product.

NMR ($CDCl_3$): δ 0.82 (3H, d), 1.05 (3H, d), 1.45 (9H, s), 2.2 (3H, s) 4.3 (1H, m), 5.25 (1H, br.d).

(3S)-Boc-valyl-4-methyl-3-aminopentan-2-one

c) 4-Methyl-3(S)-t-butyloxycarbonylaminopentan-2-one is stirred with 4N HCl/dioxane at room temperature for 30 minutes, concentrated under high vacuum and then evaporated from $CH_2Cl_2$ several times to remove residual HCl. The resulting hydrochloride salt is dissolved in $CH_2Cl_2$ and neutralized with one equivalent of triethylamine. Boc-L-valine (one equiv.) and dicyclohexylcarbodiimide (one equiv.) are then added and the reaction is stirred at room temperature overnight. The reaction mixture is filtered, washed with 1 N $Na_2CO_3$, dried over $Na_2SO_4$ and concentrated to yield the crude dipeptide, which is then purified by silica gel chromatography.

(4S,5S)-benzyloxycarbonylalanyl-alanyl-(6-phenyl-5-amino-4-hydroxy-hexanoyl)-valyl-(4-methyl-3(S)-aminopentan-2-one)

d) The compound of Example 92(c) is treated with 4N HCl/dioxane at room temperature for 30 min, concentrated under high vacuum and evaporated from $CH_2Cl_2$ several times to remove residual HCl to yield (3S) valyl-4-methyl-3-aminopentan-2-one, hydrochloride. Substituting this compound for valine isobutylamide in the procedure of Example 89 provides the titled compound.

## Example 93

Preparation of-(5S) Boc-5-amino-6-phenyl-trans-hex-3-enoic acid

monomethyl trans-3-hexenoic acid

a) Diazald® (21.4 g, 100 mmol) was dissolved in ether and added dropwise to a solution of KOH (5 g), water (8 ml) and ether (25 ml) at 65 °C. The resulting diazomethane was distilled directly into a solution of β-trans-hydromuconic acid (10 g, 69 mmol). The solution was evaporated and the residue purified by flash chromatography on silica gel using 2% acetic acid/chloroform to give 1.24 g of the desired product.
NMR ($CDCl_3$): δ 10.85 (1H, s), 5.75 (2H, m), 3.68 (3H, s), 3.23 (4H, m).

(4S)-methyl 6-(2-oxo-4-(phenylmethyl)-3-oxazolidinyl)-trans-hex-3-enoate

b) Monomethyl trans-3-hexenoic acid (1.24 g, 7.8 mol) was dissolved in 50 ml THF along with triethylamine (1.42 ml, 10.2 mmol) and cooled to -78 °C. Pivaloyl chloride (1.06 ml, 8.6 mmol) was added, the reaction stirred for 15 min at -78 °C and then allowed to warm to 0 °C for 45 min.

(S)-Benzyloxazolidinone (2.5 g, 14.1 mmol) was dissolved in 30 ml THF and cooled to -78 °C under argon. Butyllithium 5.6 ml of 2.5 M in hexanes) was added slowly and the mixture stirred 5 min. The mixed anhydride from above was cooled to -78 °C and the lithiated oxazolidinone was added via cannula. Reaction was stirred at -78 °C for 15 min and then allowed to warm to room temperature over 1.5 hr. The reaction was quenched with 100 ml 1M $NaHSO_4$ and the THF removed under reduced pressure. The aqueous residue was extracted with $CH_2Cl_2$ and the combined extracts were washed with 0.25 M $NaHSO_4$, dried over $MgSO_4$ and evaporated to dryness. The crude product was purified by flash chromatography on silica gel in 30% ethyl acetate/hexane to give 1.40 g of the desired product.
NMR ($CDCl_3$): δ 7.3 (5H, m), 5.8 (2H, m), 4.7 (1H, m), 4.2 (2H, d), 3.62 (3H, s), 3.1 (6H, m).
FAB-MS, m/z 318 $(M+H)^+$.

(4S,5S)-methyl 6-(2-oxo-4-(phenylmethyl)-3-oxazolidinyl)-5-(phenylmethyl)-trans-hex-3-enoate

c) The oxazolidinone from Example 93(b) (3.9 mmol) was dissolved in 50 ml THF and cooled to -78 °C. Potassium hexamethyldisilazide (7.8 ml of 0.5 M in THF, 3.9 mmol) was added and the mixture stirred at -78 °C for 15 min. Benzyl bromide (0.93 ml, 7.8 mmol) was added and the mixture was allowed to warm to room temperature and stir overnight. The reaction mixture was concentrated and the residue purified by gravity chromatography on silica gel using 20% ethyl acetate/hexane to yield 0.38 g of the desired product (and 0.09 g of the 5(R) isomer).
NMR ($CDCl_3$): δ 7.27 (10H, m), 7.0 (2H, m), 5.73 (2H, m), 4.75 (3H, m) 4.05 (2H, m), 3.60 (3H, s), 3.0 (7H, m).
FAB-MS: m/z 408 $(M+H)^+$.

(5S)-monomethyl 5-(phenylmethyl)-trans-hex-3-enoate

d) The alkylated oxazolidinone of Example 93(c) (0.38 g, 0.93 mmol) was dissolved in 13.9 ml THF and 4.6 ml water and cooled to 0 °C. Hydrogen peroxide (0.63 ml of 30% solution) was added followed by lithium hydroxide (78.1 mg, 2 equiv.) After stirring at 0 °C for 30 min., the reaction was quenched by the addition of 4.5 ml 1.5 M $Na_2SO_3$. The THF was removed under reduced pressure and the pH of the aqueous solution was adjusted to 10 with $NaHCO_3$ and washed with ethyl acetate and ether. The pH was then lowered to 1 with 1 M HCl and extracted with ethyl acetate. The extracts were combined, dried over $Na_2SO_4$ and evaporated to give the crude product. Purification by flash chromatography on silica gel using 2% acetic acid:chloroform gave 50 mg of the desired product.
NMR ($CDCl_3$): δ 9.43 (1H, s), 7.25 (5H, m), 5.64 (2H, m), 3.65 (3H, s), 3.05 (5H, m).

(5S)-monomethyl 5-(phenylmethyl)-trans-hex-3-enamide

e) The carboxylic acid of Example 93(d) (50 mg, 0.2 mmol) was dissolved in 3 ml toluene and cooled to -5 °C. Triethylamine (28 ml, 0.2 mmol) was added followed by ethyl chloroformate (16 ml, 0.2 mmol). After stirring one hr at -5 °C, ammonia was bubbled into the solution which was then allowed to warm to room temperature and stir for 2 days. The solution was evaporated and the residue dissolved in ethyl acetate, washed with 0.5 M $NaHCO_3$ and dried over $MgSO_4$. Evaporation of the solvent gave 50 mg of the desired product.
NMR ($CDCl_3$): δ 7.25 (5H, m), 5.84 (2H, m), 3.68 (3H, s), 3.0 (5H, m).

(5S)-methyl 5-amino-6-phenyl-trans-hex-3-enoate

f) The carboxamide of Example 93(e) is dissolved in acetonitrile-water (4:1) and I,I-bis(trifluoro-acetoxy)-iodosobenzene (1.5 equiv.) is added and the reaction is stirred at room temperature until complete. The reaction mixture is acidified to pH 2 with 1 N HCl and washed with ether. The pH of the aqueous solution is then adjusted to 10 with 1N NaOH and the product is extracted with ethyl acetate. The extracts are dried over $Na_2SO_4$ and evaporated to give the desired product.

(5S)-methyl 5-t-butyloxycarbonylamino-6-phenyl-trans-hex-3-enoate

g) The compound of Example 93(f) is dissolved in DMF along with triethylamine (3 equiv.). Di-t-butyldicarbonate (3 equiv.) is added and the reaction allowed to proceed at room temperature overnight. The solvent is removed under reduced pressure and the product is purified by flash chromatography on silica gel.

(5S)-Boc-5-amino-6-phenyl-trans-hex-3-enoic acid

h) The compound of Example 93(g) is dissolved in 1:1 dioxane:1 N NaOH (1 equiv. NaOH) and stirred at room temperature until the reaction is complete. The solution is acidified with 1 N HCl and extracted with ethyl acetate to yield the desired product.

Example 94

Preparation of (5S)-alanyl-alanyl-(5-amino-6-phenyl-trans-hex-3-enoyl)-valyl-valinamide

The protected peptidyl resin (5S) Boc-Ala-Ala-(5-amino-6-phenyl-trans-hex-3-enoyl)-Val-Val-BHA is prepared according to the usual method of Example 1. The peptidyl resin is cleaved and deprotected by treatment with 10 ml anhydrous liquid HF/1 ml anisole at 0 °C for 1 hour. After removal of the HF under vacuum, the resin is washed with diethyl ether, air dried and extracted with 2 x 50 ml glacial acetic acid, which is lyophilized to yield the crude peptide. The crude product is purified by gel filtration on Sephadex® G-15 using 10% aqueous acetic acid as eluant.

Example 95

Preparation of Asn-Tyr-Pro-Val-Val-$NH_2$

The protected peptidyl resin Boc-Asn-Tyr(BrZ)-Pro-Val-Val-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 145 mg crude product. The crude peptide (100 mg) was dissolved in 1% HOAc and purified on a 2.6 x 70 cm Sephadex® G-15 column. The appropriate fractions were pooled and lyophilized to yield 84.4 mg purified product.
HPLC: $k'$ = 2.95 (Hamilton PRP-1®, 5% $CH_3CN$/ 0.1% TFA to 40% $CH_3CN$/ 0.1% TFA, 15 min.).
MS (FAB): m/z 590 $(M+H)^+$

Example 96

Preparation of Ala-Asn-Tyr-Pro-Val-Val-$NH_2$

The protected peptidyl resin Boc-Ala-Asn-Tyr(BrZ)-Pro-Val-Val-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 136.6 mg crude product. The crude peptide was dissolved in 1% HOAc and purified on a 2.6 x 70 cm Sephadex® G-15 column. The appropriate fractions were pooled and lyophilized to yield 116.7 mg of purified product.
HPLC: $k'$ = 2.47 (Hamilton PRP-1®, 5% $CH_3CN$/ 0.1% TFA to 40% $CH_3CN$/ 0.1% TFA, 15 min.).
MS (FAB): m/z 661 $(M+H)^+$

Example 97

Preparation of Ser-Gln-Asn-Tyr-Pro-Val-Val

The protected peptidyl resin BocSer(Bzl)-Gln-Asn-Tyr(BrZ)-Pro-Val-Val-Merrifield resin was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 248.8 mg crude product. The crude peptide (90 mg) was dissolved in 1% HOAc and purified on a 2.6 x 70 cm Sephadex® G-15 column. The appropriate fractions were pooled and lyophilized to yield 58.8 mg purified product.

HPLC: k′ = 2.86 (Hamilton PRP-1®, 10% $CH_3CN$/ 0.1% TFA to 40% $CH_3CN$/ 0.1% TFA, 15 min.).
MS (FAB): m/z 806 $(M+H)^+$

## Example 98

### Preparation of Ser-Gln-Asn-Cha-Pro-Val-Val-$NH_2$

The protected peptidyl resin Boc-Ser(Bzl)-Gln-Asn-Cha-Pro-Val-Val-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 316 mg crude product. The crude peptide (100 mg) was dissolved in 1% HOAc and purified on a 2.6 x 70 cm Sephadex® G-15 column. The appropriate fractions were pooled and lyophilized to yield 88.1 mg purified product.
HPLC: k′ = 3.31 (Hamilton PRP-1®, 5% $CH_3CN$/ 0.1% TFA to 40% $CH_3CN$/ 0.1% TFA, 15 min.)
MS (FAB): m/z 795 $(M+H)^+$
Amino Acid Analysis: Asp 1.00, Ser 0.72, Glu 1.02, Pro 0.97, Val 2.01.

## Example 99

### Preparation of Ac-Ser-Gln-Asn-Cha-Pro-Val-Val-$NH_2$

The protected peptidyl resin Ac-Ser(Bzl)-Gln-Asn-Cha-Pro-Val-Val-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 280 mg crude product. The crude peptide (100 mg) was dissolved in 1% HOAc and purified on a 2.6 x 70 cm Sephadex® G-15 column. The appropriate fractions were pooled and lyophilized to yield 74.1 mg purified product.
HPLC: k′ = 3.27 (Hamilton PRP-1®, 5% $CH_3CN$/ 0.1% TFA to 40% $CH_3CN$/ 0.1% TFA, 15 min.)
MS (FAB): m/z 837 $(M+H)^+$
Amino Acid Analysis: Asp 1.00, Ser 0.75, Glu 0.99, Pro 0.96, Val 1.90.

## Example 100

### Preparation of Ac-Ser-Gln-Asn-Tyr-Pro-Val-$NH_2$

The protected peptidyl resin Ac-Ser(Bzl)-Gln-Asn-Tyr(BrZ)-Pro-Val-BHA was prepared in the usual manner on a 1.0 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 145 mg of crude product. The crude peptide (130 mg) was dissolved in 1% HOAc and purified on a 2.6 x 70 cm Sephadex® G-15 column. The appropriate fractions were pooled and lyophilized to yield 101 mg of purified product.
MS (FAB): m/z 748.7 $(M+H)^+$
HPLC: k′ = 2.08 (Hamilton PRP-1®, 5% $CH_3CN$/0.1% TFA to 40% $CH_3CN$/0.1% TFA, 15 min.).
Amino Acid Analysis: Asp 1.00, Ser 0.55, Glu 0.98, Pro 1.19, Val 0.95, Tyr 0.99.

## Example 101

Preparation of Ac-Asp-Gln-Asn-Tyr-Pro-Val-Arg-$NH_2$

The protected peptidyl resin Ac-Asp(OBzl)-Gln-Asn-Tyr(BrZ)-Pro-Val-Arg(Tos)-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 404 mg crude product. The crude peptide (100 mg) was dissolved in 1% HOAc and purified on a 2.6 x 70 cm Sephadex® G-15 column. The appropriate fractions were pooled and lyophilized to yield 84.2 mg partially purified product. The partially purified peptide was further purified by preparative hplc (Hamilton PRP-1®, 11% $CH_3CN$/ 0.1% TFA) to yield 78.8 mg purified product.
HPLC: $k'$ = 2.04 (Hamilton PRP-1®, 5% $CH_3CN$/ 0.1% TFA to 40% $CH_3CN$/ 0.1% TFA, 15 min.)
MS (FAB): m/z 932 $(M+H)^+$
Amino Acid Analysis: Asp 1.88, Glu 0.90, Pro 0.76, Val 1.00, Tyr 0.33, Arg 1.01.

## Example 102

Preparation of Asp-Gln-Asn-Tyr-Pro-Val-Val-$NH_2$

The protected peptidyl resin Boc-Asp(OBzl)-Gln-Asn-Tyr(BrZ)-Pro-Val-Val-BHA was prepared in the usual manner on a 1.0 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 671 mg crude product. The crude peptide (100 mg) was dissolved in 1% HOAc and purified on a 2.6 x 70 cm Sephadex® G-15 column. The appropriate fractions were pooled and lyophilized to yield 74.5 mg purified product.
HPLC: $k'$ = 2.44 (Hamilton PRP-1®, 5% $CH_3CN$/ 0.1% TFA to 40% $CH_3CN$/ 0.1% TFA, 15 min.)
MS (FAB): m/z 833 $(M+H)^+$
Amino Acid Analysis: Asp 2.00,Glu 0.98, Pro 0.96, Val 2.04, Tyr 0.34.

## Example 103

Preparation of Ac-Ala-Ala-Ser-Gln-Asn-Tyr-Pro-Val-Val-$NH_2$

The protected peptidyl resin Ac-Ala-Ala-Ser(Bzl)-Gln-Asn-Tyr(BrZ)-Pro-Val-Val-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield the crude product.

## Example 104

Preparation of Ac-Ala-Gln-Gly-Tyr-Pro-Val-Val-$NH_2$

The protected peptidyl resin Ac-Ala-Gln-Gly-Tyr(BrZ)-Pro-Val-Val-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 275 mg crude product. The crude peptide was purified by countercurrent distribution using the system n-BuOH-HOAc-$H_2O$ 4:1:5, yielding 235 mg purified product.
HPLC: $k'$ = 4.48 (Beckman Ultrasphere® ODS, 5% $CH_3CN$/ 0.1% TFA to 40% $CH_3CN$/ 0.1% TFA, 15 min.)

MS (FAB): m/z 831 $(M+H)^+$
Amino Acid Analysis: Asp 1.02, Glu 1.00, Pro 0.97, Val 1.23, Tyr 0.87, Ala 1.04.

Example 105

Preparation of D-Ser-Gln-Asn-Tyr-Pro-Val-Val-$NH_2$

The protected peptidyl resin Boc-D-Ser(Bzl)-Gln-Asn-Tyr(BrZ)-Pro-Val-Val-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 271 mg crude product. The crude peptide (98 mg) was dissolved in 10% HOAc and purified on a 2.6 x 70 cm Sephadex® G-15 column. The appropriate fractions were pooled and lyophilized to yield 73 mg purified product.
HPLC: $k'$ = 4.45 (Beckman Ultrasphere® ODS, 5% $CH_3CN$/ 0.1% TFA to 40% $CH_3CN$/ 0.1% TFA, 15 min.)
MS (FAB): m/z 805 $(M+H)^+$
Amino Acid Analysis: Asp 1.00, Glu 0.99, Pro 1.11, Val 1.89, Tyr 0.57, Ser 0.68.

Example 106

Preparation of Ac-D-Ser-Gly-Asn-Tyr-Pro-Val-Val-$NH_2$

The protected peptidyl resin Ac-D-Ser(Bzl)-Gly-Asn-Tyr(BrZ)-Pro-Val-Val-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 248 mg crude product. The crude peptide (93 mg) was dissolved in 10% HOAc and purified on a 2.6 x 70 cm Sephadex® G-15 column. The appropriate fractions were pooled and lyophilized to yield 75 mg purified product.
HPLC: $k'$ = 4.16 (Beckman Ultrasphere® ODS, 5% $CH_3CN$/ 0.1% TFA to 40% $CH_3CN$/ 0.1% TFA, 15 min.)
MS (FAB): m/z 847 $(M+H)^+$
Amino Acid Analysis: Asp 1.00, Ser 0.67, Glu 0.99, Pro 0.93, Val 2.00, Tyr 0.30.

Example 107

Preparation of Ser-Gln-Asn-Tyr-Pro-Ala-Val-$NH_2$

The protected peptidyl resin Boc-Ser(Bzl)-Gln-Asn-Tyr(BrZ)-Pro-Ala-Val-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 202 mg crude product. The crude peptide (102 mg) was dissolved in 1% HOAc and purified on a 2.6 x 70 cm Sephadex® G-15 column. The appropriate fractions were pooled and lyophilized to yield 74 mg purified product.
HPLC: $k'$ = 3.96 (Beckman Ultrasphere® ODS, 5% $CH_3CN$/ 0.1% TFA to 40% $CH_3CN$/ 0.1% TFA, 15 min.)
MS (FAB): m/z 777 $(M+H)^+$
Amino Acid Analysis: Asp 1.05, Ser 0.91, Glu 1.00, Ala 1.02, Pro 0.86, Val 1.09, Tyr 0.61.

Example 108

Preparation of $Ac-Ser-Gln-Asn-Tyr-Pro-Gly-Val-NH_2$

The protected peptidyl resin Ac-Ser(Bzl)-Gln-Asn-Tyr(BrZ)-Pro-Gly-Val-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 153 mg crude product. The crude peptide (100 mg) was dissolved in 10% HOAc and purified on a 2.6 x 70 cm Sephadex® G-15 column. The appropriate fractions were pooled and lyophilized to yield 76 mg purified product.
HPLC: $k'$ = 3.87 (Beckman Ultrasphere® ODS, 5% $CH_3CN$/ 0.1% TFA to 40% $CH_3CN$/ 0.1% TFA, 15 min.)
MS (FAB): m/z 805 $(M+H)^+$
Amino Acid Analysis: Asp 1.00, Ser 0.84, Glu 0.95, Gly 1.00, Pro 0.97, Val 1.06, Tyr 0.77.

Example 109

Preparation of $Ac-Ser-Gln-Asn-Tyr-Pro-Val-Val-Arg-NH_2$

The protected peptidyl resin Ac-Ser(Bzl)-Gln-Asn-Tyr(BrZ)-Pro-Val-Val-Arg(Tos)-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 329 mg of crude product. The crude peptide was purified by countercurrent distribution in the system $n-BuOH-HOAc-H_2O$ 4:1:5. The appropriate fractions were pooled and lyophilized to yield 172 mg of purified product.
MS (FAB): m/z 1003 $(M+H)^+$
HPLC: $k'$ = 3.71 (Beckman Ultrasphere® $C_{18}$, 5% $CH_3CN$/0.1% TFA to 40% $CH_3CN$/0.1% TFA, 15 min.).
Amino Acid Analysis: Asp 1.00, Ser 0.73, Glu 0.98, Pro 0.98, Val 1.84, Tyr 1.01, Arg 0.99.

Example 110

Preparation of $Ac-Ser-Gln-Asn-Tyr-Pro-Glu-Val-NH_2$

The protected peptidyl resin Ac-Ser(Bzl)-Gln-Asn-Tyr(BrZ)-Pro-Glu(OBzl)-Val-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 184 mg crude product. The crude peptide (92 mg) was dissolved in 10% HOAc and purified on a 2.6 x 70 cm Sephadex® G-15 column. The appropriate fractions were pooled and lyophilized to yield 64 mg purified product.
HPLC: $k'$ = 3.83 (Beckman Ultrasphere® ODS, 5% $CH_3CN$/ 0.1% TFA to 40% $CH_3CN$/ 0.1% TFA, 15 min.)
MS (FAB): m/z 877 $(M+H)^+$
Amino Acid Analysis: Asp 1.00, Ser 0.77, Glu 2.03, Pro 0.87, Val 1.08, Tyr 1.01.

Example 111

Preparation of $Ser-Gln-Asn-Tyr-Pro-Glu-Val-NH_2$

The protected peptidyl resin Boc-Ser(Bzl)-Gln-Asn-Tyr(BrZ)-Pro-Glu(OBzl)-Val-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 216 mg crude product. The crude peptide (91 mg) was dissolved in 10% HOAc and purified on a 2.6 x 70 cm Sephadex® G-15 column. The appropriate fractions were pooled and lyophilized to yield 67 mg purified product.
HPLC: $k'$ = 3.35 (Beckman Ultrasphere® ODS, 5% $CH_3CN$/ 0.1% TFA to 40% $CH_3CN$/ 0.1% TFA, 15 min.)
MS (FAB): m/z 835 $(M+H)^+$
Amino Acid Analysis: Asp 0.97, Ser 0.90, Glu 2.03, Pro 1.00, Val 0.84.

## Example 112

### Preparation of Ac-Ser-Gln-Gly-Tyr-Pro-Lys-Val-$NH_2$

The protected peptidyl resin Ac-Ser(Bzl)-Gln-Gly-Tyr(BrZ)-Pro-Lys($Cl_2Z$)-Val-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 280 mg crude product. The crude peptide was purified by countercurrent distribution using the system n-BuOH-HOAc-$H_2O$ 4:1:5, yielding 242 mg purified product.
HPLC: $k'$ = 4.41 (Beckman Ultrasphere® ODS, 5% $CH_3CN$/ 0.1% TFA to 40% $CH_3CN$/ 0.1% TFA, 15 min.)
MS (FAB): m/z 876 $(M+H)^+$
Amino Acid Analysis: Asp 1.00, Ser 0.68, Glu 1.01, Pro 0.82, Val 1.14, Tyr 1.06, Lys 1.00.

## Example 113

### Preparation of Ac-Ser-Gln-Asn-Tyr-Pro-$NH_2$

The protected peptidyl resin Ac-Ser(Bzl)-Gln-Asn-Tyr(BrZ)-Pro-BHA was prepared in the usual manner on a 1.0 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 400 mg crude product. The crude peptide (100 mg) was dissolved in 1% HOAc and purified on a 2.6 x 70 cm Sephadex® G-15 column. The appropriate fractions were pooled and lyophilized to yield 92.8 mg of purified product.
MS (FAB): m/z 649 $(M+H)^+$
HPLC: $k'$ = 1.57 (Hamilton PRP-1®, 5% $CH_3CN$/0.1% TFA to 40% $CH_3CN$/0.1% TFA, 15 min.).
Amino Acid Analysis: Asp 1.00, Ser 0.92, Glu 1.03, Pro 0.87, Tyr 0.95.

## Example 114

### Preparation of Ac-Ser-Gln-Asn-Tyr-Pro-Val-Val-Gln-Asn-$NH_2$

The protected peptidyl resin Ac-Ser(Bzl)-Gln-Asn-Tyr(BrZ)-Pro-Val-Val-Gln-Asn-BHA was prepared in the usual manner on a 1.0 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 862 mg of crude product. The crude peptide was purified by countercurrent distribution in the system n-BuOH-HOAc-$H_2O$

4:1:5. The appropriate fractions were pooled and lyophilized to yield 713 mg of purified product.
MS (FAB): m/z 1089 $(M+H)^+$
HPLC: $k'$ = 2.17 (Hamilton PRP-1®, 5% $CH_3CN$/0.1% TFA to 40% $CH_3CN$/0.1% TFA, 15 min.).
Amino Acid Analysis: Asp 1.81, Ser 0.97, Glu 2.00, Pro 0.88, Val 1.81, Tyr 1.23.

## Example 115

### Preparation of Ac-Ser-Gln-Asn-Phe-Pro-Val-Val-$NH_2$

The protected peptidyl resin Ac-Ser(Bzl)-Gln-Asn-Phe-Pro-Val-Val-BHA was prepared in the usual manner on a 1.0 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 573 mg of crude product. The crude peptide (30 mg) was by preparative hplc (Hamilton PRP-1®, 17% $CH_3CN$/0.1 % TFA) to give 24.5 mg of purified product.
MS (FAB): m/z 831 $(M+H)^+$
HPLC: $k'$ = 3.02 (Hamilton PRP-1®, 5% $CH_3CN$/0.1% TFA to 40% $CH_3CN$/0.1% TFA, 15 min.).
Amino Acid Analysis: Asp 1.06, Ser 0.86, Glu 1.00, Pro 1.02, Val 1.95, Phe 1.00.

## Example 124

### Preparation of Boc-L-valine isopropylamide

Boc-L-Valine (0.5 g, 2.3 mmol) was dissolved in 25 ml DMF along with isopropylamine (0.39 ml, 4.6 mmol), benzotriazol-1-yloxytris(dimethylamino)-phosphonium hexafluorophosphate (2.03 g, 4.6 mmol), 1-hydroxybenzotriazole (0.62 g, 4.6 mmol) and diisopropylethylamine (1.2 ml, 6.9 mmol). The reaction was stirred at room temperature for 2 hr. then evaporated to dryness. The residue was dissolved in ethyl acetate and washed with 1 N $NaHSO_4$, 1 N $NaHCO_3$ and water and then dried over $Na_2SO_4$. THe solvent was removed under reduced pressure to give 0.53 g crude product. The product was purified by flash chromatography on silica gel using 25% MeOH/$CHCl_3$ to yield 358 mg purified product.
MS (DCI), m/z 259 $(M+H)^+$
NMR ($CDCl_3$): $\delta$ 0.90 ppm, d, 0.98 ppm d, 6H; 1.16 ppm, d, 6H; 1.35 ppm, s, 9H; 4.1 ppm, dd, 1H; 5.25 ppm, d, 1H; 6.14 ppm, d, 1H.

## Example 117

### Preparation of Ac-Tyr-Pro-Val-Val-$NH_2$

The protected peptidyl resin Ac-Tyr(BrZ)-Pro-Val-Val-BHA was prepared in the usual manner on a 0.5 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 98.7 mg crude product. The crude peptide was dissolved in 1% HOAc and purified on a 2.6 x 70 cm Sephadex® G-15 column. The appropriate fractions were pooled and lyophilized to yield 78.5 mg purified product.
HPLC: $k'$ = 2.96 (Hamilton PRP-1®, 5% $CH_3CN$/ 0.1% TFA to 40% $CH_3CN$/ 0.1% TFA, 15 min.).
MS (FAB): m/z 518 $(M+H)^+$

## Example 118

Preparation of Ac-Arg-Ala-Ser-Gln-Asn-Tyr-Pro-$NH_2$

The protected peptidyl resin Ac-Arg(Tos)-Ala-Ser(Bzl)-Gln-Asn-Tyr(BrZ)-Pro-BHA was prepared in the usual manner on a 1.0 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 621 mg crude product. The crude peptide was purified by countercurrent distribution in the system n-BuOH-EtOAC-HOAc-$H_2O$ 2:2:1:5. The appropriate fractions were pooled and lyophilized to yield 549 mg partially purified product. This partially purified peptide (100 mg) was dissolved in 10% HOAc and purified on a 2.6 x 70 cm Sephadex® G-15 column. The appropriate fractions were pooled and lyophilized to yield 94 mg of purified product.
MS (FAB): m/z 876 $(M+H)^+$
HPLC: $k'$ = 2.78(Beckman Ultrasphere® $C_{18}$, 5% $CH_3CN$/0.1% TFA to 40% $CH_3CN$/0.1% TFA, 15 min.).
Amino Acid Analysis: Asp 1.00, Ser 1.03, Glu 1.01, Pro 0.91, Ala 1.02, Arg 0.99, Tyr 0.94.

## Example 119

Preparation of Ac-Ser-Gln-Asn-Tyr-Pro

The protected peptidyl resin Ac-Ser(Bzl)-Gln-Asn-Tyr(BrZ)-Pro-Merrifield resin was prepared in the usual manner on a 1.0 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 458.5 mg crude product, which was used without further characterization.

## Example 120

Preparation of Ac-Ser-Gln-Asn-Tyr-Pro-Val-HNiPr

Boc-Val isopropylamide 1034 (24.4 mg, 154 mmol)was deprotected by treatment with 50% TFA/$CH_2Cl_2$ for 20 min at room temperature. The reaction mixture was evaporated to dryness and the residue evaporated from $CH_2Cl_2$ six times, treated with 7% diisopropyl-amine/$CH_2Cl_2$ and evaporated three times and then evaporated from $CH_2Cl_2$ four times. The residue was dissolved in 25 ml DMF along with the peptide 1035 (50 mg, 77 mmol), benzotriazol-1-yloxytris(dimethylamino)phosphonium hexafluorophosphate (68 mg, 154 mmol), 1-hydroxybenzotriazole (20.8 mg, 154 mmol) and diisopropylethylamine (40.2 ml, 231 mmol) and the reaction was allowed to proceed overnight at room temperature. The reaction mixture was evaporated to dryness and the residue dissolved in MeOH and purified by preparative hplc (Beckman Ultrasphere® ODS, 5% $CH_3CN$/ 0.1% TFA to 40% $CH_3CN$/ 0.1% TFA, 15 min.) to give 41 mg purified peptide.
HPLC: $k'$ = 4.97 (Beckman Ultrasphere® ODS, 5% $CH_3CN$/ 0.1% TFA to 40% $CH_3CN$/ 0.1% TFA, 15 min.)
MS (FAB): m/z 790.5 $(M+H)^+$
Amino Acid Analysis: Asp 1.00, Ser 0.72, Glu 1.02, Pro 1.13, Val 0.65, Tyr 0.83.

## Example 121

Preparation of Ac-Arg-Lys-Ile-Leu-Phe-Leu-Asp-Gly-$NH_2$

The protected peptidyl resin Boc-Arg(Tos)-Lys($Cl_2$Bzl)-Ile-Leu-Phe-Leu-Asp(OBzl)-Gly-BHA was prepared in the usual manner on a 1.0 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 520 mg crude product. The crude peptide was purified by countercurrent distribution in the system n-BuOH-HOAc-$H_2O$ 4:1:5. The appropriate fractions were pooled and lyophilized to yield 40 mg partially purified product. The partially purified peptide (50 mg) was further purified by preparative hplc (Hamilton PRP-1®, 23% $CH_3CN$/ 0.1% TFA) to yield 37.5 mg purified peptide.
HPLC: k′ = 3.81 (Hamilton PRP-1®, 5% $CH_3CN$/ 0.1% TFA to 40% $CH_3CN$/ 0.1% TFA, 15 min.)
MS (FAB): m/z 1002 $(M+H)^+$
Amino Acid Analysis: Asp 1.00, Gly 1.05, Ile 0.91, Leu 2.08, Lys 0.94, Arg 1.07, Phe 1.29.

## Example 122

### Preparation of Ac-Arg-Lys-Ile-Leu-(4′NO₂)Phe-Leu-Asp-Gly-NH₂

The protected peptidyl resin Ac-Arg(Tos)-Lys($Cl_2$Z)-Ile-Leu-(4′$NO_2$)Phe-Leu-Asp(OBzl)-Gly-BHA was prepared in the usual manner on a 1.0 mmol scale. The peptide was cleaved from the resin with anhydrous liquid HF at 0 °C for 1 hr. After removal of the HF, the resin was washed with ether, air-dried and extracted with 3 x 30 ml 10% HOAc. The HOAc extracts were diluted with water and lyophilized to yield 980 mg crude product. The crude peptide was purified by countercurrent distribution using the system n-BuOH-HOAc-$H_2O$ 4:1:5, yielding 853 mg partially purified product. The partially purified peptide (50 mg) was further purified by preparative hplc (Hamilton PRP-1®, 23% $CH_3CN$/ 0.1% TFA) to give 43.4 mg purified peptide.
HPLC: k′ = 3.87 (Hamilton PRP-1®, 5% $CH_3CN$/ 0.1% TFA to 40% $CH_3CN$/ 0.1% TFA, 15 min.)
MS (FAB): m/z 1047.7 $(M+H)^+$
Amino Acid Analysis: Asp 1.00, Gly 1.04, Ile 0.89, Leu 2.00, Arg 0.91, (4′$NO_2$)Phe 0.97, Lys 0.91.

### Preparation of (4S,5S)-4-(tert-butyldimethylsiloxy)-5-(tert-butyloxycarbonyl)-amino-6-phenylhexanoic acid

#### (S)-tert-butyloxycarbonylphenylalanine N-methoxy-N-methyl amide.

a) The titled compound was prepared by following the procedure of Org. Syn., 67, 69 (1988), except using Boc-(L)-Phe in place of Boc-(L)-Leu.
$[a]_{365}$ = + 34° (c 1, ethanol).

#### (6S)-5-oxo-6-(tert-butyloxycarbonyl)amino-7-phenylhept-1-ene

b) A flame-dried, 2-liter 3-neck flask fitted with mechanical stirrer, thermometer, condenser, Ar bubbler, and addition funnel containing neat 4-bromo-1-butene (90.2 ml, 0.889 mol), was charged with Mg powder (32 g, 1.3 mol), and dry ether (600 ml). A 2 ml portion of the bromobutene was added, and the stirring mixture was warmed to 30 °C with a water bath until the reaction initiated (ca. 5 min). The remainder of the bromobutene was then added over 1.5 hr, at a rate such that a gentle reflux was maintained. The mixture was heated to reflux for an additional 30 min, then was cooled to 5 °C. A solution of the compound of Example 123(a) (54.0 g, 0.175 mol) in ether (130 ml) was added in portions over 15 min, with the reaction temperature maintained below 12 °C. After 1 hr vigorous stirring at 5 °C, the mixture was poured cautiously into 500 ml cold (5-10 °C) 3N HCl. The layers were separated and the aqueous layer was extracted with two 200 ml portions of ether. The combined organic layers were washed with 5% $NaHCO_3$ and brine, then were dried over $MgSO_4$, filtered and concentrated by rotary evaporation to afford the crude product (49.4 g, 0.163 mol, 93% yield) as a white solid, sufficiently pure for use in the next reaction. A portion of the product was purified by flash chromatography (silica, 10% ethyl acetate in hexanes) for analysis.
mp: 74-75 °C.

$[a]_D$ = -36.8° (c 1, ethanol).

(5S)-4-oxo-5-(tert-butyloxycarbonyl)amino-6-phenylhexanoic acid

c) The compound of Example 123(b) (45.0 g, 0.148 mol) was rapidly stirred with benzene (550 ml), water (550 ml) and acetic acid (115 ml) at 3° C. Tetrabutylammonium bromide (490 mg) was added, followed by $KMnO_4$ (83 g). The stirring mixture was allowed to warm to room temperature. After 2.5 hr more $KMnO_4$ (14 g) was added. After an additional 30 min, the reaction mixture was cooled to 5° C and 500 ml saturated aqueous $KHSO_4$ was added. The mixture was stirred 30 min, then filtered. The layers were separated, the aqueous layer was extracted with four 200 ml portions of ethyl acetate. The combined organic layers were washed with brine, dried over $MgSO_4$, filtered and concentrated to an oil. The oil was crystallized from 350 ml of 6:1 hexane:ethyl acetate to provide the titled compound (33.3 g, 70% yield) as a white solid.
$[a]_D$ = -36.1°.

(5S)-4-oxo-5-(tert-butyloxycarbonyl)amino-6-phenylhexanoic acid, benzyl ester.

d) To a stirred solution of the compound of Example 123(c) (33.2 g) in acetonitrile (500 ml) at 4° C was added diazabicycloundecane (15.7 g), followed by benzyl bromide (26.0 g). The mixture was stirred with warming to room temperature for 3 hr, then was concentrated to 70 ml by rotary evaporation. The mixture was diluted with $CH_2Cl_2$, washed with 1 N HCl, 5% $NaHCO_3$, and brine, dried over $MgSO_4$ and concentrated to a yellow solid. The solid was triturated with 3:1 ether:hexane to provide the titled compound (33.0 g, 78% yield).
mp: 86-87.5° C.
$[a]_D$ = -27.8°

(4S,5S)-4-(tert-butyldimethylsiloxy)-5-(tert-butyloxycarbonyl)amino-6-phenylhexanoic acid benzyl ester.

e) A solution of the compound of Example 123(d) (28.0 g) in methanol (840 ml) was cooled to 5° C, and $NaBH_4$ (1.30 g) was added in one portion with stirring. After 30 min acetic acid (10 ml) was added and the mixture was concentrated by rotary evaporation, then diluted with $CH_2Cl_2$ and washed with water. The organic layer was dried ($Na_2SO_4$) and concentrated to provide the crude diastereomeric alcohols as a white solid (27.7 g, 99% yield). The crude alcohols were dissolved in DMF (140 ml) along with imidazole (177 g) and tert-butyldimethylsilyl chloride (20.7 g). The mixture was allowed to stand at room temperature for 3 days, then was diluted with 500 ml cold 1N HCl and extracted with six 100 ml portions of $CH_2Cl_2$. The combined organic extracts were washed with 3x100 ml water, dried ($Na_2SO_4$) and concentrated under vacuum with warming to 40° C to provide an oil (42 g). The two isomers were separated by preparative HPLC in four equal portions on 2.5 Kg (3 in x 1 m) Vydac HS silica using 90:10 hexane ethyl acetate at 200 ml/min to provide 9.0 g of the titled compound as an oil.
HPLC: RT 9.18 min (4S isomer), 12.2 min (4R isomer) (YMC silica A-003, 4.6 mm x 25 cm, UV detection at 254 nm, 91:9 hexane:ethyl acetate, 1.0 ml/min),(4R):(4S) = 3:1.
$[a]_D$ = -14.4°

(4S,5S)-4-(tert-butyldimethylsiloxy)-5-(tert-butyloxycarbonyl)-amino-6-phenylhexanoic acid

f) The compound of Example 123(e) (7.7 g) was dissolved in ethyl acetate (150 ml) with 5% Pd on carbon (0.75 g) and shaken under 25 psi $H_2$ for 4 hr. The mixture was filtered through Celite® and concentrated under vacuum with warming to provide the titled compound as a colorless glass (6.3 g, 98 % yield).
$[a]_D$ = -24.7°

Example 124

Preparation of(5S)-benzyloxycarbonylalanyl-alanyl-(5-amino-6-phenyl-trans-hex-3-enoyl)-valyl-valinamide

The compound of Example 94 is dissolved in DMF to which is added 2 equiv. of triethylamine and 1.5 equiv. N-(benzyloxycarbonyloxy)succinimide. The extent of reaction is monitored by hplc and the product is purified directly from the reaction mixture by preparative hplc using an acetonitrile-water-0.1% trifluoroacetic acid system on a Hamilton PRP-1® polystyrene column.

Example 125

Preparation of (5S)-benzyloxycarbonylalanyl-alanyl-(5-amino-6-diphenyl-cis-3,4-dihydroxy-hexanoyl)-valyl-valinamide

The compound of Example 124 is dissolved in THF along with N-methylmorpholine N-oxide (3 equiv.) Osmium tetroxide (0.04 equiv. of a 2.5% solution in t-butanol) is added and the reaction allowed to proceed at room temperature. When the reaction is complete, the reaction mixture is partitioned between ethyl acetate and brine, the brine back-washed with ethyl acetate and the combined ethyl acetate layers are washed with 10% $Na_2SO_3$, 1 N $NaHSO_4$, dried over $Na_2SO_4$ and evaporated to give the crude product as a mixture of diastereomeric cis alcohols. The products are purified by preparative hplc using an acetonitrile-water-0.1% trifluoroacetic acid system on a Hamilton PRP-1% polystyrene column.

Example 126

Preparation of (4S,5S)-alanylalanyl-(6-phenyl-5-amino-4-hydroxyhexanoyl)-valyl-valine

The protected peptidyl resin (4S,5S) Boc-Ala-Ala-(6-phenyl-5-amino-4-t-butyldimethyl-silyloxyhexanoyl)-Val-Val-Merrifield resin is prepared on the according to the usual method. The peptidyl resin is cleaved and deprotected by treatment with 10 ml anhydrous liquid HF/1 ml anisole at 0 °C for 1 hour. After removal of the HF under vacuum, the resin is washed with diethyl ether, air dried and extracted with 2 x 50 ml glacial acetic acid, which is lyophilized to yield the crude peptide. The crude product is purified by gel filtration on Sephadex® G-15 using 10% aqueous acetic acid as eluant.

Example 127

(4S,5S)-benzyloxycarbonylalanyl-alanyl-(6-phenyl-5-amino-4-hydroxyhexanoyl)-valyl-valine

The compound of Example 126 is dissolved in DMF to which is added 2 equiv. of triethylamine and 1.5 equiv. N-(benzyloxycarbonyloxy)succinimide. The extent of reaction is monitored by hplc and the product is purified directly from the reaction mixture by preparative hplc using an acetonitrile-water-0.1% trifluoroacetic acid system on a Hamilton PRP-1® polystyrene column.

Example 128

Preparation of (4S,5S)-benzyloxycarbonylalanyl-alanyl-(6-phenyl-5-amino-4-hydroxyhexanoyl)-valyl-valyl-(5-aminopentyl)amide

mono-Boc-1,5-diaminopentane

a) A solution of 1,5-diaminopentane (14.0 ml, 120 mmol) in 70 ml $CH_2Cl_2$ was treated with di-t-butyldicarbonate (7.24 g, 33.2 mmol) at 0 °C and was then stirred overnight at room temperature. The reaction mixture was diluted with 75 ml $CHCl_3$, washed with 5% aqueous sodium carbonate, dried over $MgSO_4$ and evaporated. The residue was dissolved in a minimum volume of 1 N HCl (10 ml) and washed with diethyl ether. The aqueous layer was then basified to pH 10 with 2 N NaOH and extracted with ethyl acetate. The extracts were combined, dried over $MgSO_4$ and evaporated to give 1.6 g of the desired product.

NMR ($CDCl_3$): δ 1.43 (17 H, br.s), 2.53-2.83 (2 H, m), 2.90-3.10 (2H, m), 4.80-5.10 (1 H, br.s).
MS (CI): m/z 237 $(M+Cl)^-$.

(4S,5S)-benzyloxycarbonylalanyl-alanyl-(6-phenyl-5-amino-4-hydroxyhexanoyl)-valyl-valyl-(5-aminopentyl)-amide

b) The compound of Example 126 is dissolved in DMF with mono-Boc-1,5-diamino-pentane (3 equiv.), 1-hydroxybenzotriazole (1.5 equiv.) and diisopropylcarbodiimide (1.5 equiv.) and the reaction is stirred at room temperature and monitored by hplc. When the reaction is complete, the reaction mixture is evaporated to dryness and the residue treated with trifluoroacetic acid at 0 °C for 30 minutes. The trifluoroacetic acid is removed under reduced pressure and the residue is dissolved in methanol and purified by reverse phase hplc using an acetonitrile-water-0.1% trifluoroacetic acid system on a Hamilton PRP-1® polystyrene column.

## Example 129

Preparation of (4S,5S)-benzyloxycarbonylalanyl-alanyl-(6-phenyl-5-amino-4-hydroxyhexanoyl)-valyl-valyl-(5-guanidinopentyl)amide

The compound of Example 128 is dissolved in water and the pH adjusted to 10 with 3 N NaOH. After cooling to 0 °C, an aqueous solution of O-methylisourea sulfate adjusted to pH 10 with 3 N NaOH is added. The reaction is monitored by hplc and the pH continually adjusted to 10 with the addition of 3 N NaOH. After completion of the reaction, the pH is lowered to 4.5 by the addition of 1% acetic acid and the product is purified by reverse phase hplc using an acetonitrile-water-0.1% trifluoroacetic acid system on a Hamilton PRP-1® polystyrene column.

## Example 130

Preparation of (4S,5S)-alanyl-alanyl-6-phenyl-5-amino-4-hydroxyhexanoyl)-valyl-valinamide

The protected peptidyl resin (4S,5S) Boc-Ala-Ala-(6-phenyl-5-amino-4-t-butyldimethyl-silyloxyhexanoyl)-Val-Val-BHA is prepared according to the usual method of Example 1. The peptidyl resin is cleaved and deprotected by treatment with ml anhydrous liquid HF/1 ml anisole at 0 °C for 1 hour. After removal of the HF under vacuum, the resin is washed with diethyl ether, air dried and extracted with 2 x 50 ml glacial acetic acid, which is lyophilized to yield the crude peptide. The crude product is purified by gel filtration on Sephadex® G-15 using 10% aqueous acetic acid as eluant.

## Example 131

Preparation of (4S,5S)-alanyl-(6-phenyl-5-amino-4-hydroxyhexanoyl)-valyl-valinamide

The protected peptidyl resin (4S,5S) Boc-Ala-(6-phenyl-5-amino-4-t-butyldimethyl-silyloxyhexanoyl)-Val-Val-BHA is prepared according to the usual method. The peptidyl resin is cleaved and deprotected by treatment with 10 ml anhydrous liquid HF/1 ml anisole at 0 °C for 1 hour. After removal of the HF under vacuum, the resin is washed with diethyl ether, air dried and extracted with 2 x 50 ml glacial acetic acid, which is lyophilized to yield the crude peptide. The crude product is purified by gel filtration on Sephadex® G-15 using 10% aqueous acetic acid as eluant.

## Example 132

Preparation of (4S,5S)-benzyloxycarbonylalanyl-alanyl-(6-phenyl-5(S)-amino-4(S)-hydroxyhexanoyl)-valyl-valinamide

The compound of Example 130 is dissolved in DMF to which is added 1 equiv. of triethylamine and 1.5 equiv. N-(benzyloxycarbonyloxy)succinimide. The extent of reaction is monitored by hplc and the product is purified directly from the reaction mixture by preparative hplc using an acetonitrile-water-0.1% trifluoroacetic acid system on a Hamilton PRP-1® polystyrene column.

## Example 133

Preparation of (4S,5S)-alanylalanyl-(6-phenyl-5-amino-4-hydroxyhexanoyl)-valine

The protected peptidyl resin (4S,5S) Boc-Ala-Ala-(6-phenyl-5-amino-4-t-butyldimethyl-silyloxyhexanoyl)-Val-Merrifield resin is prepared according to the usual method. The peptidyl resin is cleaved and deprotected by treatment with 10 ml anhydrous liquid HF/1 ml anisole at 0 °C for 1 hour. After removal of the HF under vacuum, the resin is washed with diethyl ether, air dried and extracted with 2 x 50 ml glacial acetic acid, which is lyophilized to yield the crude peptide. The crude product is purified by gel filtration on Sephadex® G-15 using 10% aqueous acetic acid as eluant.

## Example 134

Preparation of (4S,5S)-benzyloxycarbonylalanylalanyl-(6-phenyl-5-amino-4-hydroxyhexanoyl)-valine

The compound of Example 133 is dissolved in DMF to which is added 2 equiv. of triethylamine and 1.5 equiv. N-(benzyloxycarbonyloxy)succinimide. The extent of reaction is monitored by hplc and the product is purified directly from the reaction mixture by preparative hplc using an acetonitrile-water-0.1% trifluoroacetic acid system on a Hamilton PRP-1® polystyrene column.

## Example 135

Preparation of (4S,5S)-benzyloxycarbonylalanylalanyl-(6-phenyl-5-amino-4-hydroxyhexanoyl)-valyl-(1-aminomethyl-2-methyl)propylamide)

carbobenzyloxy-L-valinol

a) L-valinol is dissolved in water containing 1 equiv. of NaOH and cooled to 5 °C in an ice bath. Benzyl chloroformate (1.1 equiv) and 4 N NaOH (1.2 equiv.) are added alternately in several portions. When the reaction is complete, the reaction mixture is extracted with ethyl acetate and the extracts are combined, dried over $Na_2SO_4$, and concentrated. The crude product is purified by flash chromatography on silica gel.

1-phthaloylamino-2-carbobenzyloxyamino-3-methylbutane

b) Carbobenzyloxy-L-valinol is dissolved in THF along with triphenylphosphine (1.1 equiv.) and phthalimide (1.1 equiv.) and cooled to -5 °C in a salt-ice bath. Diisopropyl-azidodicarboxylate (1.1 equiv.) is added and the reaction stirred for 1 hr. at -5 °C and allowed to warm to room temperature. After stirring overnight at room temperature, the reaction mixture is evaporated and the residue is purified by flash chromatography to yield the deisred compound.

1-butyloxycarbonylamino-2-carbobenzyloxyamino-3-methylbutane

c) 1-Phthaloylamino-2-carbobenzyloxyamino-3-methylbutane is dissolved in ethanol and treated at room temperature with hydrazine hydrate (2 equiv.). When the reaction is complete by tlc, the reaction mixture is eavporated and the residue dissolved in DMF. Di-t-butyldicarbonate (3 equiv.) and triethylamine (3 equiv.) are added and the reaction is stirred at room temperature overnight. The reaction mixture is eavporated and the residue is purified by flash chromatography on silica gel to yield the desired compound.

(4S,5S)-benzyloxycarbonylalanyl-alanyl-(6-phenyl-5-amino-4-hydroxyhexanoyl)-valyl-(1-aminomethyl-2-methyl)propylamide

d) 1-t-Butyloxycarbonylamino-2-carbobenzyloxyamino-3-methylbutane is dissolved in methanol and hydrogenated over 10% palladium on carbon at 40 psig for 1 hr. The reaction mixture is filtered through a pad of celite, evaporated to dryness to give 1-t-butyloxycarbonyl-2-amino-3-methylbutane.

(4S,5S)-benzyloxycarbonylalanyl-alanyl-(6-phenyl-5-amino-4-hydroxyhexanoyl)-valyl-(1-aminomethyl-2-methyl)propylamide

e) The compound of Example 134 is dissolved in DMF along with 1-t-butyloxycarbonyl-2-amino-3-methylbutane (3 equiv.), 1-hydroxybenzotriazole (1.5 equiv.) and diisopropylcarbodiimide (1.5 equiv.) and the reaction is stirred at room temperature and monitored by hplc. When the reaction is complete, the reaction mixture is evaporated to dryness and the residue treated with trifluoroacetic acid at 0 °C for 30 minutes. The trifluoroacetic acid is removed under reduced pressure and the residue is dissolved in methanol and purified by reverse phase hplc using an acetonitrile-water-0.1% trifluoroacetic acid system on a Hamilton PRP-1® polystyrene column.

## Example 136

Preparation of (2R,5S)-2-(phenylmethyl)-5-t-butyloxycarbonylamino-6-phenylhex-3-enoic acid

2(S)-t-butyloxycarbonylamino-3-phenylpropanol

a) Boc-L-Phenylalanine is dissolved in THF containing triethylamine (1 equiv.) cooled to -5 °C. Ethyl chloroformate (21 equiv.) in THF is added dropwise over 15 min. and the reaction is stirred 30 min at -5 °C. The reaction mixture is filtered and added dropwise over 30 min. to a solution of sodium borohydride (2.5 equiv.) in water at 0 °C. The reaction is allowed to warm to room temperature and is stirred 2 hr. The reaction mixture is acidified with 1 N HCl, the aqueous layer separated and washed with ether. The ether

and THF layers are combined, washed with 10% NaOH, water, dried over $Na_2SO_4$ and concentrated to yield the titled compound.

(2S)-1-(methylsulfonyl)oxo-2-t-butyloxycarbonylamino-3-phenylpropane

b) The compound of Example 136(a) is dissolved in $CH_2Cl_2$ containing triethylamine (1.5 equiv.) and cooled to -10 °C. To this is added methanesulfonyl chloride (1.1 equiv.) over 10 min. After stirring for 10 min and -10 °C, the reaction mixture is extracted with ice water, cold 10% HCl, saturated $NaHCO_3$, and brine. The organic layer was dried over $Na_2SO_4$ and concentrated to give the titled compound.

(2S)-1-phenylthio-2-t-butyloxycarbonylamino-3-phenylpropane

c) Thiophenol (4 equiv.) is dissolved in a mixture of THF and methanol. Sodium methoxide (4 equiv.) is added and the reaction is stirred at room temperature for 15 min. The compound of Example 136(b) (1 equiv.) is added and the temperature raised to 50 °C for 2.5 hr. The reaction mixture is diluted with 10% NaOH, extracted with $CH_2Cl_2$, and evaporated to give the titled compound.

(2S)-1-(benzenesulfonyl)-2-t-butyloxycarbonylamino-3-phenylpropane

d) The compound of Example 136(c) is dissolved in $CH_2Cl_2$, cooled to 0 °C and m-chloroperbenzoic acid (3.5 equiv.) is added. The reaction is allowed to warm to room temperature and stir for 1 hr.. The reaction mixture is partitioned between $CH_2Cl_2$ and saturated $NaHSO_4$, and the organic layer washed with 1 N $NaHCO_3$, dried over $Na_2SO_4$ and evaporated to give the titled compound.

(4R)-(2-oxo-4-(phenylmethyl)-3-oxazolidinyl)-5-methylpent-3-enoate

e) The titled compound is prepared according to the procedure of Example 93(b), substituting 4-methylpent-3-enoic acid for monomethyl trans-hex-3-enoic acid, and (R)-benzyloxazolidinone for (S)-benzyloxazolidinone.

(2R,4R)-(2-oxo-4-(phenylmethyl)-3-oxazolidinyl)-2-(phenylmethyl)-5-methylpent-3-enoate

f) Using the compound of Example 136(e), the titled compound is prepared according to the procedure of Example 93 (c).

(2R) 2-(phenylmethyl)-5-methylpent-3-en-1-ol

g) The compound of Example 136(f) in THF is cooled to -78 °C and treated with $LiAlH_4$ (1 equiv.). After stirring 30 min. at -78 °C, the reaction is allowed to warm to 0 °C over 1 hr. After 30 min. at 0 °C, the reaction mixture is then cooled again to -78 °C and 1 ml ethyl acetate is added. The reaction is quenched with saturated aqueous ammonium chloride at -78 °C, allowed to warm to room temperature and diluted with water. The reaction mixture is extracted with ether and the extracts are dried over $Na_2SO_4$ and evaporated to yield the titled compound, which is purified by flash chromatography on silica gel.

(2R)-1-(tetrahydropyranyl)oxy-2-(phenylmethyl)-5-methyl-3-pentene

h) The compound of Example 136(g) is dissolved in $CH_2Cl_2$ and dihydropyran (1.5 equiv.) is added along with pyridinium p-toluenesulfonate (0.1 equiv.) and stirred at room temperature for 4 hrs. The solution is diluted with ether, washed with brine, dried over $Na_2SO_4$ and evaporated to yield the titled compound.

(2S)-2-(phenylmethyl)-3-tetrahydropyranyloxypropionaldehyde

i) The compound of Example 136(h) is dissolved in $CH_2Cl_2$ cooled to -78 °C and treated with ozone until a blue color persists. The reaction is quenched with dimethylsulfide, allowed to warm to room temperature and concentrated by evaporation to yield titled compound, which is used without further purification.

(2R,5S)-1-(tetrahydropyranyl)oxo-2-(phenylmethyl)-5-t-butyloxycarbonylamino-6-phenylhex-3-ene

j) A solution of the sulfone of Example 136(d) in THF is cooled to -78 °C and is treated with methyllithium (2 equiv. of 1.6 M solution in ether) over 5 min. and is stirred at -78 °C for 20 min. In a separate flask, the aldehyde of Example 136(i) (2.75 equiv) in THF is cooled to -78 °C and treated with diisobutylaluminum methoxide (2.25 equiv.) in THF (prepared by the addition of an equimolar amount of methanol to 1 M diiosbutylaluminum hydride in THF). The aldehyde solution is transferred to the sulfone dianion via cannula and stirred at -78 °C for 30 min. The reaction is quenched by the addition of saturated aqueous ammonium chloride and the product extracted with ether, dried over $MgSO_4$ and concentrated under reduced pressure.

The crude β-hydroxysulfone is dissolved in methanol and cooled to 0 °C. Disodium hydrogen phosphate (4 equiv.) is added followed by 5% sodium amalgum (12 equiv.). After stirring 4 hr. at 0 °C, the reaction is diluted with water and extracted with $CH_2Cl_2$, dried over $MgSO_4$, and concentrated. The crude product is purified by flash chromatography on silica gel to afford the titled compound.

(2R,5S)-2-(phenylmethyl)-5-t-butyloxycarbonylamino-6-phenylhex-3-enoic acid

k) The compound of Example 136(j) is dissolved in acetone, cooled to 0 °C and treated dropwise with Jones reagent (3 equiv.). After stirring for 3 hr., the reaction mixture is diluted with water and extracted with ether. The ethereal extracts are washed with 5% NaOH and the aqueous washings are combined, acidified to pH 2 with 10% HCl and extracted with ether. The ether extracts are dried over $Na_2SO_4$ and evaporated to give the titled compound.

## Example 137

Preparation of-(2R,5S) alanylalanyl-(2-(phenylmethyl)-5-amino-6-phenyl-trans-hex-3-enoyl)-valyl-valinamide

The protected peptidyl resin (2R,5S) Boc-Ala-Ala-(2-(phenylmethyl)-5-amino-6-phenyl-trans-hex-3-enoyl)-Val-Val-BHA is prepared on the according to the usual method of Example 1. The peptidyl resin is cleaved and deprotected by treatment with 10 ml anhydrous liquid HF/1 ml anisole at 0 °C for 1 hour. After removal of the HF under vacuum, the resin is washed with diethyl ether, air dried and extracted with 2 x 50 ml glacial acetic acid, which is lyophilized to yield the crude peptide. The crude product is purified by gel filtration on Sephadex® G-15 using 10% aqueous acetic acid as eluant to yield the titled compound.

## Example 138

Preparation of (2R,5S)-benzyloxycarbonylalanylalanyl-(2-(phenylmethyl)-5-amino-6-phenyl-trans-hex-3-enoyl)-valyl-valinamide

The compound of Example 137 is dissolved in DMF to which is added 2 equiv. of triethylamine and 1.5 equiv N-(benzyloxycarbonyloxy)succinimide. The extent of reaction is monitored by hplc and the product is purified directly from the reaction mixture by preparative hplc using an acetonitrile-water-0.1% trifluoroacetic acid system on a Hamilton PRP-1® polystyrene column.

Example 139

Preparation of (6S)-6-(tert-butyloxycarbonyl)amino-7-phenyl-5-oxo-heptanoic acid

Using the procedure of Example 12, except substituting 5-bromo-1-pentene for 4-bromo-1-butene, the titled compound is prepared.

Using the detailed procedures described herein in Examples 19, 34, 76, 77, 78, 88, and 135, the following specific compounds are prepared:

a) (5S,6S)-6-(carbobenzyloxy-alanylalanyl)amino-7-phenyl-5-hydroxy-(1-oxo)heptyl-valyl valine methyl ester;

b) (5S,6S)-6-(carbobenzyloxy-alanyl)amino-7-phenyl-5-hydroxy-(1-oxo)heptyl-valyl valine methyl ester;

c) (5S,6S)-6-(carbobenzyloxy-alanylalanyl)amino-7-phenyl-5-hydroxy-(1-oxo)heptyl-valyl valinamide;

d) (5S,6S)-6-(carbobenzyloxy-alanyl)amino-7-phenyl-5-hydroxy-(1-oxo)heptyl-valyl valinamide;

e) (5S,6S)-6-(carbobenzyloxy-alanylalanyl)amino-7-phenyl-5-hydroxy-(1-oxo)heptyl-valyl valinol;

f) (5S,6S)-6-(carbobenzyloxy-alanyl)amino-7-phenyl-5-hydroxy-(1-oxo)heptyl-valyl valinol;

g) (5S,6S)-6-(carbobenzyloxy-alanylalanyl)amino-7-phenyl-5-hydroxy-(1-oxo)heptyl-valyl valin-((S)-1-amino-3-methylbutyl-2-)amide; and

h) (5S,6S)-6-(carbobenzyloxy-alanyl)amino-7-phenyl-5-hydroxy-(1-oxo)heptyl-valyl valin-((S)-1-amino-3-methylbutyl-2-)amide.

Example 140

Using the procedure of Example 151 to prepare (2R,5S)-2-methyl-5-(tert-butyloxycarbonyl)amino-6-phenyl-4-oxo-hexanoic acid and employing the detailed procedures described herein in Examples 19, 34, 76, 77, 78, 88 and 135, the following specific compounds are prepared:

a) (2R,4S,5S)-5-(carbobenzyloxy-alanylalanyl)amino-6-phenyl-4-hydroxy-2-methyl-(1-oxo)hexyl-valyl valine methyl ester;

b) (2R,4S,5S)-5 (carbobenzyloxy-alanylalanyl)amino-6-phenyl-4-hydroxy-2-methyl-(1-oxo)hexyl-valyl valinamide;

c) (2R,4S,5S)-5-(carbobenzyloxy-alanyl)amino-6-phenyl-4-hydroxy-2-methyl-(1-oxo)hexyl-valyl valinamide;

d) (2R,4S,5S)-5-(carbobenzyloxy-alanylalanyl)amino-6-phenyl-4-hydroxy-2-methyl-(1-oxo)hexyl-valyl valinol;

e) (2R,4S,5S)-5-(carbobenzyloxy-alanyl)amino-6-phenyl- 4-hydroxy-2-methyl-(1-oxo)hexyl-valyl valinol;

f) (2R,4S,5S)-5-(carbobenzyloxy-alanylalanyl)amino-6-phenyl-4-hydroxy-2-methyl-(1-oxo)hexyl-valyl valin-((S)-1-amino-3-methylbutyl-2-)amide;

g) (2R,4S,5S)-5-(carbobenzyloxy-alanyl)amino-6-phenyl-4-hydroxy-2-methyl-(1-oxo)hexyl-valyl valin-(-(S)-1-amino-3-methylbutyl-2-)amide;

h) (2R,4S,5S)-5-(carbobenzyloxy-alanylalanyl)amino-6-phenyl-4-hydroxy-2-methyl-(1-oxo)hexyl-valyl valine; and

i) (2R,4S,5S)-5-(carbobenzyloxy-alanyl)amino-6-phenyl-4-hydroxy-2-methyl-(1-oxo)hexyl-valyl valine.

Example 141

Preparation of (2R,5S)-2-benzyl-5-(tert-butyloxycarbonyl)amino-6-phenyl-4-oxo-hexanoic acid

Using the procedure of Example 151, except using 3-bromo-2-phenylmethyl-1-benzyloxypropane for 3-bromo-2-methyl-1-benzyloxypropane, the titled compound is prepared.

Using (2R,5S)-2-benzyl-5-(tert-butyloxycarbonyl)amino-6-phenyl-4-oxo-hexanoic acid and employing the detailed procedures described herein in Examples 19, 34, 76, 77, 78, 88 and 135, the following specific

compounds are prepared:

a) (2R,4S,5S)-5-(carbobenzyloxy-alanylalanyl)amino-6-phenyl-4-hydroxy-2-benzyl-(1-oxo)hexyl-valyl valine methyl ester;

b) (2R,4S,5S)-5-(carbobenzyloxy-alanyl)amino-6-phenyl-4-hydroxy-2-benzyl-(1-oxo)hexyl-valyl valine methyl ester;

c) (2R,4S,5S)-5-(carbobenzyloxy-alanylalanyl)amino-6-phenyl-4-hydroxy-2-benzyl-(1-oxo)hexyl-valyl valinamide;

d) (2R,4S,5S)-5-(carbobenzyloxy-alanyl)amino-6-phenyl-4-hydroxy-2-benzyl-(1-oxo)hexyl-valyl valinamide;

e) (2R,4S,5S)-5-(carbobenzyloxy-alanylalanyl)amino-6-phenyl-4-hydroxy-2-benzyl-(1-oxo)hexyl-valyl valinol;

f) (2R,4S,5S)-5-(carbobenzyloxy-alanyl)amino-6-phenyl-4-hydroxy-2-benzyl-(1-oxo)hexyl-valyl valinol;

g) (2R,4S,5S)-5-(carbobenzyloxy-alanylalanyl)amino-6-phenyl-4-hydroxy-2-benzyl-(1-oxo)hexyl-valyl valin-((S)-1- amino-3-methylbutyl-2-)amide;

h) (2R,4S,5S)-5-(carbobenzyloxy-alanyl)amino-6-phenyl-4-hydroxy-2-benzyl-(1-oxo)hexyl-valyl valin-(-(S)-1-amino-3-methylbutyl-2-)amide;

i) (2R,4S,5S)-5-(carbobenzyloxy-alanylalanyl)amino-6-phenyl-4-hydroxy-2-benzyl-(1-oxo)hexyl-valyl valine; and

j) (2R,4S,5S)-5-(carbobenzyloxy-alanyl)amino-6-phenyl-4-hydroxy-2-benzyl-(1-oxo)hexyl-valyl valine.

## Example 142

### Preparation of (3RS,5S)-3-isobutyl-5-(tert-butyloxycarbonyl)amino-6-phenyl-4-oxo-hexanoic acid

#### 4-hydroxy-6-methyl-hept-1-ene

a) To a solution of isobutyraldehyde (1.0 g, (1.6 mmol) in anhydrous THF (30 ml)at 0 ° C, allylmagnesium bromide (12.2 mmol) is added. After addition the reaction is allowed to warm to room temperature and stirred for an additional 2 h. The reaction is quenched by pouring into a stirred mixture of 5% HCl and diethyl ether. The organic layer is separated, washed with water and brine and dried over $MgSO_4$. Filtration and evaporation of the solvent yields the titled compound.

#### 4-bromo-6-methyl-hept-1-ene

b) The compound of Example 142(a) (0.8 g, .625 mmol) is dissolved in $CH_2Cl_2$ (25 ml) and treated with mesyl chloride (0.72 g,.625 mmol) and triethylamine (0.63 g, .625 mmol) in methylene chloride. After stirring 3 h at room temperature, the reaction is poured into cold 5% HCl and diethyl ether. Standard extractive workup affords the crude mesylate. The crude mesylate is treated with an excess of LiBr (3 equiv.) in dry DMF at 120 ° C for 6 h. The reaction is cooled and the reaction mixture is diluted with water. Standard extractive workup with pentane yields the titled compound, which is purified by distillation.

#### (3RS,5S)-3-isobutyl-5-(tert-butyloxycarbonyl)amino-6-phenyl-4-oxo-hexanoic acid

c) Using the procedure of Example 12, except substituting 4-bromo-6-methyl-hept-1-ene for 4-bromo-1-butene, yields the titled compound.

Using the detailed procedures described herein in Examples 19, 34, 76, 77 and 78, the following specific compounds are prepared:

a1) (3SR,4S,5S)-5-(carbobenzyloxy-alanylalanyl)amino-6-phenyl-4-hydroxy-3-isobutyl-(1-oxo)hexyl-valyl valine methyl ester;

b1) (3SR,4S,5S)-5-(carbobenzyloxy-alanyl)amino-6-phenyl-4-hydroxy-3-isobutyl-(1-oxo)hexyl-valyl valine methyl ester;

c1) (3SR,4S,5S)-5-(carbobenzyloxy-alanylalanyl)amino-6-phenyl-4-hydroxy-3-isobutyl-(1-oxo)hexyl-valyl valinamide; and

d1) (3SR,4S,5S)-5-(carbobenzyloxy-alanyl)amino-6-phenyl-4-hydroxy-3-isobutyl-(1-oxo)hexyl-valyl valinamide.

## Example 143

### Preparation of (3RS,5S)-3-methyl-5-(tert-Butyloxycarbonyl)amino-6-phenyl-4-oxo-hexanoic acid

Using the procedure of Example 142, except substituting acetaldehyde for isobutyraldehyde, the titled compound is prepared.

Using the detailed procedures described herein in Examples 19, 34, 76, 77, and 78, the following specific compounds are prepared:

a) (3SR,4S,5S)-5-(carbobenzyloxy-alanylalanyl)amino-6-phenyl-4-hydroxy-3-methyl-(1-oxo)hexyl-valyl valine methyl ester;

b) (3SR,4S,5S)-5-(carbobenzyloxy-alanyl)amino-6-phenyl-4-hydroxy-3-methyl-(1-oxo)hexyl-valyl valine methyl ester;

c) (3SR,4S,5S)-5-(carbobenzyloxy-alanylalanyl)amino-6-phenyl-4-hydroxy-3-methyl-(1-oxo)hexyl-valyl valinamide; and

d) (3SR,4S,5S)-5-(carbobenzyloxy-alanyl)amino-6-phenyl-4-hydroxy-3-methyl-(1-oxo)hexyl-valyl valinamide.

## Example 144

### Preparation of (3RS,5S)-3-benzyl-5-(tert-butyloxycarbonyl)amino-6-phenyl-4-oxo-hexanoic acid

Using the procedure of Example 142, except substituting phenylacetaldehyde for isobutyraldehyde, the titled compound is prepared.

Using the detailed procedures described herein in Examples 19, 34, 76, 77 and 78, the following specific compounds are prepared:

a) (3SR,4S,5S)-5-(carbobenzyloxy-alanylalanyl)amino-6-phenyl-4-hydroxy-3-benzyl-(1-oxo)hexyl-valyl valine methyl ester;

b) (3SR,4S,5S)-5-(carbobenzyloxy-alanyl)amino-6-phenyl-4-hydroxy-3-benzyl-(1-oxo)hexyl-valyl valine methyl ester;

c) (3SR,4S,5S)-5-(carbobenzyloxy-alanylalanyl)amino-6-phenyl-4-hydroxy-3-benzyl-(1-oxo)hexyl-valyl valinamide; and

d) (3SR,4S,5S)-5-(carbobenzyloxy-alanyl)amino-6-phenyl-4-hydroxy-3-benzyl-(1-oxo)hexyl-valyl valinamide.

## Example 145

### Preparation of (5S)-5-(tert-Butyloxycarbonyl)amino-5-phenyl-4-oxo-pentanoic acid

Using procedure of Example 12, except substituting Boc-phenylglycine for Boc-phenylalanine, the titled compound is prepared.

Using the detailed procedures described herein in Examples 34, 76, 77 and 78, the following specific compounds are prepared:

a) (5S,4S)-5-(carbobenzyloxy-alanylalanyl)amino-5-phenyl-4-hydroxy-(1-oxo)pentyl-valyl valine methyl ester; and

b) (5S,4S)-5-(carbobenzyloxy-alanyl)amino-5-phenyl-4-hydroxy-(1-oxo)pentyl-valyl valine methyl ester.

## Example 146

### Preparation of (5S)-5-(tert-Butyloxycarbonyl)amino-7-phenyl-4-oxo-heptanoic acid

Using the procedure of Example 12, except substituting Boc-homophenylalanine in place of Boc-phenylalanine, the titled compound is prepared.

Using the detailed procedures described herein in Examples 34, 76, 77 and 78 the following specific compounds are prepared:

a) (4S,5S)-5-(carbobenzyloxy-alanylalanyl)amino-7-phenyl-4-hydroxy-(1-oxo)heptyl-valyl valine methyl ester; and

b) (4S,5S)-5-(carbobenzyloxy-alanyl)amino-7-phenyl-4-hydroxy-(1-oxo)heptyl-valyl valine methyl ester.

## Example 147

### Preparation of (5S)-5(carbobenzyloxyalanyl)amino-4-hydroxy-1-oxo-6-phenyl-hexyl-valine, isobutyl amide

#### (5S)-5-(tert-butyloxy)amino-1,4-dioxo-6-phenylhexyl-valine, isobutylamide

a) A solution of the compound of Example 12(b) (1.0 mmol) and NMM (1.2 mmol) in THF (5 ml.) at -40° is treated in dropwise fashion with isobutyrylchloroformate (1.0 mmol). After 15 min., a solution of L-valine isobutylamide (1.2 mmol) in DMF (2 ml.) is added. The mixture is stirred with warming to room temperature overnight, then is poured into 4% aq. HCl and extracted with $CHCl_3$. The organic layer is washed with 5% $NaHCO_3$, then $H_2O$, and concentrated to yield the titled compound.

#### (5S)-5-(tert-butyloxycarbonyl)-4-hydroxy-1-oxo-6-phenyl-hexyl-valine, isobutyl amide

b)The product of Example 147(a) is reduced with $NaBH_4$ (1. equiv) in methanol. Dilute HCl is added and the product is isolated by extraction with $CHCl_3$. Preparative HPLC in silica using a $CH_2Cl_2/CH_3OH$ system yields the titled compound, along with its hydroxy epimer.

#### (5S)-5-amino-4-hydroxy-1-oxo-6-phenyl-hexyl-valine, isobutyl amide

c) The product of Example 147(b) is dissolved in a minimal volume of neat TFA. After 5 minutes, methanol is added, followed by 1 equivalent of conc. HCl. The solvents are removed in vacuo to yield the titled compound.

#### (5S)-5-(carbobenzyloxyalanyl)amino-4-hydroxy-1-oxo-6-phenyl-hexyl-valine isobutyl amide

d) A solution of Cbz-alanine (0.50 mmol) and NMM (0.60 mmol) in THF (5 ml.) at -40° is treated dropwise with isobutyrylchloroformate (0.50 mmol). After 15 minutes, more NMM (0.60 mmol) is added, followed by a solution of the product of Example 147(c) (0.60 mmol) in DMF (2 ml.). The mixture is allowed to warm to room temperature with stirring overnight. 5% HCl is added and the product is isolated by extraction with $CHCl_3$.

## Example 148

Preparation of (5S)-5-(carbobenzyloxyalanylalanyl)amino-4-hydroxy-1-oxo-6-phenyl-hexyl valine, isobutylamide

The titled compound is prepared as described in Example 147(d), except Cbz-alanylalanine (0.50 mmol) is used in place of Cbz-alanine.

## Example 149

Preparation of 5-(myristyl)amino-4-hydroxy-1-oxo-6-phenyl-hexyl valine, isobutyl amide

To a solution of the product of Example 147(c) (0.10 mmol) in pyridine (2 ml.) is added myristic anhydride (0.12 mmol). After 12 hours the solution is diluted with 10% HCl and extracted with ethyl acetate. The organic layer is washed with 5% $NaHCO_3$ and $H_2O$, then concentrated to afford the titled compound.

Preparation of 5-(stearyl)amino-4-hydroxy-1-oxo-6-phenyl-hexyl valine, isobutyl amide

The titled compound is prepared as in Example 149 except substituting stearic anhydride for myristic anhydride.

## Example 151

Preparation of 5-(carbobenzyloxyalanyl)amino-4-hydroxy-2-methyl-1-oxo-6-phenyl-hexylvalyl valine, methyl ester

5-(tert-butyloxycarbonyl)amino-4-oxo-2-methyl-6-phenyl-1-benzyloxy hexane

a) The titled compound is prepared as in the procedure of Example 12(a), except instead of 4-bromo-1-butene, the 3-bromo-2-methyl-1-benzyloxy-propane (J. Med. Chem. 30, 374, (1987)) is used.

5-(tert-butyloxycarbonyl)amino-4-oxo-2-methyl-1-hydroxy-6-phenyl hexane

b) The titled compound is prepared by hydrogenolysis of the product of Example 151(a) using the procedure of Example 86(b).

5-(tert-butyloxycarbonyl)amino-4-oxo-2-methyl-6-phenyl-hexanoic acid

c) To a mixture of the product of Example 151(b) and 5:1:1 benzene/$H_2O$/AcOH is added $(n\text{-}Bu)_4NBr$ (0.01 eq.). $KMnO_4$ (3.5 eq.) is added and the mixture is stirred for 2 hours, then sat. aqueous sodium bisulfite is added. After several minutes, the mixture is acidified to pH 1-2 with sat. aqueous $KHSO_4$, then is extracted with EtOAC. The organic layer is concentrated and the residue is purified by silica chromatography (ethyl acetate/hexane/HOAc) to yield the titled compound.

5-(carbobenzyloxy alanyl)amino-4-hydroxy-1-oxo-6-phenyl-hexylvalyl valine, methyl ester

d) The compound of Example 151(c) is coupled to valyl valine methyl ester using the method of Example 35(a) except substituting the compound of Example 151(c) for 5-(tert-butyloxycarbonyl)amino-4-oxo-6-(4-benzyloxy-phenyl)hexanoic acid. The ketone is reduced with $NaBH_4$ using the procedure of

Example 84(b). The amino group is deprotected by the method of Example 35(c) and coupled to Cbz-alanine by the method of Example 72(a) to yield the titled compound.

Example 152

Preparation of 7-methyl-5-(carbobenzyloxyalanyl)amino-3,4-dihydroxy-2-phenylmethyl-1-oxo-octyl valine, isobutylamide

7-methyl-4,5-0,(tert-butyloxycarbonyl)N-isopropylidine-3-hydroxy-2-phenylmethyl-octanoic acidic benzyl ester

a) A solution of 2,2-dimethyl-3 (tert-butyloxy carbonyl)-4-isopropyl-5-formyl-1,3 oxazolidine (1.0 mmol) (J. Med. Chem. 30, 976 (1987)) in THF (2 ml) is added to a -78° solution of 1.1 mmol lithio-benzyl-3-phenylpropionate (prepared from lithium diisopropyl amide (1.1 mmol) and benzyl-3-phenylpropionate (1.1 mmol)). The mixture is stirred at -78° for 1 hour, then allowed to warm to room temperature and is diluted with pH 7 phosphate buffer. The mixture is extracted with chloroform, the organic layer is concentrated, and the residue is purified by flash chromatography to yield the titled compound as a mixture of isomers.

7-methyl-5-(tert-butyloxycarbonyl)amino-3,4-dioxyisopropylidine-2-phenylmethyl-octanoic acid, benzyl ester

b) A solution of the product of Example 152(a) (0.5 mmol) in $CH_2Cl_2$ (10 ml) is stirred with camphorsulfonic acid (0.1 mmol) overnight. Excess solid $NaHCO_3$ is added with acetone (10 ml), the mixtue is stirred vigorously for 1 hour, and filtered through Celite. Concentration and flash chromatography provides the titled compound.

7-methyl-5(tert-butyloxycarbonyl)amino-3,4-dioxy-isopropylidene-2-phenylmethyl-octanoic acid

c) A solution of the product of Example 152(b) (0.2 mmol) in methanol (5 ml) is stirred with 10% Pd on activated carbon (20 mg) for 8 hours under 1 atm. $H_2$. The mixture is filtered through Celite and concentrated to yield the titled compound.

7-methyl-5(tert-butyloxyamino)-3,4-dioxy-isopropylidine-2-phenylmethyl-1-oxo-octyl valine, isobutyl amide

d) The titled compound is prepared by the procedure of Example 87(a) except substituting the product of Example 152(c) for 5-(tert-butyloxycarbonyl)amino-4-oxo-6-phenyl hexanoic acid, and eliminating the wash with 5% HCl in the workup.

7-methyl-5-amino-3,4-dihydroxy-2-phenylmethyl-1-oxo-octyl valine, isobutyl amide, hydrochloride

e) The titled compound is prepared from the product of Example 152(d) by the procedure of Example 147(c).

7-methyl-5-(carbobenzyloxyalanyl)amino-3,4-dihydroxy-2-phenylmethyl-1-oxo-octyl valine, isobutyl amide

f) The titled compound is prepared by the procedure of Example 147(d), except substituting the product of Example 152(e).

Example 153

Preparation of (4S,5S)-5-(alanylalanyl)amino-4-hydroxy-1-oxo-6-(4-hydroxophenyl)-hexyl-valyl valinol

(4S,5S)-5-(carbobenzyloxy-alanylalanyl)amino-4-hydroxy-1-oxo-6-(4-benzyloxyphenyl)-hexyl-valyl valine

a) The compound of Example 72(a) (1 mmol) is dissolved in methanol (10 ml) and potassium hydroxide (1.5 mm) in $H_2O$ (2 ml) is added dropwise. The reaction is monitored by thin layer chromatography until hydrolysis of the ester is complete. The reaction is neutralized with 5% HCl, the methanol is evaporated under reduced pressure and the residue is dissolved in $CHCl_3$. The organic extract is washed with 5% HCl and water and dried over $MgSO_4$. Filtration and evaporation of the solvent provides the titled compound.

(4S,5S)-5-(carbobenzyloxy-alanylalanyl)amino-4-hydroxy-1-oxo-6-(4-benzyloxyphenyl)-hexyl-valyl valinol

b) A solution of the compound of Example 153(a) (.5 mmol) is dissolved in $CH_2Cl_2$ (5 ml) under an Ar atmosphere and cooled to O° C. Borane methyl sulfide (.55 mmol) is added dropwise. The reaction is stirred .5 hr. at 5° C and 2 hr. at room temperature. Methanol (3 ml) is added, followed by acetic acid (2 ml) and the solution is stirred at room temperature for an additional 2 hr. The solvents are evaporated under reduced pressure, the residue is dissolved in $CHCl_3$, and washed successively with 5% HCl, $H_2O$ and 5% $NaHCO_3$. The organic extract is dried over $MgSO_4$, filtered and the solvent is evaporated to yield the titled compound.

(4S,5S)-5-alanylalanyl)amino-4-hydroxy-1-oxo-6-(4-hydroxophenyl)-hexyl-valyl valinol

c) To a solution of the compound of Example 153(b) (.2 mmol) in acetic acid, 10% palladium (150 mg) on activated carbon is added. Hydrogen is bubbled through the mixture for 60 min. and the reaction is stirred under a hydrogen atmosphere for 20 hr. The suspension is filtered through Celite® and the solvent is evaporated under reduced pressure to yield the titled compound.

## Example 154

Preparation of 4-methyl-3-[5-(alanylalanyl)amino-4-hydroxy-1-oxo-6-(4-hydroxophenyl)-hexyl-valyl]amino-pentan-2-one

4-methyl-3-[5-(carbobenzyloxy-alanylalanyl)amino-4-hydroxy-1-oxo-6-(4-benzyloxyphenyl)-hexyl-valyl]amino-pentan-2-one

a) The compound of Example 153(a) (.2 mmol) is dissolved in methylene chloride (4 ml) at 0° C and oxalyl chloride (.25 mmol) is added followed by 1 drop DMF. After stirring for .5 hr. at 0° C and .5 hr. at room temperature, the methylene chloride solution is added dropwise to a solution of an excess of diazomethane (>4 mmol) in a mixture of $Et_2O$ and THF. After stirring at 10° C for 1 hr., acetic acid is added until nitrogen evolution ceases. The solvents are evaporated, the residue is dissolved in $CHCl_3$ , washed with water and dried over $MgSO_4$. Filtration and evaporation of the solvent yields the titled compound.

4-methyl-3-[5-(alanylalanyl)amino-4-hydroxy-1-oxo-6-(4-hydroxyphenyl)-hexyl-valyl]amino-pentan-2-one

b) To a solution of the compound of Example 154(a) (.1 mmol) in acetic acid, 10% palladium (80 mg) on activated carbon is added. Hydrogen is bubbled through the mixture for 60 min. and the reaction is stirred under a hydrogen atmosphere for 20 hr. The suspension is filtered through Celite® and the solvent is

evaporated under reduced pressure to yield the titled compound.

Example 155

Preparation of Ac-Arg-Ala-Ser-Gln-Asn-Tyr-Pro-Val-Val-$NH_2$

Using the procedure of Example 1, except substituting Boc-Phe for Boc-Tyr(BrZ), the titled compound was prepared.

Example 156

Using the method of Example 58, except substituting Boc-His(Tos), Boc-Val, Boc-Ile, Boc-Lys(Clz), Boc-Ser(Bzl), Boc-D-Arg(Tos) or Boc-Met for Boc-Ala, yields the following peptides:
His-Ser-Gln-Asn-Tyr-Pro-Val-Val-$NH_2$;
Val-Ser-Gln-Asn-Tyr-Pro-Val-Val-$NH_2$;
Ile-Ser-Gln-Asn-Tyr-Pro-Val-Val-$NH_2$;
Lys-Ser-Gln-Asn-Tyr-Pro-Val-Val-$NH_2$;
D-Arg-Ser-Gln-Asn-Tyr-Pro-Val-Val-$NH_2$;
Ser-Ser-Gln-Asn-Tyr-Pro-Val-Val-$NH_2$; and
Met-Ser-Gln-Asn-Tyr-Pro-Val-Val-$NH_2$.

Example 157

Using the method of Example 39, except beginning the sequence with Boc-Ala-BHA, Boc-Gly-BHA, Boc-Ile-BHA, Boc-Leu-BHA or Boc-Met-BHA, yields the following peptides.
Ac-Ser-Gln-Asn-Tyr-Pro-Val-Ala-$NH_2$;
Ac-Ser-Gln-Asn-Tyr-Pro-Val-Gly-$NH_2$;
Ac-Ser-Gln-Asn-Tyr-Pro-Val-Ile-$NH_2$;
Ac-Ser-Gln-Asn-Tyr-Pro-Val-Leu-$NH_2$; and
Ac-Ser-Gln-Asn-Tyr-Pro-Val-Met-$NH_2$.

Example 158

Using the method of Example 39, except substituting Boc-Ala, Boc-Gly, Boc-Ile, Boc-Arg(Tos), Boc-Leu or Boc-Met for Boc-Val in the second residue of the peptide, yields the following peptides.
Ac-Ser-Gln-Asn-Tyr-Pro-Ala-Val-$NH_2$;
Ac-Ser-Gln-Asn-Tyr-Pro-Gly-Val-$NH_2$;
Ac-Ser-Gln-Asn-Tyr-Pro-Ile-Val-$NH_2$;
Ac-Ser-Gln-Asn-Tyr-Pro-Arg-Val-$NH_2$.
Ac-Ser-Gln-Asn-Tyr-Pro-Leu-Val-$NH_2$; and
Ac-Ser-Gln-Asn-Tyr-Pro-Met-Val-$NH_2$.

Example 159

Using the method of Example 40, except substituting Boc-His(Tos), Boc-Val, Boc-Ile, Boc-Leu, Boc-Thr-(Bzl) or Boc-Gln for Boc-Ala in the fifth residue of the peptide, yields the following peptides.
Ac-Ser-Ala-His-Tyr-Pro-Val-Val-$NH_2$;

Ac-Ser-Ala-Val-Tyr-Pro-Val-Val-$NH_2$;
Ac-Ser-Ala-Leu-Tyr-Pro-Val-Val-$NH_2$;
Ac-Ser-Ala-Thr-Tyr-Pro-Val-Val-$NH_2$; and
Ac-Ser-Ala-Gln-Tyr-Pro-Val-Val-$NH_2$.

## Example 160

### Preparation of (5S,4RS)-5-(tert-butoxycarbonylserylalanylalanyl)amino-4-hydroxy-1-oxo-6-phenylhexyl-valyl valine

The compound of Example 34(d) (50 mg, 6.5 X $10^{-5}$ mole) is dissolved in methanol (5 ml) and water (1 ml) containing potassium carbonate (27 mg, 19.5 X $10^{-5}$ mole) is added dropwise. After stirring for 6 hr. at room temperature, the reaction mixture is diluted with ethyl acetate and water. The pH is adjusted to 3.0 with dil. HCl and the aqueous layer is separated. The organic layer is washed with water (1X) and dried over $MgSO_4$. Filtration and evaporation of the ethyl acetate yields the titled compound.

## Example 161

### Preparation of (5S,4RS)-5-[(tert-butoxycarbonyl-serylalanylalanyl)-amino]-4-hydroxy-1-oxo-6-phenylhexyl-valyl valine-diethylamide

The compound of Example 160 (60 mg, 7.8 X $10^{-5}$ mole) is dissolved in anhydrous THF (4 ml) at -40°C under an argon atmosphere and N-methyl morpholine (10.2 mg, 1.01 X $10^{-4}$ mole) is added, followed by isobutyrlchloroformate (11 mg, 7.8 X $10^{-5}$ mole). After stirring for 15 min., diethyl amine (11 mg, 1.56 X $10^{-4}$ mole) is added. After stirring for an additional 30 min. at -40°C the reaction mixture is stirred at room temperature. The reaction is diluted with ethyl acetate and washed successively with 5% HCl, 5% sodium bicarbonate and brine. The organic extract is dried over $MgSO_4$, filtered and evaporated in vacuo to yield the titled compound.

Using the same procedure, except substituting n-butylamine for the diethylamine, yields (5S,4RS)-5-[-(tert-butoxycarbonyl-serylalanylalanyl)amino]-4-hydroxy-1-oxo-6-phenylhexyl-valyl valine-butylamide

## Example 162

### Preparation of (5S,4RS)-5-[(tert-butoxycarbonyl-serylalanylalanyl)amino[-4-hydroxy-1-oxo-6-phenylhexyl valine-isobutylamide

#### (5S,4RS)-5-[(tert-butoxycarbonyl-serylalanylalanyl)amino[-4-hydroxy-1-oxo-6-phenylhexyl valine methyl ester

a.) Using the procedure of Example 34, except substituting valine methyl ester for valyl valine methyl ester, the titled compound is produced.

#### (5S,4RS)-5-[(tert-butoxycarbonyl-serylalanylalanyl)amino[-4-hydroxy-1-oxo-6-phenylhexyl valine

b.) Using the procedure of Example 160, except substituting the peptide of Example 162(a), the titled carboxylic acid is produced.

(5S,4RS)-5-[(tert-butoxycarbonyl-serylalanylalanyl)amino[-4-hydroxy-1-oxo-6-phenylhexyl valine-isobutylamide

c.) Using the peptide of Example 162(b) and the procedure of Example 161, except substituting isobutylamine for diethylamine, the titled compound is produced.

## Example 163

### Liposomal Dosage Unit Composition

Phosphatidylcholine (1.4 g) and phosphatidylglycerol (.6g) are dissolved in 300 ml of a 20% methanol in chloroform solvent and evaporated to dryness. A solution of the peptide (30 mg in 200 ml of phosphate buffered saline) is added to the dry phospholipid film which is allowed to equilibrate at room temperature for 1-2 hr. The liposome dispersion formed is then vortexed to insure uniform mixing. The resulting suspension is extruded through a 0.2μ polycarbonate filter five times to produce a uniform size distribution. If necessary the suspension can be dialysed or ultracentrifuged to remove non-encapsulated peptide.

## Example 164

### Liposomal Dosage Unit Composition

In a beaker, cholesterol (49 mg) and oleic acid (.358 g) are warmed to 65° C for 20-30 min. Maintaining the temperature, phospholipids (1 g) are added slowly, ensuring complete wetting by the oleic acid. A solution of arginine (.22 g in 3.37 g water) at 40° C is added in small aliquots and thoroughly mixed into the slurry. Mixing is maintained at 40° C. After equilibration for one week, the peptide (150 mg) is mixed thoroughly into the gel. The pH is adjusted to 7.0 with acetic acid if necessary. Phosphate buffered saline (pH 7.4) is added in small aliquots with vortexing to achieve a concentration of 200 mg of liposomal gel per ml. Liposomes form spontaneously. This procedure produces 5 g of liposomal suspension. A standard dosage unit is 1 g of liposomal suspension.

## Example 165

### Parenteral Dosage Unit Composition

A preparation which contains 25 mg of a peptide of this invention is prepared as follows:

25 mg of the peptide is dissolved in 15 ml of distilled water. The solution is filtered under sterile conditions in to a 25 ml multi-dose ampoule and lyophilized. The powder is reconstituted by addition of 20 ml of 5% dextrose in water(D5W) for intravenous or intramuscular injection. The dosage is thereby determined by the injection volume. This solution is also suitable for use in other methods for administration, such as addition to a bottle or bag for IV drip infusion.

## Example 166

### Oral Dosage Unit Composition

A capsule for oral administration is prepared by mixing and milling 35 mg of the peptide with 75 mg of

lactose and 5 mg of magnesium stearate. The resulting powder is screened and filled into a hard gelatin capsule.

The above description fully discloses how to make and use this invention. This invention, however, is not limited to the precise embodiments described herein, but encompasses all modifications within the scope of the claims which follow.

## Claims

1. A peptide of the formula:

$A\text{-}B\text{-}(Q)_a\text{-}(C)_b\text{-}(D)_c\text{-}M\text{-}(W)_d\text{-}(X)_e\text{-}Y\text{-}Z$ (I)

wherein:

A is BocNH, CbzNH, H, $R'R''N$, $R''CONR'$ or DnsNH, or if a, b and c are 0 and Y is a covalent bond, then A is H, Boc, Cbz, $R''$ or $R''CO$;

B is one or more D or L amino acids, β-Ala or is a covalent bond;

C and D are the same or different and are Glx, Asx, Ala, β-Ala, Arg, Gly, Ile, Leu, Lys, Ser, Thr, Val, Met or His;

Q is a D or L amino acid and is Ser, Thr, Asp, His, Cys, Arg or Ala;

W is Pro or Δ3-dehydro-Pro;

X is Ala, Gly, Ile, Leu, Val, Met, Lys, Glx or Asx;

Y is one or more D or L amino acids, or is a covalent bond;

Z is $CO_2R''''$, $CONR'R''''$, $COR'$, $CH_2OH$,$CH_2NR'R''R''$ or H, or if d and e are 0 and Y is a covalent bond, Z is $OR''''$ or $NR'R''''$;

a, b, c, d and e are each independently 0 or 1, provided that c and e are not simultaneously 0;

M is Cha, Phe($4'R_a$) or $-NHCHR_1R_2-$;

wherein:

$R_1$ is independently $C_{1-5}Alk$, $(CH_2)_nSC_{1-5}Alk$, $(CH_2)_nOC_{1-5}Alk$, $(CH_2)_nC_{3-7}$cycloalkyl or $(CH_2)_mC_6H_4\text{-}R_a$;

$R_a$ is halogen, $OR'$, $NO_2$, $NH_2$ or H;

$R_2$ is $(CH_2)_n\text{-}$, $CHR_3(CH_2)_mCO\text{-}$, $CO(CH_2)_mCO\text{-}$, $CHR_3CHR_3'(CH_2)_mCO\text{-}$, $CHR_3CHR_3'CHR_1(CH_2)_mCO\text{-}$, $CHR_3CHR_1CHR'(CH_2)_mCO\text{-}$, $CHR_3CHR'CHR_1(CH_2)_mCO\text{-}$, $COCR_1CHR'(CH_2)_mCO\text{-}$, $COCF_2CR'R''(CH_2)_mCO\text{-}$, $COCF_2(CH_2)_mCO\text{-}$, $CH=CR'CHR''(CH_2)_mCO\text{-}$, $COCH=CR'(CH_2)_mCO\text{-}$, $CH_2S(O)_pCHR'(CH_2)_mCO\text{-}$, $CH_2NR'CHR''(CH_2)_mCO\text{-}$, $P=O(OR')(CH_2)_mCO\text{-}$,

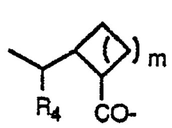 , 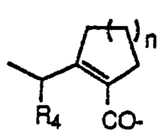 , 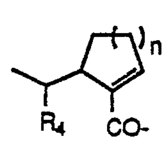 , 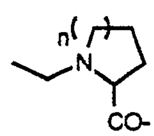 , 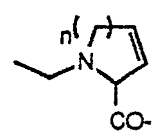 ,

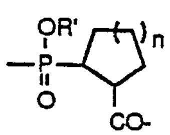 or 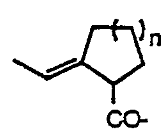 ;

$R_3$ and $R_3'$ are each independently OH, H or $NH_2$;

$R_4$ is OH, H, $NH_2$ or =0;

m is independently 0, 1, 2 or 3;

n is independently 1 or 2;

p is 0, 1 or 2;

$R'$ is H or $C_{1-5}Alk$;

$R''$ is H or $C_{1-18}Alk$;

$R''''$ is H, $C_{1-5}Alk$, $C_{3-6}$cycloalkyl, $(CH_2)_nC_6H_5$, $(CH_2)_nC_5H_4N$, $(CH_2)_nOH$, $(CH_2)_nNH_2$, or$(CH_2)_nNHC(NH)NH_2$; and pharmaceutically acceptable salts thereof.

2. A compound according to claim 1 in which B is one or two D or L amino acids chosen from the group consisting of Ala, β-Ala, Gly, Val, Ile, Leu, Met, His, Lys, Arg, Glx, Asx, Cys, Ser and Thr, or is a covalent bond, and Y is one to three D or L amino acids chosen from the group consisting of Ala, Gly, Ile, Leu, Met, Val, Arg, Lys, Thr, Ser, Cys, Glx and Asx, or is a covalent bond.

3. A compound according to claim 1 or 2 in which Q is a D or L amino acid chosen from the group consisting of Ser, Thr, Asp and His, and D is chosen from the group consisting of Glx, Asx, Ser, Ala, β-Ala,

Ile, Leu, Val and Met.

4. A compound according to any one of claims 1 to 3 in which d is 0 and M is $-NHCHR_1R_2$.

5. A compound according to any one of claims 1 to 4 in which $R_1$ is $CH_2C_6H_4-R_a$, $CH_2C_6H_{11}$ or $C_{1-5}Alk$, and $R_2$ is $CHR_3CHR_3'(CH_2)_mCO-$, $CHR_3CHR_3'CHR_1CO-$, $CHR_3CHR_1CHR'CO-$, $CHR_3(CH_2)_mCO-$, $CO(CH_2)_mCO-$,$COCR_1CHR'(CH_2)_mCO-$, $CHR_3CF_2(CH_2)_mCO-$, $-COCF_2(CH_2)_mCO-$, $-P=O(OR')(CH_2)_mCO-$,

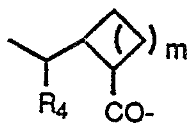 , 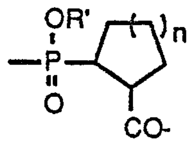 or 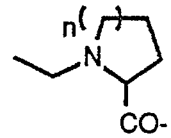 .

6. A compound according to any one of claims 1 to 5 in which Y is one amino acid chosen from the group consisting of Ala, Gly, Ile, Met, Asx, Arg and Val, or is a covalent bond and C and D are chosen from the group consisting of Gln, Asn and Ala.

7. A compound according to any one of claims 1 to 6 in which $R_2$ is $CH(OH)CH_2CH_2CO-$.

8. A compound according to any one of claims 1 to 7 in which B is a covalent bond and a is 0.

9. A compound according to claim 8 in which b is 0.

10. A compound according to claim 1 which is:
2-(acetyl-serylglutaminylasparaginyl)amino-3-phenylpropyl-prolylvalyl valinamide;
2-(serylglutaminylasparaginyl)amino-3-phenylpropylprolylvalyl valinamide;
4-(acetyl-serylglutaminylasparaginyl)amino-3-hydroxy-1-oxo-5-phenylpentylprolylvalyl valinamide;
4-(acetyl-serylglutaminylasparaginyl)amino-3-hydroxy-1-oxo-5-phenylpentylvalyl valinamide;
4-(seryl glutaminylasparaginyl)amino-3(S)-hydroxy-1-oxo-5-phenyl pentylvalyl valinamide;
5-(carbobenzyloxy-alanylalanyl)amino-6-phenyl-4-hydroxy(1-oxo)hexyl-valyl valinol;
carbobenzyloxy-alanyl-(3-hydroxy-5-amino-6-phenyl)hexanoyl-valyl-(4-aminomethyl)pyridine; or
5-(carbobenzyloxyalanylalanyl)amino-4-hydroxy-6-phenyl(1-oxo)hexyl-valine isobutylamide;
and pharmaceutically acceptable salts thereof.

11. A compound according to claim 1 which is:
2-((2-(tert-butoxycarbonyl-serylalanylalanyl)amino-1-oxo-3-phenylpropyl)cyclopentylcarbonylvalyl valine methyl ester;
2-(2-(serylalanylalanyl)amino-1-oxo-3-phenylpropyl)cyclopentylcarbonylvalylvaline methyl ester;
2-(2-(tert-butoxycarbonyl-serylalanylalanyl)amino-1-hydroxy-3-phenylpropyl)cyclopentylcarbonylvalyl valine methyl ester;
2-(-1-hydroxy-3-phenyl-2-(serylalanylalanyl)aminopropyl)cyclopentylcarbonylvalylvaline methyl ester;
2-((1-methoxy-1-(2-phenyl-1-(tert-butyloxycarbonylserylalanylalanyl)amino)ethyl)phosphinyl)-cyclopentylcarbonyl-valylvaline methyl ester;
2-((1-hydroxy-1-(2-phenyl-1-(serylalanylalanyl)amino) ethyl)phosphinyl)cyclopentylcarbonyl-valyl valine methyl ester;
5-(tert-butoxycarbonyl-serylalanylalanyl)amino-4-hydroxy-1-oxo-6-phenylhexyl-valyl valine methyl ester;
5-(tert-butoxycarbonyl-serylalanylalanyl)amino-4-hydroxy-1-oxo-6-(4-hydroxy-phenyl)hexyl-valyl valine methyl ester;
4-(tert-butoxycarbonyl-serylalanylalanyl)amino-2,2-difluoro-3-hydroxy-1-oxo-5-phenylpentyl-valyl valine methyl ester;
4-(serylalanylalanyl)amino-2,2-difluoro-3-hydroxy-1-oxo-5-phenylpentyl-valyl valine methyl ester;
4-(serylalanylalanyl)amino-2,2-difluoro-1,3-dioxo-5-phenylpentyl-valyl valine methyl ester;
5-(t-butyloxycarbonylserylalanylalanyl)amino-4-hydroxy-6-(4-hydroxy)phenyl-1-oxo-hexyl-valyl amide
5-((carbobenzyloxy-D-seryl)alanylalanyl)amino-4-hydroxy-1-oxo-6-phenylhexyl-valyl valine methyl ester;
5-((D-seryl)alanylalanyl)amino-4-hydroxy-1-oxo-6-phenylhexyl-valyl valine methyl ester;
5-(t-butyloxycarbonylserylalanylalanyl)amino-6-(4-hydroxy)phenyl-4-hydroxy-1-oxo-hexyl-valyl valine;
5-(serylalanylalanyl)amino-6-(4-hydroxy)phenyl-4-hydroxy-1-oxo-hexyl-valyl valine;
2-((1-hydroxy-2-(serylalanylalanyl)amino-3-cyclohexyl)propyl)cyclopentanecarbonyl-valyl valine methyl ester;
(4S,5S) 5-(carbobenzyloxyalanyl)amino-4-hydroxy-1-oxo-6-phenylhexyl-valyl valine methyl ester;
(4S,5S) 5-(alanyl)amino-4-hydroxy-1-oxo-6-phenylhexylvalyl valine methyl ester;
(4S,5S) 5-(tert-butyloxycarbonylalanyl)amino-4-hydroxy-1-oxo-6-(4-hydroxyphenyl)hexyl-valyl valine methyl ester;
(4S,5S)-5-alanylamino-4-hydroxy-1-oxo-6-(4-hydroxyphenyl)-hexyl-valyl valine methyl ester;

(4S,5S) 5-(alanylalanyl)amino-4-hydroxy-1-oxo-6-(4-hydroxyphenyl)hexyl-valyl valine methyl ester;
(4S,5S) 5-[(carbobenzyloxy-β-alanyl)alanyl]amino-4-hydroxy-1-oxo-6-(4-hydroxyphenyl)hexyl-valyl valine methyl ester;
(4S,5S) 5-[(β-alanyl)alanyl]amino-4-hydroxy-1-oxo-6-(4-hydroxyphenyl)hexyl-valyl valine methyl ester;
(4S,5S) 5-(alanylalanyl)amino-4-hydroxy-1-oxo-6-cyclohexyl-hexyl-valyl valine methyl ester;
(4S,5S) 5-(carbobenzyloxyalanylalanyl)amino-4-hydroxy-1-oxo-6-phenylhexyl-valyl valine methyl ester;
(4S,5S) 5-(alanylalanyl)amino-4-hydroxy-1-oxo-6-phenylhexyl-valyl valine methyl ester;
(4RS,5S) 5-amino-6-(4-hydroxy)phenyl-4-hydroxy-1-oxo-hexyl-valyl valine benzyl ester;
(1R,2R)-2-(((1S,2S)-1-hydroxy-2-amino-3-cyclohexyl)propyl)cyclopentanecarbonyl-valyl valine methyl ester;
(4S,5S) 5-amino-4-hydroxy-1-oxo-6-phenylhexyl-valyl valine methyl ester;
(4S,5S)-5-(tert-butyloxycarbonyl)amino-4-hydroxy-1-oxo-6-phenylhexyl-valyl valine methyl ester;
(4S,5S) 5-(methoxycarbonyl-alanylalanyl)amino-6-phenyl-4-hydroxy-(1-oxo)hexyl-valyl valine; or
(4RS,5S) 5-((tert-butyloxycarbonyl)isoleucyl)amino-7-methyl-4-hydroxy-1-oxo-octyl-leucyl-aspartic acid methyl ester; or the carboxamides thereof;
and pharmaceutically acceptable salts thereof.

12. A compound according to any one of claims 4-11 for use in a medicament.

13. A pharmaceutical composition which comprises a compound according to any one of claims 4-11 and a pharmaceutically acceptable carrier.

14. A pharmaceutical composition which comprises a compound according to any one of claims 4-11 , azidothymidine and a pharmaceutically acceptable carrier.

15. An intermediate compound of the formula:

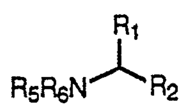

wherein:
$R_1$ is independently $C_{1-5}Alk$, $-(CH_2)_nSC_{1-5}Alk$, $-(CH_2)_nOC_{1-5}Alk$, $-(CH_2)_nC_{3-7}$cycloalkyl or $-(CH_2)_mC_6H_4-R_a$;
$R_a$ is halogen, $NO_2$, OH, $OC_{1-5}Alk$, $NH_2$, or H;
$R_2$ is $(CH_2)_n-X$, $(CH_2)_nCO-X$, $CHR_3CHR_1CHR'(CH_2)_mCO-$, $COCR_1CHR'(CH_2)_mCO-$, $CHR_3CF_2(CH_2)_mCO-X$, $COCF_2CR'R''(CH_2)_mCO-X$, $CHR_3(CH_2)_mCO-X$, $CO(CH_2)_mCO-X$, $COCR_1CHR'(CH2)_mCO-X$,

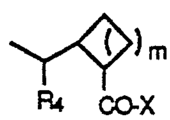 , 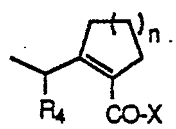 , 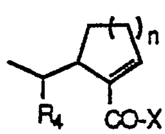 , 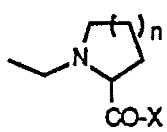 , 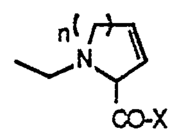 ,

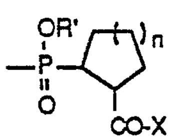 or 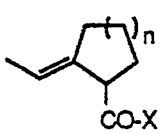 ;

$R_3$ is H, $NH_2$ or OH;
$R_4$ is H, $NH_2$, OH, or =O;
$R_5$ is H;
$R_6$ is $CH_3CO$, $C_{1-5}Alk$, Dns, Cbz or Boc, or taken together $R_5$ and $R_6$ are phthalimido;
X is H, halogen, OH, $OC_{1-5}Alk$, $NHR'R''$ or $OCOC_{1-5}Alk$;
R' and R'' are H or $C_{1-5}Alk$;
m is independently 0, 1, 2 or 3; and
n is independently 1 or 2;
provided that $R_2$ is not $CHR_3CH_2CO-X$, $COCH_2CO-X$, $COCF_2CO-X$ or $CHR_3CF_2CO-X$ when $R_1$ is phenylmethylene, isobutyl or cyclohexylmethylene, and $R_2$ is not $CH(OH)CH_2CH_2CO-X$ or $COCH_2CH_2CO-X$, when $R_1$ is isobutyl.

16. A compound according to claim 15 which is:

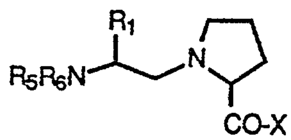

wherein:
$R_1$ is $C_{1-5}Alk$, $-(CH_2)_nSC_{1-5}Alk$, $-(CH_2)_nOC_{1-5}Alk$, $(CH_2)_nC_{3-7}$cycloalkyl or $-(CH_2)_mC_6H_4-R_a$;
$R_a$ is halogen, $NO_2$, OH, $OC_{1-5}Alk$, $NH_2$ or H;
m is 0, 1, 2 or 3;
n is 1 or 2;
$R_5$ is H;
$R_6$ is $CH_3CO$, $C_{1-5}Alk$, Dns, Cbz or Boc, or taken together $R_5$ and $R_6$ are phthalimido;
X is H, halogen, OH, $OC_{1-5}Alk$, $NHR'R''$ or $OCOC_{1-5}Alk$; and
$R'$ and $R''$ are H or $C_{1-5}Alk$.

17. A compound according to claim 15 which is:

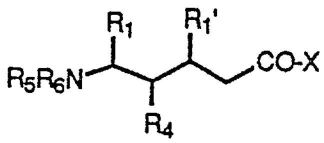

wherein:
R is $C_{1-5}Alk$, $-(CH_2)_nSC_{1-5}Alk$, $-(CH_2)_nOC_{1-5}Alk$, $(CH_2)_nC_{3-7}$cycloalkyl or $-(CH_2)_mC_6H_4-R_a$;
$R_1'$ is H, $C_{1-5}Alk$, $-(CH_2)_nSC_{1-5}Alk$, $-(CH_2)_nOC_{1-5}Alk$, $(CH_2)_nC_{3-7}$cycloalkyl or $-(CH_2)_mC_6H_4-R_a$;
$R_4$ is OH, $NH_2$, H or =O;
$R_a$ is halogen, $NO_2$, OH, $OC_{1-5}Alk$, $NH_2$ or H;
m is independently 0, 1, 2 or 3;
n is independently 1 or 2;
$R_5$ is H;
$R_6$ is $CH_3CO$, $C_{1-5}Alk$, Dns, Cbz or Boc, or taken together $R_5$ and $R_6$ are phthalimido;
X is H, halogen, OH, $OC_{1-5}Alk$, $NHR'R''$ or $OCOC_{1-5}Alk$; and
$R'$ and $R''$ are H or $C_{1-5}Alk$;
provided that $R_1$ is not isobutyl when $R_1'$ is H.

18. A compound according to claim 17 in which $R_1'$ is H, $R_1$ is $CH_2C_6H_4-R_a$ and $R_4$ is OH or =O.

19. A compound according to claim 15 which is:

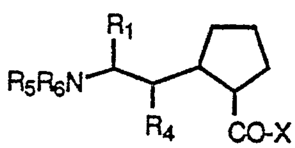

wherein:
$R_1$ is $C_{1-5}Alk$, $-(CH_2)_nSC_{1-5}Alk$, $-(CH_2)_nOC_{1-5}Alk$, $(CH_2)_nC_{3-7}$cycloalkyl or $-(CH_2)_mC_6H_4-R_a$;
$R_a$ is halogen, $NO_2$, OH, $OC_{1-5}Alk$, $NH_2$ or H;
$R_4$ is H, $NH_2$, OH, or =O;
m is 0, 1, 2 or 3;
n is 1 or 2;
$R_5$ is H;
$R_6$ is $CH_3CO$, $C_{1-5}Alk$, Dns, Cbz or Boc, or taken together $R_5$ and $R_6$ are phthalimido;
X is H, halogen, OH, $OC_{1-5}Alk$, $NHR'R''$ or $OCOC_{1-5}Alk$; and
$R'$ and $R''$ are H or $C_{1-5}Alk$.

20. A compound according to claim 15 which is:

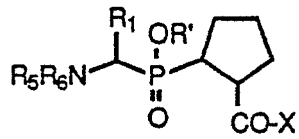

wherein:

$R_1$ is $C_{1-5}$Alk, $-(CH_2)_nSC_{1-5}$Alk, $-(CH_2)_nOC_{1-5}$Alk, $(CH_2)_nC_{3-7}$cycloalkyl or $-(CH_2)_mC_6H_4-R_a$;
$R_a$ is halogen, $NO_2$, OH, $OC_{1-5}$Alk, $NH_2$ or H;
m is 0, 1, 2 or 3;
n is 1 or 2;
$R_5$ is H;
$R_6$ is $CH_3CO$, $C_{1-5}$Alk, Dns, Cbz or Boc, or taken together $R_5$ and $R_6$ are phthalimido;
X is H, halogen, OH, $OC_{1-5}$Alk, $NHR'R''$ or $OCOC_{1-5}$Alk; and
$R'$ and $R''$ are H or $C_{1-5}$Alk.

Claims for the following Contracting State: GR

1. A process for preparing a compound of the formula:

A-B-$(Q)_a$-$(C)_b$-$(D)_c$-M-$(W)_d$-$(X)_e$-Y-Z (I)

and pharmaceutically acceptable salts thereof,

wherein:

A is BocNH, CbzNH, H, $R'R''N$, $R''CONR'$ or DnsNH, or if a, b and c are 0 and Y is a covalent bond, then A is H, Boc, Cbz, $R''$ or $R''CO$;
B is one or more D or L amino acids, β-Ala or is a covalent bond;
C and D are the same or different and are Glx, Asx, Ala, β-Ala, Arg, Gly, Ile, Leu, Lys, Ser, Thr, Val, Met or His;
Q is a D or L amino acid and is Ser, Thr, Asp, His, Cys, Arg or Ala;
W is Pro or Δ3-dehydro-Pro;
X is Ala, Gly, Ile, Leu, Val, Met, Lys, Glx or Asx;
Y is one or more D or L amino acids, or is a covalent bond;
Z is $CO_2R''''$, $CONR'R''''$, $COR'$, $CH_2OH$, $CH_2NR'R''''$ or H, or if d and e are 0 and Y is a covalent bond, Z is $OR''''$ or $NR'R''''$;
a, b, c, d and e are each independently 0 or 1, provided that c and e are not simultaneously 0;
M is Cha, Phe($4'R_a$) or $-NHCHR_1R_2-$;

wherein:

$R_1$ is independently $C_{1-5}$Alk, $(CH_2)_nSC_{1-5}$Alk, $(CH_2)_nOC_{1-5}$Alk, $(CH_2)_nC_{3-7}$cycloalkyl or $(CH_2)_mC_6H_4-R_a$;
$R_a$ is halogen, $OR'$, $NO_2$, $NH_2$ or H;
$R_2$ is $(CH_2)_n-$, $CHR_3(CH_2)_mCO-$, $CO(CH_2)_mCO-$, $CHR_3CHR_3'(CH_2)_mCO-$, $CHR_3CHR_3'CHR_1(CH_2)_mCO-$, $CHR_3CHR_1CHR'(CH_2)_mC0-$, $CHR_3CHR'CHR_1(CH_2)_mCO-$, $COCR_1CHR'(CH_2)_mCO-$, $COCF_2CR'R''(CH_2)_mCO-$, $COCF_2(CH_2)_mCO-$, $CH=CR'CHR''(CH_2)_mCO-$, $COCH=CR'(CH_2)_mCO-$, $CH_2S(O)_pCHR'(CH_2)_mCO-$, $CH_2NR'CHR''(CH_2)_mCO-$, $P=O(OR')(CH_2)_mCO-$,

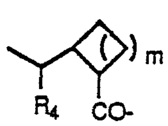 , 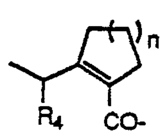 , 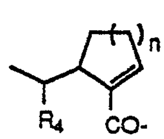 , 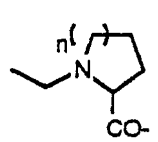 , 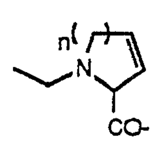 ,

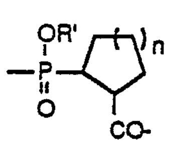 or 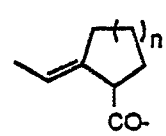 ;

$R_3$ and $R_3'$ are each independently OH, H or $NH_2$;
$R_4$ is OH, H, $NH_2$ or =0;

m is independently 0, 1, 2 or 3;
n is independently 1 or 2;
p is 0, 1 or 2;
$R'$ is H or $C_{1-5}$Alk;
R" is H or $C_{1-18}$Alk;
$R''''$ is H, $C_{1-5}$Alk, $C_{3-6}$cycloalkyl, $(CH_2)_nC_6H_5$, $(CH_2)_nC_5H_4N$, $(CH_2)_nOH$, $(CH_2)_nNH_2$, or$(CH_2)_nNHC(NH)NH_2$; which comprises, (a) coupling the appropriate amino acid residues, (b) optionally removing any protective groups, (c) optionally modifying the amino or carboxy terminus of the peptide and (d) optionally forming a pharmaceutically acceptable salt thereof.

2. A process according to claim 1 in which B is one or two D or L amino acids chosen from the group consisting of Ala, β-Ala, Gly, Val, Ile, Leu, Met, His, Lys, Arg, Glx, Asx, Cys, Ser and Thr, or is a covalent bond, and Y is one to three D or L amino acids chosen from the group consisting of Ala, Gly, Ile, Leu, Met, Val, Arg, Lys, Thr, Ser, Cys, Glx and Asx, or is a covalent bond.

3. A process for preparing a compound according to claim 1 or 2 in which Q is a D or L amino acid chosen from the group consisting of Ser, Thr, Asp and His, and D is chosen from the group consisting of Glx, Asx, Ser, Ala, β-Ala, Ile, Leu, Val and Met.

4. A process for preparing a compound according to any one of claims 1 to 3 in which d is 0 and M is $-NHCHR_1R_2$.

5. A process according to any one of claims 1 to 4 in which $R_1$ is $CH_2C_6H_4$-$R_a$, $CH_2C_6H_{11}$ or $C_{1-5}$Alk, and $R_2$ is $CHR_3CHR_3'(CH_2)_mCO$-, $CHR_3CHR_3'CHR_1CO$-, $CHR_3CHR_1CHR'CO$-, $CHR_3(CH_2)_mCO$-, $CO(CH_2)_mCO$-, $COCR_1CHR'(CH_2)_mCO$-, $CHR_3CF_2(CH_2)_mCO$-, $-COCF_2(CH_2)_mCO$-, $-P=O(OR')(CH_2)_mCO$-,

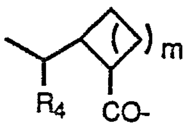 , 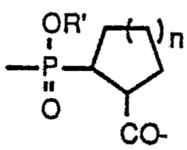 or 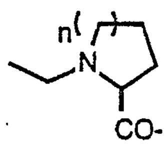 .

6. A process for preparing a compound according to any one of claims 1 to 5 in which Y is one amino acid chosen from the group consisting of Ala, Gly, Ile, Met, Asx, Arg and Val, or is a covalent bond and C and D are chosen from the group consisting of Gln, Asn and Ala.

7. A process for preparing a compound according to any one of claims 1 to 6 in which $R_2$ is $CH(OH)$-$CH_2CH_2CO$-.

8. A process for preparing a compound according to any one of claims 1 to 7 in which B is a covalent bond and a is 0.

9. A process for preparing a compound according to claim 8 in which b is 0.

10. A process for preparing a compound according to claim 9 in which either c or e are 0.

11. A process according to claim 1 for preparing a compound which is:
2-(acetyl-serylglutaminylasparaginyl)amino-3-phenylpropyl-prolylvalyl valinamide;
2-(serylglutaminylasparaginyl)amino-3-phenylpropylprolylvalyl valinamide;
4-(acetyl-serylglutaminylasparaginyl)amino-3-hydroxy-1-oxo-5-phenylpentylprolylvalyl valinamide;
4-(acetyl-serylglutaminylasparaginyl)amino-3-hydroxy-1-oxo-5-phenylpentylvalyl valinamide;
4-(seryl glutaminylasparaginyl)amino-3(S)-hydroxy-1-oxo-5-phenyl pentylvalyl valinamide;
5-(carbobenzyloxy-alanylalanyl)amino-6-phenyl-4-hydroxy(1-oxo)hexyl-valyl valinol;
carbobenzyloxy-alanyl-(3-hydroxy-5-amino-6-phenyl)hexanoyl-valyl-(4-aminomethyl)pyridine; or
5-(carbobenzyloxyalanylalanyl)amino-4-hydroxy-6-phenyl(1-oxo)hexyl-valine isobutylamide;
and pharmaceutically acceptable salts thereof.

12. A process according to claim 1 for preparing a compound which is:
2-((2-(tert-butoxycarbonyl-serylalanylalanyl)amino-1-oxo-3-phenylpropyl)cyclopentylcarbonylvalyl valine methyl ester;
2-(2-(serylalanylalanyl)amino-1-oxo-3-phenylpropyl)cyclopentylcarbonylvalylvaline methyl ester;
2-(2-(tert-butoxycarbonyl-serylalanylalanyl)amino-1-hydroxy-3-phenylpropyl)cyclopentylcarbonylvalyl valine methyl ester;
2-(-1-hydroxy-3-phenyl-2-(serylalanylalanyl)aminopropyl)cyclopentylcarbonylvalylvaline methyl ester;
2-((1-methoxy-1-(2-phenyl-1-(tert-butyloxycarbonylserylalanylalanyl)amino)ethyl)phosphinyl)-cyclopentylcarbonyl-valylvaline methyl ester;
2-((1-hydroxy-1-(2-phenyl-1-(serylalanylalanyl)amino) ethyl)phosphinyl)cyclopentylcarbonyl-valyl valine

methyl ester;
5-(tert-butoxycarbonyl-serylalanylalanyl)amino-4-hydroxy-1-oxo-6-phenylhexyl-valyl valine methyl ester; 5-(tert-butoxycarbonyl-serylalanylalanyl)amino-4-hydroxy-1-oxo-6-(4-hydroxy-phenyl)hexyl-valyl valine methyl ester;
4-(tert-butoxycarbonyl-serylalanylalanyl)amino-2,2-difluoro-3-hydroxy-1-oxo-5-phenylpentyl-valyl valine methyl ester;
4-(serylalanylalanyl)amino-2,2-difluoro-3-hydroxy-1-oxo-5-phenylpentyl-valyl valine methyl ester;
4-(serylalanylalanyl)amino-2,2-difluoro-1,3-dioxo-5-phenylpentyl-valyl valine methyl ester;
5-(t-butyloxycarbonylserylalanylalanyl)amino-4-hydroxy-6-(4-hydroxy)phenyl-1-oxo-hexyl-valyl amide
5-((carbobenzyloxy-D-seryl)alanylalanyl)amino-4-hydroxy-1-oxo-6-phenylhexyl-valyl valine methyl ester;
5-((D-seryl)alanylalanyl)amino-4-hydroxy-1-oxo-6-phenylhexyl-valyl valine methyl ester;
5-(t-butyloxycarbonylserylalanylalanyl)amino-6-(4-hydroxy)phenyl-4-hydroxy-1-oxo-hexyl-valyl valine;
5-(serylalanylalanyl)amino-6-(4-hydroxy)phenyl-4-hydroxy-1-oxo-hexyl-valyl valine;
2-((1-hydroxy-2-(serylalanylalanyl)amino-3-cyclohexyl)propyl)cyclopentanecarbonyl-valyl valine methyl ester;
(4S,5S) 5-(carbobenzyloxyalanyl)amino-4-hydroxy-1-oxo-6-phenylhexyl-valyl valine methyl ester;
(4S,5S) 5-(alanyl)amino-4-hydroxy-1-oxo-6-phenylhexyl-valyl valine methyl ester;
(4S,5S) 5-(tert-butyloxycarbonylalanyl)amino-4-hydroxy-1-oxo-6-(4-hydroxyphenyl)hexyl-valyl valine methyl ester;
(4S,5S)-5-alanylamino-4-hydroxy-1-oxo-6-(4-hydroxyphenyl)-hexyl-valyl valine methyl ester;
(4S,5S) 5-(alanylalanyl)amino-4-hydroxy-1-oxo-6-(4-hydroxyphenyl)hexyl-valyl valine methyl ester;
(4S,5S) 5-[(carbobenzyloxy-β-alanyl)alanylamino-4-hydroxy-1-oxo-6-(4-hydroxyphenyl)hexyl-valyl valine methyl ester;
(4S,5S) 5-[(β-alanyl)alanyl]amino-4-hydroxy-1-oxo-6-(4-hydroxyphenyl)hexyl-valyl valine methyl ester;
(4S,5S) 5-(alanylalanyl)amino-4-hydroxy-1-oxo-6-cyclohexyl-hexyl-valyl valine methyl ester;
(4S,5S) 5-(carbobenzyloxyalanylalanyl)amino-4-hydroxy-1-oxo-6-phenylhexyl-valyl valine methyl ester;
(4S,5S) 5-(alanylalanyl)amino-4-hydroxy-1-oxo-6-phenylhexyl-valyl valine methyl ester;
(4RS,5S) 5-amino-6-(4-hydroxy)phenyl-4-hydroxy-1-oxo-hexyl-valyl valine benzyl ester;
(1R,2R)-2-(((1S,2S)-1-hydroxy-2-amino-3-cyclohexyl)propyl)cyclopentanecarbonyl-valyl valine methyl ester;
(4S,5S) 5-amino-4-hydroxy-1-oxo-6-phenylhexyl-valyl valine methyl ester;
(4S,5S)-5-(tert-butyloxycarbonyl)amino-4-hydroxy-1-oxo-6-phenylhexyl-valyl valine methyl ester;
(4S,5S) 5-(methoxycarbonyl-alanylalanyl)amino-6-phenyl-4-hydroxy-(1-oxo)hexyl-valyl valine; or
(4RS,5S) 5-((tert-butyloxycarbonyl)isoleucyl)amino-7-methyl-4-hydroxy-1-oxo-octyl-leucyl-aspartic acid methyl ester; or the carboxamides thereof;
and pharmaceutically acceptable salts thereof.

13. The use of a compound according to any one of claims 4-11 in the manufacture of a medicament for the treatment of viral disease.

14. A process for preparing a pharmaceutical composition which comprises bringing into association a compound according to any one of claims 4-11 and a pharmaceutically acceptable carrier.

15. A process for preparing a pharmaceutical composition which comprises bringing into association a compound according to any one of claims 4-11, azidothymidine and a pharmaceutically acceptable carrier.

16. A compound of the formula:

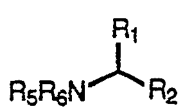

wherein:
R is independently $C_{1-5}Alk$, $-(CH_2)_nSC_{1-5}Alk$, $-(CH_2)_nOC_{1-5}Alk$, $-(CH_2)_nC_{3-7}$cycloalkyl or $-(CH_2)_mC_6H_4-R_a$;
$R_a$ is halogen, $NO_2$, OH, $OC_{1-5}Alk$, $NH_2$, or H;
$R_2$ is $(CH_2)_n$-X, $(CH_2)_nCO$-X, $CHR_3CHR_1CHR'(CH_2)_mCO$-, $COCR_1CHR'(CH_2)_mCO$-, $CHR_3CF_2(CH_2)_mCO$-X, $COCF_2CR'R''(CH_2)_mCO$-X, $CHR_3(CH_2)_mCO$-X, $CO(CH_2)_mCO$-X, $COCR_1CHR'(CH_2)_mCO$-X,

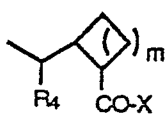 , 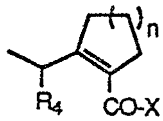 , 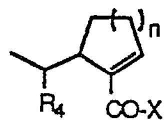 , 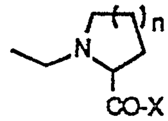 , 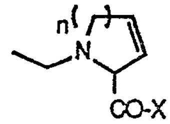 ,

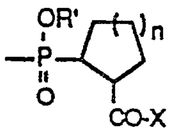 or 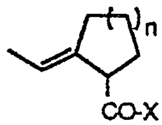 ;

$R_3$ is H, $NH_2$ or OH;
$R_4$ is H, $NH_2$, OH, or =O;
$R_5$ is H;
$R_6$ is $CH_3CO$, $C_{1-5}Alk$, Dns, Cbz or Boc, or taken together $R_5$ and $R_6$ are phthalimido;
X is H, halogen, OH, $OC_{1-5}Alk$, $NHR'R''$ or $OCOC_{1-5}Alk$;
$R'$ and $R''$ H or $C_{1-5}Alk$;
m is independently 0, 1, 2 or 3; and
n is independently 1 or 2;
provided that $R_2$ is not $CHR_3CH_2CO$-X, $COCH_2CO$-X, $COCF_2CO$-X or $CHR_3CF_2CO$-X when $R_1$ is phenylmethylene, isobutyl or cyclohexylmethylene, and $R_2$ is not $CH(OH)CH_2CH_2CO$-X or $COCH_2CH_2CO$-X, when $R_1$ is isobutyl.

17. A compound according to claim 16 which is:

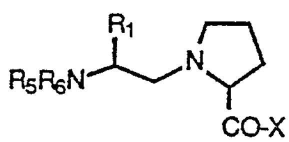

wherein:
$R_1$ is $C_{1-5}Alk$, $-(CH_2)_nSC_{1-5}Alk$, $-(CH_2)_nOC_{1-5}Alk$, $-(CH_2)_nC_{3-7}$cycloalkyl or $-(CH_2)_mC_6H_4-R_a$;
$R_a$ is halogen, $NO_2$, OH, $OC_{1-5}Alk$, $NH_2$ or H;
m is 0, 1, 2 or 3;
n is 1 or 2;
$R_5$ is H;
$R_6$ is $CH_3CO$, $C_{1-5}Alk$, Dns, Cbz or Boc, or taken together $R_5$ and $R_6$ are phthalimido;
X is H, halogen, OH, $OC_{1-5}Alk$, $NHR'R''$ or $OCOC_{1-5}Alk$;
$R'$ and $R''$ are H or $C_{1-5}Alk$.

18. A compound according to claim 16 which is:

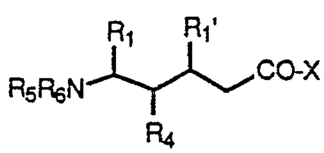

wherein:
$R_1$ is $C_{1-5}Alk$, $-(CH_2)_nSC_{1-5}Alk$, $-(CH_2)_nOC_{1-5}Alk$, $-(CH_2)_nC_{3-7}$cycloalkyl or $-(CH_2)_mC_6H_4-R_a$; $R_1'$ is H, $C_{1-5}Alk$, $-(CH_2)_nSC_{1-5}Alk$, $-(CH_2)_nOC_{1-5}Alk$, $-(CH_2)_nC_{3-7}$cycloalkyl or $-(CH_2)_mC_6H_4-R_a$;
$R_4$ is OH, $NH_2$, H or =O;
$R_a$ is halogen, $NO_2$, OH, $OC_{1-5}Alk$, $NH_2$ or H;
m is independently 0, 1, 2 or 3;
n is independently 1 or 2;
$R_5$ is H;
$R_6$ is $CH_3CO$, $C_{1-5}Alk$, Dns, Cbz or Boc, or taken together $R_5$ and $R_6$ are phthalimido;
X is H, halogen, OH, $OC_{1-5}Alk$, $NHR'R''$ or $OCOC_{1-5}Alk$;
$R'$ and $R''$ are H or $C_{1-5}Alk$;

provided that $R_1$ is not isobutyl when $R_1'$ is H.

19. A compound according to claim 18 in which $R_1'$ is H, $R_1$.is $CH_2C_6H_4$-$R_a$ and $R_4$ is OH or =O.

20. A compound according to claim 16 which is:

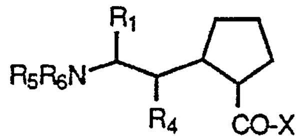

wherein:
$R_1$ is $C_{1-5}$Alk, -$(CH_2)_n$S$C_{1-5}$Alk, -$(CH_2)_n$O$C_{1-5}$Alk, $(CH_2)_n$$C_{3-7}$cycloalkyl or -$(CH_2)_m$$C_6H_4$-$R_a$;
$R_a$ is halogen, $NO_2$, OH, O$C_{1-5}$Alk, $NH_2$ or H;
$R_4$ is H, $NH_2$, OH, or =O;
m is 0, 1, 2 or 3;
n is 1 or 2;
$R_5$ is H;
$R_6$ is $CH_3CO$, $C_{1-5}$Alk, Dns, Cbz or Boc, or taken together $R_5$ and $R_6$ are phthalimido;
X is H, halogen, OH, O$C_{1-5}$Alk, NHR$'$R$''$ or OCO$C_{1-5}$Alk;
R$'$ and R$''$ are H or $C_{1-5}$Alk.

21. A compound according to claim 16 which is:

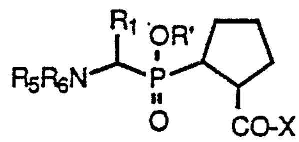

wherein:
$R_1$ is $C_{1-5}$Alk, -$(CH_2)_n$S$C_{1-5}$Alk, -$(CH_2)_n$O$C_{1-5}$Alk, -$(CH_2)_n$$C_{3-7}$cycloalkyl or -$(CH_2)_m$$C_6H_4$-$R_a$;
$R_a$ is halogen, $NO_2$, OH, O$C_{1-5}$Alk, $NH_2$ or H;
m is 0, 1, 2 or 3;
n is 1 or 2;
$R_5$ is H;
$R_6$ is $CH_3CO$, $C_{1-5}$Alk, Dns, Cbz or Boc, or taken together $R_5$ and $R_6$ are phthalimido;
X is H, halogen, OH, O$C_{1-5}$Alk, NHR$'$R$''$ or OCO$C_{1-5}$Alk; and
R$'$ and R$''$ are H or $C_{1-5}$Alk.

Claims for the following Contracting State: ES

1. A process for preparing a compound of the formula:
A-B-$(Q)_a$-$(C)_b$-$(D)_c$-M-$(W)_d$-$(X)_e$-Y-Z (I)
and pharmaceutically acceptable salts thereof,
wherein:
A is BocNH, CbzNH, H, R$'$R$''$N, R$''$CONR$'$ or DnsNH, or if a, b and c are 0 and Y is a covalent bond, then A is H, Boc, Cbz, R$''$ or R$''$CO;
B is one or more D or L amino acids, β-Ala or is a covalent bond;
C and D are the same or different and are Glx, Asx, Ala, β-Ala, Arg, Gly, Ile, Leu, Lys, Ser, Thr, Val, Met or His;
Q is a D or L amino acid and is Ser, Thr, Asp, His, Cys, Arg or Ala;
W is Pro or Δ3-dehydro-Pro;
X is Ala, Gly, Ile, Leu, Val, Met, Lys, Glx or Asx;
Y is one or more D or L amino acids, or is a covalent bond;
Z is $CO_2$R$''''$, CONR$'$R$''''$, COR$'$, $CH_2OH$, $CH_2$NR$'$R$''''$ or H, or if d and e are 0 and Y is a covalent bond, Z is OR$''''$ or NR$'$R$''''$;
a, b, c, d and e are each independently 0 or 10, provided that c and e are not simultaneously 0;
M is Cha, Phe(4$'$$R_a$) or -NHCH$R_1$$R_2$-;
wherein:
$R_1$ is independently $C_{1-5}$Alk, $(CH_2)_n$S$C_{1-5}$Alk, $(CH_2)_n$O$C_{1-5}$Alk, $(CH_2)_n$$C_{3-7}$cycloalkyl or $(CH_2)_m$$C_6H_4$-$R_a$;

$R_a$ is halogen, $OR'$, $NO_2$, $NH_2$ or H;
$R_2$ is $(CH_2)_n$-, $CHR_3(CH_2)_mCO$-, $CO(CH_2)_mCO$-, $CHR_3CHR_3'(CH_2)_mCO$-, $CHR_3CHR_3'CHR_1(CH_2)_mCO$-, $CHR_3CHR_1CHR'(CH_2)_mCO$-, $CHR_3CHR'CHR_1(CH_2)_mCO$-, $COCR_1CHR'(CH_2)_mCO$-, $COCF_2CR'R''(CH_2)_mCO$-, $COCF_2(CH_2)_mCO$-, $CH=CR'CHR''(CH_2)_mCO$-, $COCH=CR'(CH_2)_mCO$-, $CH_2S(O)_pCHR'(CH_2)_mCO$-, $CH_2NR'CHR''(CH_2)_mCO$-, $P=O(OR')(CH_2)_mCO$-,

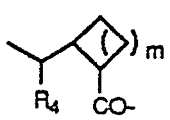 , 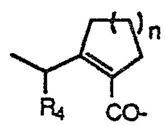 , 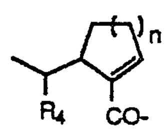 , 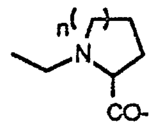 , 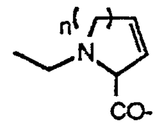 ,

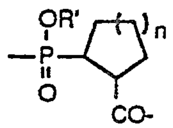 or 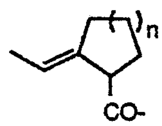 ;

$R_3$ and $R_3'$ are each independently OH, H or $NH_2$; $R_4$ is OH, H, $NH_2$ or =0;
m is independently 0, 1, 2 or 3;
n is independently 1 or 2;
p is 0, 1 or 2;
$R'$ is H or $C_{1-5}$Alk;
$R''$ is H or $C_{1-18}$Alk;
$R''''$ is H, $C_{1-5}$Alk, $C_{3-6}$cycloalkyl, $(CH_2)_nC_6H_5$, $(CH_2)_nC_5H_4N$, $(CH_2)_nOH$, $(CH_2)_nNH_2$, or$(CH_2)_nNHC(NH)NH_2$;
which comprises, (a) coupling the appropriate amino acid residues, (b) optionally removing any protective groups, (c) optionally modifying the amino or carboxy terminus of the peptide and (d) optionally forming a pharmaceutically acceptable salt thereof.

2. A process according to claim 1 in which B is one or two D or L amino acids chosen from the group consisting of Ala, β-Ala, Gly, Val, Ile, Leu, Met, His, Lys, Arg, Glx, Asx, Cys, Ser and Thr, or is a covalent bond, and Y is one to three D or L amino acids chosen from the group consisting of Ala, Gly, Ile, Leu, Met, Val, Arg, Lys, Thr, Ser, Cys, Glx and Asx, or is a covalent bond.

3. A process for preparing a compound according to claim 1 or 2 in which Q is a D or L amino acid chosen from the group consisting of Ser, Thr, Asp and His, and D is chosen from the group consisting of Glx, Asx, Ser, Ala, β-Ala, Ile, Leu, Val and Met.

4. A process for preparing a compound according to any one of claims 1 to 3 in which d is 0 and M is $-NHCHR_1R_2$.

5. A process according to any one of claims 1 to 4 in which $R_1$ is $CH_2C_6H_4$-$R_a$, $CH_2C_6H_{11}$ or $C_{1-5}$Alk, and $R_2$ is $CHR_3CHR_3'(CH_2)_mCO$-, $CHR_3CHR_3'CHR_1CO$-, $CHR_3CHR_1CHR'CO$-, $CHR_3(CH_2)_mCO$-, $CO(CH_2)_mCO$-, $COCR_1CHR'(CH_2)_mCO$-, $CHR_3CF_2(CH_2)_mCO$-, $-COCF_2(CH_2)_mCO$-, $-P=O(OR')(CH_2)_mCO$-,

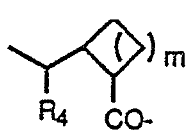 , 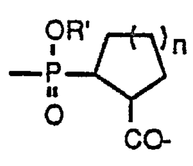 or 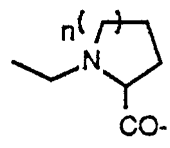 .

6. A process for preparing a compound according to any one of claims 1 to 5 in which Y is one amino acid chosen from the group consisting of Ala, Gly, Ile, Met, Asx, Arg and Val, or is a covalent bond and C and D are chosen from the group consisting of Gln, Asn and Ala.

7. A process for preparing a compound according to any one of claims 1 to 6 in which $R_2$ is $CH(OH)$-$CH_2CH_2CO$-.

8. A process for preparing a compound according to any one of claims 1 to 7 in which B is a covalent bond and a is 0.

9. A process for preparing a compound according to claim 8 in which b is 0.

10. A process for preparing a compound according to claim 9 in which either c or e are 0.

11. A process according to claim 1 for preparing a compound which is:
2-(acetyl-serylglutaminylasparaginyl)amino-3-phenylpropyl-prolylvalyl valinamide;
2-(serylglutaminylasparaginyl)amino-3-phenylpropylprolylvalyl valinamide;

4-(acetyl-serylglutaminylasparaginyl)amino-3-hydroxy-1-oxo-5-phenylpentylprolylvalyl valinamide;
4-(acetyl-serylglutaminylasparaginyl)amino-3-hydroxy-1-oxo-5-phenylpentylvalyl valinamide;
4-(seryl glutaminylasparaginyl)amino-3(S)-hydroxy-1-oxo-5-phenyl pentylvalyl valinamide;
5-(carbobenzyloxy-alanylalanyl)amino-6-phenyl-4-hydroxy(1-oxo)hexyl-valyl valinol;
carbobenzyloxy-alanyl-(3-hydroxy-5-amino-6-phenyl)hexanoyl-valyl-(4-aminomethyl)pyridine; or
5-(carbobenzyloxyalanylalanyl)amino-4-hydroxy-6-phenyl(1-oxo)hexyl-valine isobutylamide;
and pharmaceutically acceptable salts thereof.

12. A process according to claim 1 for preparing a compound which is:
2-((2-(tert-butoxycarbonyl-serylalanylalanyl)amino-1-oxo-3-phenylpropyl)cyclopentylcarbonylvalyl valine methyl ester;
2-(2-(serylalanylalanyl)amino-1-oxo-3-phenylpropyl)cyclopentylcarbonylvalylvaline methyl ester;
2-(2-(tert-butoxycarbonyl-serylalanylalanyl)amino-1-hydroxy-3-phenylpropyl)cyclopentylcarbonylvalyl valine methyl ester;
2-(-1-hydroxy-3-phenyl-2-(serylalanylalanyl)aminopropyl)cyclopentylcarbonylvalylvaline methyl ester;
2-((1-methoxy-1-(2-phenyl-1-(tert-butyloxycarbonylserylalanylalanyl)amino)ethyl)phosphinyl)-cyclopentylcarbonyl-valylvaline methyl ester;
2-((1-hydroxy-1-(2-phenyl-1-(serylalanylalanyl)amino) ethyl)phosphinyl)cyclopentylcarbonyl-valyl valine methyl ester;
5-(tert-butoxycarbonyl-serylalanylalanyl)amino-4-hydroxy-1-oxo-6-phenylhexyl-valyl valine methyl ester;
5-(tert-butoxycarbonyl-serylalanylalanyl)amino-4-hydroxy-1-oxo-6-(4-hydroxy-phenyl)hexyl-valyl valine methyl ester;
4-(tert-butoxycarbonyl-serylalanylalanyl)amino-2,2-difluoro-3-hydroxy-1-oxo-5-phenylpentyl-valyl valine methyl ester;
4-(serylalanylalanyl)amino-2,2-difluoro-3-hydroxy-1-oxo-5-phenylpentyl-valyl valine methyl ester;
4-(serylalanylalanyl)amino-2,2-difluoro-1,3-dioxo-5-phenylpentyl-valyl valine methyl ester;
5-(t-butyloxycarbonylserylalanylalanyl)amino-4-hydroxy-6-(4-hydroxy)phenyl-1-oxo-hexyl-valyl amide
5-((carbobenzyloxy-D-seryl)alanylalanyl)amino-4-hydroxy-1-oxo-6-phenylhexyl-valyl valine methyl ester;
5-((D-seryl)alanylalanyl)amino-4-hydroxy-1-oxo-6-phenylhexyl-valyl valine methyl ester;
5-(t-butyloxycarbonylserylalanylalanyl)amino-6-(4-hydroxy)phenyl-4-hydroxy-1-oxo-hexyl-valyl valine; 5-(serylalanylalanyl)amino-6-(4-hydroxy)phenyl-4-hydroxy-1-oxo-hexyl-valyl valine;
2-((1-hydroxy-2-(serylalanylalanyl)amino-3-cyclohexyl)propyl)cyclopentanecarbonyl-valyl valine methyl ester;
(4S,5S) 5-(carbobenzyloxyalanyl)amino-4-hydroxy-1-oxo-6-phenylhexyl-valyl valine methyl ester;
(4S,5S) 5-(alanyl)amino-4-hydroxy-1-oxo-6-phenylhexylvalyl valine methyl ester;
(4S,5S) 5-(tert-butyloxycarbonylalanyl)amino-4-hydroxy-1-oxo-6-(4-hydroxyphenyl)hexyl-valyl valine methyl ester;
(4S,5S)-5-alanylamino-4-hydroxy-1-oxo-6-(4-hydroxyphenyl)-hexyl-valyl valine methyl ester;
(4S,5S) 5-(alanylalanyl)amino-4-hydroxy-1-oxo-6-(4-hydroxyphenyl)hexyl-valyl valine methyl ester;
(4S,5S) 5-[(carbobenzyloxy-$\beta$-alanyl)alanyl]amino-4-hydroxy-1-oxo-6-(4-hydroxyphenyl)hexyl-valyl valine methyl ester;
(4S,5S) 5-[($\beta$-alanyl)alanyl]amino-4-hydroxy-1-oxo-6-(4-hydroxyphenyl)hexyl-valyl valine methyl ester;
(4S,5S) 5-(alanylalanyl)amino-4-hydroxy-1-oxo-6-cyclohexyl-hexyl-valyl valine methyl ester;
(4S,5S) 5-(carbobenzyloxyalanylalanyl)amino-4-hydroxy-1-oxo-6-phenylhexyl-valyl valine methyl ester;
(4S,5S) 5-(alanylalanyl)amino-4-hydroxy-1-oxo-6-phenylhexyl-valyl valine methyl ester;
(4RS,5S) 5-amino-6-(4-hydroxy)phenyl-4-hydroxy-1-oxo-hexyl-valyl valine benzyl ester;
(1R,2R)-2-(((1S,2S)-1-hydroxy-2-amino-3-cyclohexyl)propyl)cyclopentanecarbonyl-valyl valine methyl ester;
(4S,5S) 5-amino-4-hydroxy-1-oxo-6-phenylhexyl-valyl valine methyl ester;
(4S,5S)-5-(tert-butyloxycarbonyl)amino-4-hydroxy-1-oxo-6-phenylhexyl-valyl valine methyl ester;
(4S,5S) 5-(methoxycarbonyl-alanylalanyl)amino-6-phenyl-4-hydroxy-(1-oxo)hexyl-valyl valine; or
(4RS,5S) 5-((tert-butyloxycarbonyl)isoleucyl)amino-7-methyl-4-hydroxy-1-oxo-octyl-leucyl-aspartic acid methyl ester; or the carboxamides thereof;
and pharmaceutically acceptable salts thereof.

13. The use of a compound according to any one of claims 4-11 in the manufacture of a medicament for the treatment of viral disease.

14. A process for preparing a pharmaceutical composition which comprises bringing into association a compound according to any one of claims 4-11 and a pharmaceutically acceptable carrier.

15. A process for preparing a pharmaceutical composition which comprises bringing into association a compared according to any one of claims 4-11, azidothymidine and a pharmaceutically acceptable carrier.

16. A process for preparing a compound of the formula:

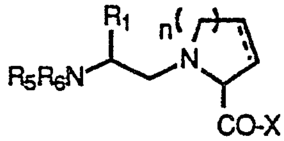

wherein:

$R_1$ is $C_{1-5}$Alk, -$(CH_2)_n$S$C_{1-5}$Alk, -$(CH_2)_n$O$C_{1-5}$Alk, -$(CH_2)_n$$C_{3-7}$cycloalkyl or -$(CH_2)_m$$C_6H_4$-$R_a$;
$R_a$ is halogen, $NO_2$, OH, O$C_{1-5}$Alk, $NH_2$ or H;
$R_5$ is H;
$R_6$ is $CH_3CO$, $C_{1-5}$Alk, Dns, Cbz or Boc, or taken together $R_5$ and $R_6$ are phthalimido;
m is independently 0, 1, 2 or 3;
n is independently 0 or 1;
-- indicates either a single or double bond;
X is H, halogen, OH, O$C_{1-5}$Alk, NHR′R″ or OCO$C_{1-5}$Alk;
R′ and R″ are H or $C_{1-5}$Alk,
which comprises,

a) reacting a compound of the formula:

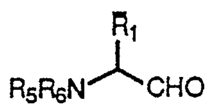

with an ester of proline, Δ3-Pro or 2-carboxypiperidine, under reducing conditions; and optionally thereafter,

b) converting the ester to a carboxylic acid, ester or amide; and

c) converting the acid to an ester, amide, anhydride or acyl halide.

17. A process for preparing a compound of the formula:

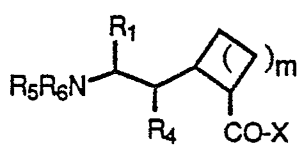

wherein:

$R_1$ is $C_{1-5}$Alk, -$(CH_2)_n$O$C_{1-5}$Alk, -$(CH_2)_n$$C_{3-7}$cycloalkyl or -$(CH_2)_m$$C_6H_4$-$R_a$;
$R_4$ is OH, $NH_2$, H or =O;
$R_a$ is halogen, $NO_2$, OH, O$C_{1-5}$Alk, $NH_2$ or H;
m is independently 0, 1, 2 or 3;
n is 0 or 1;
$R_5$ is H;
$R_6$ is $CH_3CO$, $C_{1-5}$Alk, Dns, Cbz or Boc, or taken together $R_5$ and $R_6$ are phthalimido;
X is H, halogen, OH, O$C_{1-5}$Alk, NHR′R″ or OCO$C_{1-5}$Alk; and
R′ and R″ are H or $C_{1-5}$Alk;
which comprises,

a) oxidizing to a keto-carboxylic acid, a compound of the formula:

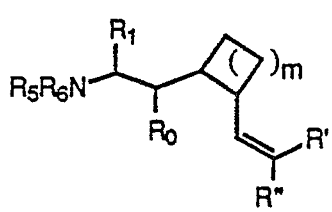

wherein $R_0$ is OH or =O;
and thereafter in any order,

b) optionally reducing or reductively aminating the keto group; and

c) optionally converting the carboxylic acid to an ester, amide, aldehyde, anhydride or acyl halide.

18. A process for preparing a compound of the formula:

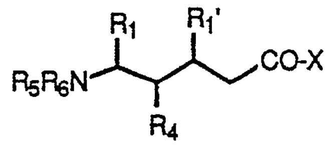

wherein:
$R_1$ is $C_{1-5}$Alk, -$(CH_2)_n$O$C_{1-5}$Alk, -$(CH_2)_n$$C_{3-7}$cycloalkyl or -$(CH_2)_m$$C_6H_4$-$R_a$;
$R_1'$ is H, $C_{1-5}$Alk, -$(CH_2)_n$O$C_{1-5}$Alk, -$(CH_2)_n$$C_{3-7}$cycloalkyl or -$(CH_2)_m$$C_6H_4$-$R_a$;
$R_4$ is OH, $NH_2$, H or =O;
$R_a$ is halogen, $NO_2$, OH, O$C_{1-5}$Alk, $NH_2$ or H;
m is independently 0, 1, 2 or 3;
n is independently 0 or 1;
$R_5$ is H;
$R_6$ is $CH_3CO$, $C_{1-5}$Alk, Dns, Cbz or Boc, or taken together $R_5$ and $R_6$ are phthalimido;
X is H, halogen, OH, O$C_{1-5}$Alk, NHR$'$R$''$ or OCO$C_{1-5}$Alk; and
R$'$ and R$''$ are H or $C_{1-5}$Alk, provided that $R_1$ is not isobutyl;
which comprises,

a) oxidizing to a carboxylic acid, a compound of the formula:

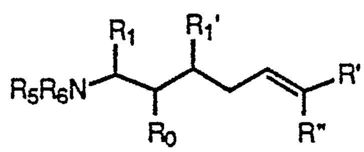

wherein $R_0$ is OH or =O;
and thereafter in any order,

b) optionally reducing or reductively aminating the keto group;

c) optionally converting the carboxylic acid to an ester, amide, aldehyde, anhydride or acyl halide.

19. A process according to claim 18 in which $R_1'$ is H, $R_1$ is $CH_2C_6H_4$-$R_a$ and $R_4$ is OH or =O.

20. A process for preparing a compound of the formula:

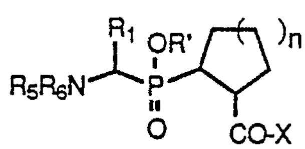

wherein:
$R_1$ is $C_{1-5}$Alk, -$(CH_2)_n$O$C_{1-5}$Alk, $(CH_2)_n$$C_{3-7}$cycloalkyl or -$(CH_2)_m$$C_6H_4$-$R_a$;
$R_a$ is halogen, $NO_2$, OH, O$C_{1-5}$Alk, $NH_2$ or H;
m is 0, 1, 2 or 3;
n is independently 0 or 1;
$R_6$ is $CH_3CO$, $C_{1-5}$Alk, Dns, Cbz or Boc, or taken together $R_5$ and $R_6$ are phthalimido;
X is H, halogen, OH, O$C_{1-5}$Alk, NHR$'$R$''$ or OCO$C_{1-5}$Alk; and
R$'$ and R$''$ are H or $C_{1-5}$Alk;
which comprises,

a) oxidizing to a carboxylic acid, a compound of the formula:

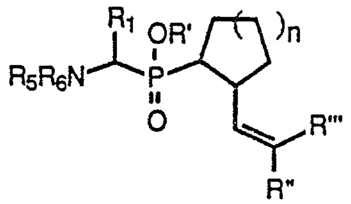

and thereafter,

b) optionally hydrolyzing the alkyl phosphinate to the phosphinic acid; and

c) optionally converting the carboxylic acid to an ester, amide, aldehyde, anhydride or acyl halide.